# EUROPEAN PATENT APPLICATION

(11) **EP 4 488 264 A1**
(43) Date of publication of application: **08.01.2025**
(21) Application number: 23762735.1
(22) Date of filing: 15.02.2023
(51) Int. Cl.: C07D 213/40, C07D 239/26, C07D 241/12, C07D 277/28, C07D 231/12, C07D 233/64, A61K 31/16, A61K 38/05, A61K 31/4402, A61K 31/426, A61P 31/12

(54) **KETO AMIDE DERIVATIVES AND PHARMACEUTICAL USE THEREOF**

(30) Priority: 01.03.2022 CN 202210199543
(71) Applicant: WestVac Biopharma Co., Ltd., Chengdu, Sichuan 610000 (CN)
(72) Inventor: YANG, Shengyong, Chengdu, Sichuan 610000 (CN); LI, Linli, Chengdu, Sichuan 610000 (CN)
(74) Representative: Altmann Stößel Dick Patentanwälte PartG mbB
(86) International application number: PCT/CN2023/076078
(87) International publication number: WO 2023/165334

(57) **Abstract**

The present invention provides a class of ketoamide derivatives and pharmaceutical uses thereof, and belongs to the fields of organic synthesis & pharmaceutical technology. Specifically provided are the compound represented by formula I, or a pharmaceutically acceptable salt thereof, or a stereoisomer thereof, or an optical isomer thereof, or a deuterated compound thereof. The compound can effectively inhibit the activity of SARS-CoV-2 M^{pro}, and can be used in the manufacturer of SARS-CoV-2 M^{pro} inhibitors, blocking the replication and transcription of SARS-CoV-2 virus in patients. The compound of the present invention has very good application prospects in the manufacturer of SARS-CoV-2 M^{pro} inhibitors, anti-SARS-CoV-2 medicaments, as well as the medicaments for preventing and/or treating Corona Virus Disease 2019 (COVID-19).

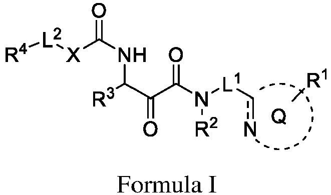

## Description

### Technology of the invention

The present invention belongs to the fields of organic synthesis & pharmaceutical technology, and specifically relates to ketoamide derivatives with SARS-CoV-2 M^{pro} inhibitory activity as well as preparation methods therefor and uses thereof.

### Background of the invention

In 2019, coronavirus pneumonia (COVID-19, also known as novel coronavirus pneumonia) was caused by severe acute respiratory syndrome coronavirus type 2 (SARS-CoV-2, also known as novel coronavirus). By February 2022, more than 400 million people had been infected, causing nearly 6 million deaths. Currently, only two oral anti-SARS-CoV-2 medicaments are on the market. One is Molnupiravir developed by Merck Co., Inc., which targets RdRp, but as a nucleoside analogue, it may have mutagenic side effects and genotoxicity, and thereby its safety needs more data for further confirmation. The other is Nirmatrelvir **(PF-07321332),** an oral medicament developed by Pfizer Inc., which targets the main protease of the virus. However, due to its susceptibility to cytochrome CYP enzyme metabolism in the human body, it needs to be combined with Ritonavir to play its antiviral effect. Therefore, there is an urgent need to develop safe and effective oral anti-SARS-CoV-2 medicaments.

The genome RNA of the coronavirus is approximately 30 kb, with a 5'-cap-shaped structure and a 3'-poly-a tail, and contains at least 6 open reading frames (ORFs). The first ORF (ORF1a/b) accounts for about two-thirds of the genome length and directly translates two polyproteins: ppla and pplab. There is an a-1 frame shift between ORFla and ORF1b. These polyproteins are assembled with a main protease (abbreviated as M^{pro}; also known as 3C like protease (3CL^{pro})) and papain like proteases (PL^{pro}), and transformed into 16 non-structural proteins. The non-structural proteins participate in the production of subgenomic RNA, encoding four main structural proteins (envelope (E), membrane (M), spike (S), and nucleocapsid (N) proteins) and other auxiliary proteins, so as to complete the replication and invasion process of the virus.

M^{pro} can hydrolyze and cleave overlapping poly-proteins ppla and pplab into functional proteins, which is a crucial step in the virus replication process. Enzymes necessary for virus replication, such as RdRp or nsp13, cannot fully function and complete replication without prior protein hydrolysis and release. Therefore, inhibiting M^{pro} of the virus can prevent the production of infectious viral particles, thereby alleviating disease symptoms.

M^{pro} is conserved in coronaviruses, and the substrates of M^{pro} in different coronaviruses share some common characteristics: amino acids from the N-terminus to the C-terminus are numbered in a paired form (-P4-P3-P2-P1↓P1'-P2'-P3'), with cleavage sites between P1 and P1'. Specifically, M^{pro} has a unique substrate preference for glutamine at the P1 site (Leu-Gln↓(Ser, Ala, Gly)), which is not present in host proteases, indicating that targeting the viral M^{pro} is feasible for high selectivity. Therefore, the absolute dependence of the virus on the correct function of this protease, coupled with the lack of homologous human proteases, makes M^{pro} become an ideal antiviral target.

Therefore, it is urgent to develop an oral medicament that can effectively inhibit the M^{pro} enzyme activity of SARS-CoV-2 virus.

### Summary of the invention

The object of the present invention is to provide a novel ketoamide derivative and pharmaceutical uses thereof.

The present invention provides a compound represented by formula I, or a pharmaceutically acceptable salt thereof, or a stereoisomer thereof, or an optical isomer thereof, or a deuterated compound thereof: 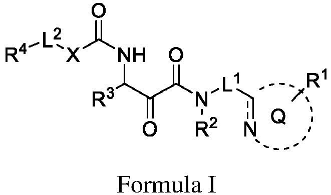
wherein, Q is a nitrogen-containing heteroaromatic ring or a nitrogen-containing fused heteroaromatic ring;
R¹ is selected from the group consisting of H, unsubstituted or halogenated C₁₋₈ alkyl, unsubstituted or halogenated C₁₋₈ alkoxy, halogen, 5-6-membered aryl, 5-6-membered heteroaryl, 3-8-membered saturated heterocyclyl, 3-8-membered saturated cycloalkyl, and NHCOR^{1a}; R^{1a} is selected from C₁₋₈ alkyl, and unsubstituted or halogenated following groups: 3-8-membered saturated heterocyclyl, 3-8-membered saturated cycloalkyl, 5-6-membered aryl, 5-6-membered heteroaryl;
R² is selected from the group consisting of H, C₁₋₈ alkyl, C₁₋₈ alkoxy, halogen, 5-6-membered aryl, 5-6-membered heteroaryl, 3-8-membered saturated heterocyclyl, 3-8-membered saturated cycloalkyl;
R³ is selected from the group consisting of H, C₁₋₈ alkyl, C₁₋₈ alkoxy, halogen, 5-6-membered aryl, 5-6-membered heteroaryl, 3-8-membered saturated heterocyclyl, 3-8-membered saturated cycloalkyl, and CH₂R^{3a}; R^{3a} is selected from unsubstituted or halogenated following groups: 3-8-membered saturated heterocyclyl, 3-8-membered saturated cycloalkyl, 5-6-membered aryl, 5-6-membered heteroaryl, and fused aryl;
R⁴ is selected from the group consisting of H, and any one of the substituted or unsubstituted following groups: C₁₋₈ alkyl, C₁₋₈ alkoxy, 3-8-membered saturated cycloalkyl, 3-8-membered saturated heterocyclyl, 5-6-membered aryl, 5-6-membered heteroaryl, bridged group, fused aryl, fused heteroaryl, or (5-6-membered saturated heterocycle)-fused 5-6-membered aryl;
The substituents of the substituent group are R^{4a}, R^{4b}, R^{4c}, and R^{4d}, and each independently selected from the group consisting of H, halogen, phenyl, cyano, hydroxyl, ester group, trimethylsilyl, -(CH₂)ₘ-SO₂R', and -COOR"; alternatively, selected from any one of the substituted (with one or more of halogen, cyano, haloalkyl, and haloalkoxy) or unsubstituted following groups: C₁₋₈ alkyl, C₁₋₈ alkoxy, benzyl, pyridyl or 3-6-membered saturated cycloalkyl; said R' and R" are each independently selected from C₁₋₈ alkyl; M is an integer from 0 to 3;
or, said R^{4a} and R^{4b} are linked to form halogenated or unsubstituted 3-6-membered saturated carboncycle or carbon heterocycle;
L¹ is selected from the group consisting of absence, substituted or unsubstituted - (CH₂)ₙ- or -O-(CH₂)ₙ-, -O-, -NHCO-, -NHSO₂-, -CONH-, -NHCOO-, -NHCONH-, and - NH-; n is any integer from 1 to 3; the substituent of said L¹ is selected from the group consisting of C₁₋₃ alkyl, C₁₋₃ alkoxy or phenyl;
X is selected from the group consisting of absence, CR⁵R⁶, NR⁵R⁶, O or SO₂; R⁵ and R⁶ are each independently selected from the group consisting of:
   H, halogen, and R^{5a}-, R^{5b}-, R^{5c}-substituted or unsubstituted C₁₋₈ alkyl; or R⁵ and R⁶ are linked to form R^{6a}-, R^{6b}-substituted or unsubstituted 3-8-membered saturated cycloalkyl or 3-8-membered saturated heterocyclyl;
   R^{5a}, R^{5b}, and R^{5c} are each independently selected from the group consisting of H, alkynyl, hydroxyl, -CONH₂, -N(CH₃)₂, halogen, C₁₋₈ alkoxy, 5-6-membered aryl, and 5-6-membered heteroaryl; or any two of R^{5a}, R^{5b}, and R^{5c} are linked to form 3-8-membered saturated cycloalkyl or 3-8-membered saturated heterocyclyl;
   R^{6a} and R^{6b} are each independently selected from the group consisting of H, alkenyl, halogen, and halogen-substituted or unsubstituted C₁₋₈ alkyl; or R^{6a} and R^{6b} are linked to form 5-6-membered aryl;
   L² is selected from the group consisting of absence, 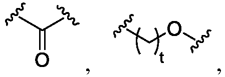 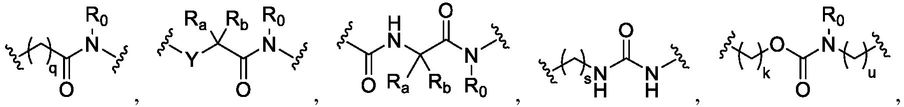 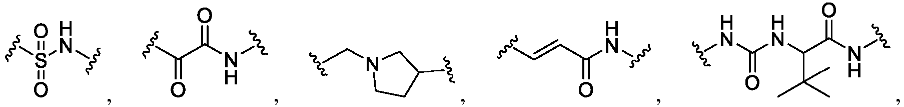 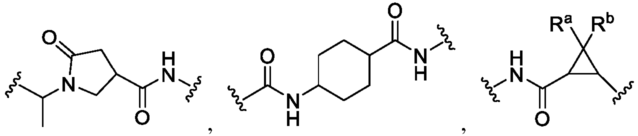 or Rₐ-, R_{b}-substituted or unsubstituted 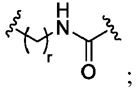
   wherein, R₀ is selected from H and C₁₋₃ alkyl; or R₀ and R₅ are linked to form the substituted or unsubstituted following structure: bridged ring, 3-6-membered saturated ring or (3-6-membered saturated ring)-fused benzene; the substituent of said substituted structure is C₁₋₃ alkyl or halogen;
   Rₐ and R_{b} are each independently selected from the group consisting of H, cyano, C_{1~5} alkyl, and 3-6-membered cycloalkyl; or Rₐ and R_{b} are linked to form 3-6-membered saturated carboncycle;
   Y is O or S;
   t is any integer from 1 to 3, q is any integer from 0 to 4, r is any integer from 0 to 3, s is any integer from 0 to 3, k is any integer from 0 to 3, and u is any integer from 0 to 3.

Further, said Q is selected from the group consisting of 5-6-membered N-containing heteroaromatic ring, (5-membered N-containing heteroaromatic ring)-fused (6-membered N-containing heteroaromatic ring) or (6-membered N-containing heteroaromatic ring)-fused (6-membered N-containing heteroaromatic ring).

More further, Q is selected from the group consisting of

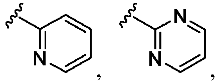

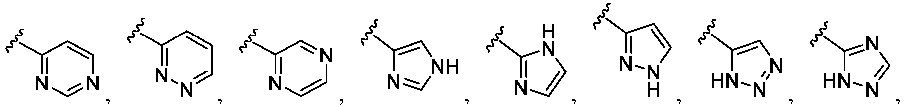

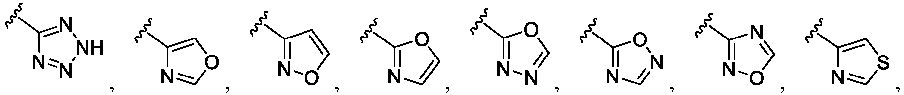

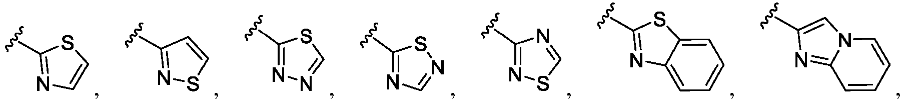

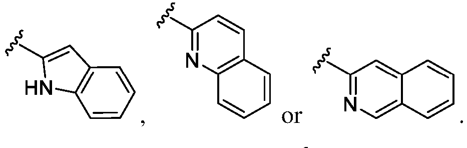

More further, said Q is

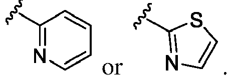

Further, R¹ is selected from the group consisting of H, halogen, halogenated or unsubstituted C₁₋₈ alkyl or unsubstituted C₁₋₈ alkoxy.

More further, R¹ is selected from the group consisting of H, halogen, halogenated or unsubstituted C₁₋₃ alkyl or unsubstituted C₁₋₃ alkoxy.

More further, R¹ is selected from the group consisting of H, F, Cl, CH₃, CH₃O or - CF₃.

Further, said R² is selected from the group consisting of H or C₁₋₈ alkyl.

More further, said R² is H or CH₃.

Further, said R³ is selected from the group consisting of H or CH₂R^{3a}; R^{3a} is selected from the unsubstituted or halogenated following groups: C₁₋₄ alkyl, 5-6-membered cycloalkyl, 5-6-membered aryl, 5-6-membered heteroaryl or fused aryl.

More further, said R³ is selected from the group consisting of H or CH₂R^{3a}; R^{3a} is selected from the unsubstituted or halogenated following groups: C₁₋₂ alkyl, 5-6-membered cycloalkyl, 5-6-membered aryl, 5-6-membered heteroaryl or naphthyl.

More further, said R³ is selected from the group consisting of H or CH₂R^{3a}; R^{3a} is selected from the group consisting of phenyl, ethyl, cyclohexyl, furyl, naphthyl or F-substituted phenyl.

Further, said R⁴ is selected from any one of the substituted or unsubstituted following groups: C₁₋₄ alkyl, C₁₋₄ alkoxy, 3-7-membered saturated cycloalkyl, 4-6-membered saturated heterocyclyl, 5-6-membered aryl, 5-6-membered heteroaryl, bridged group, naphthyl, (5-6-membered aromatic heterocyclyl)-fused phenyl or (5-6-membered saturated heterocyclyl)-fused phenyl.

The substituents of the substituent group are R^{4a}, R^{4b}, R^{4c}, and R^{4d}, and each independently selected from the group consisting of H, halogen, phenyl, cyano, hydroxyl, ester group, trimethylsilyl, -(CH₂)ₘ-SO₂R', and -COOR"; alternatively, selected from any one of the halogen-substituted or unsubstituted following groups: C₁₋₃ alkyl, C₁₋₃ alkoxy or 3-4-membered saturated cycloalkyl; said R' and R" are each independently selected from C₁₋₄ alkyl; M is an integer from 0 to 2;
or, any two of said R^{4a}, R^{4b}, R^{4c} and R^{4d} are linked to form halogenated or unsubstituted 3-6-membered saturated carboncycle or heterocycle.

More further, said R⁴ is selected from any one of the substituted or unsubstituted following groups: C₁₋₄ alkyl, C₁₋₄ alkoxy, 3-7-membered saturated cycloalkyl, 4-6-membered saturated heterocyclyl, 5-6-membered aryl, 5-6-membered N-containing heteroaryl, bridged group, naphthyl, benzofuranyl, benzopyridyl or (5-6-membered saturated O-containing heterocyclyl)-fused phenyl;

The substituents of the substituent group are R^{4a}, R^{4b}, R^{4c}, and R^{4d}, and each independently selected from the group consisting of H, halogen, phenyl, cyano, hydroxyl, ester group, trimethylsilyl, -(CH₂)ₘ-SO₂R', and -COOR"; alternatively, selected from any one of the halogen-substituted or unsubstituted following groups: C₁₋₃ alkyl, C₁₋₃ alkoxy or 3-membered saturated cycloalkyl; said R' and R" are each independently selected from C₁₋₄ alkyl; m is 0 or 1;
or, any two of said R^{4a}, R^{4b}, R^{4c} and R^{4d} are linked to form halogenated or unsubstituted 3-6-membered saturated carboncycle or O-containing heterocycle.

More further, said R⁴ is 4-6-membered saturated cycloalkyl substituted with F.

More further, said R⁴ is 4-6-membered saturated cycloalkyl substituted with two fluorines.

More further, said R⁴ is 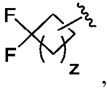 and z is an integer from 1 to 3.

Further, said R⁴ is selected from any one of the substituted or unsubstituted following groups: -CH₃, -OCH₃,

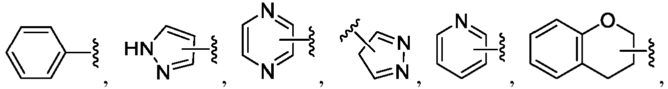

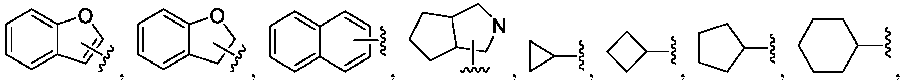

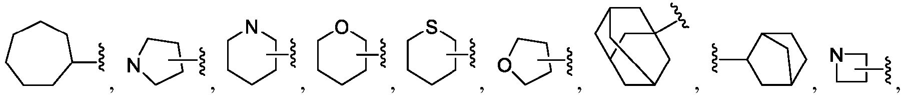

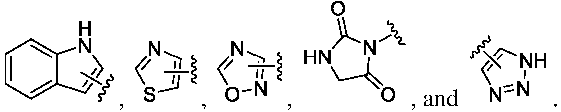

More further, said R⁴ is selected from any one of the substituted or unsubstituted following groups: -CH₃, CF₃, -OCH₃, -OCF₃, -OC(CH₃)₃,

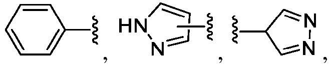

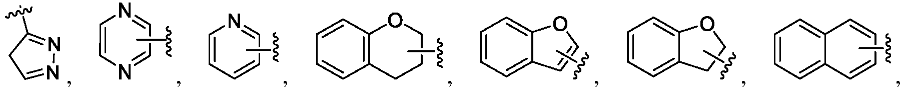

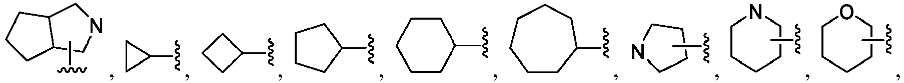

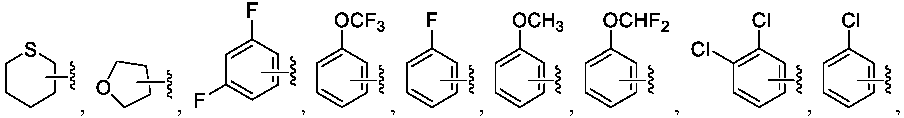

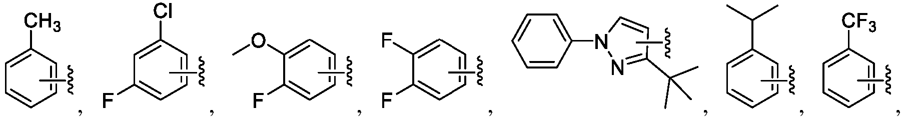

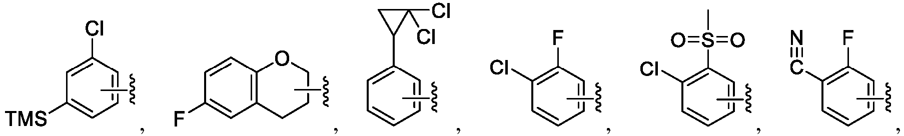

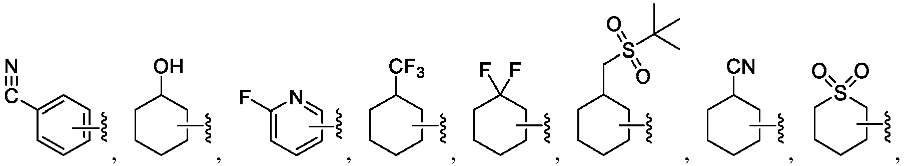

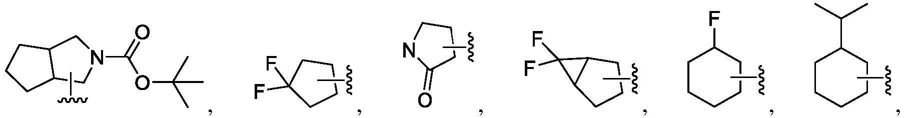

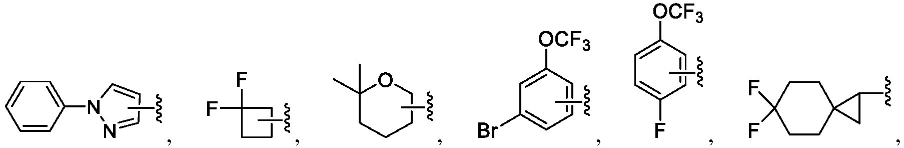

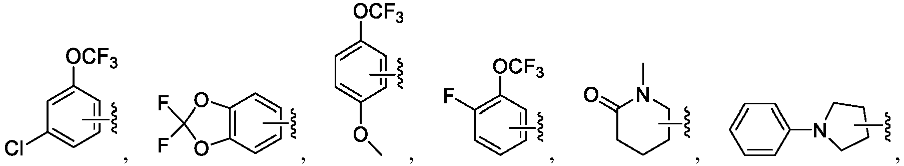

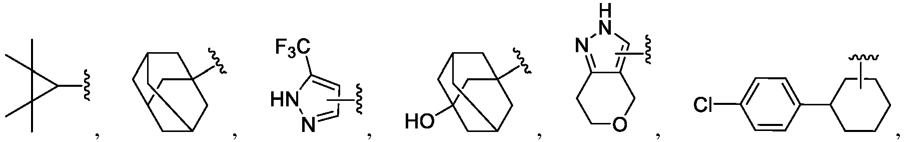

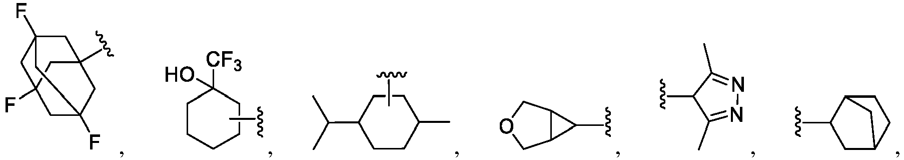

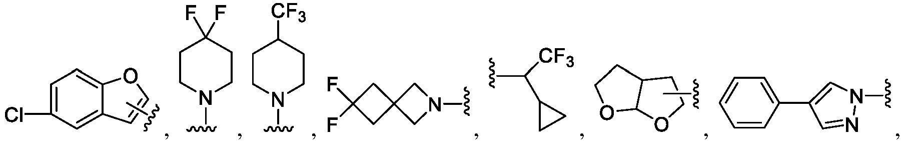

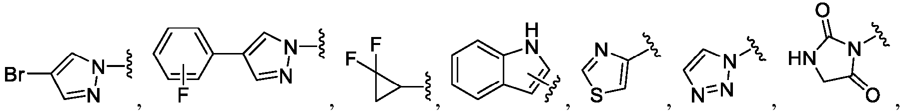

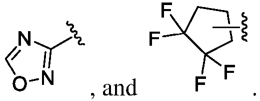

Further, said L¹ is selected from substituted or unsubstituted -(CH₂)ₙ-, and n is any integer from 1 to 3; said substituted substituent is C₁₋₃ alkyl or phenyl.

More further, said L¹ is selected from substituted or unsubstituted -CH₂-; said substituted substituent is methyl or phenyl.

Further, said X is selected from the group consisting of absence, CR⁵R⁶ or NR⁵R⁶; R⁵ and R⁶ are each independently selected from the group consisting of:
H, F, and R^{5a}-, R^{5b}-, R^{5c}-substituted or unsubstituted C₁₋₅ alkyl; or R⁵ and R⁶ are linked to form R^{6a}-, R^{6b}-substituted or unsubstituted 3-6-membered saturated cycloalkyl or 4-membered saturated O-containing heterocyclyl;
R^{5a}, R^{5b}, and R^{5c} are each independently selected from the group consisting of H, ethynyl, hydroxyl, -CONH₂, -CONHCH₃, -N(CH₃)₂, F, methoxy, phenyl, methylsulfonyl, amino, carboxyl, methoxycarbonyl, azaphenyl; or any two of R^{5a}, R^{5b}, and R^{5c} are linked to form (3-6)-membered saturated cycloalkyl or (5-6)-membered saturated O-containing heterocyclyl;
R^{6a} and R^{6b} are each independently selected from the group consisting of H, vinyl, F, F-substituted methyl; or R^{6a} and R^{6b} are linked to form phenyl;
L² is selected from the group consisting of absence, 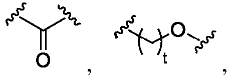 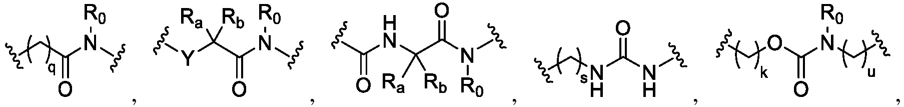 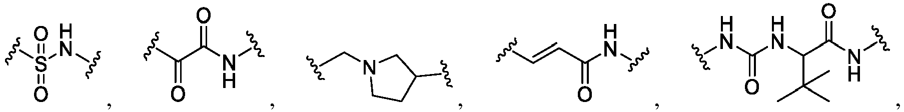 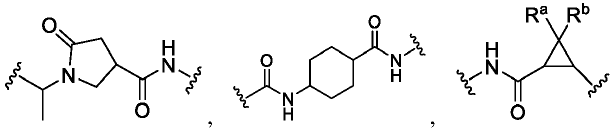 or Rₐ-, R_{b}-substituted or unsubstituted 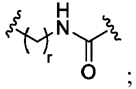
wherein, R₀ is H and C₁₋₃ alkyl; or, R₀ and R₅ are linked to form the following substituted or unsubstituted structures: 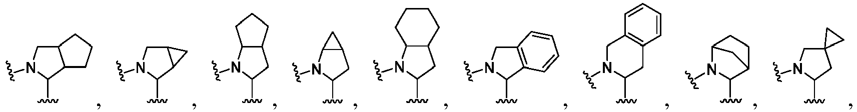 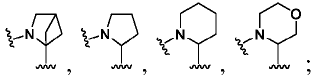 the substituent of said substituted structure is methyl or F;
Rₐ and R_{b} are each independently selected from the group consisting of H, cyano, butyl, and 6-membered cycloalkyl; or Rₐ and R_{b} are linked to form 3-membered carboncycle;
Y is O or S;
t is 0 or 1, q is any integer from 0 to 4, r is 0 or 1, s is 0 or 1, k is 0 or 1, and u is 0 or 1.

More further, said X is CR⁵R⁶, L² is 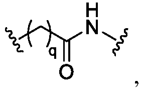 and q is 0 or 1.

More further, said X is selected from the group consisting of absence,

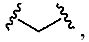

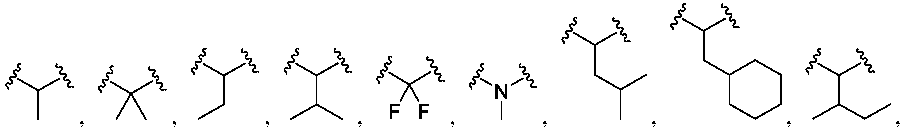

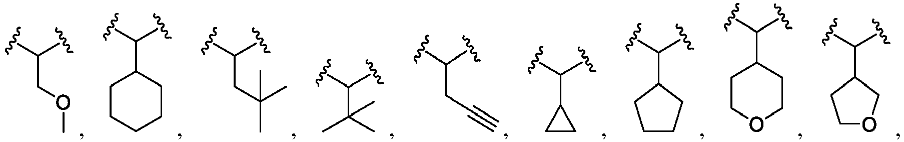

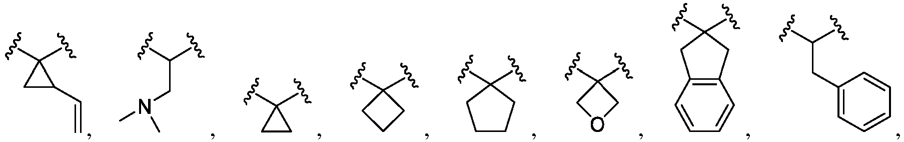

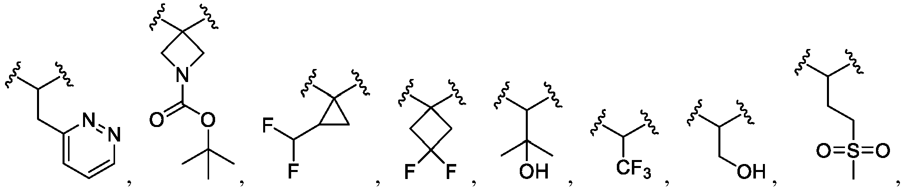

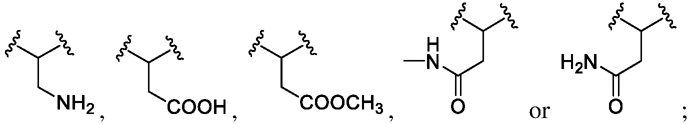

L² is selected from the group consisting of absence, 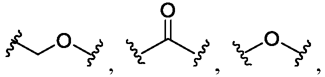 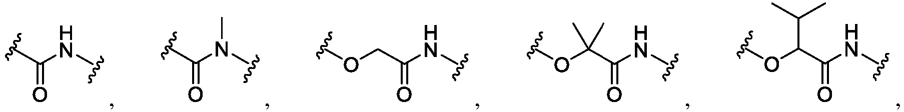 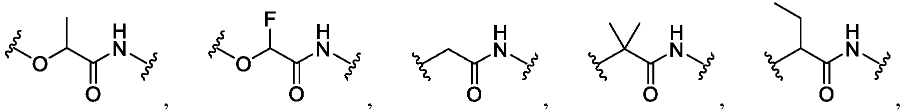 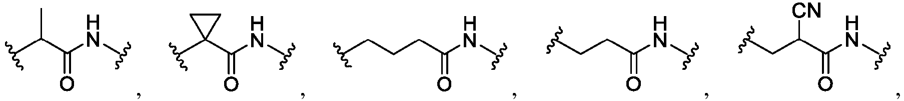 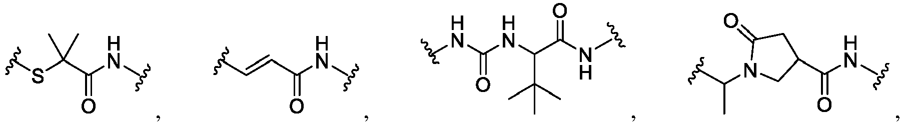 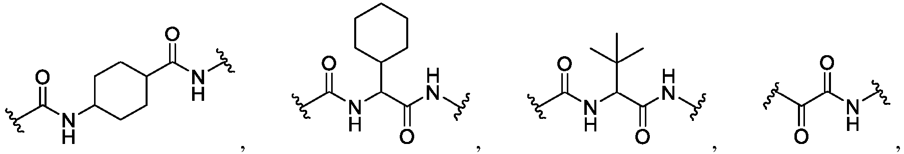 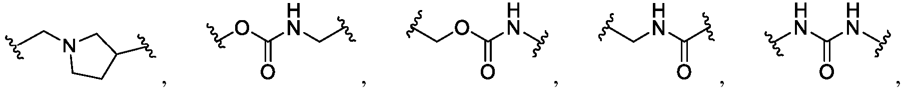 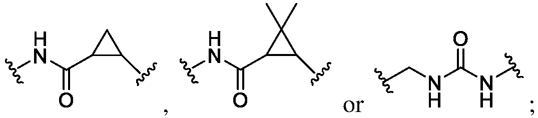 or L² and X are linked to form any one of the following structures:

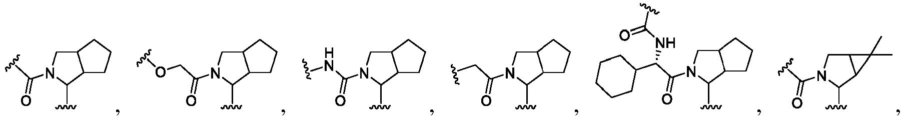

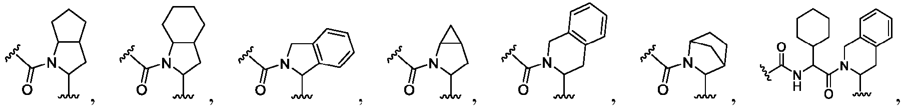

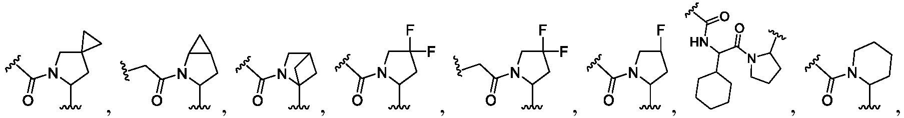

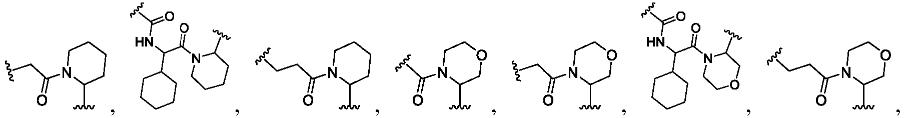

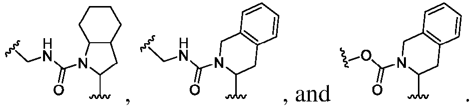

More further, said X is 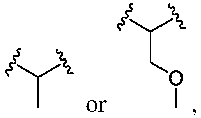 and L² is

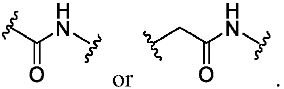

Further, the structure of above compound is as represented by formula II or formula III:

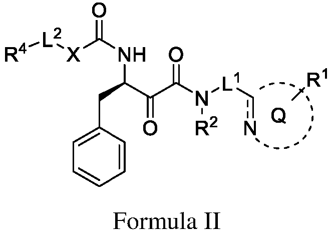

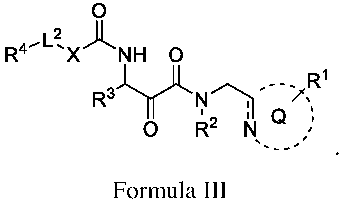

More further, the structure of above compound is as represented by formula II-A:

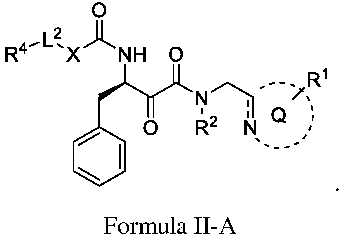

More further, the structure of above compound is as represented by formula II-A-a, formula II-A-b, formula II-A-c or formula II-A-d:

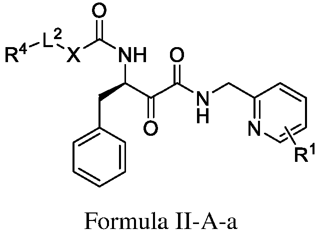

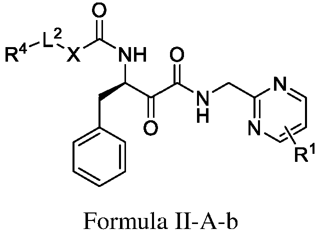

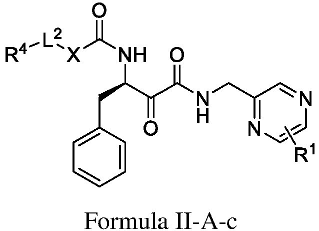

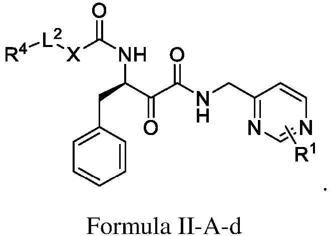

More further, the structure of above compound is as represented by formula II-A-a-1:

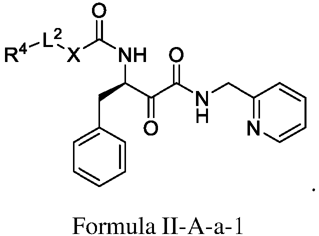

More further, said compound has any one of the following structures:

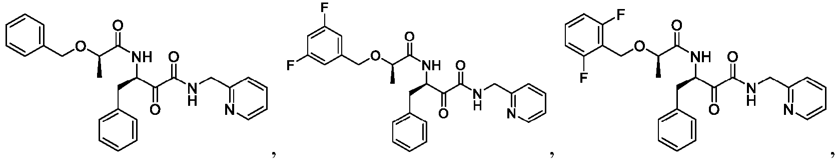

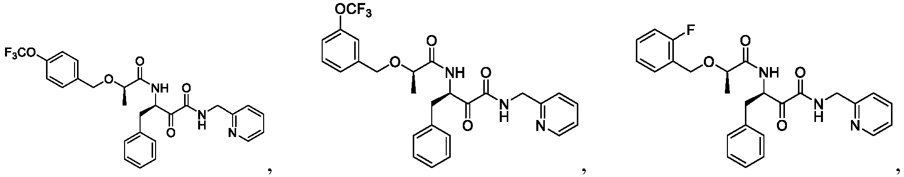

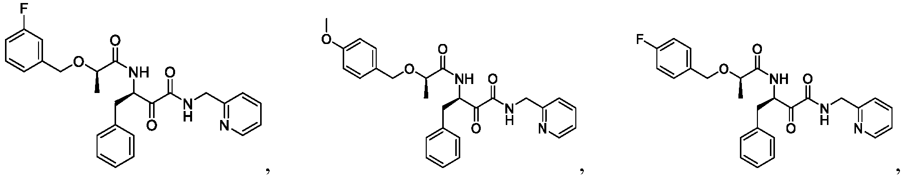

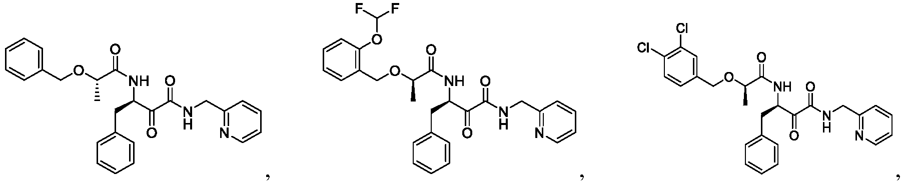

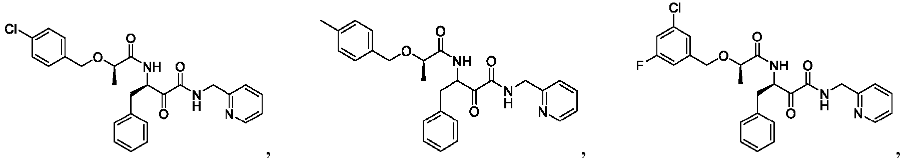

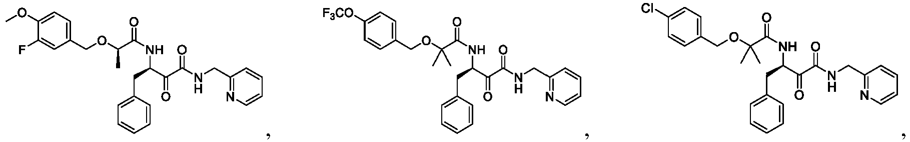

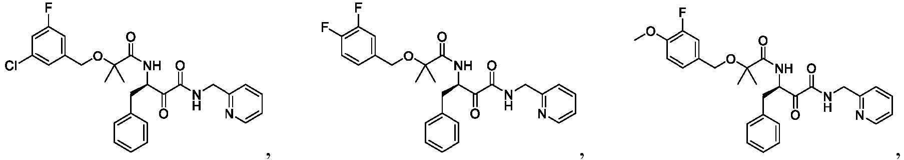

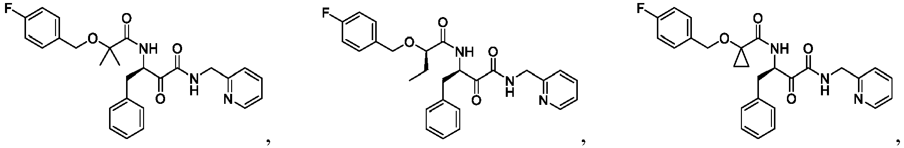

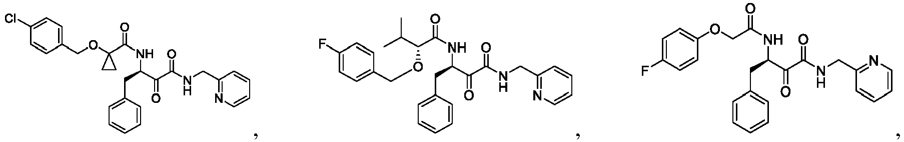

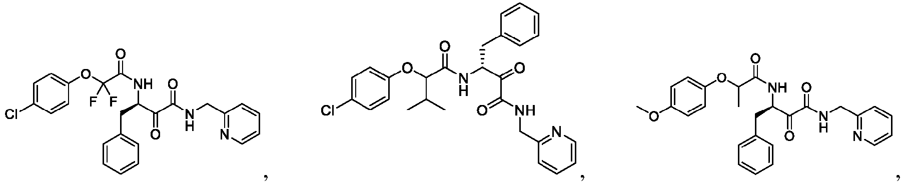

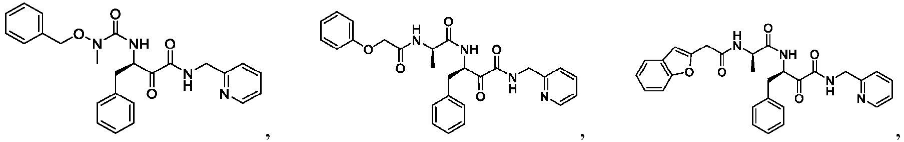

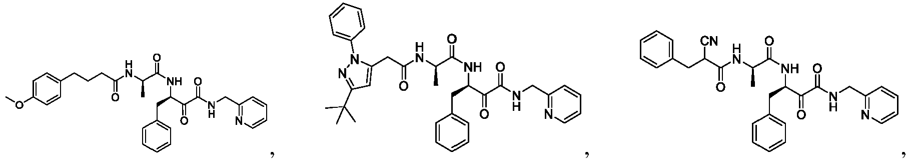

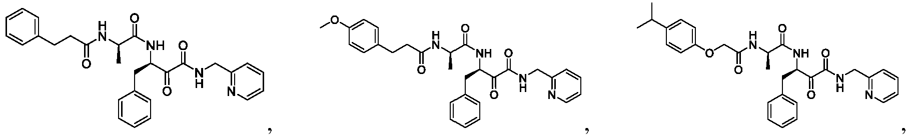

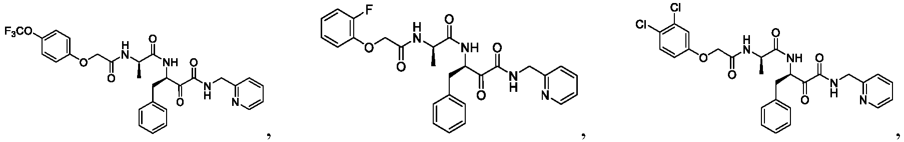

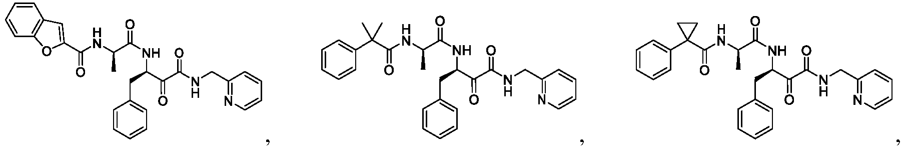

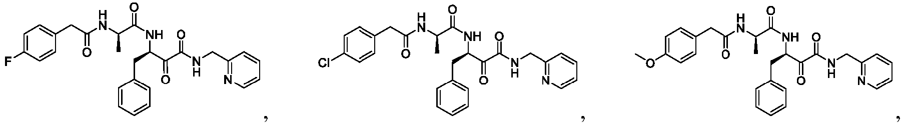

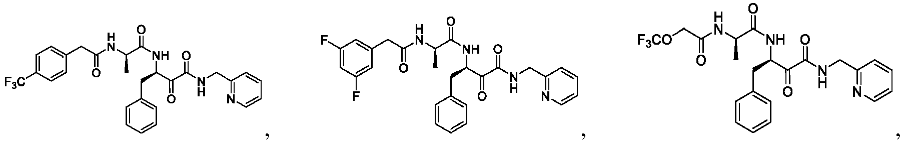

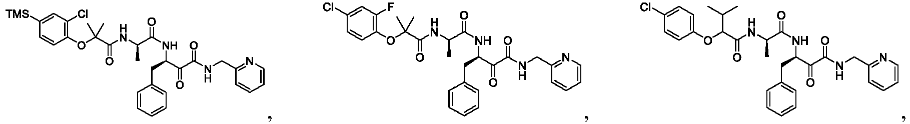

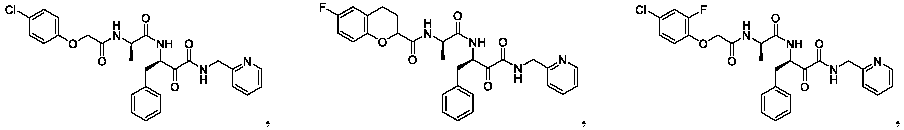

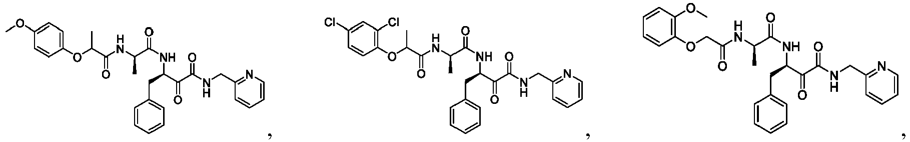

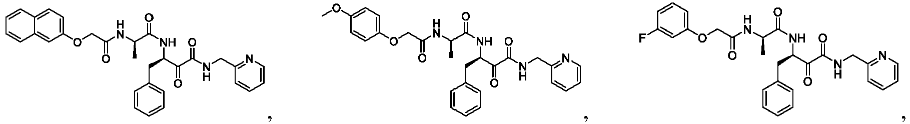

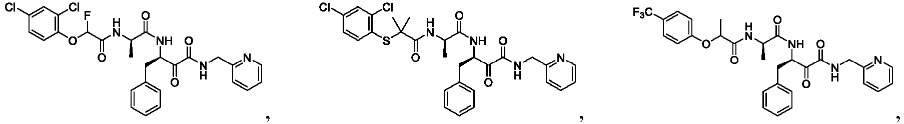

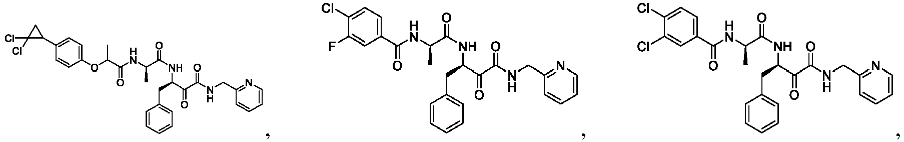

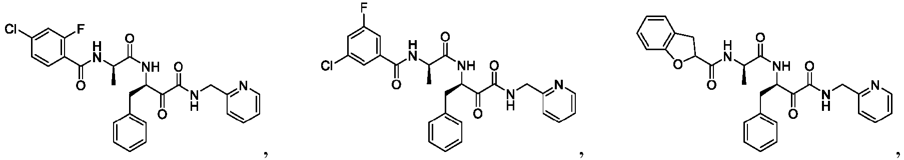

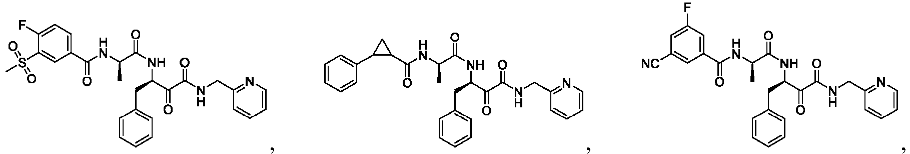

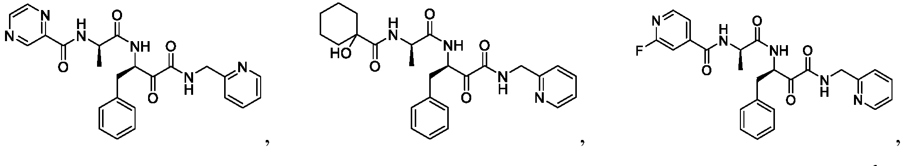

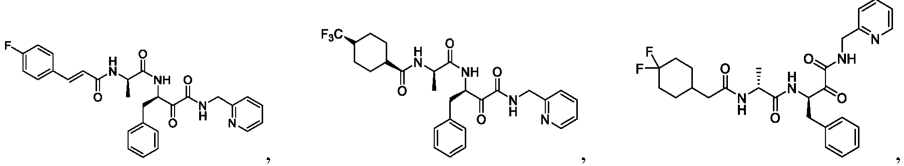

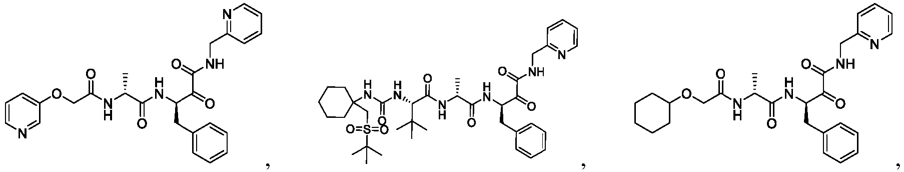

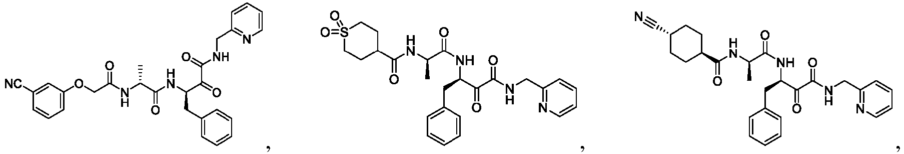

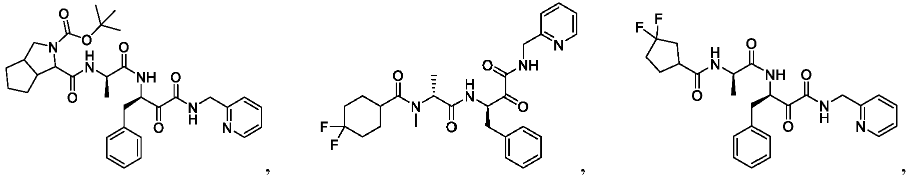

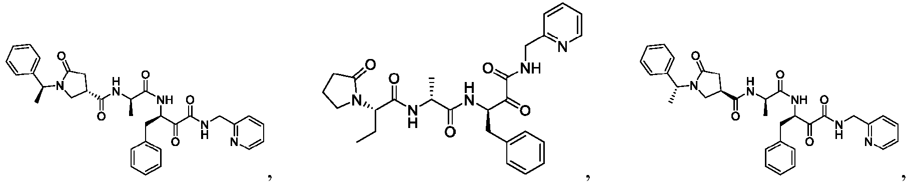

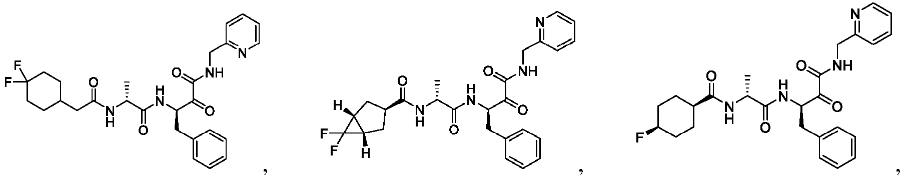

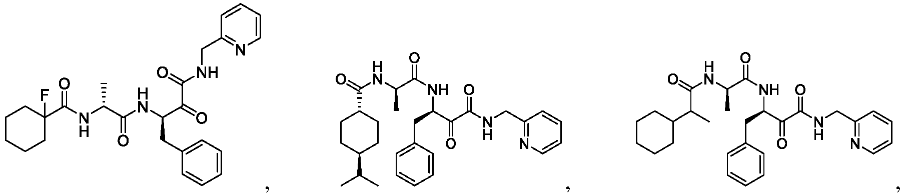

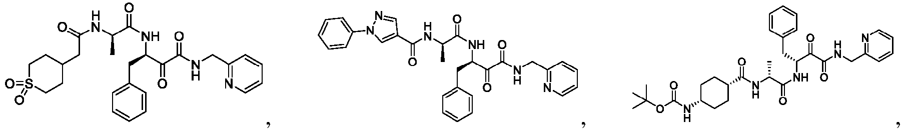

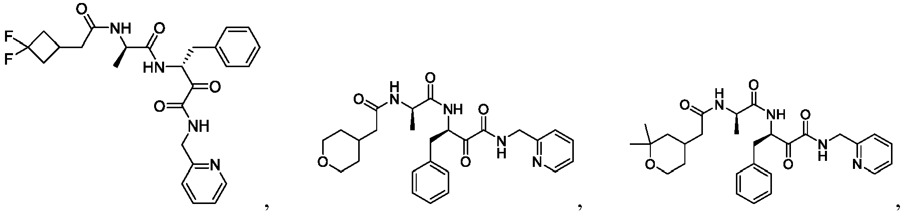

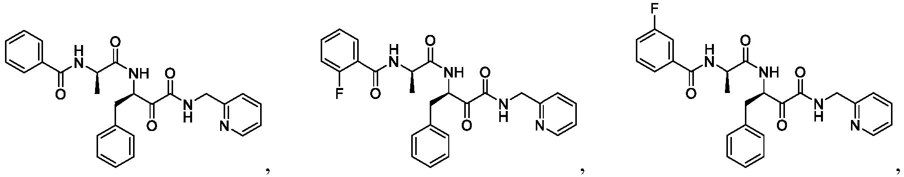

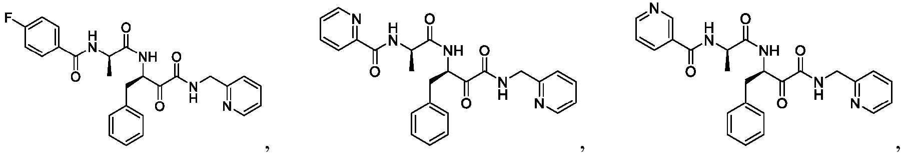

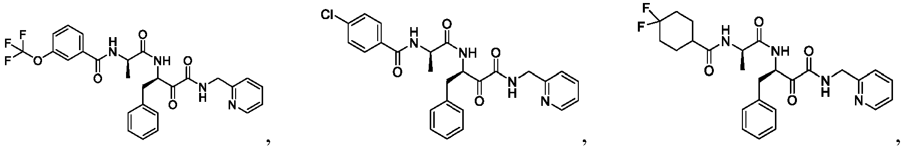

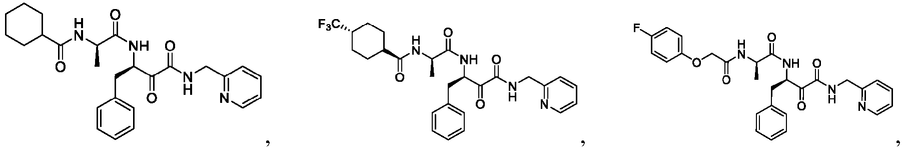

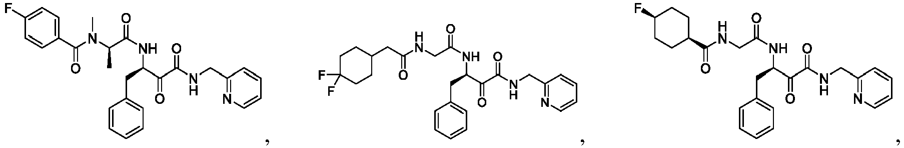

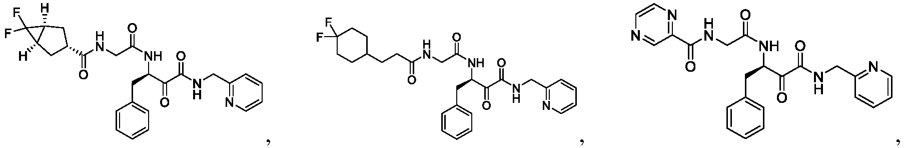

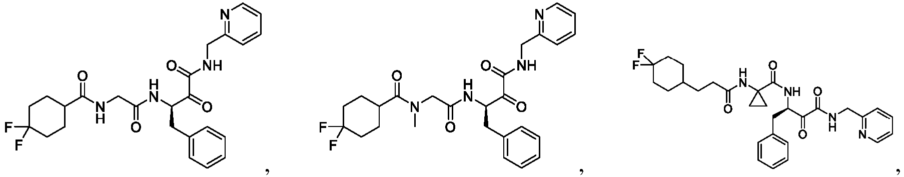

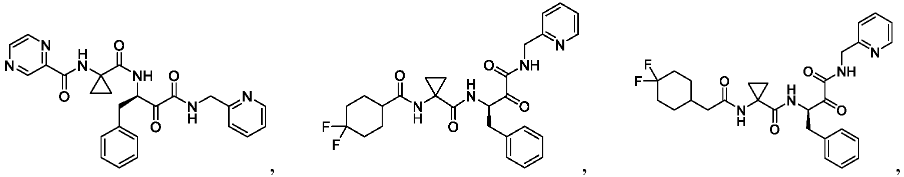

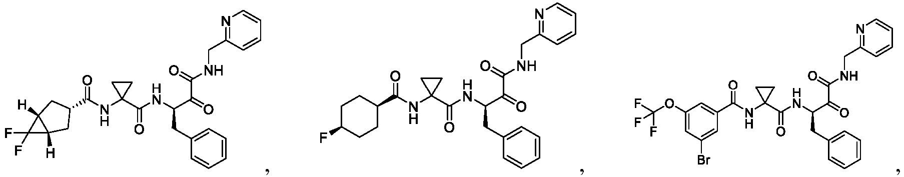

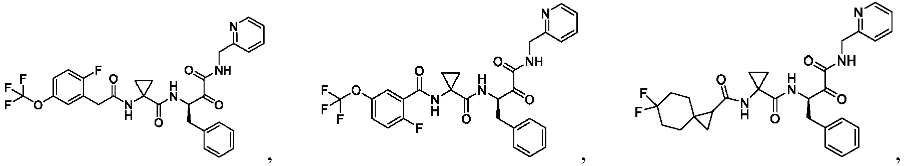

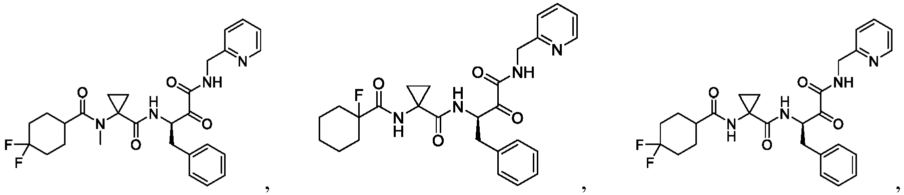

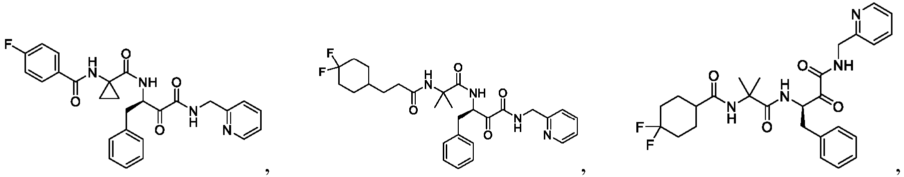

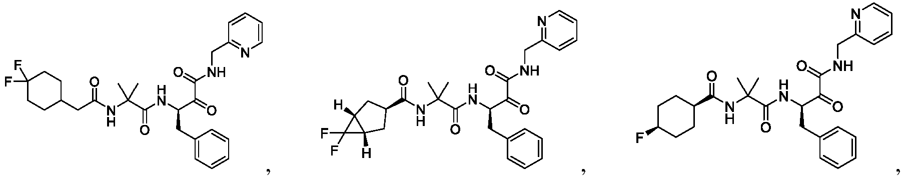

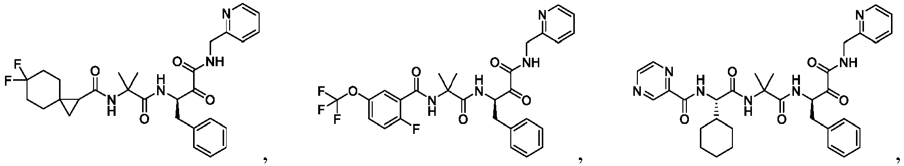

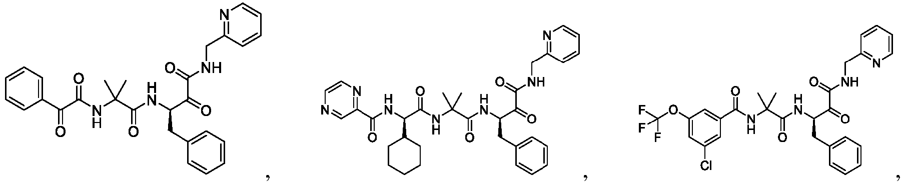

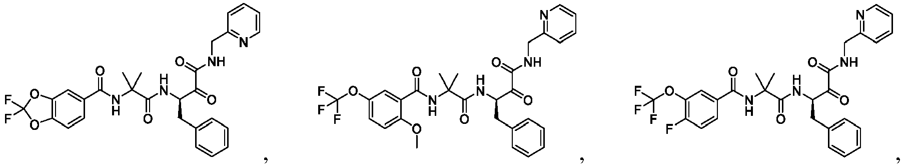

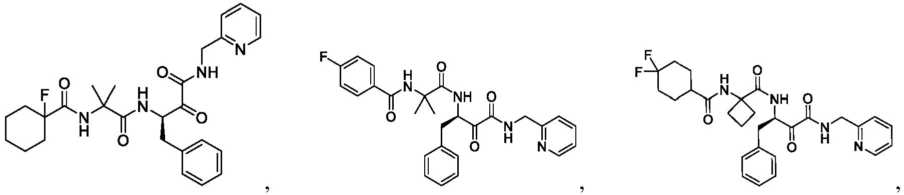

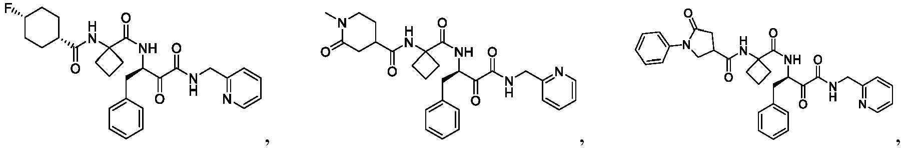

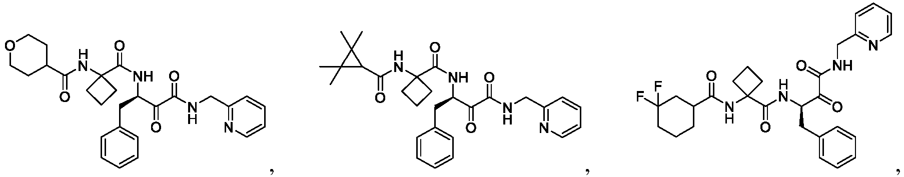

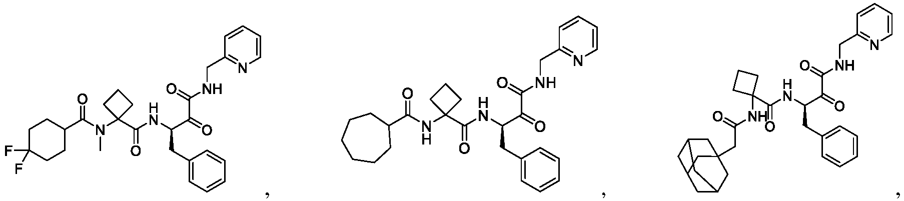

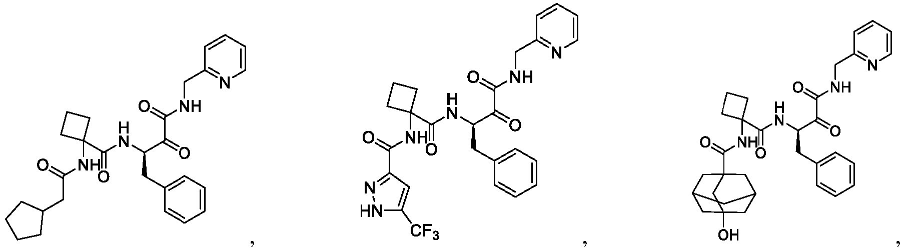

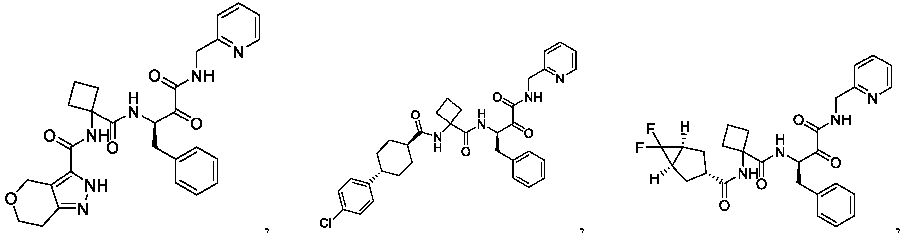

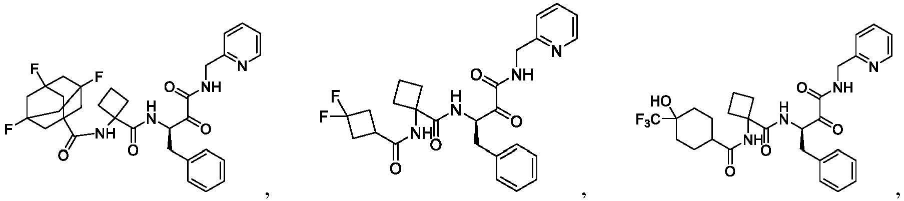

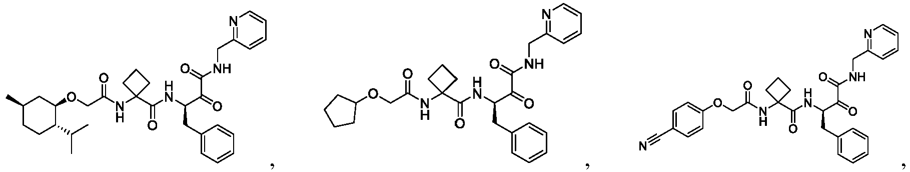

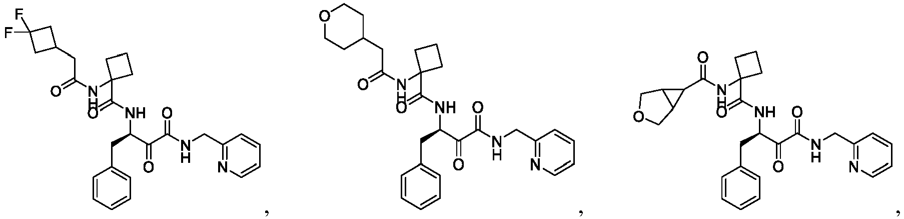

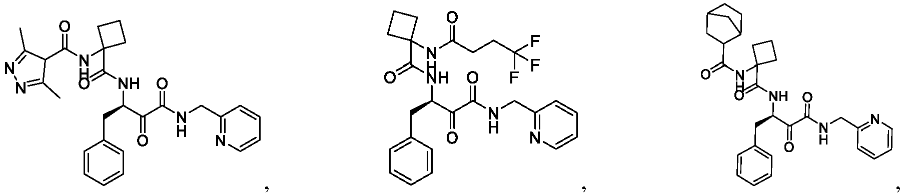

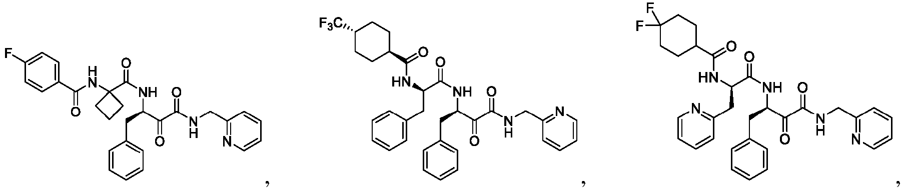

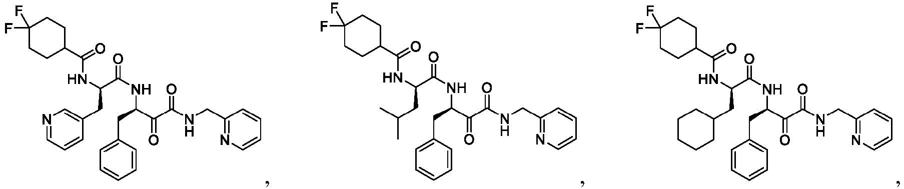

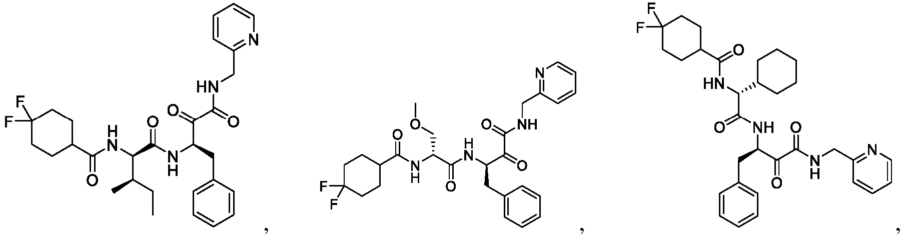

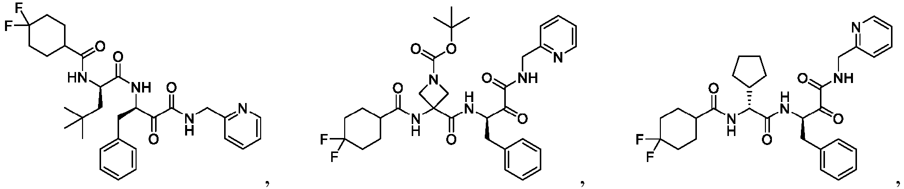

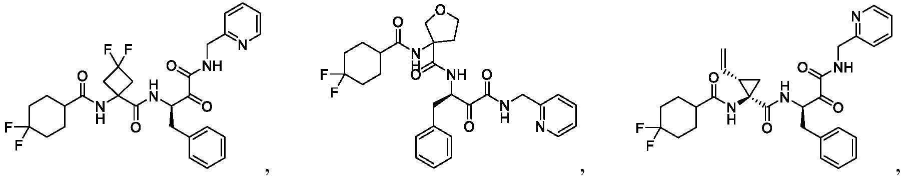

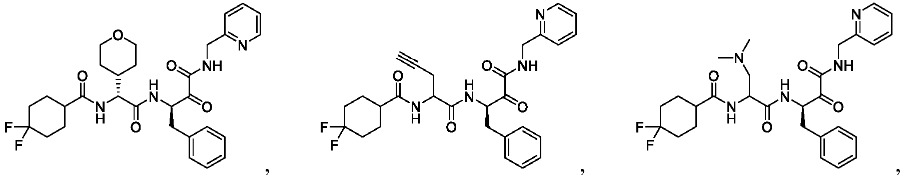

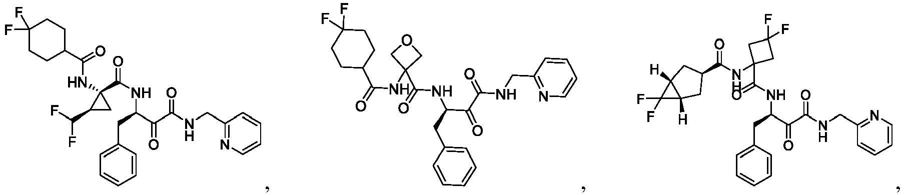

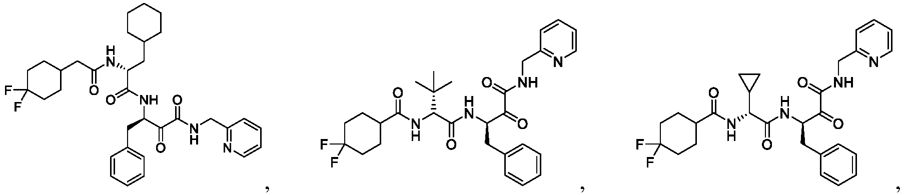

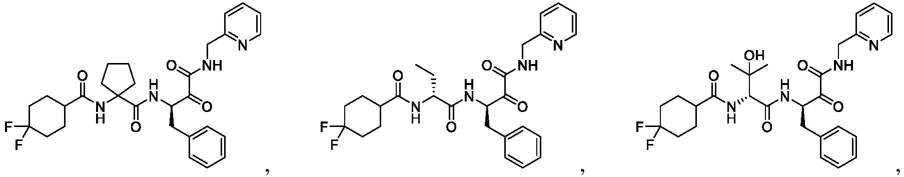

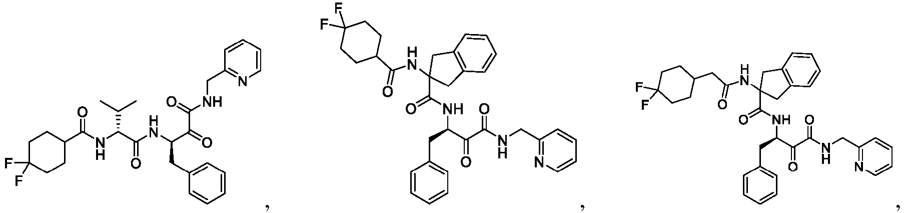

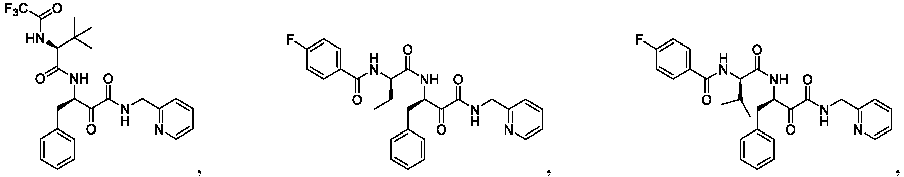

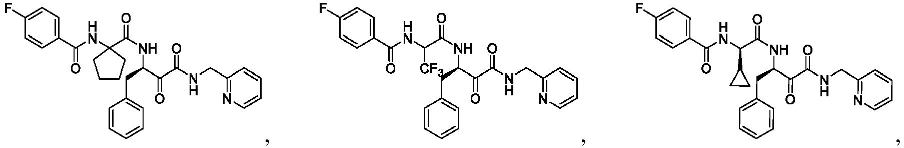

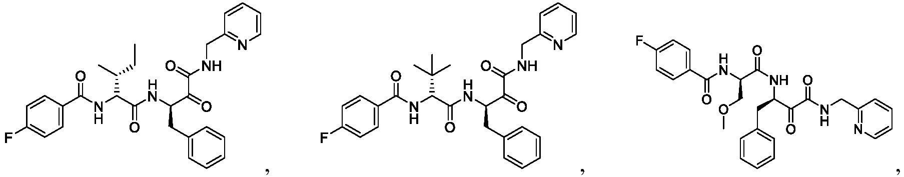

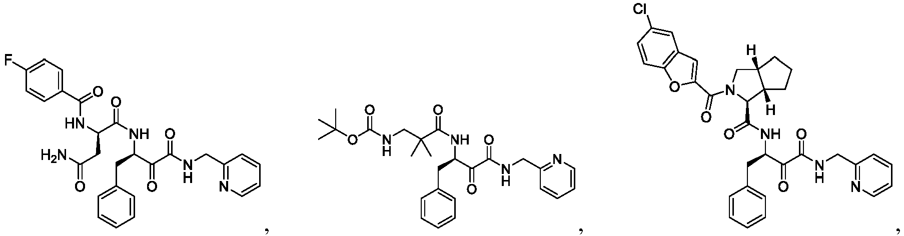

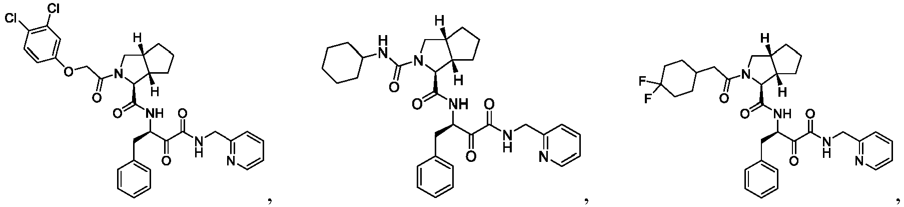

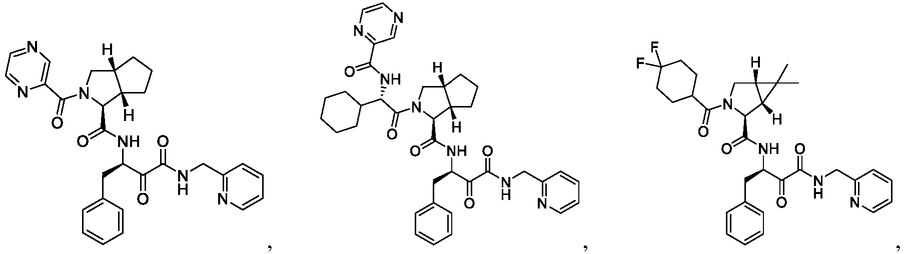

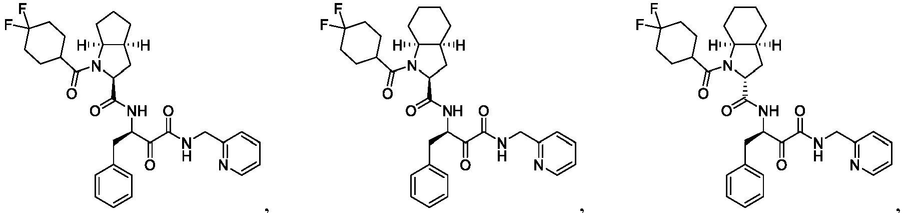

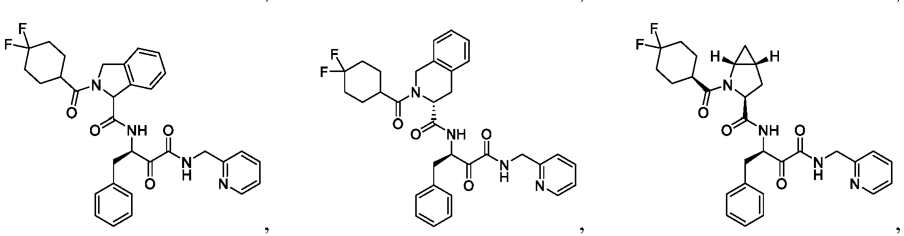

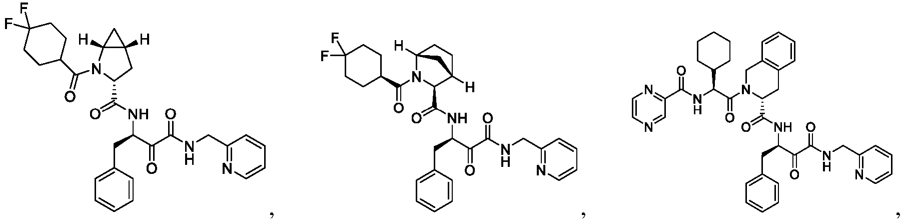

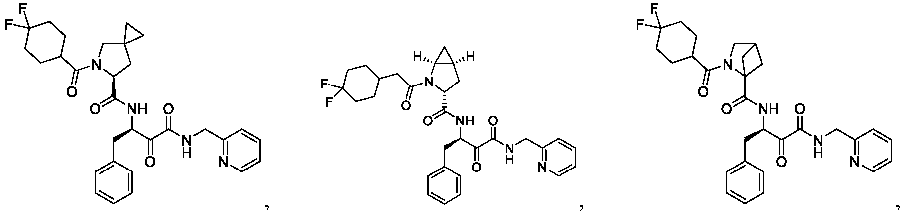

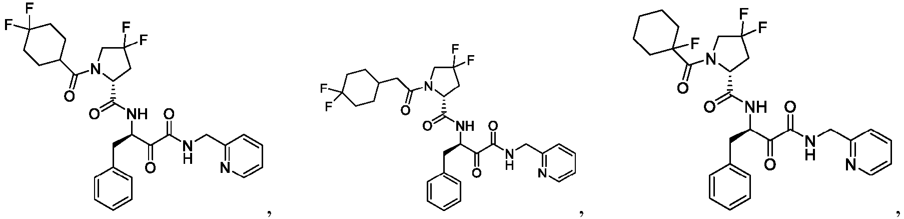

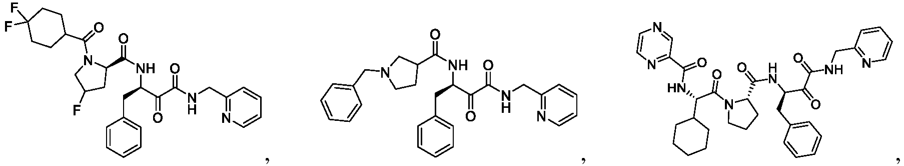

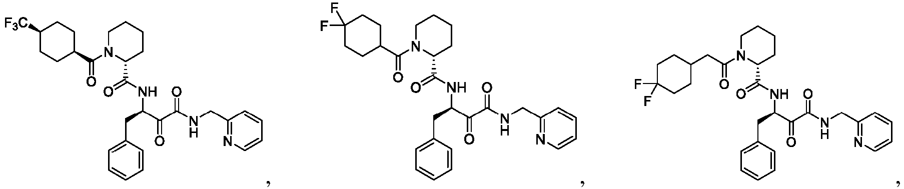

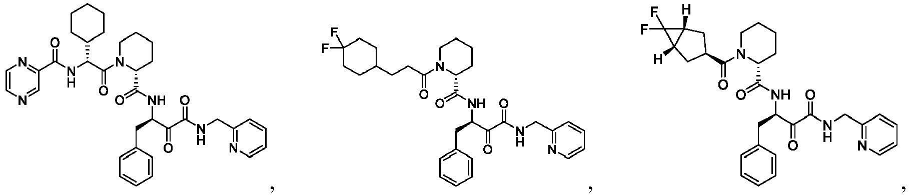

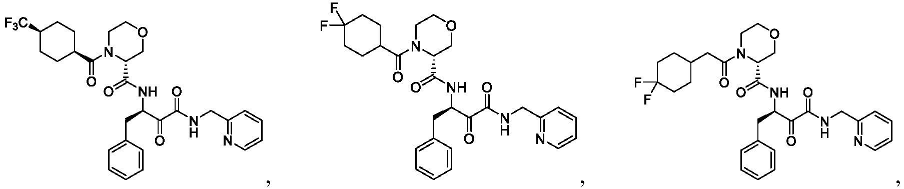

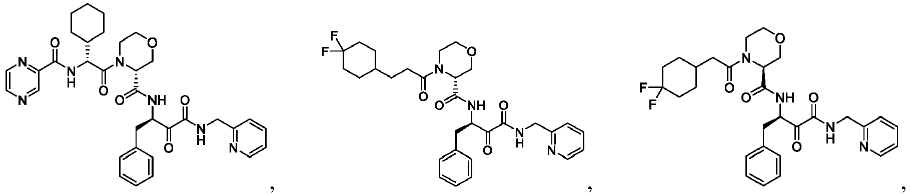

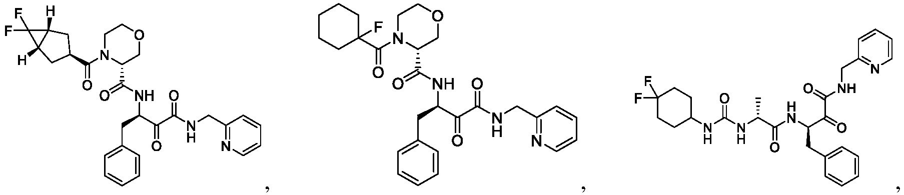

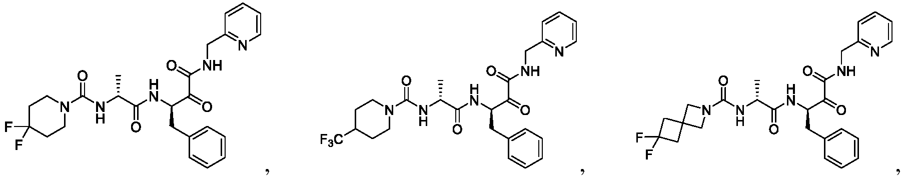

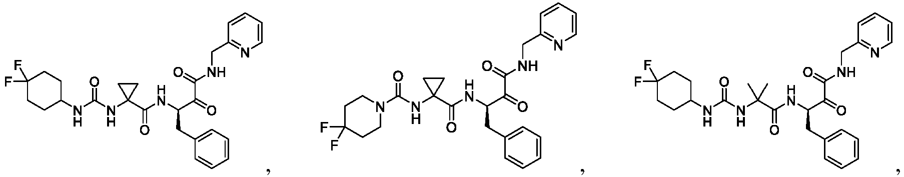

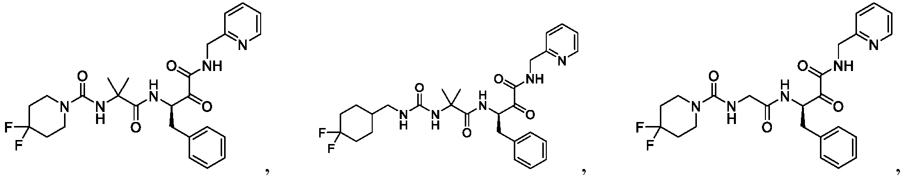

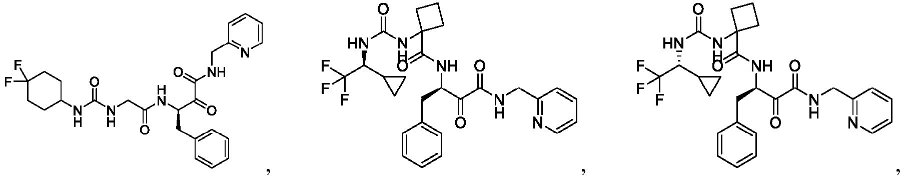

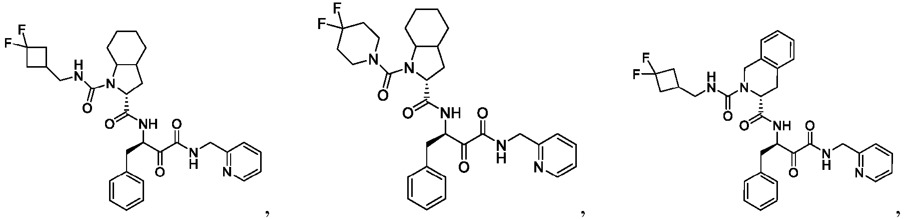

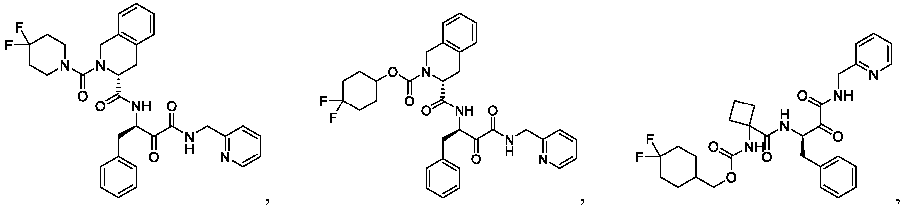

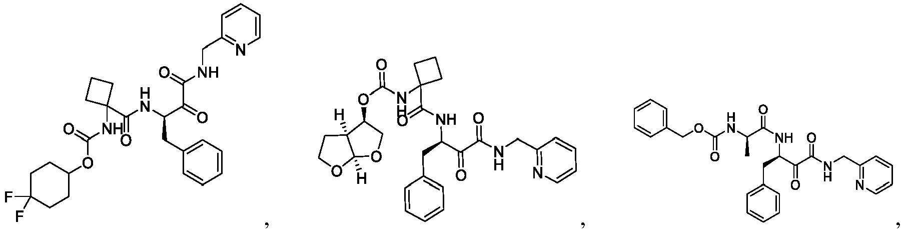

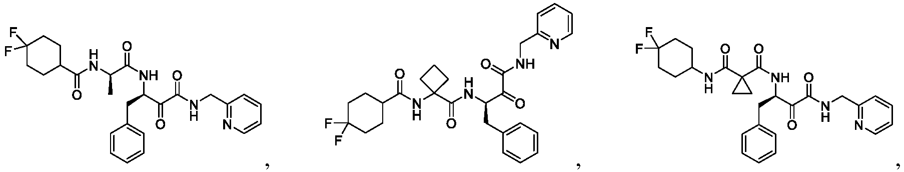

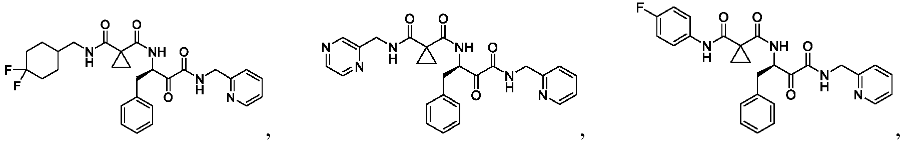

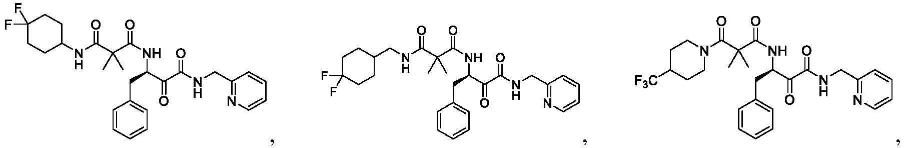

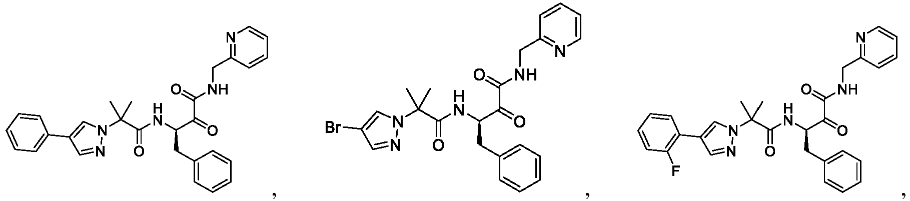

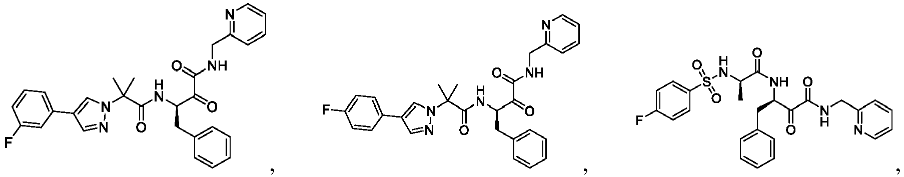

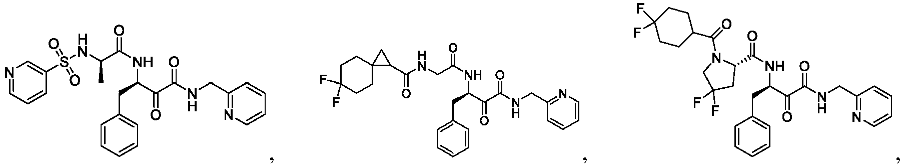

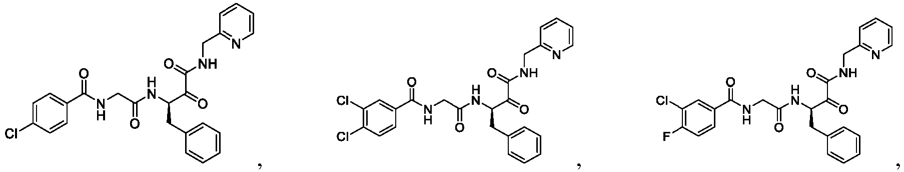

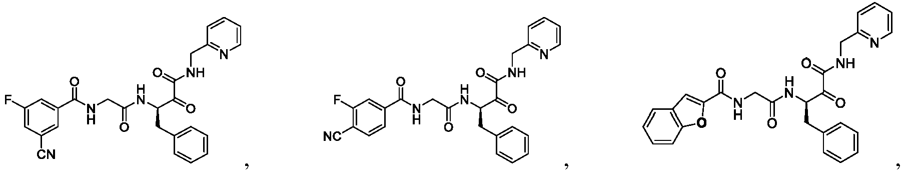

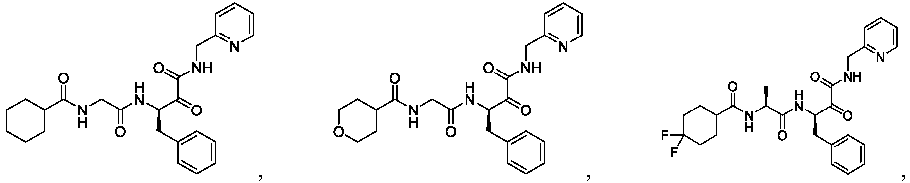

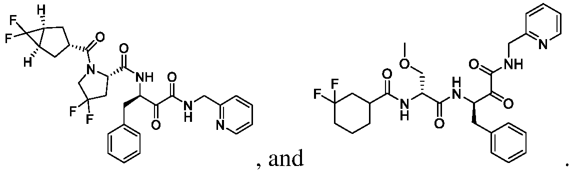

Further, the structure of said compound is as represented by formula II-A-a-2:

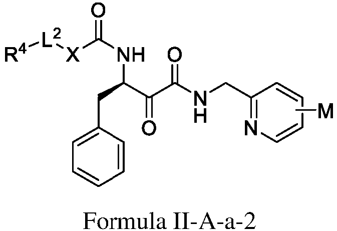

M is selected from the group consisting of F, Cl, CH₃, CHsO or -CF₃.

More further, said compound has any one of the following structures: 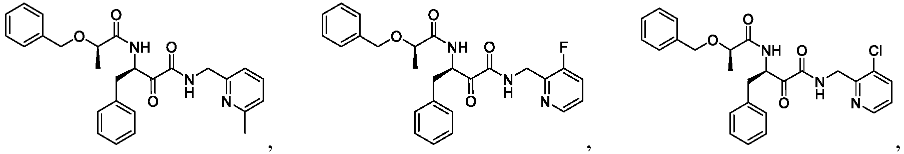 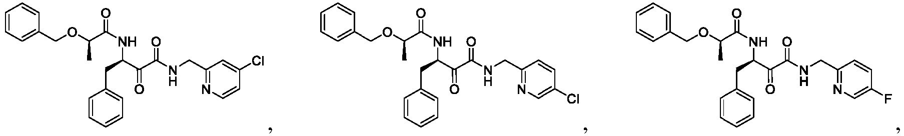 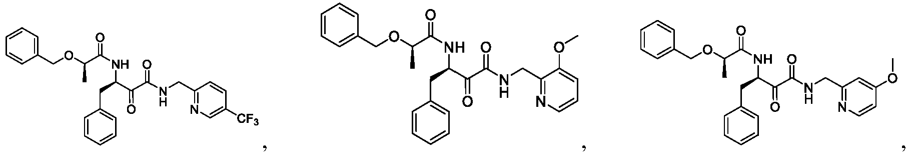 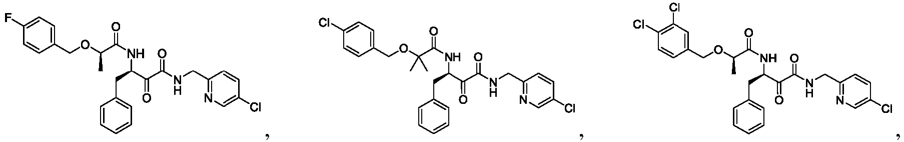 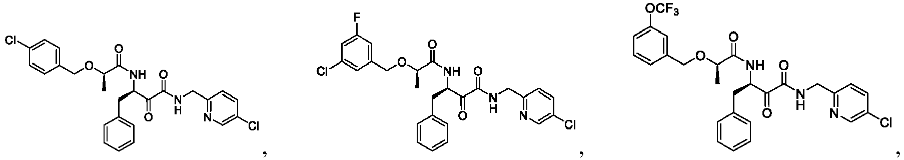 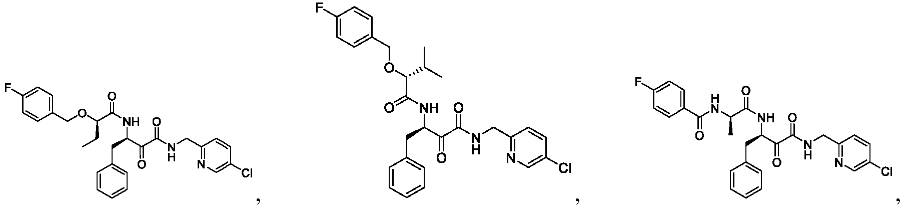 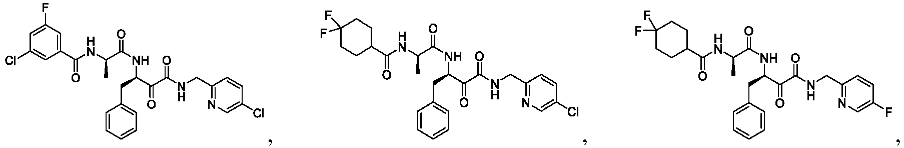 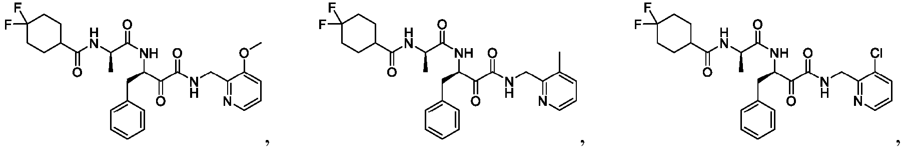 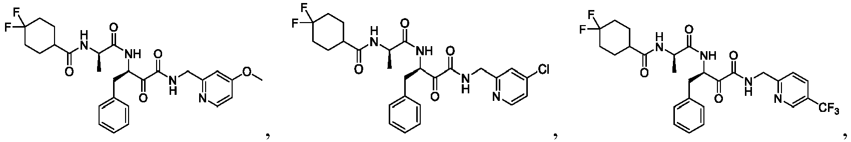 and

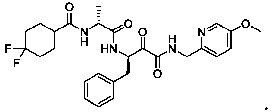

Further, the structure of said compound is as represented by formula II-A-b-1:

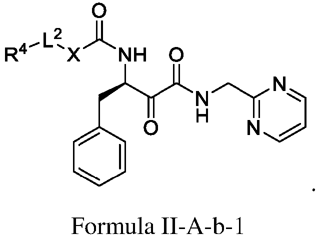

More further, said compound has the following structures:

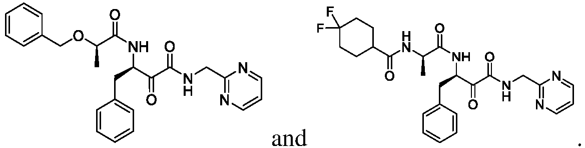

Further, the structure of said compound is as represented by formula II-A-c-1:

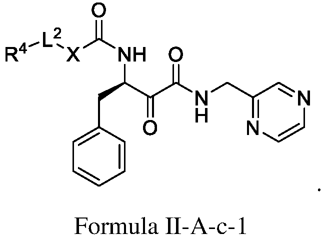

More further, said compound has the following structure:

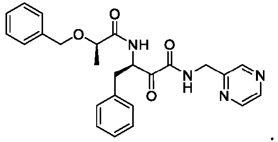

Further, the structure of said compound is as represented by formula II-A-d-1:

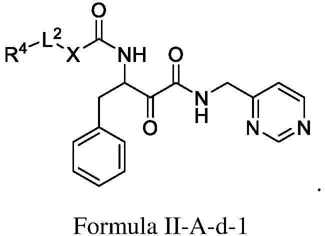

More further, said compound has the following structures:

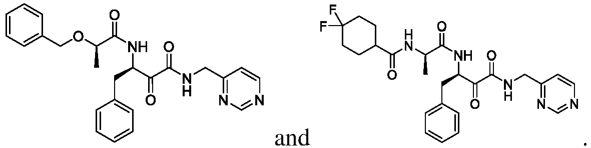

Further, the structure of said compound is as represented by formula II-A-e, formula II-A-f, formula II-A-g, formula II-A-h, formula II-A-i, formula II-A-i1, formula II-A-i2, formula II-A-i3, formula II-A-i4 or formula II-A-i5: 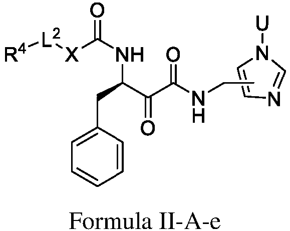 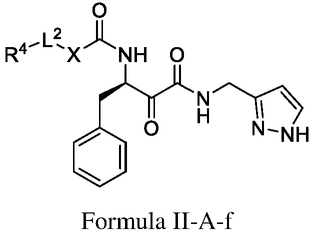 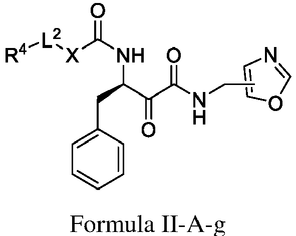 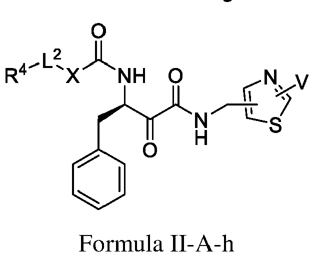 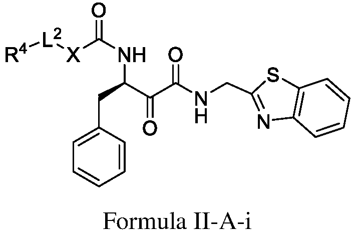 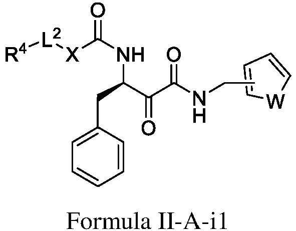 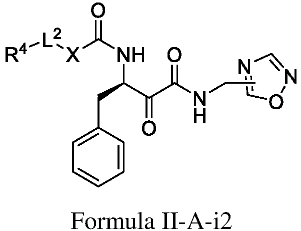 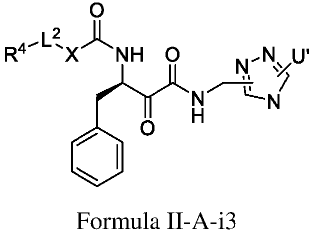 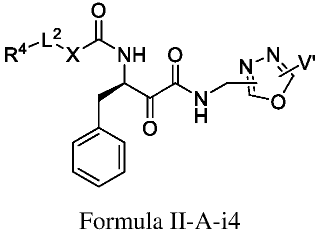 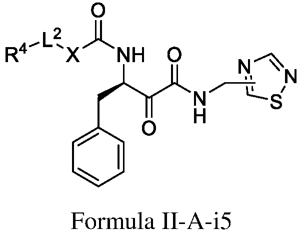 wherein, U, V, U', and V' are each independently selected from the group consisting of H, C₁₋₃ alkyl or halogen, and preferably are H, CH₃ or Cl; W is O or S.

More further, the structure of said compound is as represented by formula II-A-e-1:

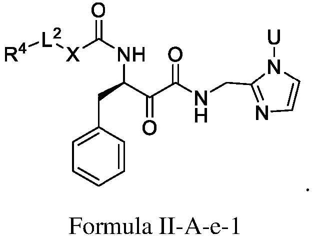

More further, said compound has any one of the following structures:

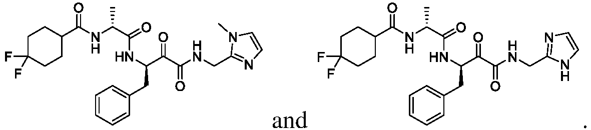

Further, the structure of said compound is as represented by formula II-A-e-2:

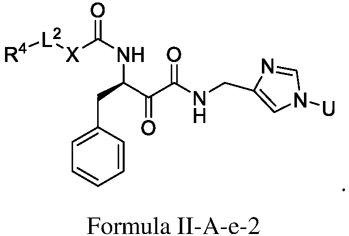

More further, the structure of said compound is as represented by the following:

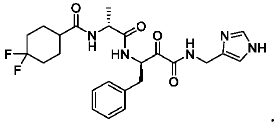

More further, the structure of said compound is as represented by any one of the following structures:

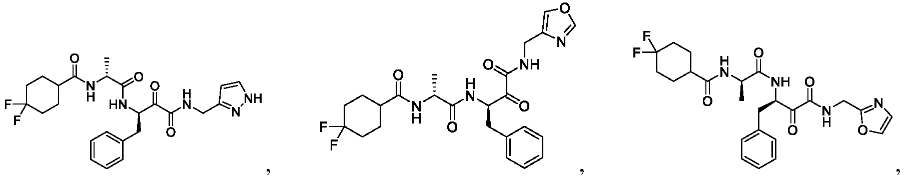

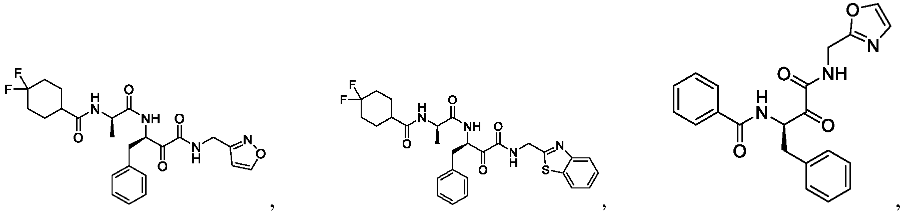

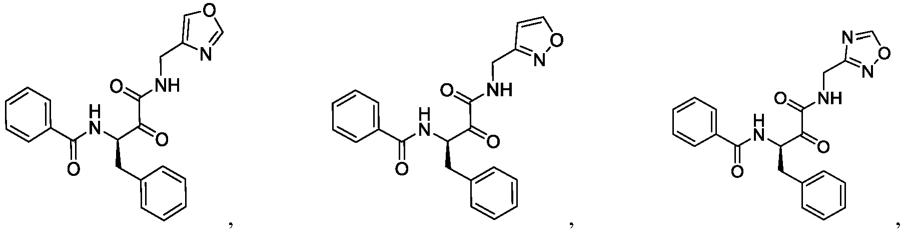

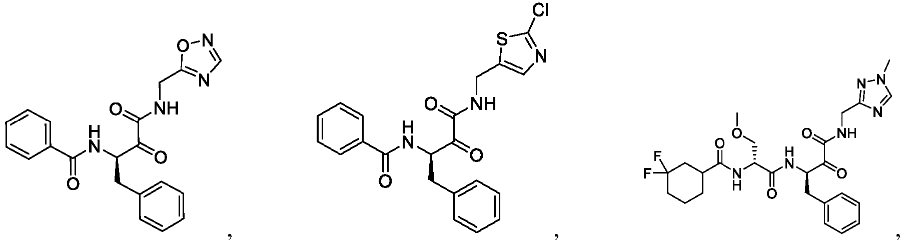

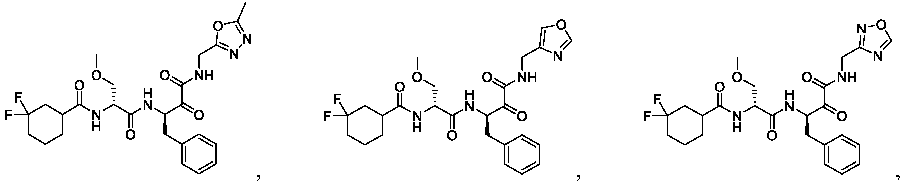

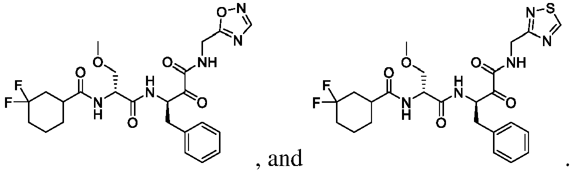

Further, the structure of said compound is as represented by formula II-A-h-1:

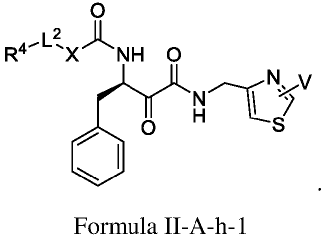

More further, the structure of said compound is as represented by any one of the following structures:

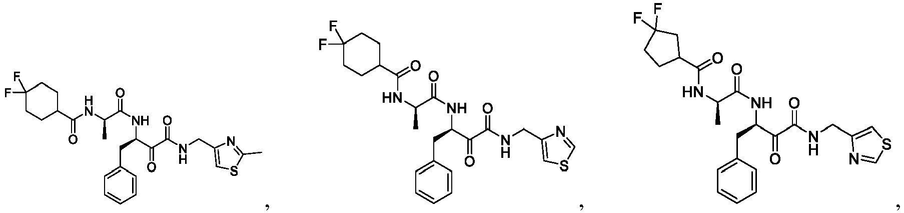

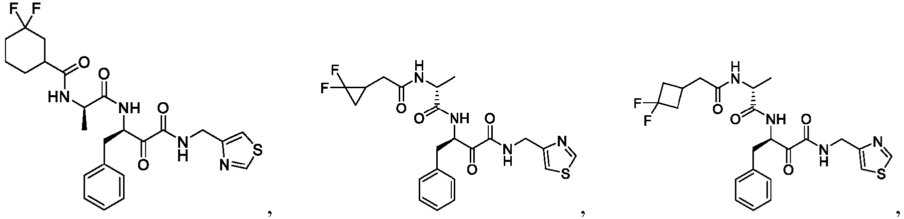

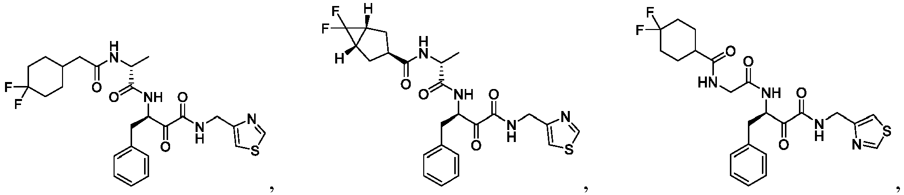

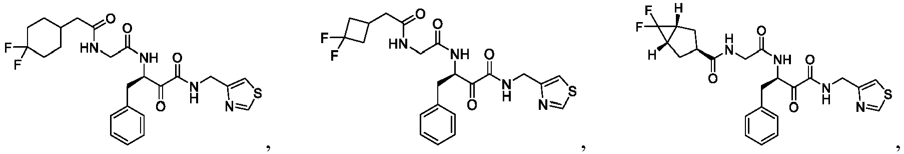

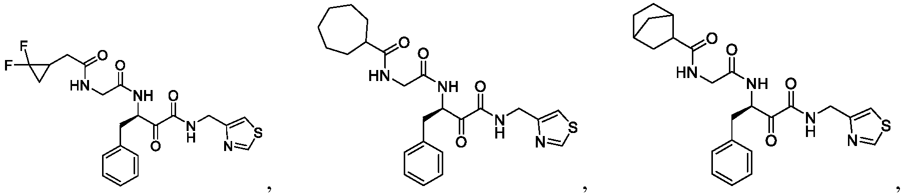

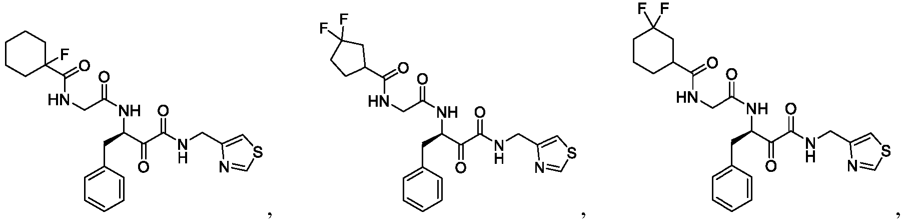

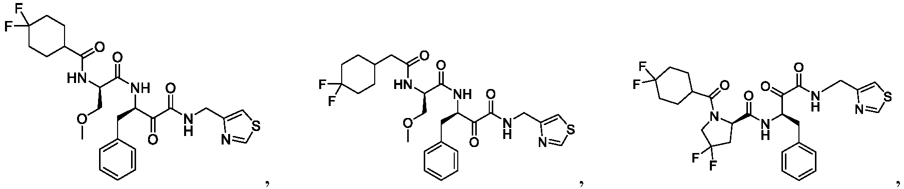

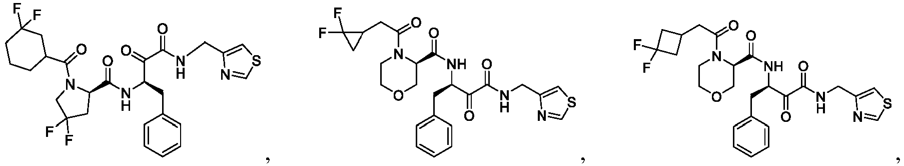

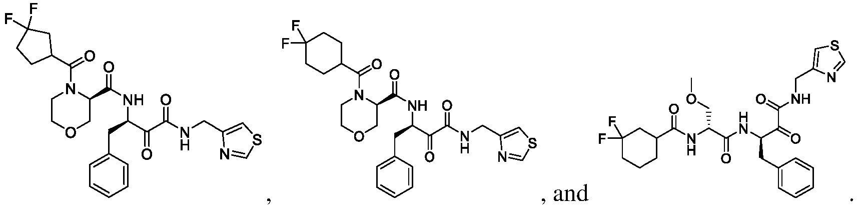

Further, the structure of said compound is as represented by formula II-A-h-2:

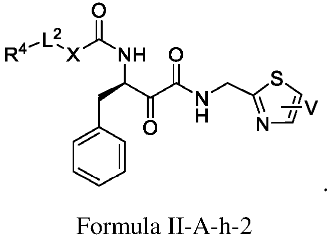

More further, the structure of said compound is as represented by any one of the following structures: 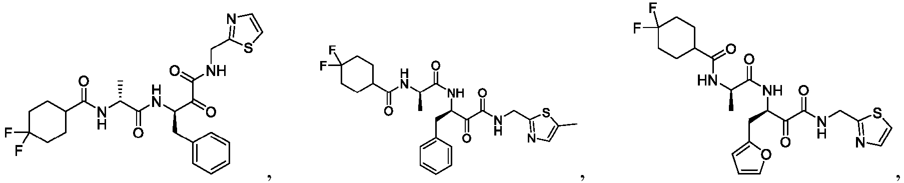 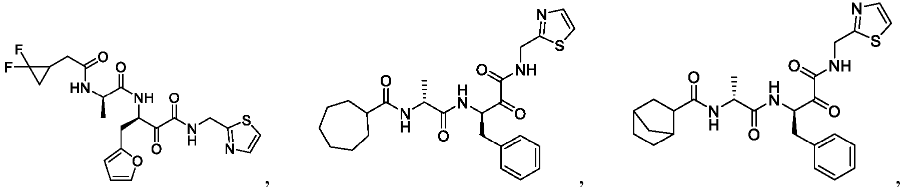 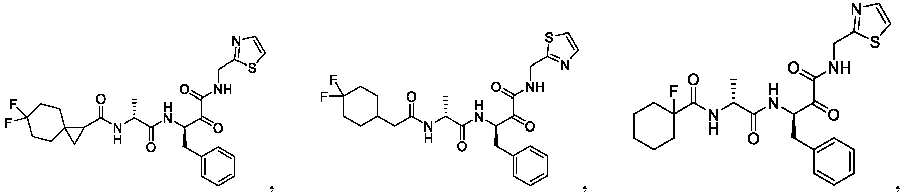 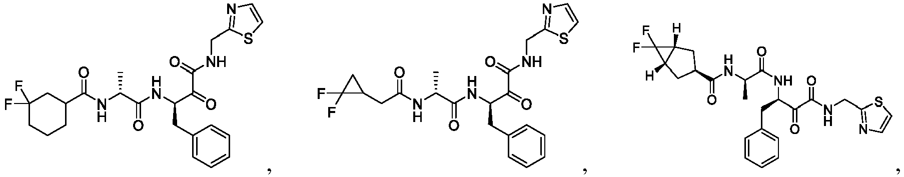 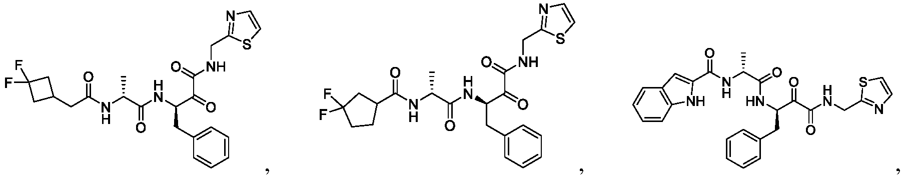 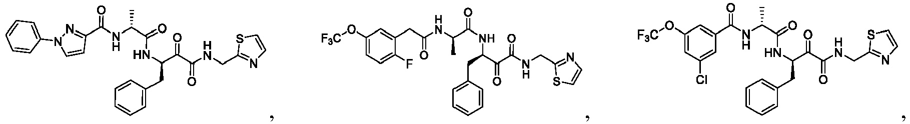 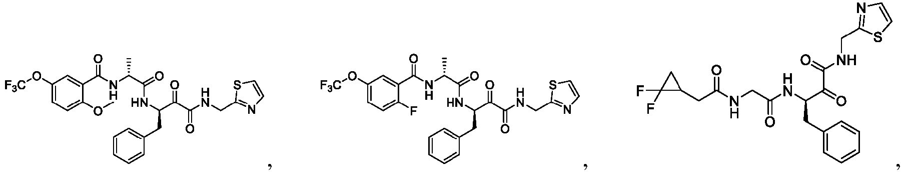 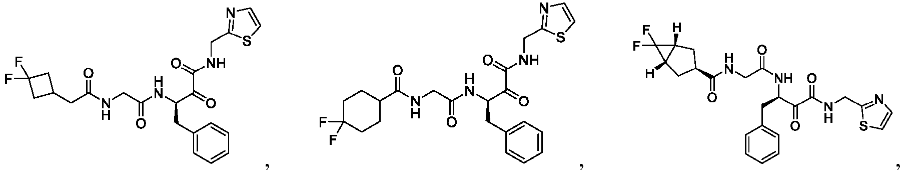 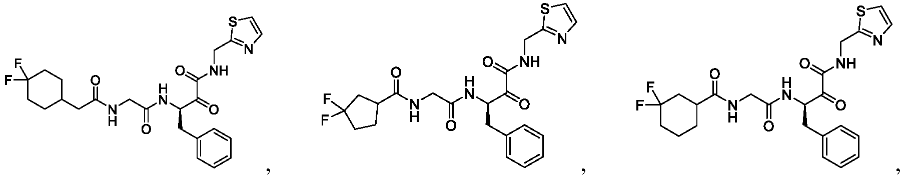 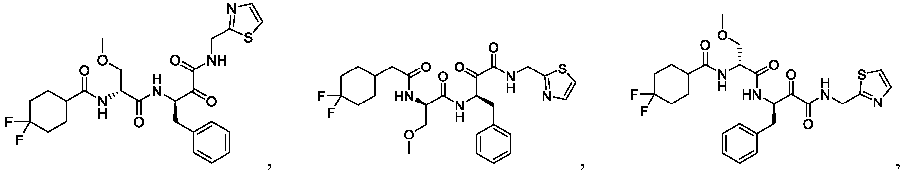 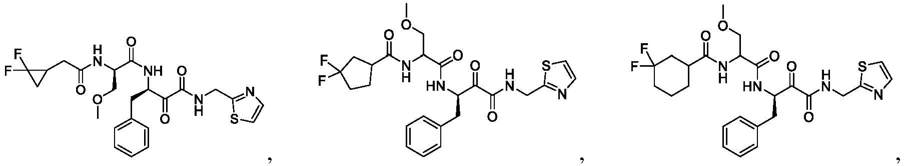 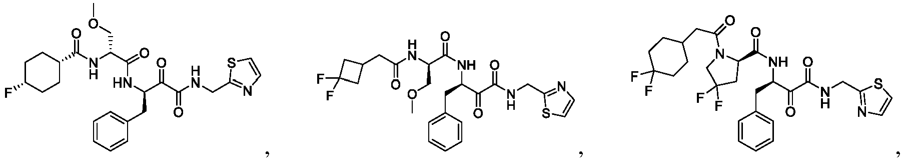 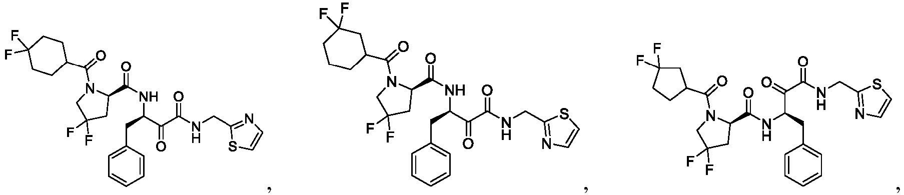 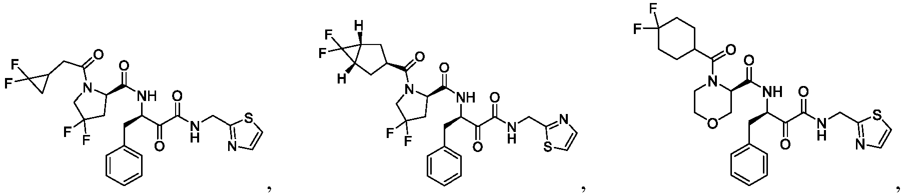 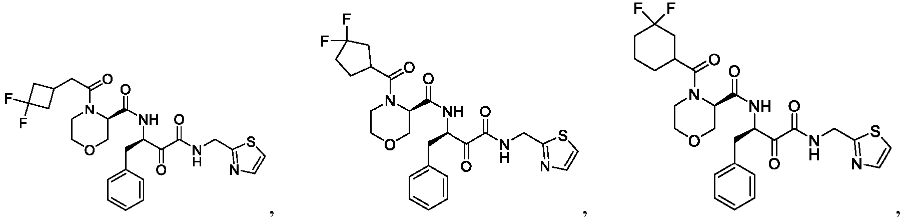 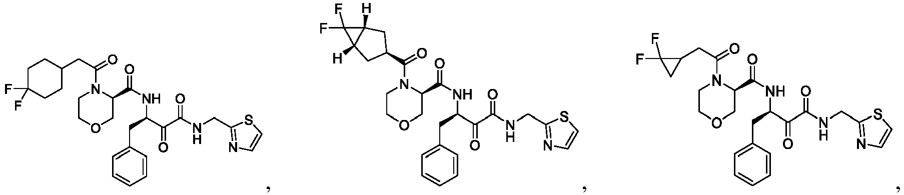 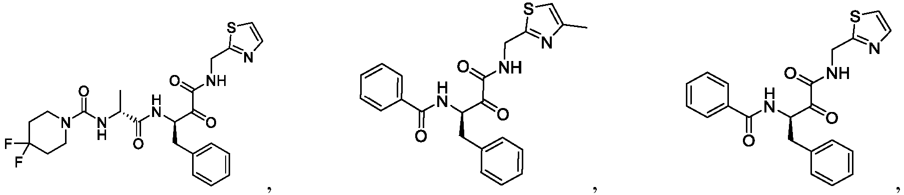 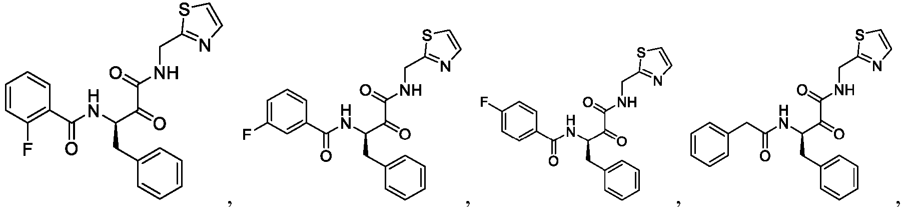 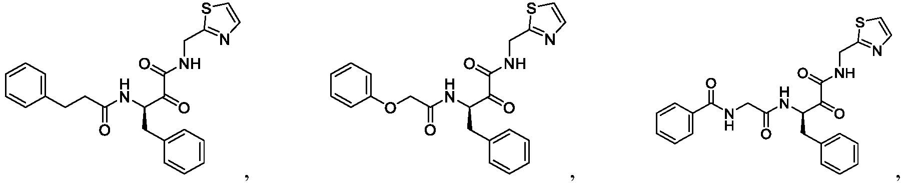 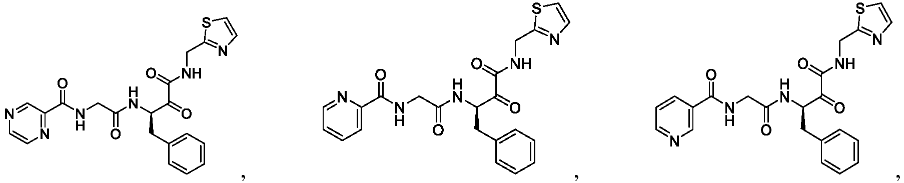 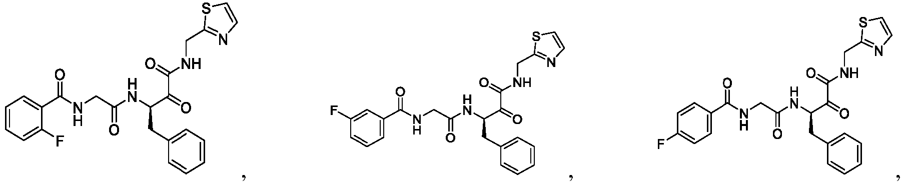 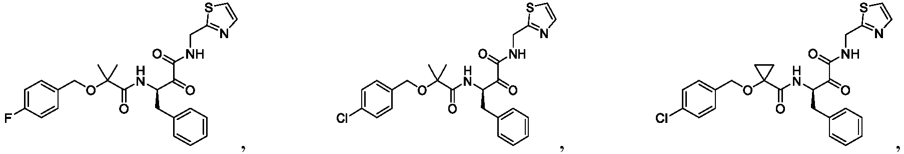 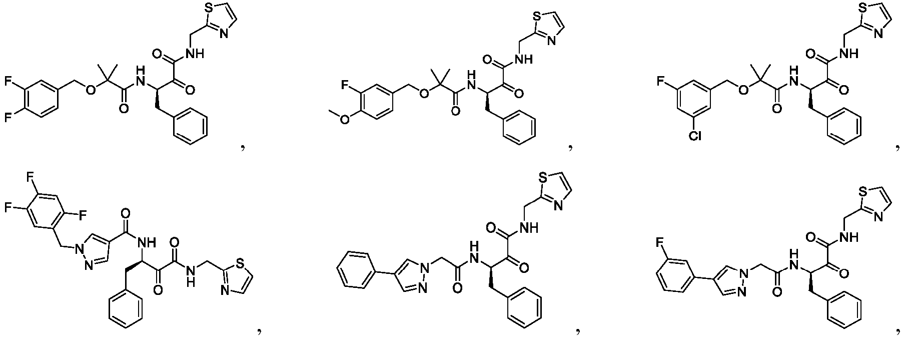 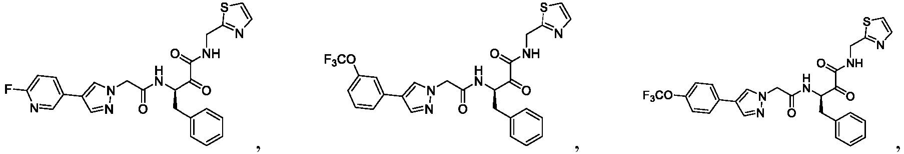 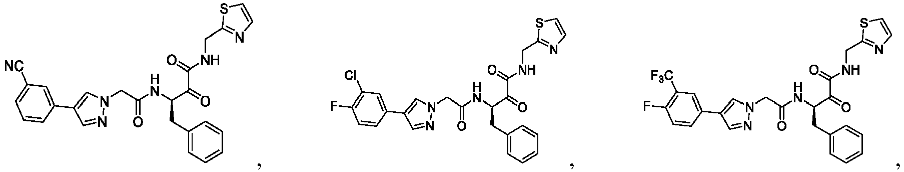 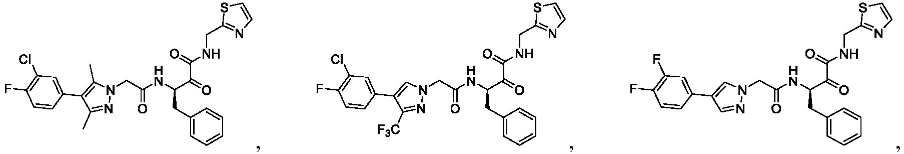 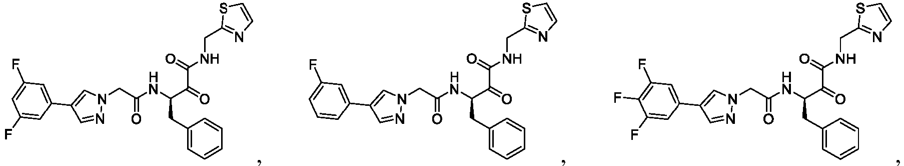 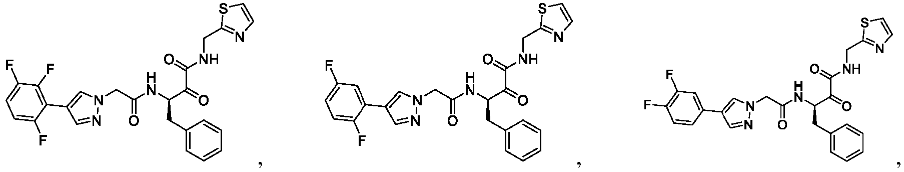 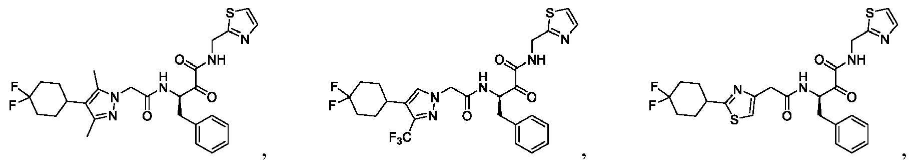 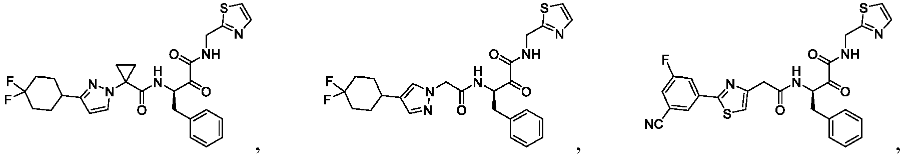 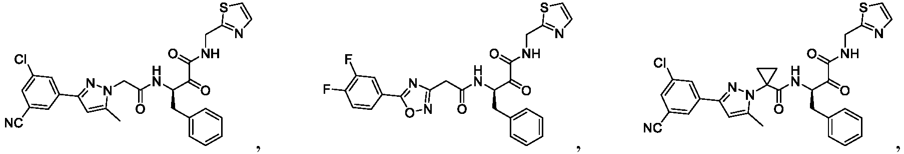 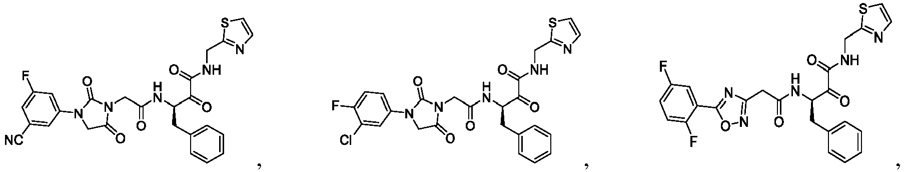 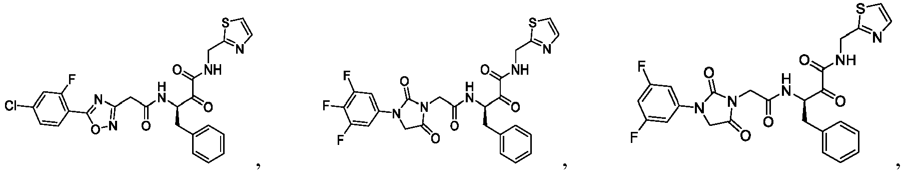 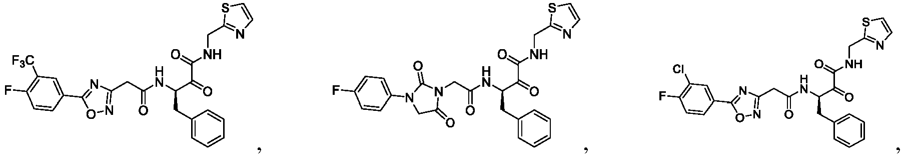 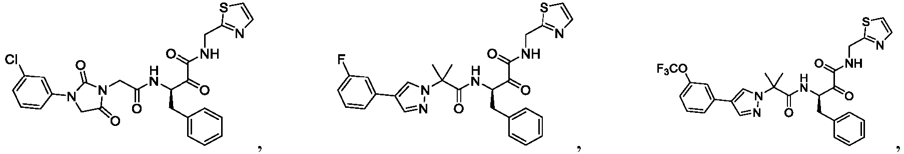 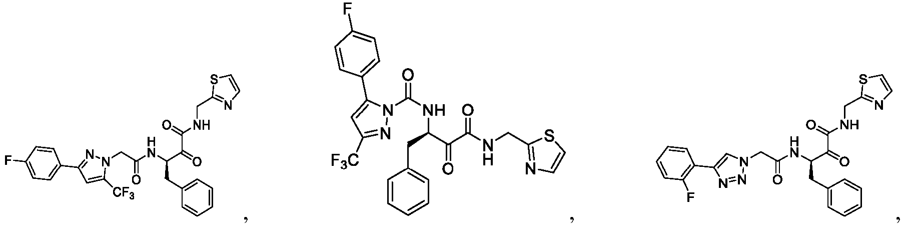 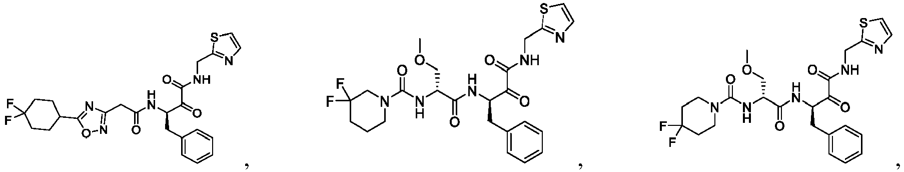 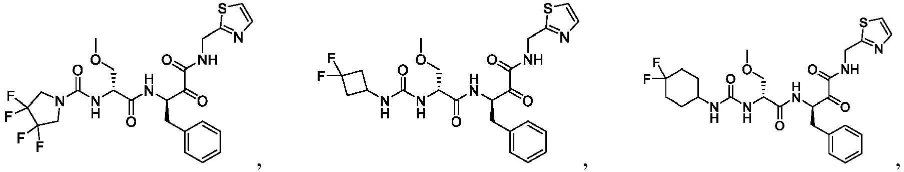 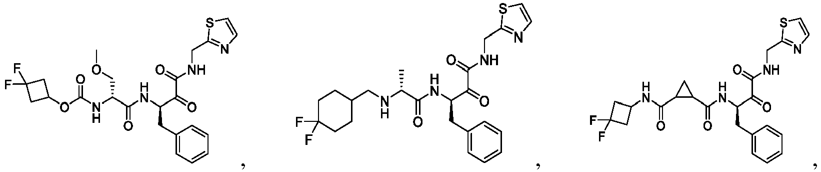 and

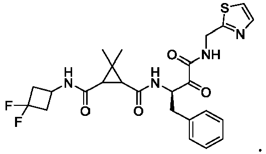

Further, the structure of said compound is as represented by formula II-A-I'-2:

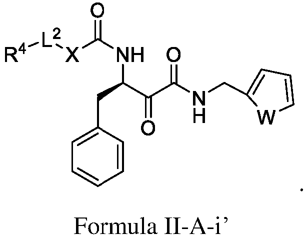

More further, the structure of said compound is as represented by any one of the following structures:

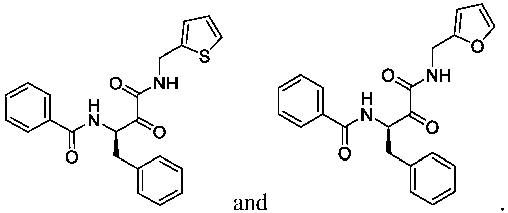

Further, the structure of said compound is as represented by formula II-A-j: 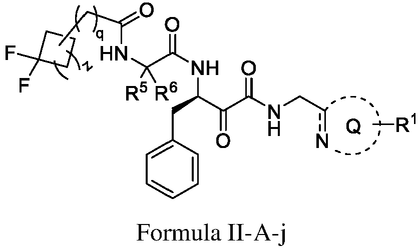
wherein, z is any integer from 1 to 3; q is any integer from 0 to 4; at least one of R⁵ and R⁶ is not H;
preferably, z is 1, 2, or 3; q is either 0 or 1.

More further, the structure of said compound is as represented by formula II-A-j-1:

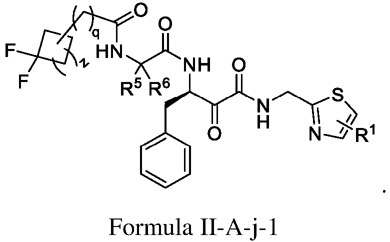

More further, said compound has any one of the following structures:

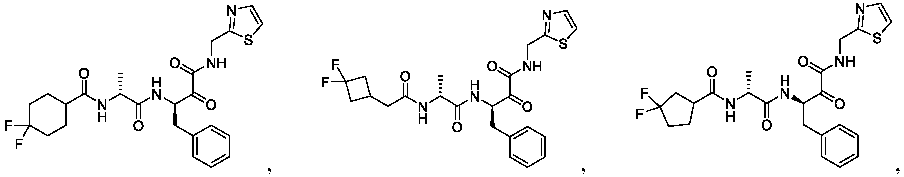

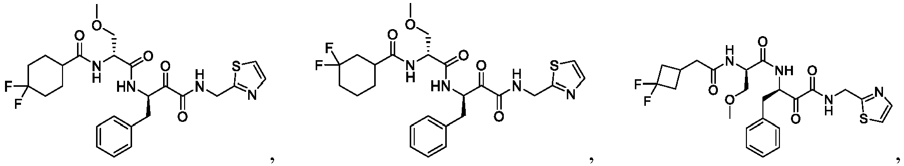

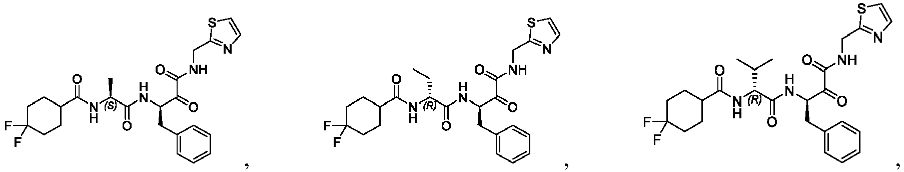

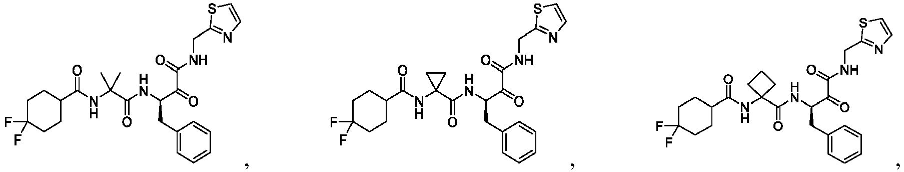

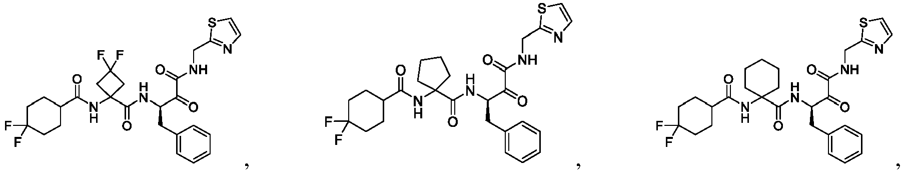

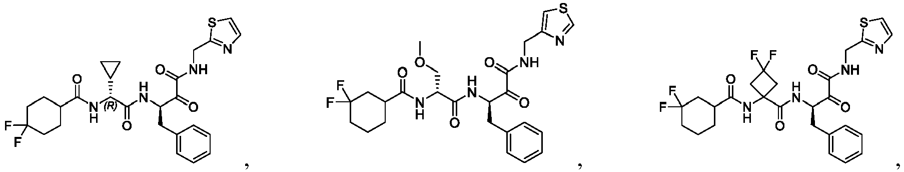

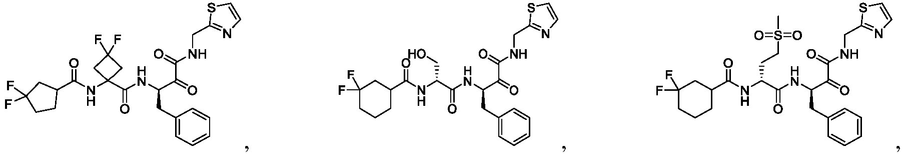

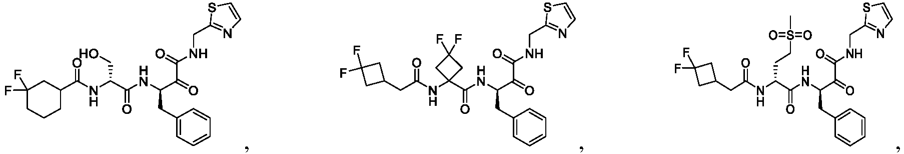

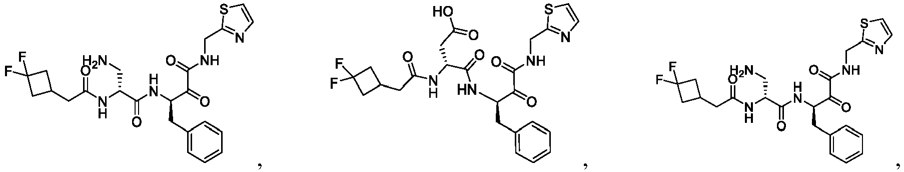

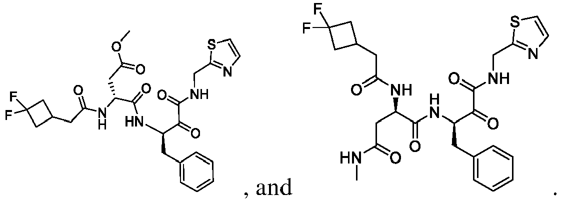

Further, the structure of said compound is as represented by formula II-B, II-C or II-D:

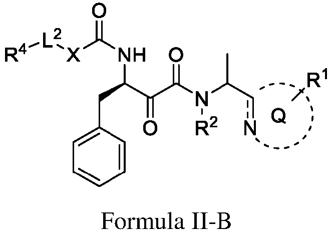

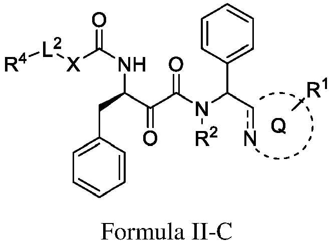

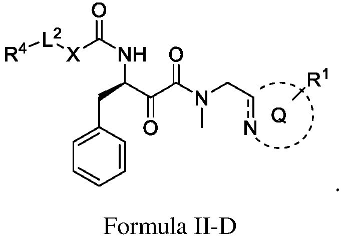

More further, the structure of said compound is as represented by formula II-B-a:

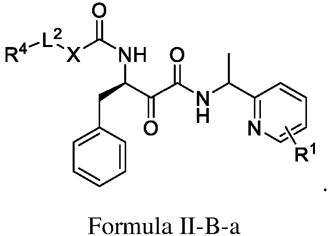

More further, the structure of said compound is as represented by formula II-B-a-1:

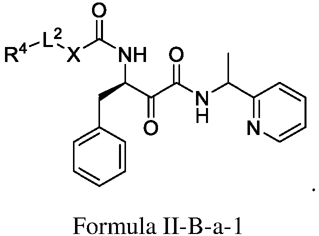

More further, said compound has the following structure:

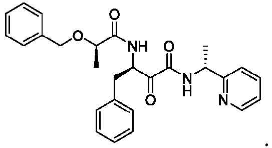

More further, the structure of said compound is as represented by formula II-C-a:

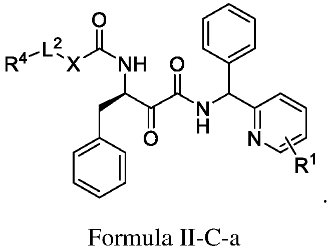

More further, the structure of said compound is as represented by formula II-C-a-1:

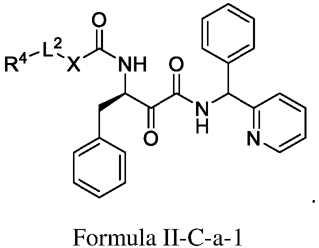

More further, said compound has the following structure:

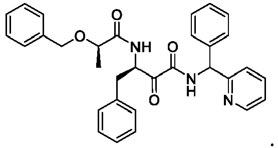

Further, the structure of said compound is as represented by formula II-D-a:

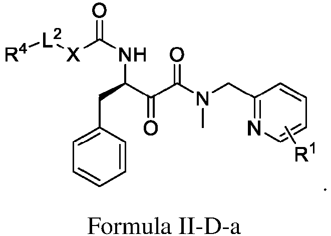

More further, the structure of said compound is as represented by formula II-D-a-1:

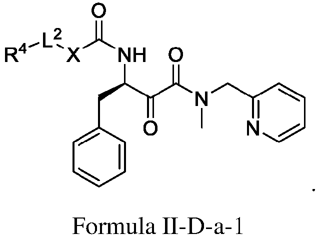

More further, said compound has the following structure:

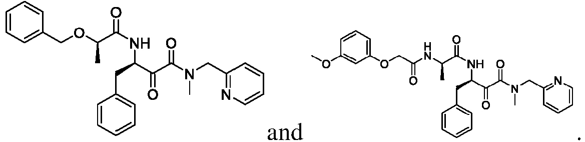

Further, the structure of said compound is as represented by formula III-A, formula III-B, formula III-C, formula III-D, formula III-E or formula III-F: 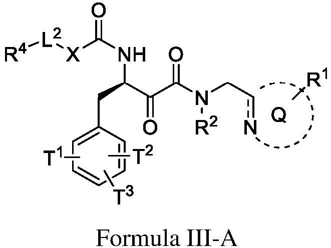 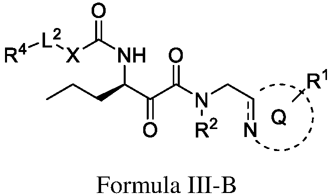 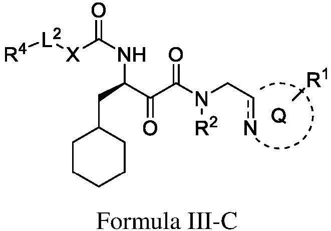 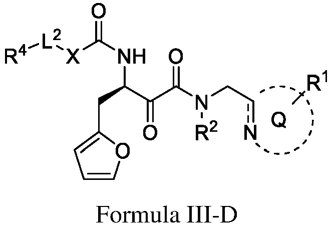 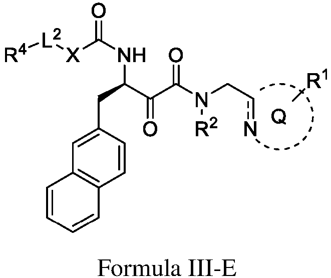 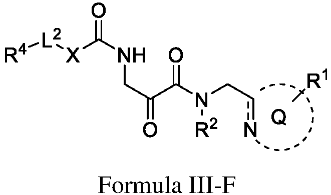 wherein, T¹, T², and T³ are each independently selected from H or halogen, and at least one of them is halogen.

More further, said T is F.

More further, said R¹ is Cl or H.

More further, the structure of the compound is as represented by formula III-A-a, formula III-A-b, formula III-B-a, formula III-C-a, formula III-C-b, formula III-D-a, formula III-D-b, formula III-E-a or formula III-F-a:

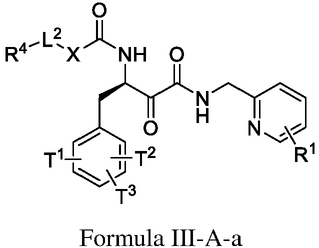

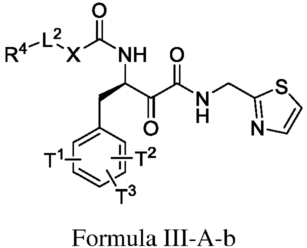

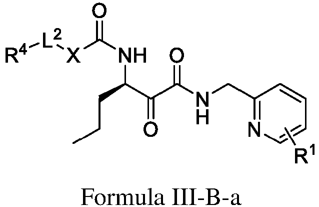

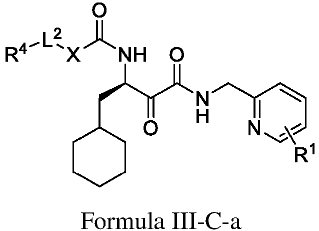

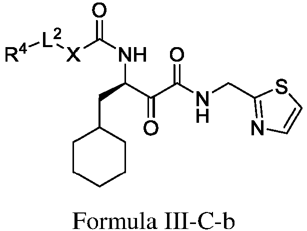

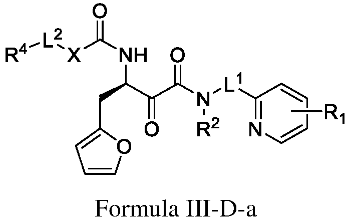

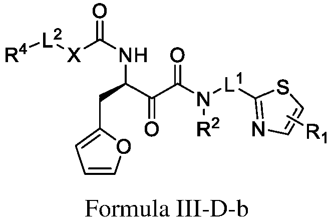

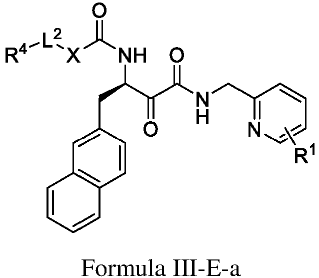

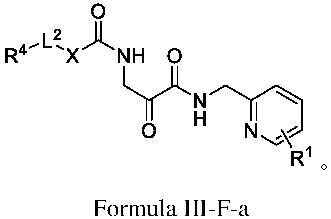

More further, said compound has any one of the following structures:

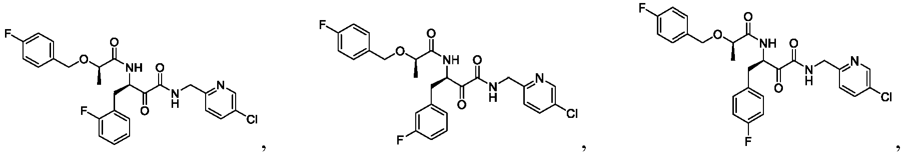

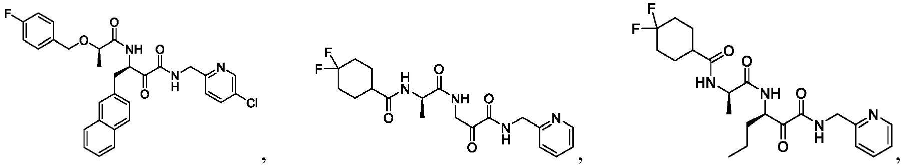

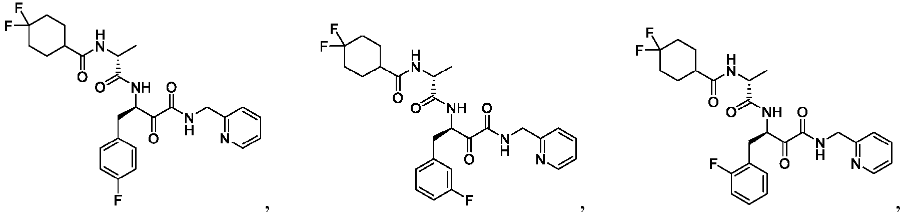

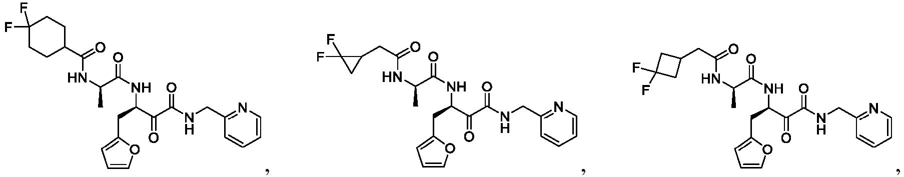

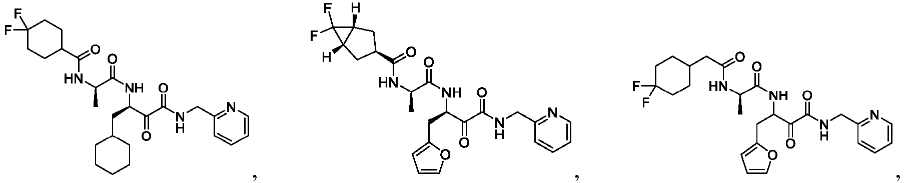

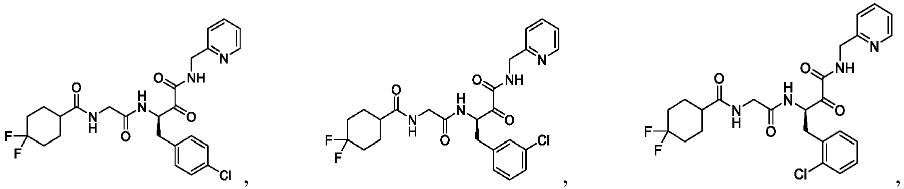

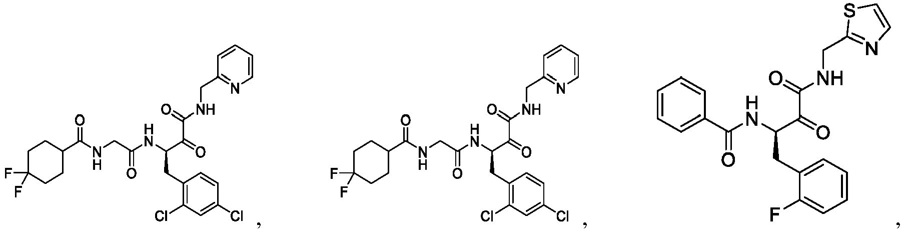

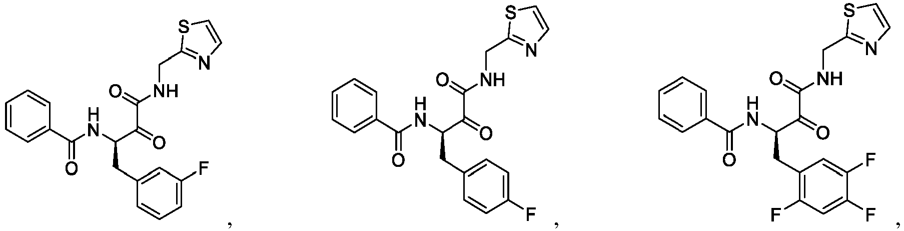

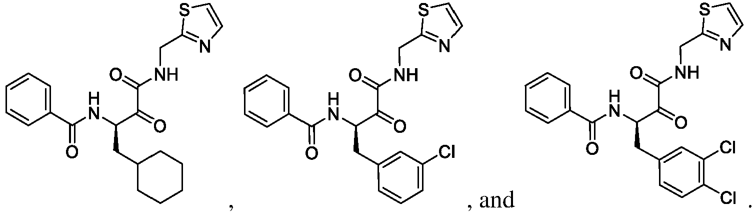

The present invention also provides a pharmaceutical composition, which is a preparation formed by the compound mentioned above, or a pharmaceutically acceptable salt thereof, or a stereoisomer thereof, or an optical isomer thereof, or a deuterated compound thereof, as the active ingredient, in association with pharmaceutically acceptable excipients.

The present invention also provides the use of above compound, or a pharmaceutically acceptable salt thereof, or a stereoisomer thereof, or an optical isomer thereof, or a deuterated compound thereof in the manufacturer of medicaments for preventing and/or treating coronavirus-related diseases.

Further, the medicaments for preventing and/or treating coronavirus-related diseases are anti-coronavirus medicaments.

More further, the anti-coronavirus medicaments are those inhibiting coronavirus infection in cells.

More further, the anti-coronavirus medicaments are the inhibitors of coronavirus proteolytic enzymes, and preferably are the inhibitors of coronavirus main proteases.

Further, the coronavirus is SARS-CoV-2, SARS-CoV, MERS-CoV, HcoV-229E, HcoV-NL63, HcoV-HKU1 or HcoV-OC43, and preferably is SARS-CoV-2.

More further, the medicaments for preventing and/or treating coronavirus-related diseases are those for preventing and/or treating Corona Virus Disease 2019 (COVID-19).

More further, the inhibitors of coronavirus main proteases are SARS-CoV-2 M^{pro} inhibitors.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

The minimum and the maximum for the content of carbon atoms in hydrocarbon groups are represented by prefixes, such as the prefix C_{a-b} alkyl indicates any alkyl having "a" to "b" carbon atoms. For example, C₁₋₈ alkyl means a straight or branched alkyl having 1-8 carbon atoms.

"Alkylene" refers to the group obtained by an alkyl losing one atom. For example, C1-3 alkylene refers to the group resulted from C1-3 alkyl losing one atom.

Herein, "substitution" means that one, two or more hydrogens in a molecule are substituted with other different atoms or molecules, including one, two or more substitutions on the same or different atoms in the molecule.

"A deuterated compound" refers to the compound obtained by substituting one or more hydrogens in a compound with deuterium.

"Pharmaceutically acceptable" refers to a carrier, vehicle, diluent, excipient, and/or formed salt that is typically chemically or physically compatible with other components contained in a pharmaceutical formulation, and physiologically compatible with the receptor.

"Salt" refers to an acidic and/or basic salt formed by combining a compound or its stereoisomer with inorganic and/or organic acids and/or bases, as well as zwitterionic salts (inner salts) and quaternary ammonium salts, such as alkyl ammonium salts. These salts can be directly obtained during the final separation and purification of the compound. It can also be obtained by mixing a compound or its stereoisomer with a certain amount of acid or base (such as equivalency). These salts may form precipitates in a solution and be collected by filtration, recovered after evaporation of solvents, or prepared by freeze-drying after reaction in an aqueous medium.

"Pharmaceutically acceptable salts" can be a compound's hydrochloride, sulfate, citrate, benzenesulfonate, hydrobromate, hydrofluorate, phosphate, acetate, propionate, succinate, oxalate, malate, succinate, fumarate, maleate, tartrate, or trifluoroacetate.

"Halogens" are fluorine, chlorine, bromine, or iodine.

"Aryl" refers to all-carbon monocyclic or fused polycyclic (i.e., the rings sharing adjacent carbon atom pairs) groups with conjugated π electron systems, such as phenyl. The aryl cannot contain heteroatoms such as N, O, or S, and the point connecting the parent must be the carbon atom in the ring with a conjugated π electron system. The aryl can be substituted or unsubstituted. "5-6-membered aryl" refers to an aryl containing 5 or 6 carbon atoms in the ring.

"Heteroaryl" refers to a heteroaromatic group containing one or more heteroatoms. The heteroatoms, as used herein, include oxygen, sulfur, and nitrogen. For example, furyl, thienyl, pyridyl, pyrazolyl, etc. The heteroaryl can be optionally substituted or unsubstituted. "5-6-membered heteroaryl" refers to a heteroaryl with a ring atom number of 5 or 6.

"Cycloalkyl" refers to saturated or unsaturated cyclic hydrocarbon substituents. For example, "3-8-membered saturated cycloalkyl" means a saturated cycloalkyl containing 3-8 ring carbon atoms.

"Heterocyclyls" refer to saturated or unsaturated cyclic hydrocarbon substituents; and the cyclic hydrocarbon carries at least one ring heteroatom (including but not limited to O, S, or N). For example, "3-8-membered saturated heterocyclyl" means a heterocyclyl having 3-8 ring atoms. "Oxygen heterocycles" refer to heteroatoms in heterocycles that are O but not S and N. And so on.

"Bridged group" refers to a polycyclic cycloalkyl, in which two rings share two adjacent carbon atoms.

"Fused aryl" refers to a polycyclic aryl, in which two rings share two adjacent carbon atoms, for example, naphthyl (i.e. (6-membered aromatic ring)-fused 6-membered aromatic ring, or benzobenzene ring), anthranyl, and phenanthrenyl.

"Fused heteroaromatic ring/fused heteroaryl" refers to a polycyclic aromatic ring/aryl containing at least one heteroatom (O, N, or S), wherein two rings share two adjacent carbons or heteroatoms. For example, (5-membered aromatic ring or heteroaromatic ring (e.g. furan, thiophene, pyrrole, pyridine ring))-fused (6-membered aromatic ring or heteroaromatic ring) (e.g. benzene, pyridine, pyridazine, pyrimidine, pyrazine ring), such as indole, or (6-membered aromatic ring or heteroaromatic ring)-fused (6-membered aromatic ring or heteroaromatic ring), such as quinoline; it can also be a combination of two or more rings, such as acridine. "N-containing fused heteroaromatic ring" refers to at least one heteroatom in the above "fused heteroaromatic ring" being N.

"(5-6-membered saturated heterocyclyl)-fused (5-6-membered aryl)" means a group formed by "5-6-membered saturated heterocycle" and "5-6-membered aromatic ring" sharing two adjacent carbons or heteroatoms.

"CDs" represents CH₃ substituted with three deuteriums.

"The substituent is an ester group" refers to the substitution of methylene CH₂ with =O to form C=O.

A "saturated heterocycle" refers to a saturated ring formed by substituting at least one carbon atom in a saturated carboncycle with O, N, and/or S. "Oxygen heterocycle" refers to a ring formed by substituting at least one carbon atom in a carboncycle with O.

Unless otherwise specified, the ring formed by connecting into a ring in the present invention includes both unsubstituted and substituted rings. For example, "R^{4a} and R^{4b} are linked to form a ring", "R^{2a} and R^{2b} are linked to form a ring".

Based on the experimental results, the present invention provides a compound that can effectively inhibit the activity of the main protease M^{pro} of novel coronavirus, and that can effectively block the replication and transcription of SARS-CoV-2 virus in patients, inhibit SARS-CoV-2 infection in cells, and provide strong support for fighting against SARS-CoV-2.

Meanwhile, the compound provided in the present invention also exhibits good *in vivo* safety and pharmacokinetic properties; it has low cardiac toxicity and is less likely to induce acute arrhythmia or even sudden death after administration.

The compound of the present invention can effectively inhibit the activity of SARS-CoV-2 M^{pro}, and has antiviral activity against SARS-CoV-2 wild-type virus strains (*in vitro*) and mutant virus strains (*in vivo* and *in vitro*)*.*

The compound of the present invention has very good application prospects in the manufacturer of SARS-CoV-2 M^{pro} inhibitors, anti-SARS-CoV-2 medicaments, and medicaments for preventing and/or treating COVID-19.

Obviously, based on the above content of the present invention, according to the common technical knowledge and the conventional means in the field, without department from the above basic technical spirits, other various modifications, alternations, or changes can further be made.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The inhibitory activity of compound **126** against SARS-CoV-2 M^{pro}.
Figure 2. The inhibitory activity of compound **275** against SARS-CoV-2 M^{pro}.
Figure 3. The inhibitory activity of compound **289** against SARS-CoV-2 M^{pro}.
Figure 4. The inhibitory activity of compound **296** against SARS-CoV-2 M^{pro}.
Figure 5. The inhibitory activity of compound **398** against SARS-CoV-2 M^{pro}.
Figure 6. Antiviral activities of some compounds according to the present invention at the cellular level.
Figure 7. Antiviral activities of compound **398** at the cellular level.
Figure 8. Viral load detection of compound **398** *in vivo* antiviral experiment.
Figure 9. Virus titer detection of compound **398** *in vivo* antiviral experiment.
Figure 10. Immunohistochemical staining and histopathological staining of compound **398** *in vivo* antiviral experiments.

### Examples

The starting materials and equipment used in the present invention are known products obtained by purchasing those commercially available.

The preparation and operating conditions for the ketoamide derivatives represented by formula I above include:

### Example 1 Preparation of compound 1

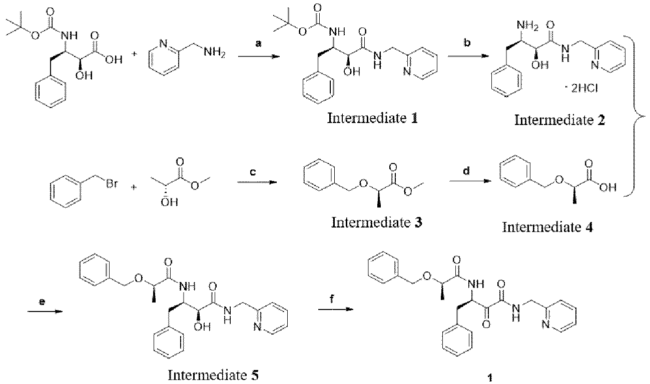

### Step a: Preparation of intermediate 1 (tert-butyl ((2R,3S)-hydroxyl-4-oxo-1-phenyl-4-(pyridin-2-ylmethyl)amino)butan-2-yl)carbamate)

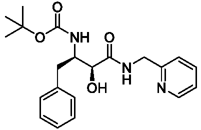

The starting material (2S,3R)-3-((tert-butoxycarbonyl)amino)-2-hydroxyl-4-phenylbutyric acid (590 mg, 2 mmol) was dissolved in dichloromethane, to which were successively added pyridine-2-ylmethylamine (217 mg, 2 mmol), N,N,N',N'-tetramethyl-O-(7-azabenzotriazol-1-yl)uronium hexafluorophosphate (1.14 g, 3 mmol), and *N,N-*diisopropylethylamine (769 mg, 6 mmol), and then the mixture was allowed to react overnight at room temperature. TLC indicated completion of the reaction. The reaction solution was respectively extracted with saturated ammonium chloride solution and saturated sodium bicarbonate solution. The combined organic phase was dried over anhydrous Na₂SO₄, and then filtered. The filtrate was concentrated. The residue was separated and purified by column chromatography to obtain the intermediate **1** (616 mg), as white solid, with a yield of 80%. MS (ESI) *m*/*z*: 386.2 [M+H]⁺.

### Step b: Preparation of intermediate 2 ((2S,3R)-3-amino-2-hydroxyl-4-phenyl-N-(pyridin-2-ylmethyl)butanamide hydrochloride)

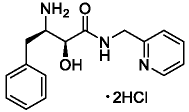

Intermediate **1** (384 mg, 1 mmol) was dissolved in dichloromethane, to which was added dioxane hydrochloride (1.25 mL, 5 mmol, 4 M) in an ice bath under nitrogen protection, and then the reaction solution was warmed to room temperature and stirred for 1-2 h. The reaction was completed by TLC detection. The reaction solution was concentrated to obtain intermediate **2** (358 mg), as white solid, which was directly used for the next reaction (step e).

### Step c: Preparation of intermediate 3 (methyl (R)-2-(benzyloxy)propionate)

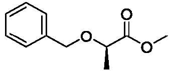

The starting material methyl (*R*)-2-hydroxylpropionate (105 mg, 1 mmol), AgO (290 mg, 1.25 mmol), and benzyl bromide (214 mg, 1.25 mmol) were dissolved in dichloromethane, and then stirred for 48 h at room temperature. After completion of the reaction by TLC detection, the reaction solution was filtered over diatomaceous earth, and then the filtrate was concentrated. The residue was separated and purified by column chromatography to obtain intermediate **3** (155 mg) as colorless oil, with a yield of 80%. MS (ESI) *m*/*z*: 195.1 [M+H]⁺.

### Step d: Preparation of intermediate 4 ((R)-2-(benzyloxy)propionic acid)

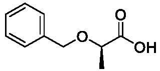

Intermediate **3** (155 mg, 0.8 mmol) was dissolved in 2 mL of ethanol and moved into an ice bath, to which was added the solution of lithium hydroxide (19 mg, 0.8 mmol) in water (2 mL) dropwise. The reaction was allowed to react for additional 30 min. TLC detection indicated completion of the reaction. The reaction solution was added into ice water, and then extracted with ethyl acetate. The pH of water phase was adjusted to be 2-3 with diluted hydrochloric acid (1M). The resultant solution was extracted with ethyl acetate. The organic phase was combined and concentrated to obtain intermediate **4** (100 mg) as colorless oil, which was directly used in the next reaction.

### Step e: Preparation of intermediate 5 ((2S,3R)-3-((R)-2-(benzyloxy)propionamido)-2-hydroxyl-4-phenyl-N-(pyridin-2-ylmethyl)butyramide)

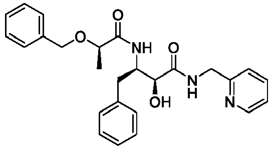

Intermediate **4** (90 mg, 0.5 mmol) was dissolved in dichloromethane, to which were successively added intermediate **2** (180 mg, 0.5 mmol), *N,N,N',N*'-tetramethyl-*O*-(7-azabenzotriazol-1-yl)uronium hexafluorophosphate (286 mg, 0.75 mmol), and *N,N-*diisopropylethylamine (258 mg, 2 mmol), and then the mixture was allowed to react overnight. TLC indicated completion of the reaction. The reaction solution was respectively extracted with saturated ammonium chloride solution and saturated sodium bicarbonate solution. The combined organic phase was dried over anhydrous Na₂SO₄, and then filtered. The filtrate was concentrated. The residue was separated and purified by column chromatography to obtain the intermediate **5** (180 mg) as yellow solid, with a yield of 80%. MS (ESI) *m*/*z*: 448.2 [M+H]⁺.

### Step f: Preparation of compound 1 ((R)-3-((R)-2-(benzyloxy)propionamido)-2-oxo-4-phenyl-N-(pyridin-2-ylmethyl)butyramide)

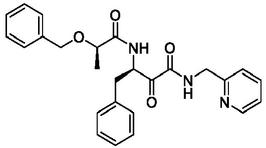

Intermediate 5 (180 mg, 0.4 mmol) was dissolved in 10 mL of dichloromethane, and then stirred in an ice bath, to which was added Dess-Martin periodinane (212 mg, 0.5 mmol) in portions, and then the reaction solution was warmed to room temperature. The reaction mixture was allowed to react under stirring for additional 2 h. After completion of the reaction, the reaction solution was diluted with dichloromethane (5 mL), and successively extracted with saturated sodium thiosulfate solution (5 mL) and saturated NaHCO₃ solution (5 mL). The combined organic phase was dried over anhydrous Na₂SO₄, and then filtered. The filtrate was concentrated. The residue was separated by column chromatography, to obtain product **1** (116 mg) as white solid, with a yield of 65%. ¹H NMR (400 MHz, DMSO-*d*₆) *δ* 9.28 (t, *J =* 6.2 Hz, 1H), 8.57-8.46 (m, 1H), 8.20 (d, *J =* 8.3 Hz, 1H), 7.82-7.67 (m, 1H), 7.42-7.15 (m, 13H), 5.34-5.23 (m, 1H), 4.44 (s, 2H), 4.42-4.35 (m, 1H), 4.31-4.23 (m, 1H), 3.87-3.80 (m, 1H), 3.25-3.18 (m, 1H), 2.94 (dd, *J* = 13.8, 9.3 Hz, 1H), 1.15 (d, *J* = 12.3, 6.7 Hz, 3H).

### Example 2 Preparation of compound 137

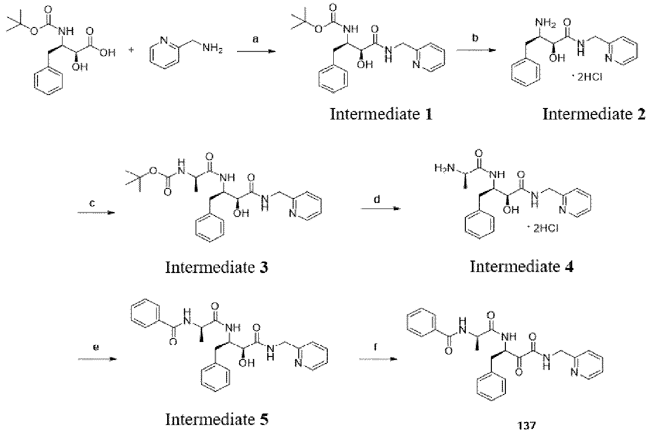

**Step a:** Same as step a in Example 1;
**Step b:** Same as step b in Example 1;

### Step c: Preparation of intermediate 3 (tert-butyl ((R)-1-(((2R,3S)-3-hydroxyl-4-oxo-1-phenyl-4-((pyridin-2-ylmethyl)amino)butan-2-yl)amino)-1-oxopropan-2-yl)carbamate)

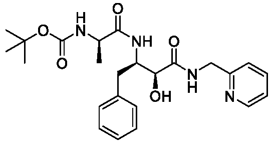

Intermediate **2** (358 mg, 1 mmol) was dissolved in dichloromethane, to which were successively added (tert-butoxycarbonyl)-D-alanine (189 mg, 1 mmol), *N,N,N',N'-*tetramethyl-*O*-(7-azabenzotriazol-1-yl)uronium hexafluorophosphate (570 mg, 1.5 mmol) and *N*,*N*-diisopropylethylamine (513 mg, 4 mmol), and then the mixture was allowed to react overnight at room temperature. TLC detection indicated completion of the reaction. The reaction solution was respectively extracted with saturated ammonium chloride solution and saturated NaHCO₃ solution, followed by extraction with saturated ammonium chloride solution and saturated NaHCO₃ solution, respectively. The combined organic phase was dried over anhydrous Na₂SO₄, and then filtered. The filtrate was concentrated. The residue was separated and purified by column chromatography, to obtain intermediate **3** (365 mg) as white solid, with a yield of 80%. MS (ESI) *m*/*z*: 457.2 [M+H]⁺.

### Step d: Preparation of intermediate 4((2S,3R)-3-((R)-2-aminopropionamido)-2-hydroxyl-4-phenyl-N-(pyridin-2-ylmethyl)butanamide hydrochloride)

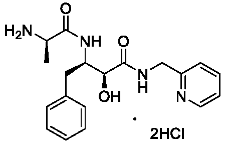

Intermediate **3** (229 mg, 0.5 mmol) was dissolved in dichloromethane, to which was added dioxane hydrochloride (0.625 mL, 2.5 mmol, 4 M) in an ice bath under nitrogen protection, and then the mixture was warmed to room temperature and stirred for 1-2 h. The reaction was completed by TLC detection. The reaction solution was concentrated to obtain intermediate **4** (214 mg) as white solid, which was directly used in the next reaction.

### Step e: Preparation of intermediate 5 (N-((R)-1-(((2R,3S)-3-hydroxyl-4-oxo-1-phenyl-4-((pyridin-2-ylmethyl)amino)butan-2-yl)amino)-1-oxopropan-2-yl)benzamide)

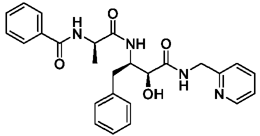

Intermediate **4** (214 mg, 0.5 mmol) was dissolved in dichloromethane, and moved into an ice bath, followed by stirring for 10 min, to which was added *N*,*N*-diisopropylethylamine (194 mg, 1.5 mmol), and then the mixture was stirred well. The solution of benzoyl chloride (71 mg, 0.5 mmol) in dichloromethane was added dropwise. The reaction mixture was allowed to react for additional 0.5-1 h. The reaction was completed by detection with TLC. To the reaction solution, was added ice water, and the resultant solution was extracted with dichloromethane. The organic phase was dried over anhydrous Na₂SO₄, and then filtered. The filtrate was concentrated. The residue was separated and purified by column chromatography, to obtain intermediate **5** (207 mg) as white solid, with a yield of 90%. MS (ESI) *m*/*z*: 461.2 [M+H]⁺.

### Step f: Preparation of product 137 (N-((R)-1-(((R)-3,4-dioxo-1-phenyl-4-((pyridin-2-ylmethyl)amino)butan-2-yl)amino)-1-oxopropan-2-yl)benzamide)

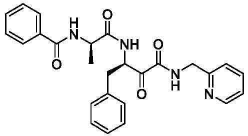

Intermediate **5** (207 mg, 0.45 mmol) was dissolved in 10 mL of dichloromethane, and then stirred in an ice bath, to which was added Dess-Martin periodinane (212 mg, 0.5 mmol) in portions, and then the reaction solution was warmed to room temperature. The reaction mixture was allowed to react under stirring for additional 2 h. After completion of the reaction, the reaction solution was diluted with dichloromethane (5 mL), and successively extracted with saturated sodium thiosulfate solution (5 mL) and saturated NaHCO₃ solution (5 mL). The combined organic phase was dried, and then filtered. The filtrate was concentrated under reduced pressure. The residue was separated by column chromatography, to obtain product **137** (134 mg) as white solid, with a yield of 65%. ¹H NMR (400 MHz, DMSO-*d*₆) *δ* 9.23 (t, *J* = 27.6, 6.1 Hz, 1H), 8.53-8.47 (m, 1H), 8.46-8.32 (m, 2H), 7.93-7.82 (m, 2H), 7.77-7.70 (m, 1H), 7.57-7.50 (m, 1H), 7.49-7.41 (m, 2H), 7.31-7.14 (m, 7H), 5.28-5.17 (m, 1H), 4.58-4.48 (m, 1H), 4.43 (s, 2H), 3.22-3.09 (m, 1H), 2.94-2.79 (m, 1H), 1.23 (s, 3H).

### Example 3 Preparation of compound 331

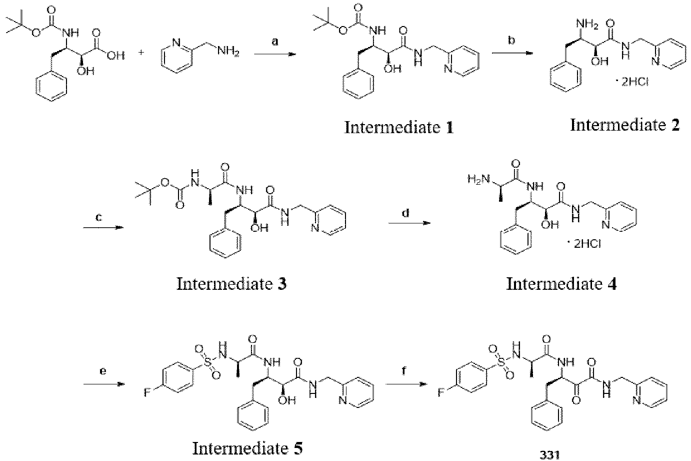

**Step a**: Same as step a in Example 2;
**Step b:** Same as step b in Example 2;
**Step c**: Same as step c in Example 2;
**Step d**: Same as step d in Example 2;

### Step e: Preparation of intermediate 5 ((2S,3R)-3-((R)-2-((4-fluorophenyl)sulfonamido) propionamido)-2-hydroxyl-4-phenyl-N-(pyridin-2-ylmethyl)butyramide)

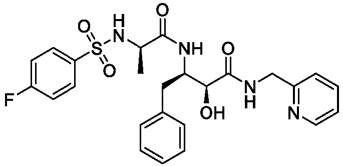

Intermediate **4** (180 mg, 0.5 mmol) was dissolved in 5 mL of dichloromethane, to which were added pyridine (118 mg, 1.5 mmol) and 4-fluorobenzenesulfonyl chloride (100 mg, 0.5 mmol), and then the mixture was allowed to react overnight. After completion of the reaction detected by TLC, the reaction solution was extracted with saturated ammonium chloride solution and saturated NaHCO₃ solution, respectively. The combined organic phase was dried over anhydrous Na₂SO₄, and then filtered. The filtrate was concentrated. The residue was separated and purified by column chromatography, to obtain intermediate **5** (174 mg) as yellow solid, with a yield of 68%. MS (ESI) *m*/*z*: 515.2 [M+H]⁺.

### Step f: Preparation of compound 331 ((R)-3-((R)-2-((4-fluorophenyl)sulfonamido) propionamido)-2-oxo-4-phenyl-N-(pyridin-2-ylmethyl)butyramide)

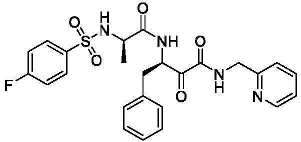

Intermediate **5** (154 mg, 0.3 mmol) was dissolved in dichloromethane, and then stirred in an ice bath, to which was added Dess-Martin periodinane (170 mg, 0.4 mmol) in portions, and then the reaction solution was warmed to room temperature. The reaction mixture was allowed to react under stirring for additional 2 h. After completion of the reaction, the reaction solution was diluted with dichloromethane, and successively extracted with saturated sodium thiosulfate solution and saturated NaHCO₃ solution. The combined organic phase was dried, and then filtered. The filtrate was concentrated under reduced pressure. The residue was separated by column chromatography, to obtain compound **331** (92 mg) as white solid, with a yield of 60%. ¹H NMR (400 MHz, DMSO-*d*₆) *δ* 9.34-9.22 (m, 1H), 8.54-8.47 (m, 1H), 8.45-8.34 (m, 2H), 8.08-7.99 (m, 1H), 7.83-7.73 (m, 2H), 7.72-7.62 (m, 2H), 7.29-7.16 (m, 7H), 5.20-4.92 (m, 1H), 4.44 (d, *J =* 18.7, 6.4 Hz, 2H), 3.94-3.84 (m, 1H), 3.16-3.05 (m, 1H), 2.82-2.65 (m, 1H), 0.97 (d, 3H).

### Example 4 Preparation of compound 296

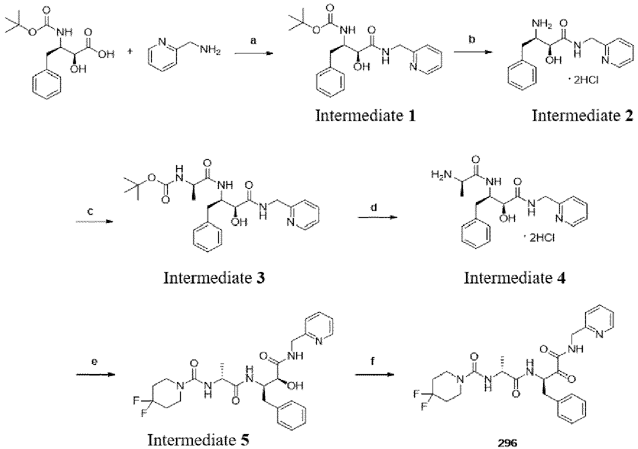

**Step a:** Same as step a in Example 2;
**Step b:** Same as step b in Example 2;
**Step c:** Same as step c in Example 2;
**Step d:** Same as step d in Example 2;

### Step e: Preparation of intermediate 5 (4,4-difluoro-N-((R)-1-(((2R,3S)-3-hydroxyl-4-oxo-1-phenyl-4-((pyridin-2-ylmethyl)amino)butan-2)-yl)amino)-1-oxopropan-2-yl)piperidin-1-formamide)

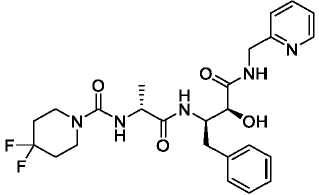

4,4-difluoropiperidine (50 mg, 0.42 mmol) was dissolved in tetrahydrofuran, to which was added triethylamine (163 µL, 1.27 mmol) at 0 °C, and then the solution of triphosgene (124 mg, 0.42 mmol) in tetrahydrofuran was added dripwise. The mixture was allowed to react for half an hour at 0 °C. The reaction solution was added into the solution of intermediate **4** (180 mg, 0.42 mmol) and triethylamine (163 µL, 1.27 mmol) in tetrahydrofuran dropwise in an ice bath. The mixture was allowed to react overnight at room temperature. The reaction was completed by TLC. The reaction solution was concentrated, and then extracted with ethyl acetate and water. The organic phase was combined, dried over anhydrous Na₂SO₄, and then filtered. The filtrate was concentrated. The residue was separated and purified by column chromatography, to obtain intermediate **5** (70 mg) as yellow solid, with a yield of 33%. MS (ESI) *m*/*z*: 504.2 [M+H]⁺.

### Step f: Preparation of product 296 (N-((R)-1-(((R)-3,4-dioxo-1-phenyl-4-((pyridin-2-ylmethyl)amino)butan-2-yl)amino)-1-oxopropan-2-yl)-4,4-difluoropiperidin-1-formamide)

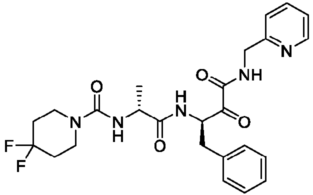

Intermediate **5** (70 mg, 0.14 mmol) was dissolved in 10 mL of dichloromethane, and then stirred in an ice bath, to which was added Dess-Martin periodinane (61 mg, 0.14 mmol) in portions, and then the reaction solution was warmed to room temperature. The reaction mixture was allowed to react under stirring for additional 2 h. After completion of the reaction by TLC, the reaction solution was diluted with dichloromethane (5 mL), and successively extracted with saturated sodium thiosulfate solution (5 mL) and saturated NaHCO₃ solution (5 mL). The combined organic phase was dried over anhydrous Na₂SO₄, and then filtered. The filtrate was concentrated. The residue was separated by column chromatography, to obtain product **58** (36 mg) as white solid, with a yield of 50%. ¹H NMR (400 MHz, DMSO-*d*₆) *δ* 9.30-9.16 (m, 1H), 8.55-8.49 (m, 1H), 8.50-8.43 (m, 1H), 7.79-7.73 (m, 1H), 7.30-7.17 (m, 7H), 6.76-6.66 (m, 1H), 4.46-4.42 (m, 1H), 4.40-4.35 (m, 1H), 4.22-4.15 (m, 1H), 3.94-3.87 (m, 1H), 3.43-3.41 (m, 4H), 3.16-3.09 (m, 1H), 2.94-2.83 (m, 1H), 1.92-1.83 (m, 4H), 1.17 (d, *J* = 7.2 Hz, 1.5H), 1.04 (d, *J =* 7.2 Hz, 1.5H).

### Example 5 Preparation of compound 313

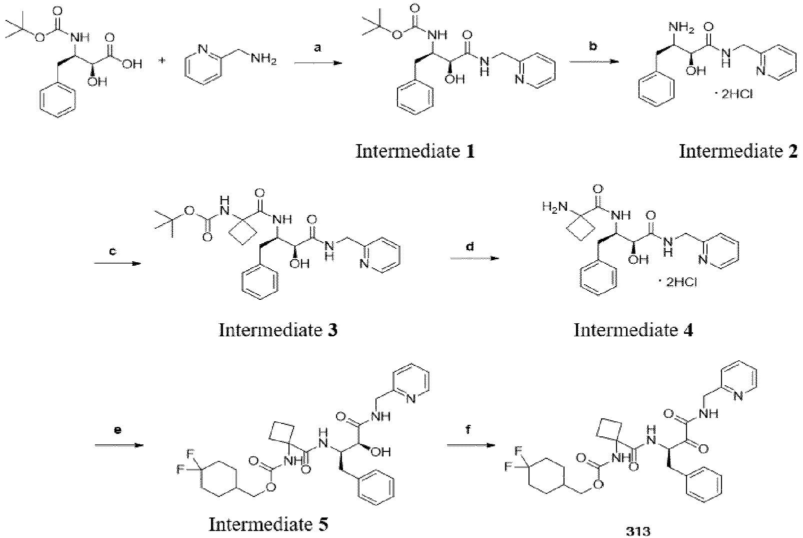

**Step a:** Same as step a in Example 2;
**Step b**: Same as step b in Example 2;

### Step c: Preparation of intermediate 3 (tert-butyl (1-(((2R,3S)-3-hydroxyl-4-oxo-1-phenyl-4-((pyridin-2-ylmethyl)amino)butan-2-yl)carbamoyl)cyclobutyl)carbamate)

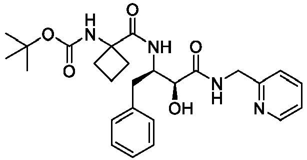

Intermediate 2 (358 mg, 1 mmol) was dissolved in dichloromethane, to which were successively added (tert-butoxycarbonyl)-1-aminocyclobutanecarboxylic acid (215 mg, 1 mmol), *N*,*N*,*N'*,*N'*-tetramethyl-*O*-(7-azabenzotriazol-1-yl)uronium hexafluorophosphate (570 mg, 1.5 mmol) and *N*,*N*-diisopropylethylamine (513 mg, 4 mmol), and then the mixture was allowed to react overnight at room temperature. TLC detection indicated completion of the reaction. The reaction solution was respectively extracted with saturated ammonium chloride solution and saturated NaHCO₃ solution, followed by extraction with saturated ammonium chloride solution and saturated NaHCO₃ solution, respectively. The combined organic phase was dried over anhydrous Na₂SO₄, and then filtered. The filtrate was concentrated. The residue was separated and purified by column chromatography, to obtain intermediate **3** (385 mg), as white solid, with a yield of 80%. MS (ESI) *m*/*z*: 483.3 [M+H]⁺.

### Step d: Preparation of intermediate 4 (1-amino-N-((2R,3S)-3-hydroxyl-4-oxo-1-phenyl-4-((pyridin-2-ylmethyl)amino)butan-2-yl)cyclobutane-1-formamide) hydrochloride

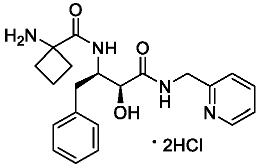

Intermediate **3** (385 mg, 0.8 mmol) was dissolved in dichloromethane, to which was added dioxane hydrochloride (1 mL, 4 mmol, 4 M) in an ice bath under nitrogen protection, and then the mixture was warmed to room temperature and stirred for 1-2 h. The reaction was completed by TLC detection. The reaction solution was concentrated to obtain intermediate **4** (360 mg), as white solid, which was directly used in the next reaction.

### Step e: Preparation of intermediate 5 ((4,4-difluorocyclohexyl)methyl(1-(((2R,3S)-3-hydroxyl-4-oxo-1-phenyl-4-((pyridin-2-ylmethyl)amino)butan-2-yl)carbamoyl)) cyclobutyl)carbamate)

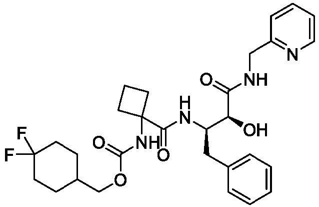

(4,4-difluorocyclohexyl)methanol (63 mg, 0.42 mmol) was dissolved in tetrahydrofuran, to which was added triethylamine (163 µL, 1.27 mmol) at 0 °C, and then the solution of triphosgene (124 mg, 0.42 mmol) in tetrahydrofuran was added dripwise. The mixture was allowed to react for 30 min at 0 °C. The reaction solution was added into the solution of intermediate **4** (180 mg, 0.42 mmol) and triethylamine (163 µL, 1.27 mmol) in tetrahydrofuran dropwise in an ice bath. The mixture was allowed to react overnight at room temperature. The reaction was completed by TLC. The reaction solution was concentrated, and then extracted with ethyl acetate and water. The organic phase was combined, dried over anhydrous Na₂SO₄, and then filtered. The filtrate was concentrated. The residue was separated and purified by column chromatography, to obtain intermediate **5** (70 mg) as yellow solid, with a yield of 30%. MS (ESI) *m*/*z*: 559.3 [M+H]⁺.

### Step f: Preparation of compound 313 ((4,4-difluorocyclohexyl)methyl(R)-(1-((3,4-dioxo-1-phenyl-4-((pyridin-2-ylmethyl)amino)butan-2-yl)carbamoyl)cyclobutyl) carbamate)

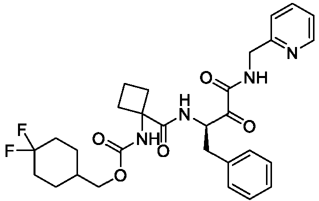

Intermediate **5** (70 mg, 0.13 mmol) was dissolved in 10 mL of dichloromethane, and then stirred in an ice bath, to which was added Dess-Martin periodinane (84 mg, 0.2 mmol), and then the reaction solution was warmed to room temperature. The reaction mixture was allowed to react under stirring for additional 2 h. After completion of the reaction by TLC, the reaction solution was diluted with dichloromethane (5 mL), and successively extracted with saturated sodium thiosulfate solution (5 mL) and saturated NaHCO₃ solution (5 mL). The combined organic phase was dried over anhydrous Na₂SO₄, and then filtered. The filtrate was concentrated. The residue was separated by column chromatography, to obtain compound **313** (38 mg) as white solid, with a yield of 55%. ¹H NMR (400 MHz, DMSO-*d*₆) *δ* 9.29-9.18 (m, 1H), 8.51 (d, 1H), 7.82-7.73 (m, 1H), 7.75-7.61 (m, 1H), 7.42-7.11 (m, 8H), 5.27-5.10 (m, 1H), 4.46 (d, *J =* 6.2 Hz, 2H), 3.87-3.74 (m, 1H), 3.19-3.10 (m, 1H), 3.02-2.85 (m, 1H), 2.43-2.29 (m, 2H), 2.08-1.96 (m, 3H), 1.86-1.66 (m, 5H), 1.28-1.20 (m, 2H).

### Example 6 Preparation of compound 52

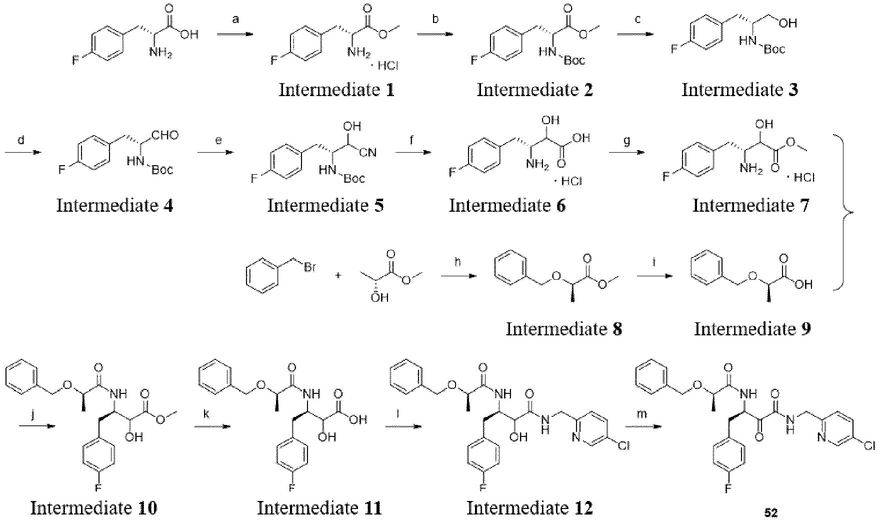

### Step a: Preparation of intermediate 1 (methyl (R)-2-amino-3-(4-fluorophenyl)propionate hydrochloride)

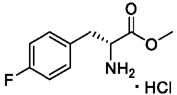

In a round-bottom flask, 4-fluoro-D-phenylalanine (1.83 g, 10 mmol) was added, and then dissolved in methanol. The reaction system was placed in an ice bath and stirred. Subsequently, to the reaction system, was slowly added 10 mL of thionyl chloride dropwise. After that, the reaction solution was heated to 65 °C and reacted overnight. The next day, the reaction system was cooled to room termperature, and evaporated under reduced pressure to remove the solvent and obtain white solid, which was directly used in the next reaction without further purification.

### Step b: Preparation of intermediate 2 (methyl (R)-2-((tert-butoxycarbonyl)amino)-3-(4-fluorophenyl)propionate)

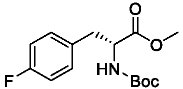

In a flask, intermediate **1** (2.34 g, 10 mmol) was added and dissolved in water, to which was added K₂CO₃ (4.14 g, 30 mmol). Subsequently, di-tert-butyl dicarbonate (2.85 g, 13 mmol) was dissolved in 20 mL of THF, and slowly added to the reaction system dropwise. After that, the reaction mixture was allowed to react for 2 h at room temperature. After completion of the reaction, the reaction system was diluted by adding 20 mL of water, and then extracted twice with ethyl acetate. The combined organic phase was dried over anhydrous Na₂SO₄, and filtered. The reaction solution was evaporated under reduced pressure to remove the solvent and obtain colorless oily liquid, which was directly used in the next reaction.

### Step c: Preparation of intermediate 3 (tert-butyl (R)-(1-(4-fluorophenyl)-3-hydroxylpropan-2-yl)carbamate)

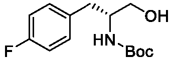

In a flask, intermediate **2** (2.97 g, 10 mmol) was added and dissolved in methanol, and then the reaction system was placed in an ice bath and stirred. Subsequently, to the reaction system, was slowly added sodium borohydride (1.89 g, 50 mmol). After that, the reaction solution was warmed to room temperature and allowed to react overnight. The next day, water was added to the reaction system to quench the reaction. The reaction solution was concentrated, and to the residue, was added water for dilution. The resultant solution was extracted twice with ethyl acetate. The combined organic phase was dried over anhydrous Na₂SO₄, and filtered. The filtrate was concentrated. The residue was separated and purified by column chromatography, to obtain intermediate **3** (1.25 g) as white solid, with a yield of 47%. MS (ESI) *m*/*z:* 270.2 [M+H]⁺.

### Step d: Preparation of intermediate 4 (tert-butyl (R)-(1-(4-fluorophenyl)-3-oxopropan-2-yl)carbamate)

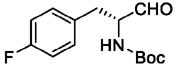

Intermediate **3** (2.69 g, 10 mmol) was dissolved in 100 mL of dichloromethane, and then stirred in an ice bath, to which was added Dess-Martin periodinane (5.08 g, 12 mmol) in portions, and then the reaction solution was warmed to room temperature. The reaction mixture was allowed to react under stirring for additional 2 h. After completion of the reaction, the reaction solution was diluted with dichloromethane, and successively extracted with saturated sodium thiosulfate solution and saturated NaHCO₃ solution. The organic phase was collected, dried, and then filtered. The filtrate was concentrated under reduced pressure. The residue was separated by column chromatography, to obtain intermediate **4** (1.87g) as white solid, with a yield of 70%. MS (ESI) *m*/*z:* 268.2 [M+H]⁺.

### Step e: Preparation of intermediate 5 (tert-butyl ((2R)-1-cyano-3-(4-fluorophenyl)-1-hydroxylpropan-2-yl)carbamate)

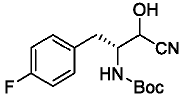

To a flask, were added intermediate **4** (2 g, 7.48 mmol) and cesium fluoride (568 mg, 3.74 mmol), and then 50 mL of methanol was added to dissolve. The reaction system was placed in an ice bath under stirring. Subsequently, to the reaction system, was slowly added trimethylsilyl cyanide (890 mg, 8.98 mmol). After that, the reaction system was stirred for 5 h at room temperature. After completion of the reaction by TLC, the reaction solution was concentrated, diluted with water, and then extracted with ethyl acetate. The combined organic phase was dried over anhydrous Na₂SO₄, and then filtered. The filtrate was concentrated to obtain intermediate **5** as yellow oil, which was directly used in the next reaction.

### Step f: Preparation of intermediate 6 ((3R)-3-amino-4-(4-fluorophenyl)-2-hydroxylbutanoic acid hydrochloride)

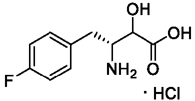

To a flask, was added intermediate **5** (2.21 g, 7.5 mmol), and then 12 mL of dioxane was added to dissolve, followed by addition of hydrochloric acid (6 N, 27 mL). The reaction system was heated to 100 °C and stirred for 12 h. After completion of the reaction by TLC, the system was cooled to room temperature, and then the reaction solution was concentrated to provide intermediate **6** as brown solid, which was directly used in the next reaction.

### Step g: Preparation of intermediate 7 (methyl (3R)-3-amino-4-(4-fluorophenyl)-2-hydroxylbutyrate hydrochloride)

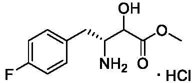

In a flask, intermediate **6** (1.8 g, 7.5 mmol) was added and dissolved in methanol, to which was added 5 mL of thionyl chloride dropwise under stirring in an ice bath. After addition, the ice bath was removed, and the reaction solution was heated under refluxing and reacted overnight. After completion of the reaction by TLC, the reaction solution was concentrated, to obtain intermediate **7,** as brown solid, which was directly used in the next reaction (step j).
**Step h:** Same as step c of Example 1;
**Step i:** Same as step d of Example 1;

### Step j: Preparation of intermediate 10 (methyl (3R)-3-((R)-2-(benzyloxy)propionamido)-4-(4-fluorophenyl)-2-hydroxylbutyrate)

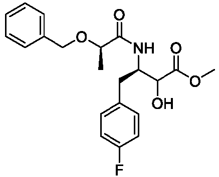

Intermediate **9** (180 mg, 1 mmol) was dissolved in dichloromethane, to which were successively added intermediate **7** (263 mg, 1 mmol), *N*,*N*,*N'*,*N'*-tetramethyl-*O*-(7-azabenzotriazol-1-yl)uronium hexafluorophosphate (456 mg, 1.2 mmol) and *N,N-*diisopropylethylamine (387 mg, 3 mmol), and then the mixture was allowed to react overnight at room temperature. After completion of the reaction by TLC, the reaction solution was respectively extracted with saturated ammonium chloride solution and saturated NaHCO₃ solution. The combined organic phase was dried over anhydrous Na₂SO₄, and then filtered. The filtrate was concentrated. The residue was separated and purified by column chromatography, to obtain intermediate **10** (316 mg), as yellow solid, with a yield of 81%. MS (ESI) *m*/*z:* 390.2 [M+H]⁺.

### Step k: Preparation of intermediate 11 ((3R)-3-((R)-2-(benzyloxy)propionamido)-4-(4-fluorophenyl)-2-hydroxylbutyric acid)

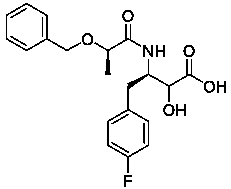

Intermediate **10** (310 mg, 0.8 mmol) was dissolved in 5 mL of ethanol, to which was added lithium hydroxide (168 mg, 4 mmol) in portions under stirring in an ice bath, and then, the reaction was allowed to react for additional 2 h. TLC detection indicated completion of the reaction. The reaction solution was added with ice water, and then extracted with ethyl acetate. The pH of water phase was adjusted to be 2-3 with diluted hydrochloric acid (1M). The resultant solution was extracted with ethyl acetate. The organic phase was combined and concentrated to obtain intermediate **4** as yellow solid, which was directly used in the next reaction.

### Step 1: Preparation of intermediate 12 ((3R)-3-((R)-2-(benzyloxy)propionamido)-N-((5-chloropyridin-2-yl)methyl)-4-(4-fluorophenyl)-2-hydroxylbutyramide)

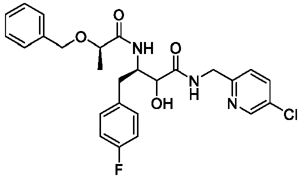

Intermediate **11** (300 mg, 0.8 mmol) was dissolved in dichloromethane, to which were successively added 5-chloro-pyridin-2-methylamine (114 mg, 0.8 mmol), *N,N,N',N'-*tetramethyl-*O*-(7-azabenzotriazol-1-yl)uronium hexafluorophosphate (456 mg, 1.2 mmol) and *N*,*N*-diisopropylethylamine (387 mg, 3 mmol), and then the mixture was allowed to react overnight. After completion of the reaction by TLC, the reaction solution was respectively extracted with saturated ammonium chloride solution and saturated NaHCO₃ solution. The combined organic phase was dried over anhydrous Na₂SO₄, and then filtered. The filtrate was concentrated. The residue was separated and purified by column chromatography, to obtain intermediate **12** (286 mg) as yellow solid, with a yield of 72%. MS (ESI) *m*/*z:* 500.2 [M+H]⁺.

### Step m: Preparation of compound 52 ((R)-3-((R)-2-(benzyloxy)propionamido)-N-((5-chloropyridin-2-yl)methyl)-4-(4-fluorophenyl)-2-oxobutyramide)

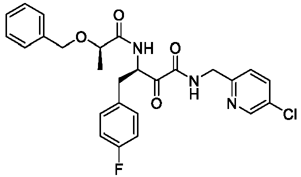

Intermediate **12** (250 mg, 0.5 mmol) was dissolved in 10 mL of dichloromethane, and then stirred in an ice bath, to which was added Dess-Martin periodinane (297 mg, 0.7 mmol), and then the reaction solution was warmed to room temperature. The reaction mixture was allowed to react under stirring for additional 2 h. After completion of the reaction, the reaction solution was diluted with dichloromethane, and successively extracted with saturated sodium thiosulfate solution and saturated NaHCO₃ solution. The combined organic phase was dried over anhydrous Na₂SO₄, and then filtered. The filtrate was concentrated. The residue was separated by column chromatography, to obtain product **52** (120 mg), as white solid, with a yield of 50%. ¹H NMR (400 MHz, DMSO-*d*₆) *δ* 9.39-9.28 (m, 1H), 8.55-8.52 (m, 1H), 8.32-8.23 (m, 1H), 7.91-7.82 (m, 1H), 7.33-7.21 (m, 5H), 7.18-7.04 (m, 4H), 5.27-5.15 (m, 1H), 4.48-4.40 (m, 2H), 4.35-4.21 (m, 3H), 3.86-3.79 (m, 2H), 3.21-3.16 (m, 1H), 2.96-2.86 (m, 1H), 1.19-1.10 (m, 3H).

### Example 7 Preparation of compound 324

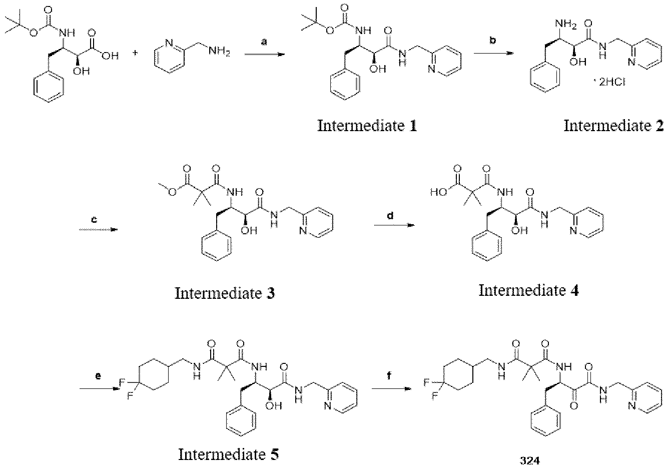

**Step a:** Same as step a in Example 2;
**Step b:** Same as step b in Example 2;

### Step c: Preparation of intermediate 3 (methyl 3-(((2R,3S)-3-hydroxyl-4-oxo-1-phenyl-4-((pyridin-2-ylmethyl)amino)butan-2-yl)amino)-2,2-dimethyl-3-oxopropionate)

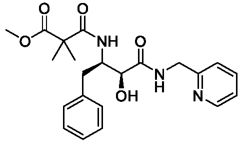

Monomethyl 2,2-dimethylmalonate (146 mg, 1 mmol) was dissolved in dichloromethane, to which were successively added intermediate **2** (356 mg, 1 mmol), *N*,*N*,*N'*,*N'*-tetramethyl-*O*-(7-azabenzotriazol-1-yl)uronium hexafluorophosphate (456 mg, 1.2 mmol), and *N*,*N*-diisopropylethylamine (516 mg, 4 mmol), and then the mixture was allowed to react overnight. After completion of the reaction by TLC, the reaction solution was respectively extracted with saturated ammonium chloride solution and saturated NaHCO₃ solution. The combined organic phase was dried over anhydrous Na₂SO₄, and then filtered. The filtrate was concentrated. The residue was separated and purified by column chromatography, to obtain intermediate **3** (310 mg) as yellow solid, with a yield of 75%. MS (ESI) *m*/*z:* 414.3 [M+H]⁺.

### Step d: Preparation of intermediate 4 (3-((((2R,3S)-3-hydroxyl-4-oxo-1-phenyl-4-((pyridin-2-ylmethyl)amino)butan-2-yl)amino)-2,2-dimethyl-3-oxopropionic acid)

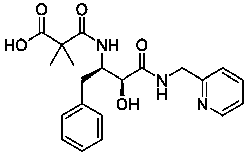

Intermediate **3** (310 mg, 0.75 mmol) was dissolved in 5 mL of ethanol, to which was added lithium hydroxide (147 mg, 3.5 mmol) in portions under stirring in an ice bath, and then, the reaction was allowed to react at room temperature for additional 2 h. TLC detection indicated completion of the reaction. The reaction solution was added with ice water, and then extracted with ethyl acetate. The pH of water phase was adjusted to be 3-4 with diluted hydrochloric acid (1M). The resultant solution was extracted with ethyl acetate. The organic phase was combined and concentrated to obtain intermediate **4** as yellow solid, which was directly used in the next reaction.

### Step e: Preparation of intermediate 5 (N¹-((4,4-difluorocyclohexyl)methyl)-N³-((2R,3S)-3-hydroxyl-4-oxo-1-phenyl-4-((pyridin-2-ylmethyl)amino)butan-2-yl)-2,2-dimethylmalonamide)

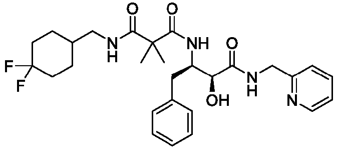

Intermediate **4** (300 mg, 0.75 mmol) was dissolved in dichloromethane, to which were successively added 4,4-difluorocyclohexylmethylamine hydrochloride (139 mg, 0.75 mmol), *N*,*N*,*N'*,*N'*-tetramethyl-*O*-(7-azabenzotriazol-1-yl)uronium hexafluorophosphate (342 mg, 0.9 mmol) and *N*,*N*-diisopropylethylamine (349 mg, 2.7 mmol), and then the mixture was allowed to react overnight. After completion of the reaction by TLC, the reaction solution was respectively extracted with saturated ammonium chloride solution and saturated NaHCO₃ solution. The combined organic phase was dried over anhydrous Na₂SO₄, and then filtered. The filtrate was concentrated. The residue was separated and purified by column chromatography, to obtain intermediate **5** (259 mg) as yellow solid, with a yield of 65%. MS (ESI) *m*/*z:* 531.3 [M+H]⁺.

### Step f: Preparation of compound 324 ((R)-N¹-((4,4-difluorocyclohexyl)methyl)-N³-(3,4-dioxo-1-phenyl-4-((pyridin-2-ylmethyl)amino)butan-2-yl)-2,2-dimethylmalonamide)

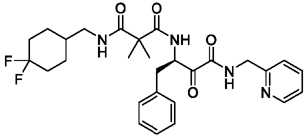

Intermediate **5** (212 mg, 0.4 mmol) was dissolved in 10 mL of dichloromethane, and then stirred in an ice bath, to which was added Dess-Martin periodinane (297 mg, 0.7 mmol) in portions, and then the reaction solution was warmed to room temperature. The reaction mixture was allowed to react under stirring for additional 2 h. After completion of the reaction, the reaction solution was diluted with dichloromethane, and successively extracted with saturated sodium thiosulfate solution and saturated NaHCO₃ solution. The organic phase was collected, dried over anhydrous Na₂SO₄, and then filtered. The filtrate was concentrated under reduced pressure. The residue was separated by column chromatography, to obtain product **324** (109 mg) as white solid, with a yield of 52%. ¹H NMR (400 MHz, Chloroform-*d*) *δ* 8.62-8.55 (m, 1H), 8.12-8.03 (m, 1H), 7.75-7.66 (m, 1H), 7.31-7.26 (m, 3H), 7.26-7.22 (m, 2H), 7.14-7.08 (m, 2H), 6.86-6.76 (m, 2H), 5.57-5.49 (m, 1H), 4.63 (d, *J =* 5.4 Hz, 2H), 3.41-3.33 (m, 1H), 3.20-3.11 (m, 1H), 3.09-2.98 (m, 2H), 2.07-2.04 (m, 1H), 1.78-1.72 (m, 2H), 1.67-1.49 (m, 4H), 1.40-1.37 (m, 3H), 1.35 (s, 3H), 1.26 (s, 2H).

### Example 8 Preparation of compound 326

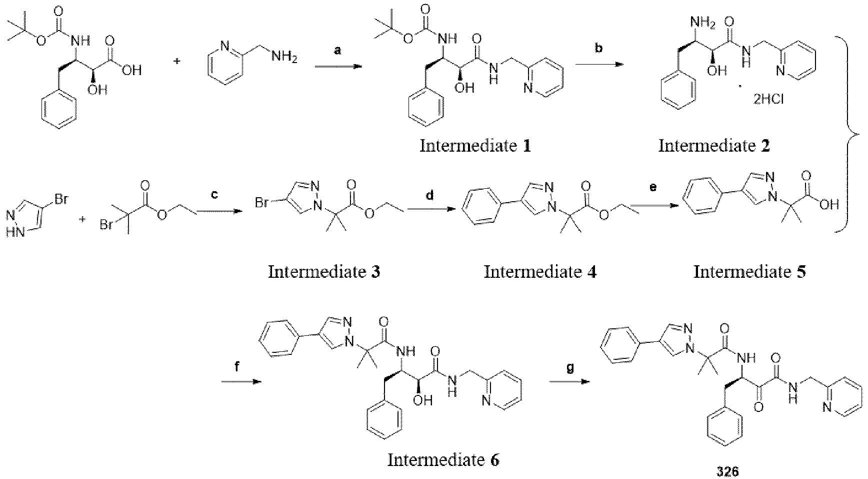

**Step a:** Same as step a in Example 2;
**Step b:** Same as step b in Example 2;

### Step c: Preparation of intermediate 3 (ethyl 2-(4-bromo-1H-pyrazol-1-yl)-2-methylpropionate)

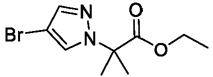

The starting material 4-bromo-1*H*-pyrazole (300 mg, 2.04 mmol) was dissolved in anhydrous DMF, and then placed in an ice bath, to which was added NaH (98 mg, 2.45 mmol) in portions, and then the solution was stirred for 30 min at room temperature. In an ice bath, ethyl 2-bromo-2-methylpropionate (478 mg, 2.45 mmol) was slowly added to the mixed solution dropwise, and then the reaction mixture was allowed to react overnight at room temperature. After the reaction was completed by TLC detection, the reaction was quenched with methanol. The reaction solution was concentrated. The residue was separated and purified by column chromatography, to obtain intermediate **3** (320 mg), as yellow solid, with a yield of 60%. MS (ESI) *m*/*z:* 261.0 [M+H]⁺.

### Step d: Preparation of intermediate 4 (ethyl 2-methyl-2-(4-phenyl-1H-pyrazol-1-yl)propionate)

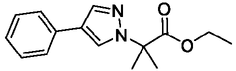

Intermediate **3** (320 mg, 1.23 mmol), phenylboronic acid (165 mg, 1.35 mmol), [1,1-bis(diphenylphosphino)ferrocene]dichloropalladium (90 mg, 0.123 mmol) and K₂CO₃ (339 mg, 2.45 mmol) were dissolved in dioxane/water (20 mL:1 mL), and then the mixture was allowed to react for 12 h at 95 °C under nitrogen protection. After completion of the reaction detected by TLC, the reaction solution was filtered over diatomaceous earth, followed by separation and purification over column chromatography, to obtain intermediate **4** (220 mg), with a yield of 70%. MS (ESI) *m*/*z:* 259.3 [M+H]⁺.

### Step e: Preparation of intermediate 5 (2-methyl-2-(4-phenyl-1H-pyrazol-1-yl)propionic acid)

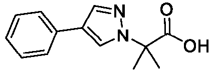

Intermediate **4** (220 mg, 0.86 mmol) was dissolved in 10 mL of ethanol, and then placed in an ice bath, to which was added the solution of lithium hydroxide (41 mg, 1.7 mmol) in water (2 mL) in an ice bath, and then the mixture was allowed to react for additional 30 min. TLC detection indicated completion of the reaction. The reaction solution was added with ice water, and then extracted with ethyl acetate. The pH of water phase was adjusted to be 2-3 with diluted hydrochloric acid (1M). The resultant solution was extracted with ethyl acetate. The organic phase was concentrated to obtain intermediate **5,** which was directly used in the next reaction.

### Step f: Preparation of intermediate 6 ((2S,3R)-2-hydroxyl-3-(2-methyl-2-(4-phenyl-1H-pyrazol-1-yl)propionamido)-4-phenyl-N-(pyridin-2-ylmethyl)butyramide)

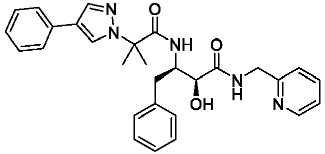

Intermediate **5** (198 mg, 0.86 mmol) was dissolved in dichloromethane, to which were successively added intermediate **2** (307 mg, 0.86 mmol), *N,N,N',N'*-tetramethyl-O-(7-azabenzotriazol-1-yl)uronium hexafluorophosphate (380 mg, 1.0 mmol) and *N,N-*diisopropylethylamine (444 mg, 3.44 mmol), and then the mixture was allowed to react overnight at room temperature. After completion of the reaction by TLC, the reaction solution was respectively extracted with saturated ammonium chloride solution and saturated NaHCO₃ solution. The combined organic phase was dried over anhydrous Na₂SO₄, and then filtered. The filtrate was concentrated. The residue was separated and purified by column chromatography, to obtain intermediate **6** (248 mg) as yellow solid, with a yield of 60%. MS (ESI) *m*/*z:* 484.2 [M+H]⁺.

### Step g: Preparation of compound 326 ((R)-3-(2-methyl-2-(4-phenyl-1H-pyrazol-1-yl)propionamido)-2-oxo-4-phenyl-N-(pyridin-2-ylmethyl)butyramide)

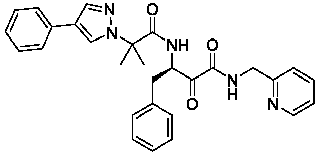

Intermediate **6** (242 mg, 0.5 mmol) was dissolved in 10 mL of dichloromethane, and then stirred in an ice bath, to which was added Dess-Martin periodinane (297 mg, 0.7 mmol) in portions, and then the reaction solution was warmed to room temperature. The reaction mixture was allowed to react under stirring for additional 2 h. After completion of the reaction, the reaction solution was diluted with dichloromethane, and successively extracted with saturated sodium thiosulfate solution and saturated NaHCO₃ solution. The organic phase was collected, dried over anhydrous Na₂SO₄, and then filtered. The filtrate was concentrated under reduced pressure. The residue was separated by column chromatography, to obtain product **326** (132 mg) as white solid, with a yield of 56%. ¹H NMR (400 MHz, DMSO-*d*₆) *δ* 9.23 (t, *J* = 6.3 Hz, 1H), 8.50 (d, *J* = 4.9 Hz, 1H), 8.26 (s, 1H), 7.97 (s, 1H), 7.76 (t, *J* = 7.9 Hz, 1H), 7.69-7.53 (m, 3H), 7.37 (t, *J* = 7.6 Hz, 2H), 7.32-7.04 (m, 8H), 5.23-5.10 (m, 1H), 4.51-4.37 (m, 2H), 3.18-3.06 (m, 1H), 2.98-2.85 (m, 1H), 1.70 (s, 6H).

### Example 9 Preparation of compound 58

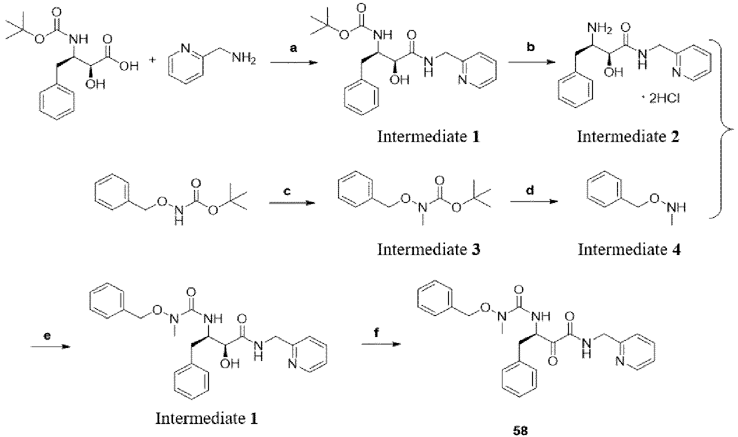

**Step a:** Same as step a in Example 2;
**Step b:** Same as step b in Example 2;

### Step c: Preparation of intermediate 3 (tert-butyl (benzyloxy)(methyl)carbamate)

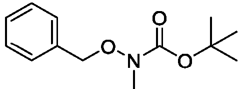

Th starting material tert-butyl N-(benzyloxy)carbamate (1000 mg, 4.48 mmol) was dissolved in DMF, to which was added NaH (269 mg, 6.73 mmol) in an ice bath, and after stirring for 30 min, CHsI ( mg, 5.38 mmol) was added. After completion of the reaction detected by TLC, the reaction solution was filtered over diatomaceous earth, and then the filtrate was concentrated. The residue was separated and purified by column chromatography, to obtain intermediate **3** (752mg), with a yield of 80%. MS (ESI) *m*/*z:* 238.1 [M+H]⁺.

### Step d: Preparation of intermediate 4 (O-benzyl-N-methylhydroxylamine)

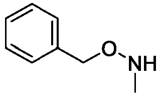

Intermediate 3 (752 mg, 3.17 mmol) was dissolved in 10 mL of dichloromethane, to which was added 10 mL (4 M) dioxane hydrochloride, and then the mixture was allowed to react for additional 30 min. After the reaction was completed by TLC, the reaction solution was rotatory evaporated to dry, which was directly used in the next reaction.

### Step e: Preparation of intermediate 5 ((2S,3R)-3-(3-(benzyloxy)-3-methylureido)-2-hydroxyl-4-phenyl-N-(pyridin-2-ylmethyl)butyramide)

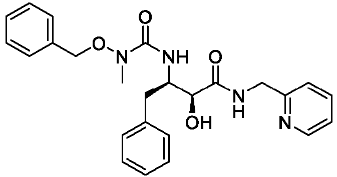

Intermediate **4** (100 mg, 0.42 mmol) was dissolved in tetrahydrofuran, to which was added triethylamine (163 µL, 1.27 mmol) at 0 °C, and then the solution of triphosgene (124 mg, 0.42 mmol) in tetrahydrofuran was added dropwise. The mixture was allowed to react for half an hour at 0 °C. The reaction solution was added into the solution of intermediate **2** (119.7 mg, 0.42 mmol) and triethylamine (163 µL, 1.27 mmol) in tetrahydrofuran dropwise at 0 °C. The mixture was allowed to react overnight at room temperature. The reaction was completed by TLC. After concentration, the reaction solution was extracted with ethyl acetate and water. The organic phase was combined, dried over anhydrous Na₂SO₄, and then filtered. The filtrate was concentrated. The residue was separated and purified by column chromatography, to obtain intermediate **5** (60 mg) as yellow solid, with a yield of 32%. MS (ESI) *m*/*z:* 449.2 [M+H]⁺.

### Step f: Preparation of product 58 ((R)-3-(3-(benzyloxy)-3-methylureido)-2-oxo-4-phenyl-N-(pyridin-2-ylmethyl)butyramide)

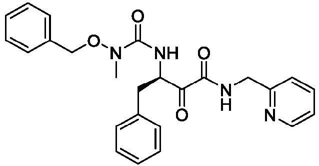

Intermediate **5** (60 mg, 0.13 mmol) was dissolved in 10 mL of dichloromethane, and then stirred in an ice bath, to which was added Dess-Martin periodinane (56 mg, 0.13 mmol), and then the reaction solution was warmed to room temperature. The reaction mixture was allowed to react under stirring for additional 2 h. After completion of the reaction, the reaction solution was diluted with dichloromethane (5 mL), and successively extracted with saturated sodium thiosulfate solution (5 mL) and saturated NaHCO₃ solution (5 mL). The organic phase was combined, dried over anhydrous Na₂SO₄, and then filtered. The filtrate was concentrated under reduced pressure. The residue was separated by column chromatography, to obtain product **58** (26 mg) as white solid, with a yield of 45%. MS (ESI) *m*/*z:* 447.2028 [M+H]⁺. ¹H NMR (400 MHz, DMSO) *δ* 9.33 (d, *J* = 5.6 Hz, 1H), 8.51 (d, *J =* 4.2 Hz, 1H), 7.99 (m, 1H), 7.74 (dt, *J* = 14.1, 6.4 Hz, 2H), 7.46 (dd, *J =* 7.6, 1.6 Hz, 1H), 7.38 (m, 3H), 7.28 (dd, *J =* 10.3, 5.1 Hz, 4H), 7.22 (m, 2H), 6.48 (s, 1H), 4.79 (s, 2H), 4.48 (m, 1H), 4.45 (s, 2H), 3.41 (m, 1H), 3.04 (m, 1H), 2.89 (s, 3H).

### Example 10 Preparation of compound 398

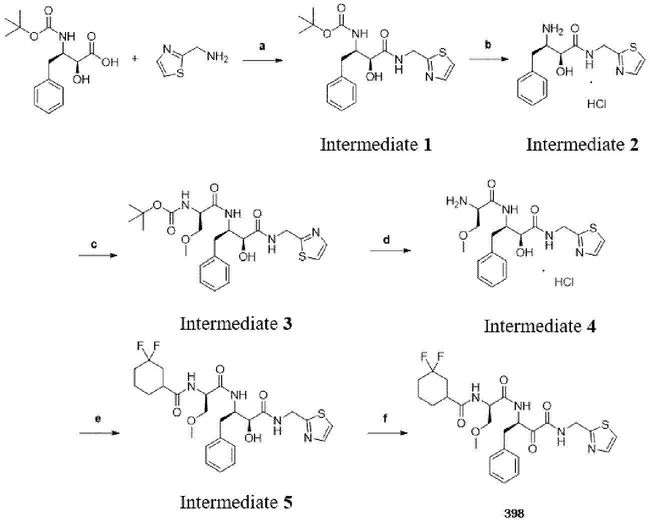

### Step a: Preparation of intermediate 1 (tert-butyl ((2R,3S)-hydroxyl-4-oxo-1-phenyl-4-(pyridin-2-ylmethyl)amino)butan-2-yl)carbamate)

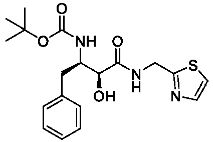

The starting material (2*S*,3*R*)-3-((tert-butoxycarbonyl)amino)-2-hydroxyl-4-phenylbutyric acid (590 mg, 2 mmol) was dissolved in DCM, to which were successively added 2-thiazolemethylamine (228 mg, 2 mmol), *N,N,N',N*'-tetramethyl-0-(7-azabenzotriazol-1-yl)uronium hexafluorophosphate (1.14 g, 3 mmol), and *N,N-*diisopropylethylamine (769 mg, 6 mmol), and then the mixture was allowed to react overnight at room temperature. The reaction solution was respectively extracted with saturated ammonium chloride solution and saturated NaHCO₃ solution. The reaction solution was concentrated. The residue was separated and purified by column chromatography, to obtain 623 mg of intermediate **1,** with a yield of 80%. MS (ESI) *m*/*z*: 392.2 [M+H]⁺.

### Step b: Preparation of intermediate 2 ((2S,3R)-3-amino-2-hydroxyl-4-phenyl-N-(thiazole-2-ylmethyl)butanamide hydrochloride)

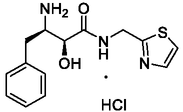

Intermediate **1** (391 mg, 1 mmol) was dissolved in dichloromethane, to which was added dioxane hydrochloride (1.25 mL, 5 mmol, 4 M) in an ice bath under nitrogen protection, and then the mixture was warmed to room temperature and stirred. After the reaction was completed by TLC, the reaction solution was concentrated to obtain 327 mg of intermediate **2,** which was directly used in the next reaction.

### Step c: Preparation of intermediate 3 (tert-butyl ((R)-1-(((2R,3S)-3-hydroxyl-4-oxo-1-phenyl-4-((thiazole-2-ylmethyl)amino)butan-2-yl)amino)-3-methoxy-1-oxopropan-2-yl)carbamate)

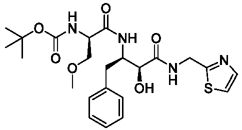

Intermediate **2** (327 mg, 1 mmol) was dissolved in dichloromethane, to which were successively added *N*-tert-butoxycarbonyl-*O*-methyl-*D*-serine (189 mg, 1 mmol), *N*,*N*,*N'*,*N'*-tetramethyl-*O*-(7-azabenzotriazol-1-yl)uronium hexafluorophosphate (570 mg, 1.5 mmol), and *N,N*-diisopropylethylamine (513 mg, 4 mmol), and then the mixture was allowed to react overnight at room temperature. The reaction solution was respectively extracted with saturated ammonium chloride solution and saturated NaHCO₃ solution. The reaction solution was concentrated. The residue was separated and purified by column chromatography, to obtain 394 mg of intermediate **3,** with a yield of 80%. MS (ESI) *m*/*z*: 493.2 [M+H]⁺.

### Step d: Preparation of intermediate 4 ((2S,3R)-3-((R)-2-amino-3-methoxypropionamido)-2-hydroxyl-4-phenyl-N-(thiazole-2-ylmethyl)butanamide hydrochloride)

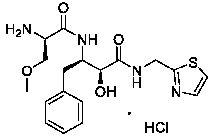

Intermediate **3** (246 mg, 0.5 mmol) was dissolved in dichloromethane, to which was added dioxane hydrochloride (0.625 mL, 2.5 mmol, 4 M) in an ice bath under nitrogen protection, and then the mixture was warmed to room temperature and stirred for 1-2 h. The reaction solution was rotatory evaporated to dry, to obtain 214 mg of intermediate **4,** which was directly used in the next reaction.

### Step e: Preparation of intermediate 5 (3,3-difluoro-N-((R)-1-(((2R,3S)-3-hydroxyl-4-oxo-1-phenyl-4-((thiazole-2-ylmethyl)amino)butan-2-yl)amino)-3-methoxy-1-oxopropan-2-yl)cyclohexane-1-formamide)

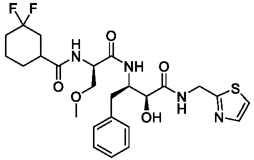

Intermediate **4** (214 mg, 0.5 mmol) was dissolved in dichloromethane, and then the reaction system was moved into an ice bath and stirred for 10 min, to which were added *N*,*N*,*N'*,*N'*-tetramethyl-*O*-(7-azabenzotriazol-1-yl)uronium hexafluorophosphate (570 mg, 1.5 mmol), *N*,*N*-diisopropylethylamine (194 mg, 1.5 mmol) and 3,3-difluorocyclohexane-1-carboxylic acid (82 mg, 0.5 mol), and then the mixture was allowed to react overnight at room temperature. After completion of the reaction, the reaction solution was respectively extracted with saturated ammonium chloride solution and saturated NaHCO₃ solution. The reaction solution was concentrated. The residue was separated and purified by column chromatography, to obtain 242 mg of intermediate **5,** with a yield of 90%. MS (ESI) *m*/*z*: 539.2 [M+H]⁺.

### Step f: Preparation of product 398 (N-((R)-1-(((R)-3,4-dioxo-1-phenyl-4-((thiazole-2-ylmethyl)amino)butan-2-yl)amino)-3-methoxy-1-oxopropan-2-yl)-3,3-difluorocyclohexane-1-formamide)

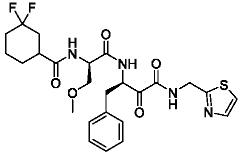

Intermediate **5** (242 mg, 0.45 mmol) was dissolved in 10 mL of dichloromethane, and then stirred in an ice bath, to which was added Dess-Martin periodinane (212 mg, 0.5 mmol), and then the reaction solution was warmed to room temperature. The reaction mixture was allowed to react under stirring for additional 2 h. After completion of the reaction, the reaction solution was diluted with dichloromethane (5 mL), and successively extracted with saturated sodium thiosulfate solution (5 mL) and saturated NaHCO₃ solution (5 mL). The organic phase was combined, dried, and then filtered. The filtrate was concentrated under reduced pressure. The residue was separated by column chromatography, to obtain product **398** (241 mg) as white solid, with a yield of 65%. ¹H NMR (400 MHz, DMSO-*d*₆) *δ* 9.59 (dt, *J =* 26.7, 6.3 Hz, 1H), 8.48-8.30 (m, 1H), 8.10 (td, *J =* 9.1, 8.4, 5.7 Hz, 1H), 7.78-7.71 (m, 1H), 7.68-7.62 (m, 1H), 7.35-7.17 (m, 5H), 5.29-5.16 (m, 1H), 4.68-4.59 (m, 2H), 4.58-4.46 (m, 1H), 3.48-3.38 (m, 1H), 3.28-3.21 (m, 2H), 3.19-3.10 (m, 3H), 2.95-2.78 (m, 1H), 2.53-2.51 (m, 1H), 2.09-1.92 (m, 2H), 1.86-1.60 (m, 4H), 1.51-1.35 (m, 1H), 1.33-1.21 (m, 1H).

### Example 11 Preparation of compound 490

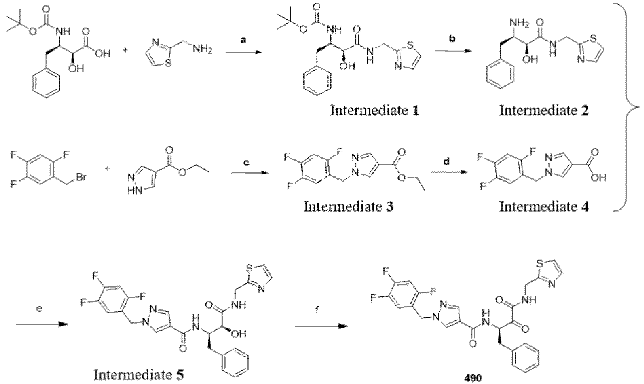

**Step a:** Same as step a in Example 10;
**Step b:** Same as step b in Example 10;

### Step c: Preparation of intermediate 3 (ethyl 1-(2,4,5-trifluorobenzyl)-1H-pyrazole-4-carboxylate)

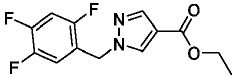

The starting material ethyl 1H-pyrazole-4-carboxylate (70 mg, 0.5 mmol) was dissolved in 10 mL of ultra dry tetrahydrofuran, and then placed in an ice bath, to which was slowly added NaH (36 mg, 1.5 mmol), followed by addition of 1-(bromomethyl)-2,4,5-trifluorobenzene (135 mg, 0.6 mmol), and then the mixture was allowed to react for half an hour, followed by reacting overnight at room temperature. After completion of the reaction detected by TLC, the reaction solution was concentrated, and then extracted with ethyl acetate and water. The organic phase was combined, dried over anhydrous Na₂SO₄, and then filtered. The filtrate was concentrated. The residue was separated and purified by column chromatography, to obtain intermediate **3** (114 mg) as yellow solid, with a yield of 80%. MS (ESI) *m*/*z:* 285.0 [M+H]⁺.

### Step d: Preparation of intermediate 4 (1-(2,4,5-trifluorobenzyl)-1H-pyrazole-4-carboxylic acid

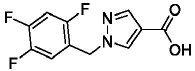

Intermediate **3** (114 mg, 0.4 mmol) was dissolved in 2 mL of ethanol, and then placed in an ice bath, to which was added the solution of lithium hydroxide (19 mg, 0.8 mmol) in water (2 mL) dropwise, and then the mixture was allowed to react for additional 30 min. TLC detection indicated completion of the reaction. The reaction solution was rotatory evaporated to dry, and then added with water. The pH was adjusted to be 2-3 with diluted hydrochloric acid (1M). The resultant solution was extracted with a suitable amount of ethyl acetate for three times. The ethyl acetate phase was combined and concentrated to obtain intermediate **4,** which was directly used in the next reaction.

### Step e: Preparation of intermediate 5 (N-((2R,3S)-3-hydroxyl-4-oxo-1-phenyl-4-((thiazole-2-ylmethyl)amino)butan-2-yl)-1-(2,4,5-trifluorobenzyl)-1H-pyrazole-4-formamide)

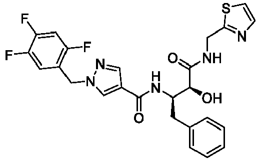

Intermediate **4** (38 mg, 0.3 mmol) was dissolved in dichloromethane, to which were successively added intermediate **2** (108 mg, 0.3 mmol), *N,N,N',N*'-tetramethyl-O-(7-azabenzotriazol-1-yl)uronium hexafluorophosphate (172 mg, 0.45 mmol) and *N,N-*diisopropylethylamine (155 mg, 1.2 mmol), and then the mixture was allowed to react overnight at room temperature. After completion of the reaction by TLC, the reaction solution was respectively extracted with saturated ammonium chloride solution and saturated NaHCO₃ solution. The combined organic phase was dried over anhydrous Na₂SO₄, and then filtered. The filtrate was concentrated. The residue was separated and purified by column chromatography, to obtain intermediate **5** (127 mg), with a yield of 80%. MS (ESI) *m*/*z:* 530.1 [M+H]⁺.

### Step f: Preparation of compound 490 ((R)-N-(3,4-dioxo-1-phenyl-4-((thiazole-2-ylmethyl))amino)butan-2-yl)-1-(2,4,5-trifluorobenzyl)-1H-pyrazole-4-formamide)

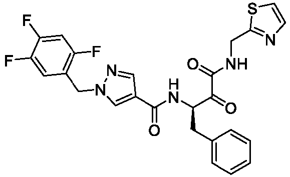

Intermediate **5** (106 mg, 0.2 mmol) was dissolved in 10 mL of dichloromethane, and then stirred in an ice bath, to which was added Dess-Martin periodinane (127 mg, 0.3 mmol), and then the reaction mixture was allowed to react in the ice bath for additional 2 h. After completion of the reaction, the reaction solution was diluted with dichloromethane (5 mL), and successively extracted with saturated sodium thiosulfate solution (5 mL) and saturated NaHCO₃ solution (5 mL). The reaction solution was still stirred for 5 min at 0 °C. The organic phase was separated. The water phase was further extracted with DCM for two times. The organic phase was combined, dried over anhydrous Na₂SO₄, and then filtered. The filtrate was concentrated. The residue was separated by column chromatography, to obtain compound **490** (82 mg) as white solid, with a yield of 78%. ¹H NMR (400 MHz, DMSO-*d*₆) *δ* 9.56 (t, *J =* 6.2 Hz, 1H), 8.56 (d, *J =* 7.2 Hz, 1H), 8.25 (s, 1H), 7.88 (s, 1H), 7.71 (d, *J =* 3.3 Hz, 1H), 7.65-7.60 (m, 1H), 7.58 (d, *J =* 3.2 Hz, 1H), 7.52-7.45 (m, 1H), 7.31-7.14 (m, 5H), 5.38 (s, 2H), 5.32-5.25 (m, 1H), 4.62 (d, *J* = 6.2 Hz, 2H), 3.23-3.15 (m, 1H), 2.94-2.85 (m, 1H). HRMS (ESI-TOF) *m*/*z* calcd. for C₂₅H₂₀F₃N₅O₃S [M+H]⁺ 528.1312, found 533.1313.

### Example 12 Preparation of compound 511

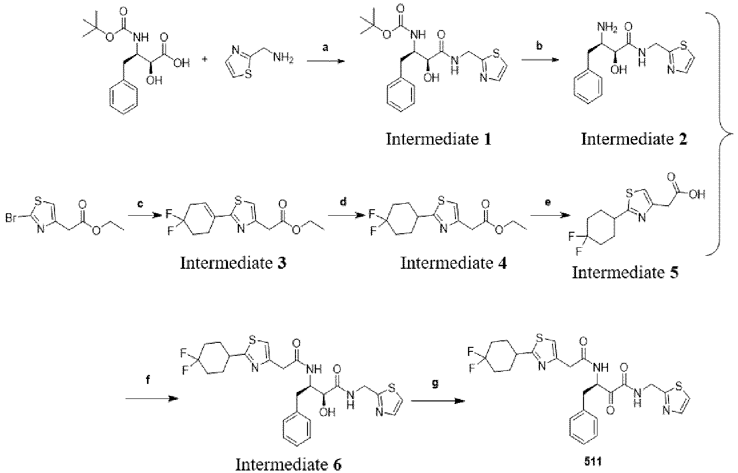

**Step a:** Same as step a in Example 10;
**Step b:** Same as step b in Example 10;

### Step c: Preparation of intermediate 3 (ethyl 2-(2-(4,4-difluorocyclohex-1-en-1-yl)thiazole-4-yl)acetate)

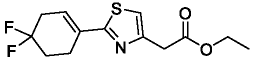

The starting material ethyl 2-bromo-4-thiazoleacetate (249 mg, 1.0 mmol), 4,4-difluorocyclohex-1-enylboronic acid pinacol ester (162 mg, 1.0 mmol), [1,1'-bis(diphenylphosphino)ferrocene]dichloropalladium (II) (73 mg, 0.1 mmol) and K₂CO₃ (176 mg, 2.0 mmol) were dissolved in 10 mL of dioxane, to which was added 0.5 mL of water, and then the reaction solution was refluxed overnight under nitrogen protection. After completion of the reaction, the reaction solution was rotatory evaporated to dry under reduced pressue, and then extracted with ethyl acetate/water. The organic phase was washed with brine, and then mixed with silica gel, followed by column chromatography, to obtain intermediate **3** (247 mg, 86%). MS (ESI) *m*/*z*: 288.1 [M+H]⁺.

### Step d: Preparation of intermediate 4 (ethyl 2-(2-(4,4-difluorocyclohexyl)thiazole-4-yl)acetate)

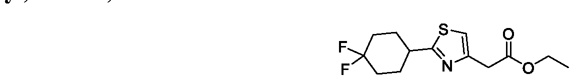

Intermediate **3** from the previous step was dissolved in 10 mL of methanol, to which was added 10% Pd/C by mass, and then the mixture was placed in a hydrogen atmosphere, and stirred overnight at room temperature. After completion of the reaction, the reaction solution was filtered over diatomaceous earth, followed by rinsing with an appropriate amount of methanol. The filtrate was rotatory evaporated to dry, to obtain 236 mg of intermediate **4,** which was directly used in the next reaction without further purification. MS (ESI) *m*/*z:* 290.1 [M+H]⁺.

### Step e: Preparation of intermediate 5 (2-(2-(4,4-difluorocyclohexyl)thiazole-4-yl)acetic acid)

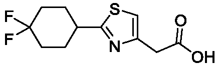

Intermediate **4** was all dissolved in MeOH/H₂O (5 mL: 5 mL), to which was added lithium hydroxide solution (1 M; 2.45 mL, 2.45 mmol), and then the reaction system was stirred for 3 h at 0 °C. After completion of the reaction, 5 mL of water was added to the reaction solution, and then the solution was adjusted to pH = 1 with hydrochloric acid, followed by extraction with ethyl acetate (10 mL×3). The combined organic phase was dried over anhydrous Na₂SO₄. The filtrate was concentrated under reduced pressure to obtain 210 mg of crude product, which was directly used in the next step without further purification. MS (ESI) (*m*/*z*): 262.1 [M+H]⁺.

### Step f: Preparation of intermediate 6 ((2S,3R)-3-(2-(2-(4,4-difluorocyclohexyl) thiazole-4-yl)acetylamino)-2-hydroxyl-4-phenyl-N-(thiazole-2-ylmethyl)butyramide)

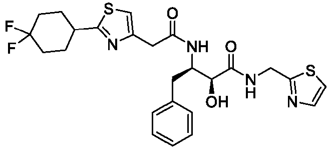

Intermediate **5** (210 mg, 0.79 mmol), intermediate **2** (238 mg, 0.82 mmol), *N,N,N',N'-*tetramethyl-O-(7-azabenzotriazol-1-yl)uronium hexafluorophosphate (373 mg, 0.95 mmol), and *N,N*-diisopropylethylamine (427 µL, 2.37 mmol) were dissolved in 10 mL of dichloromethane, and then the mixture was allowed to react for 6 h at room temperature, followed by dilution with dichloromethane. The resultant solution was sequentially washed with saturated ammonium chloride solution, saturated NaHCO₃ solution, and saturated brine, dried over anhydrous Na₂SO₄, and filtered. The filtrate was concentrated under reduced pressure, and then mixed with silica gel, followed by column chromatography, to obtain intermediate **6** (482 mg, 87%) as white oil. ¹H NMR (400 MHz, DMSO-*d*₆) *δ* 8.57 (t, *J* = 6.2 Hz, 1H), 7.74 (d, *J* = 9.0 Hz, 1H), 7.69 (d, *J* = 3.3 Hz, 1H), 7.57 (d, *J* = 3.3 Hz, 1H), 7.25 (dd, *J* = 8.1, 6.7 Hz, 2H), 7.21-7.14 (m, 3H), 7.08 (s, 1H), 6.20 (d, *J =* 5.8 Hz, 1H), 4.60-4.45 (m, 2H), 4.35-4.24 (m, 1H), 3.94 (dd, *J* = 5.8, 2.6 Hz, 1H), 3.57-3.45 (m, 2H), 3.23-3.17 (m, 1H), 2.92-2.82 (m, 1H), 2.72-2.61 (m, 1H), 2.20-1.89 (m, 6H), 1.82-1.68 (m, 2H). MS (ESI) *m*/*z:* 535.2 [M+H] ⁺.

### Step f: Preparation of compound 511 ((R)-3-(2-(2-(4,4-difluorocyclohexyl)thiazole-4-yl)acetamido)-2-oxo-4-phenyl-N-(thiazole-2-ylmethyl)butyramide)

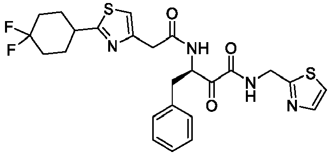

Intermediate **6** (53 mg, 0.1 mmol) was dissolved in 10 mL of dry dichloromethane, to which was added Dess-Martin periodinane (51 mg, 0.12 mmol) in portions, and then the reaction mixture was stirred at 0 °C for 2 h. The reaction was directly quenched with sodium thiosulfate solution, and the mixed system was extracted with dichloromethane (30 mL × 2). The organic layers were combined, extracted with saturated NaHCO₃ solution and saturated brine, dried over anhydrous Na₂SO₄, and then concentrated under reduced pressure. The residue was separated and purified by column chromatography to obtain product **511** (37 mg, 70%) as white solid. ¹H NMR (400 MHz, DMSO-*d*₆) *δ* 9.55 (t, *J* = 6.2 Hz, 1H), 8.51 (d, *J* = 7.1 Hz, 1H), 7.73 (d, *J* = 3.3 Hz, 1H), 7.64 (d, *J* = 3.3 Hz, 1H), 7.30-7.16 (m, 5H), 7.13 (s, 1H), 5.30-5.20 (m, 1H), 4.62 (dd, *J =* 6.2, 2.8 Hz, 2H), 3.58 (s, 2H), 3.21-3.09 (m, 2H), 2.84 (dd, *J* = 14.0, 9.0 Hz, 1H), 2.14-2.03 (m, 4H), 2.04-1.88 (m, 2H), 1.79-1.62 (m, 2H). HRMS (ESI-TOF) *m*/*z* calcd. for C₂₅H₂₆F₂N₄O₃S₂ [M+H]⁺ 533.1487, found 533.1493.

### Example 13 Preparation of compound 516

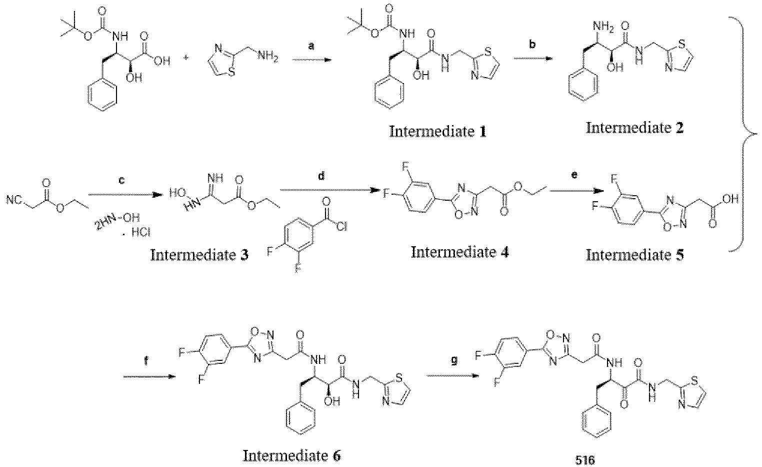

**Step a:** Same as step a in Example 10;
**Step b:** Same as step b in Example 10;

### Step c: Preparation of intermediate 3 (ethyl 3-(hydroxylamino)-3-iminopropionate)

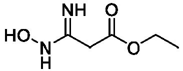

Ethyl cyanoacetate (113 mg, 1.0 mmol), hydroxylamine hydrochloride (70 mg, 1.0 mmol) and Na₂CO₃ (212 mg, 2.0 mmol) were dissolved in ethanol and then refluxed for 3-4 h. After completion of the reaction, the reaction solvent was removed by rotatory evaporation to dry under reduced pressure. To the residue, was added water. The resultant solution was extracted with ethyl acetate. The combined organic phase was dried over anhydrous Na₂SO₄, and then filtered. The filtrate was concentrated. The residue was purified by column chromatography to obtain intermediate **3** (73 mg, 50%). ¹H NMR (400 MHz, CDCl₃) *δ* 5.04 (s, 2H), 4.20 (m, 2H), 3.19 (s, 2H), 1.30 (t, *J* = 4.6, 3H). MS (ESI) *m*/*z:* 147.1 [M+H]⁺.

### Step d: Preparation of intermediate 4 (ethyl 2-(5-(3,4-difluorophenyl)-1,2,4-oxadiazol-3-yl)acetate)

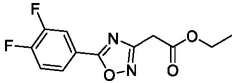

Intermediate **3** (73 mg, 0.5 mmol) obtained in the previous step and 3,4-difluorobenzoyl chloride (96 mg, 0.55 mmol) were dissolved in 10 mL of pyridine, and then the mixture was allowed to react for 20 h at 90 °C. After completion of the reaction, the solvent was rotatory evaporated to dry, and then to the residue, was added water. The resultant solution was extracted three times with ethyl acetate. The combined organic phase was dried over anhydrous Na₂SO₄, and then filtered. The filtrate was concentrated. The residue was separated and purified by column chromatography to obtain intermediate **4** (94 mg, 0.35 mmol, 70%). MS (ESI) *m*/*z:* 269.1 [M+H]⁺.

### Step e: Preparation of intermediate 5 (2-(5-(3,4-difluorophenyl)-1,2,4-oxadiazol-3-yl)acetic acid)

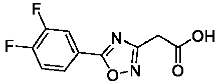

The intermediate **4** (94 mg, 0.35 mmol) obtained in the previous step was dissolved in MeOH/H₂O (2 mL: 2 mL), to which was added lithium hydroxide solution (1 M; 1.05 mL, 1.05 mmol), and then the reaction system was stirred for 3 h at 0 °C. After completion of the reaction, to the reaction solution, was added 5 mL of water, and then the resultant solution was adjusted to be pH = 1 with hydrochloric acid, followed by extraction with ethyl acetate. The combined organic phase was dried over anhydrous Na₂SO₄, and then filtered. The filtrate was concentrated under reduced pressure, to provide 72 mg of crude product, which was directly used in the next step without further purification.

### Step f: Preparation of intermediate 6 ((2S, 3R)-3-(2-(5-(3,4-difluorophenyl)-1,2,4-oxadiazol-3-yl)acetylamino)-2-hydroxyl-4-phenyl-N-(thiazole-2-ylmethyl) butyramide)

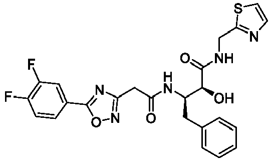

Intermediate **5** (72 mg, 0.3 mmol), intermediate **2** (87 mg, 0.3 mmol), *N,N,N',N'-*tetramethyl-O-(7-azabenzotriazol-1-yl)uronium hexafluorophosphate (136 mg, 0.36 mmol) and *N*,*N*-diisopropylethylamine (156 µL, 0.9 mmol) were dissolved in 10 mL of dichloromethane, and then the mixture was allowed to react for 6 h at room temperature. Subsequently, the reaction solution was diluted with dichloromethane, and then successively washed with saturated NH₄Cl solution, saturated NaHCO₃ solution, and saturated NaCl solution. The organic phase was dried over anhydrous Na₂SO₄, and then filtered. The filtrate was concentrated under reduced pressure. The residue was purified by column chromatography to provide intermediate **6** (126 mg, 82%) as white oil. MS (ESI) *m*/*z:* 514.1 [M+H]⁺.

### Step g: Preparation of end product 516 ((R)-3-(2-(5-(3,4-difluorophenyl)-1,2,4-oxadiazol-3-yl)acetylamino)-2-oxo-4-phenyl-N-(thiazole-2-ylmethyl)butyramide)

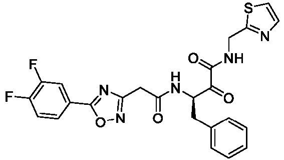

Intermediate **6** (52 mg, 0.1 mmol) was dissolved in 10 mL of dichloromethane, to which was added Dess-Martin periodinane (51 mg, 0.12 mmol) in portions, and then the reaction mixture was stirred at 0 °C for 2 h. The reaction was directly quenched with sodium thiosulfate solution, and the mixed system was extracted with dichloromethane. The organic layers were combined, successively extracted with saturated NaHCO₃ solution and brine, dried over anhydrous Na₂SO₄, and then concentrated under reduced pressure. The residue was separated and purified by column chromatography to obtain product **516** (29 mg, 57%) as white solid. ¹H NMR (400 MHz, DMSO-*d*₆) *δ* 9.60 (t, *J* = 6.3 Hz, 1H), 8.85 (d, *J =* 7.1 Hz, 1H), 8.21-8.10 (m, 1H), 8.00-7.92 (m, 1H), 7.78-7.67 (m, 2H), 7.65-7.62 (m, 1H), 7.33-7.18 (m, 5H), 5.32-5.22 (m, 1H), 4.68-4.55 (m, 2H), 3.79 (s, 2H), 3.21-3.12 (m, 1H), 2.91-2.80 (m, 1H). HRMS (ESI-TOF) *m*/*z* calcd. for C₂₄H₁₉F₂N₅O₄S [M+H]⁺512.1199, found 521.1199.

### Example 14 Preparation of compound 518

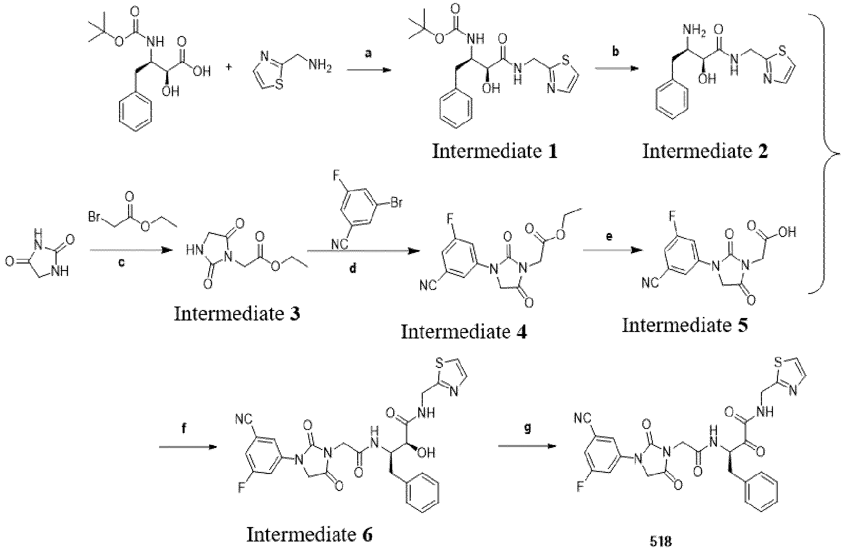

**Step a:** Same as step a in Example 10;
**Step b:** Same as step b in Example 10;

### Step c: Preparation of intermediate 3 (ethyl 2-(2,5-dioxoimidazolidin-1-yl)acetate)

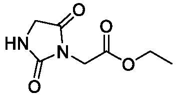

The starting material imidazolidine-2,4-dione (100 mg, 1.0 mmol), ethyl bromoacetate (165 mg, 1.0 mmol) and K₂CO₃ (276 mg, 2.0 mmol) were dissolved in the solvent acetonitrile, and then refluxed for 6-8 h. The reaction was detected by TLC. After completion of the reaction, acetonitrile was removed by rotatory evaporation to dry under reduced pressure. Subsequently, the residue was diluted with ethyl acetate, and then successively washed with water and saturated brine. The organic phase was rotatory evaporated to dry, to provide 167 mg of crude intermediate **3.** ¹H NMR (400 MHz, CDCl₃) *δ* 6.24 (s, 1H), 4.25 (s, 2H), 4.22 (m, 2H), 4.06 (s, 2H), 1.28 (t, *J =* 8.0, 3H). MS (ESI) (*m*/*z*): 187.0 [M+H]⁺.

### Step d: Preparation of intermediate 4 (ethyl 2-(3-(3-cyano-5-fluorophenyl)-2,5-dioxoimidazolidin-1-yl)acetate)

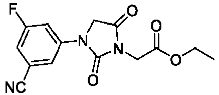

167 mg of crude intermediate **3,** 3-bromo-5-fluorobenzonitrile (178 mg, 0.9 mmol), tris(dibenzylideneacetone)dipalladium, 2-dicyclohexylphosphino-2',4',6'-triisopropylbiphenyl (102 mg, 0.1 mmol), and cesium carbonate (650 mg, 2.0 mmol) were dissolved in toluene, and refluxed overnight under nitrogen protection. After the reaction was completed, the reaction solution was concentrated under reduced pressure. The residue was diluted with ethyl acetate, and washed with water. The organic phase was dried over anhydrous sodium sulfate, mixed with silica gel, and then separated and purified by column chromatography to obtain intermediate **4** (238 mg, 87%). MS (ESI) *m*/*z*: 306.1 [M+H]⁺.

### Step e: Preparation of intermediate 5 (2-(3-(3-cyano-5-fluorophenyl)-2,5-dioxoimidazolidin-1-yl)acetic acid)

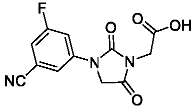

The intermediate **4** (238 mg, 0.783 mmol) obtained in the previous step was dissolved in MeOH/H₂O (5 mL/5 mL), to which was added lithium hydroxide solution (1 M; 2.35 mL, 2.35 mmol), and then the reaction system was stirred for 3 h at 0 °C. After completion of the reaction, to the reaction solution, was added 5 mL of water, and then the resultant solution was adjusted to be pH = 1 with hydrochloric acid, followed by extraction with ethyl acetate (10 mL×3). The combined organic phase was dried over anhydrous Na₂SO₄. The filtrate was concentrated under reduced pressure, to provide 220 mg of crude product, which was directly used in the next step without further purification. MS (ESI) (*m*/*z*): 278.0 [M+H]⁺.

### Step f: Preparation of intermediate 6 ((2S,3R)-3-(2-(3-(3-cyano-5-fluorophenyl)-2,5-dioxoimidazolidin-1-yl)acetylamino)-2-hydroxyl-4-phenyl-N-(thiazol-2-ylmethyl) butyramide)

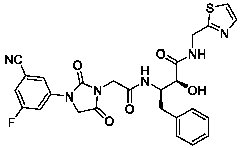

Intermediate 5 (220 mg, 0.79 mmol), intermediate **2** (230 mg, 0.79 mmol), *N,N,N',N'-*tetramethyl-O-(7-azabenzotriazol-1-yl)uronium hexafluorophosphate (360 mg, 0.95 mmol), and *N*,*N*-diisopropylethylamine (412 µL, 2.37 mmol) were dissolved in 10 mL of dichloromethane, and then the mixture was allowed to react for 6 h at room temperature. Subsequently, the reaction solution was diluted with dichloromethane, and then successively washed with saturated NH₄Cl solution, saturated NaHCO₃ solution, and saturated NaCl solution. The organic phase was dried over anhydrous Na₂SO₄, and then filtered. The filtrate was concentrated under reduced pressure, and then mixed with silica gel. The residue was purified by column chromatography to provide intermediate 6 (334 mg, 77%) as white oil. MS (ESI) *m*/*z:* 551.2 [M+H]⁺.

### Step f: Preparation of end product 518 ((R)-3-(2-(3-(3-cyano-5-fluorophenyl)-2,5-dioxoimidazolidin-1-yl)acetylamino)-2-oxo-4-phenyl-N-(thiazole-2-ylmethyl) butyramide)

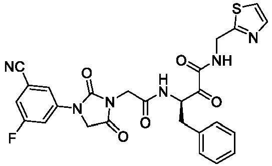

Intermediate **6** (55 mg, 0.1 mmol) was dissolved in 10 mL of super dry CH₂Cl₂, to which was added Dess-Martin periodinane (51 mg, 0.12 mmol) in portions, and then the reaction mixture was stirred at 0 °C for 2 h. The reaction was directly quenched with sodium thiosulfate solution, and the mixed system was extracted with dichloromethane (30 ml×2). The organic layers were combined, successively extracted with saturated NaHCO₃ solution and brine, dried over anhydrous Na₂SO₄, and then concentrated under reduced pressure. The residue was separated and purified by column chromatography to obtain product **518** (33 mg, 60%) as white solid. ¹H NMR (400 MHz, DMSO-*d*₆) *δ* 9.60 (t, *J=* 6.3 Hz, 1H), 8.78 (d, *J* = 7.1 Hz, 1H), 8.21 (d, *J=* 1.9 Hz, 1H), 7.73 (d, *J* = 3.2 Hz, 1H), 7.63 (d, *J =* 3.3 Hz, 1H), 7.50-7.44 (m, 1H), 7.34-7.15 (m, 6H), 5.29-5.21 (m, 1H), 4.65-4.60 (m, 2H), 4.12 (s, 2H), 3.89 (s, 2H), 3.21-3.11 (m, 1H), 2.92-2.83 (m, 1H). HRMS (ESI-TOF) *m*/*z* calcd. for C₂₆H₂₁FN₆O₅S [M+H]⁺549.1351, found 549.1355.

### Example 15 Preparation of compound 532

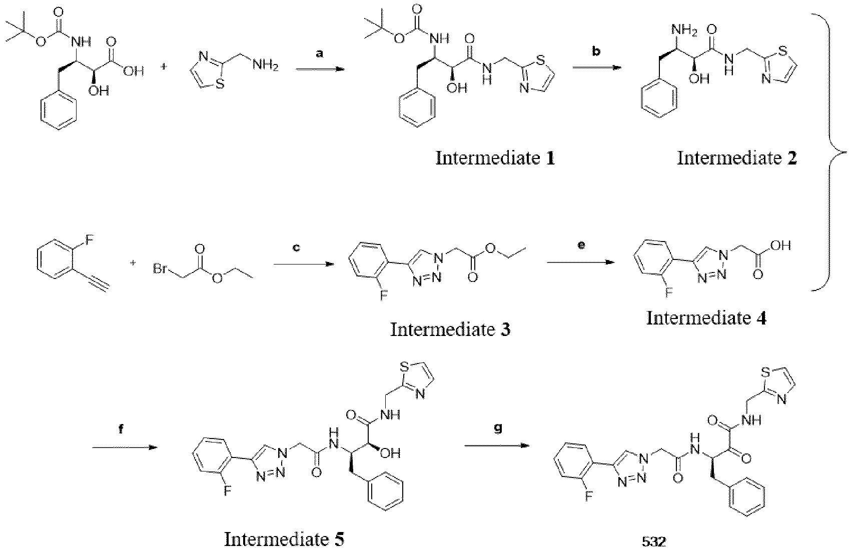

**Step a:** Same as step a in Example 10;
**Step b:** Same as step b in Example 10;

### Step c: Preparation of intermediate 3 (ethyl 2-(4-(2-fluorophenyl)-1H-1,2,3-triazol-1-yl)acetate)

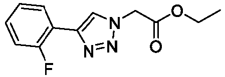

Ethyl bromoacetate (166 mg, 1.0 mmol), 1-ethynyl-2-fluorobenzene (120 mg, 1.0 mmol), sodium azide (72 mg, 1.1 mmol), sodium ascorbate (40 mg, 0.2 mmol) and copper sulfate pentahydrate (50 mg, 0.2 mmol) were weighed and placed in a 25 ml round-bottom flask, to which was added tert-butanol/water (10 mL/5 mL), and then the mixture was allowed to react at room temperature for 24 h. The reaction was monitored by TLC. After completion of the reaction, a suitable amount of water was added. The resultant solution was extracted directly with ethyl acetate. The organic phase was concentrated. The residue was purified by column chromatography to obtain 175 mg of intermediate **3,** with a yield of 70%. MS (ESI) *m*/*z:* 250.1 [M+H]⁺.

### Step d: Preparation of intermediate 4 (2-(4-(2-fluorophenyl)-1H-1,2,3-triazol-1-yl)acetic acid)

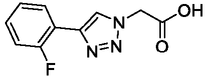

The intermediate **3** (125 mg, 0.5 mmol) obtained in the previous step was dissolved in MeOH/H₂O (2 mL : 2 mL), to which was added lithium hydroxide solution (1 M; 1.05 mL, 1.05 mmol), and then the reaction system was stirred for 3 h at 0 °C. After completion of the reaction, to the reaction solution, was added 5 mL of water, and then the resultant solution was adjusted to be pH = 1 with hydrochloric acid, followed by extraction with ethyl acetate. The combined organic phase was dried over anhydrous Na₂SO₄. The filtrate was concentrated under reduced pressure, to provide 120 mg of crude product, which was directly used in the next step without further purification.

### Step e: Preparation of intermediate 5 ((2S,3R)-3-(2-(4-(2-fluorophenyl)-1H-1,2,3-triazol-1-yl)acetamido)-2-hydroxyl-4-phenyl-N-(thiazole-2-ylmethyl)butyramide)

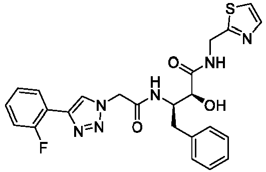

Intermediate **4** (75 mg, 0.3 mmol), intermediate **2** (87 mg, 0.3 mmol), *N,N,N',N'-*tetramethyl-*O*-(7-azabenzotriazol-1-yl)uronium hexafluorophosphate (136 mg, 0.36 mmol), and *N*,*N*-diisopropylethylamine (156 µL, 0.9 mmol) were dissolved in 10 mL of dichloromethane, and then the mixture was allowed to react for 6 h at room temperature. Subsequently, the reaction solution was diluted with dichloromethane, and then successively washed with saturated NHaCl solution, saturated NaHCO₃ solution, and saturated NaCl solution. The organic phase was dried over anhydrous Na₂SO₄, and then filtered. The filtrate was concentrated under reduced pressure. The residue was separated and purified by column chromatography to provide intermediate **5** (117 mg, 79%) as white solid. MS (ESI) *m*/*z:* 495.1 [M+H]⁺.

### Step e: Preparation of compound 532 ((R)-3-(2-(4-(2-fluorophenyl)-1H-1,2,3-triazol-1-yl)acetamido)-2-oxo-4-phenyl-N-(thiazole-2-ylmethyl)butyramide)

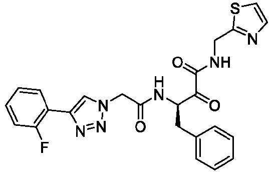

Intermediate **5** (49 mg, 0.1 mmol) was dissolved in 10 mL of CH₂Cl₂, to which was added Dess-Martin periodinane (51 mg, 0.12 mmol) in portions, and then the reaction mixture was stirred at 0 °C for 2 h. The reaction was directly quenched with sodium thiosulfate solution, and the mixed system was extracted with dichloromethane (30 mL×2). The organic layers were combined, successively extracted with saturated NaHCO₃ solution and brine, dried over anhydrous Na₂SO₄, and then concentrated under reduced pressure. The residue was separated and purified by column chromatography to obtain product **532** (24 mg, 49%) as white solid. ¹H NMR (400 MHz, DMSO-*d*₆) *δ* 9.63 (t, *J* = 6.2 Hz, 1H), 8.97 (d, *J =* 7.1 Hz, 1H), 8.31 (d, *J =* 3.8 Hz, 1H), 8.13 (td, *J* = 7.6, 1.7 Hz, 1H), 7.73 (d, *J* = 3.3 Hz, 1H), 7.62 (d, *J =* 3.3 Hz, 1H), 7.44-7.39 (m, 1H), 7.38-7.27 (m, 4H), 7.27-7.19 (m, 3H), 5.30 (dq, *J =* 11.7, 4.0 Hz, 1H), 5.24 (d, *J* = 3.7 Hz, 2H), 4.62 (dd, *J* = 6.2, 2.1 Hz, 2H), 3.25-3.13 (m, 1H), 2.96-2.82 (m, 1H). MS (ESI) *m*/*z:* 493.1 [M+H]⁺.

With reference to the method in Examples 1-15, other target compounds of the present invention were prepared. The structure and characterization data of the target compound obtained are shown in Table 1.

**Table 1. The structures and characterization data of the compounds according to the present invention.**

| **Compounds** | **Structures** | Characterization results of ¹H NMR and/or ESI-MS |
|---|---|---|
| **1** | 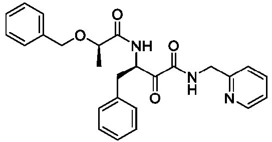 | ¹H NMR (400 MHz, DMSO-*d*₆) *δ* 9.28 (t, *J* = 6.2 Hz, 1H), 8.57-8.46 (m, 1H), 8.20 (d, *J =* 8.3 Hz, 1H), 7.82-7.67 (m, 1H), 7.42-7.15 (m, 13H), 5.34-5.23 (m, 1H), 4.44 (s, 2H), 4.42-4.35 (m, 1H), 4.31-4.23 (m, 1H), 3.87-3.80 (m, 1H), 3.25-3.18 (m, 1H), 2.94 (dd, *J =* 13.8, 9.3 Hz, 1H), 1.15 (d, *J =* 12.3, 6.7 Hz, 3H). [M+H]⁺=446.2 |
| **2** | 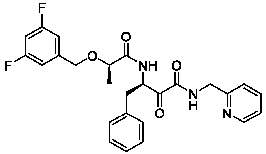 | [M+H]⁺=482.2 |
| **3** | 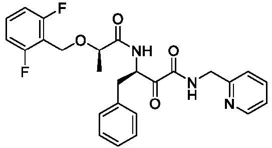 | [M+H]⁺ =482.2 |
| **4** | 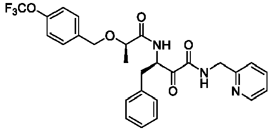 | [M+H]⁺ =530.2 |
| **5** | 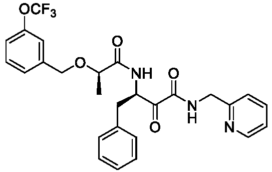 | [M+H]⁺ =530.2 |
| **6** | 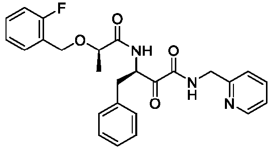 | [M+H]⁺ =464.2 |
| **7** | 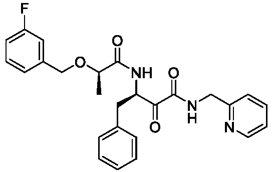 | [M+H]⁺ =464.2 |
| **8** | 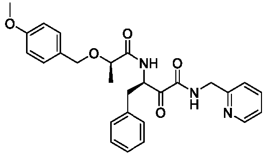 | [M+H]⁺ =476.2 |
| **9** | 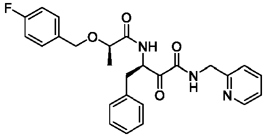 | [M+H]⁺ =464.2 |
| **10** | 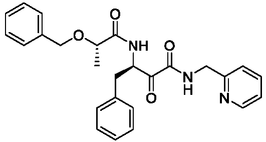 | [M+H]⁺ =446.2 |
| **11** | 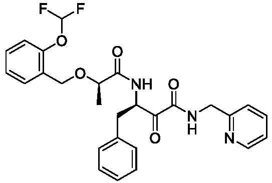 | [M+H]⁺ =512.2 |
| **12** | 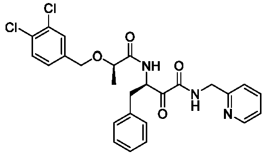 | [M+H]⁺ =514.1 |
| **13** | 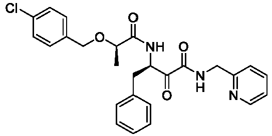 | [M+H]⁺ =480.2 |
| **14** | 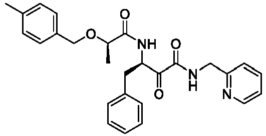 | [M+H]⁺ =460.2 |
| **15** | 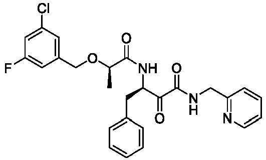 | [M+H]⁺ =498.2 |
| **16** | 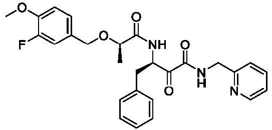 | [M+H]⁺ =494.2 |
| **17** | 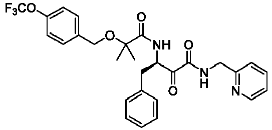 | [M+H]⁺ =544.2 |
| **18** | 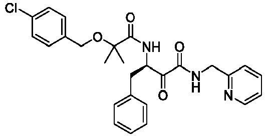 | [M+H]⁺ =494.2 |
| **19** | 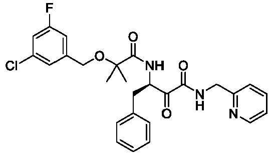 | [M+H]⁺ =512.2 |
| **20** | 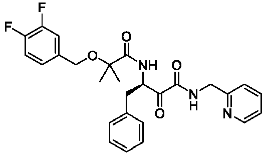 | [M+H]⁺ =496.2 |
| **21** | 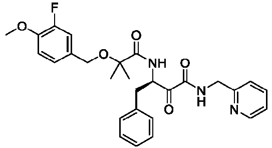 | [M+H]⁺ =508.2 |
| **22** | 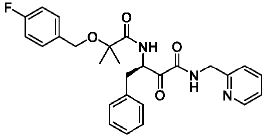 | [M+H]⁺ =478.2 |
| **23** | 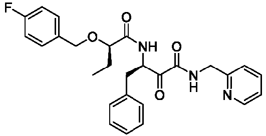 | [M+H]⁺ =478.2 |
| **24** | 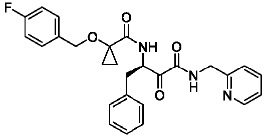 | [M+H]⁺ =476.2 |
| **25** | 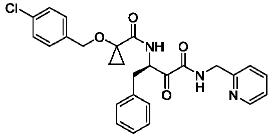 | [M+H]⁺ =492.2 |
| **26** | 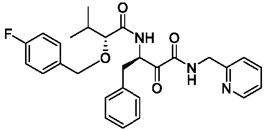 | [M+H]⁺ =492.2 |
| **27** | 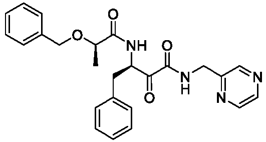 | [M+H]⁺ =447.2 |
| **28** | 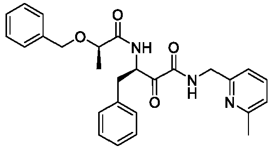 | [M+H]⁺ =460.2 |
| **29** | 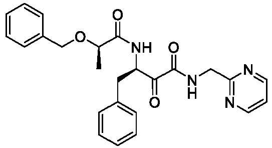 | [M+H]⁺ =447.2 |
| **30** | 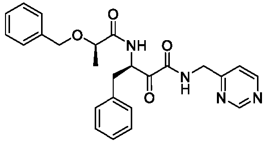 | [M+H]⁺ =447.2 |
| **31** | 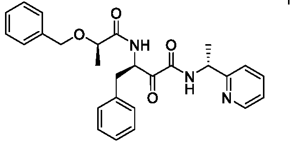 | [M+H]⁺ =460.2 |
| **32** | 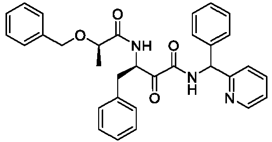 | [M+H]⁺ =522.2 |
| **33** | 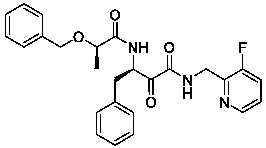 | [M+H]⁺ =464.2 |
| **34** | 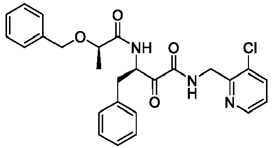 | [M+H]⁺ =480.2 |
| **35** | 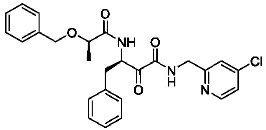 | [M+H]⁺ =480.2 |
| **36** | 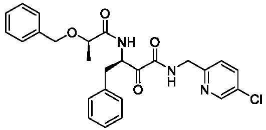 | [M+H]⁺ =480.2 |
| **37** | 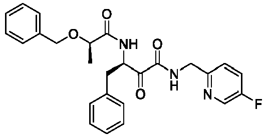 | [M+H]⁺ =464.2 |
| **38** | 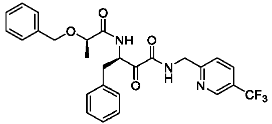 | [M+H]⁺ =514.2 |
| **39** | 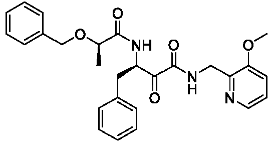 | [M+H]⁺ =476.2 |
| **40** | 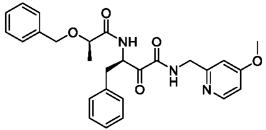 | [M+H]⁺ =476.2 |
| **41** | 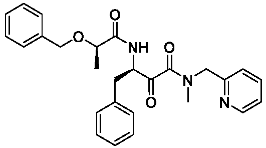 | [M+H]⁺ =460.2 |
| **42** | 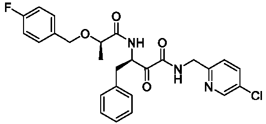 | [M+H]⁺ =498.2 |
| **43** | 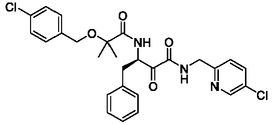 | [M+H]⁺ =528.1 |
| **44** | 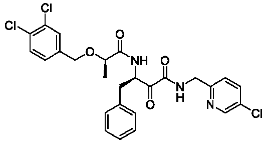 | [M+H]⁺ =548.1 |
| **45** | 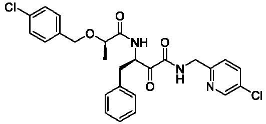 | [M+H]⁺ =514.1 |
| **46** | 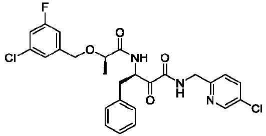 | [M+H]⁺ =532.1 |
| **47** | 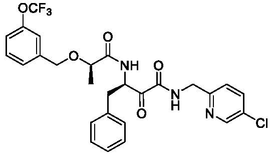 | [M+H]⁺ =564.2 |
| **48** | 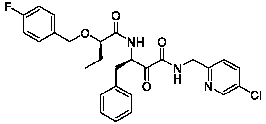 | [M+H]⁺ =512.2 |
| **49** | 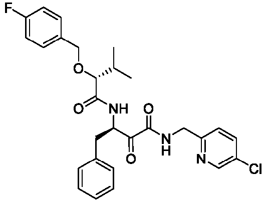 | ¹H NMR (400 MHz, Chloroform-*d*) δ 8.56-8.51 (m, 1H), 7.96-7.86 (m, 1H), 7.70-7.63 (m, 1H), 7.28-7.26 (m, 1H), 7.25-7.18 (m, 5H), 7.14-7.06 (m, 2H), 7.06-6.93 (m, 3H), 5.70-5.61 (m, 1H), 4.67-4.57 (m, 2H), 4.55-4.27 (m, 2H), 3.54 (s, 1H), 3.40-3.31 (m, 1H), 3.14-3.04 (m, 1H), 1.98-1.88 (m, 1H), 0.84 (d, *J =* 6.9 Hz, 3H), 0.78 (d, *J =* 6.8 Hz, 3H). [M+H]⁺=526.2 |
| **50** | 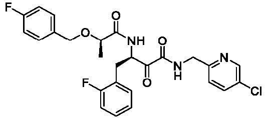 | [M+H]⁺ =516.1 |
| **51** | 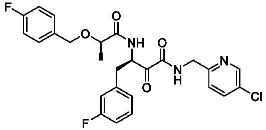 | [M+H]⁺ =516.1 |
| **52** | 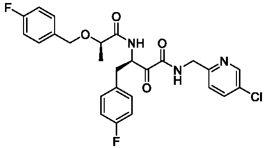 | ¹H NMR (400 MHz, DMSO-*d*₆) *δ* 9.39-9.28 (m, 1H), 8.55-8.52 (m, 1H), 8.32-8.23 (m, 1H), 7.91-7.82 (m, 1H), 7.33-7.21 (m, 5H), 7.18-7.04 (m, 4H), 5.27-5.15 (m, 1H), 4.48-4.40 (m, 2H), 4.35-4.21 (m, 3H), 3.86-3.79 (m, 2H), 3.21-3.16 (m, 1H), 2.96-2.86 (m, 1H), 1.19-1.10 (m, 3H). [M+H]⁺=516.1 |
| **53** | 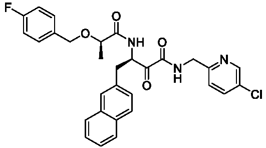 | [M+H]⁺ =548.2 |
| **54** | 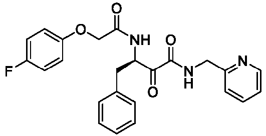 | [M+H]⁺ =436.2 |
| **55** | 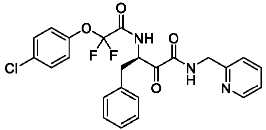 | [M+H]⁺ =488.1 |
| **56** | 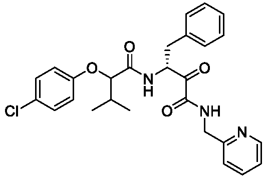 | [M+H]⁺ =494.2 |
| **57** | 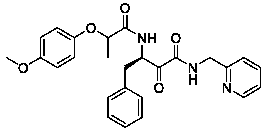 | [M+H]⁺ =462.2 |
| **58** | 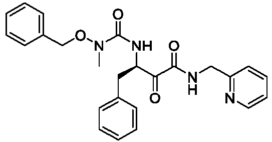 | [M+H]⁺ =447.2 |
| **59** | 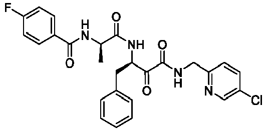 | [M+H]⁺ =511.2 |
| **60** | 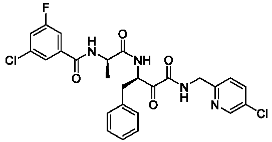 | [M+H]⁺ =545.1 |
| **61** | 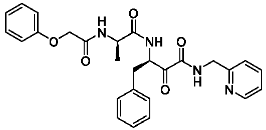 | [M+H]⁺ =489.2 |
| **62** | 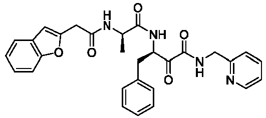 | [M+H]⁺ =513.2 |
| **63** | 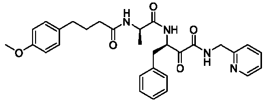 | [M+H]⁺ =531.3 |
| **64** | 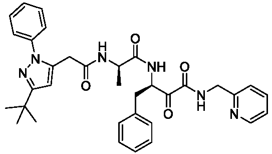 | [M+H]⁺ =595.3 |
| **65** | 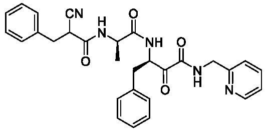 | [M+H]⁺ =512.2 |
| **66** | 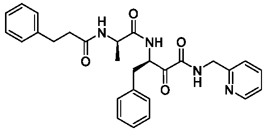 | [M+H]⁺ =487.2 |
| **67** | 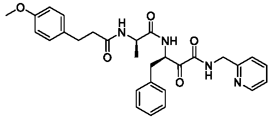 | [M+H]⁺ =517.2 |
| **68** | 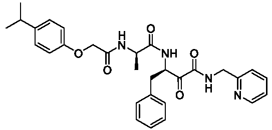 | [M+H]⁺ =531.3 |
| **69** | 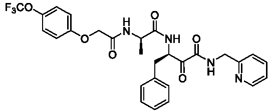 | [M+H]⁺ =573.2 |
| **70** | 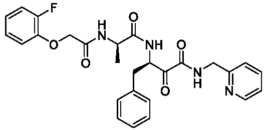 | [M+H]⁺ =507.2 |
| **71** | 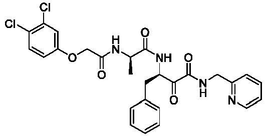 | [M+H]⁺ =557.1 |
| **72** | 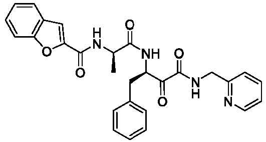 | [M+H]⁺ =499.2 |
| **73** | 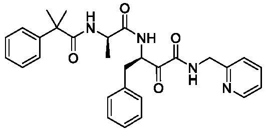 | [M+H]⁺ =501.2 |
| **74** | 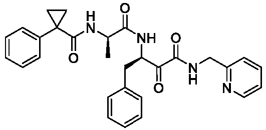 | [M+H]⁺ =499.2 |
| **75** | 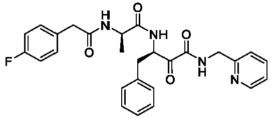 | ¹H NMR (400 MHz, Chloroform-*d*) *δ* 8.62-8.49 (m, 1H), 8.15-8.00 (m, 1H), 7.76-7.60 (m, 1H), 7.37-7.28 (m, 1H), 7.25-7.14 (m, 5H), 7.14-7.05 (m, 2H), 7.04-6.90 (m, 2H), 6.89-6.73 (m, 1H), 6.33-6.11 (m, 1H), 5.61-5.47 (m, 1H), 4.74-4.54 (m, 2H), 4.53-4.41 (m, 1H), 3.54-3.44 (m, 2H), 3.39-3.26 (m, 1H), 3.10-2.95 (m, 1H), 1.24 (d, *J* = 6.9 Hz, 1.5H), 1.17 (d, *J* = 7.0 Hz, 1.5H). |
| | | [M+H]⁺=491.2 |
| **76** | 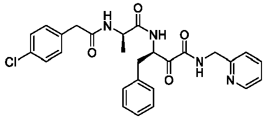 | [M+H]⁺=507.2 |
| **77** | 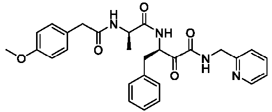 | [M+H]⁺=503.2 |
| **78** | 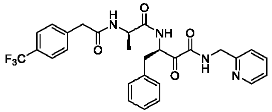 | [M+H]⁺=541.2 |
| **79** | 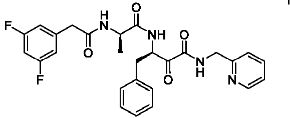 | [M+H]⁺=509.2 |
| **80** | 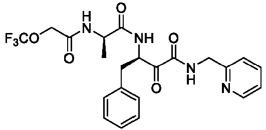 | [M+H]⁺=481.2 |
| **81** | 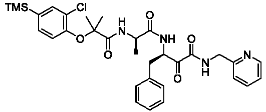 | [M+H]⁺=623.2 |
| **82** | 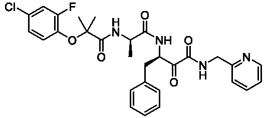 | [M+H]⁺=569.2 |
| **83** | 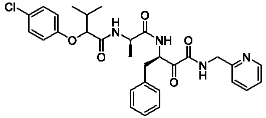 | [M+H]⁺=565.2 |
| **84** | 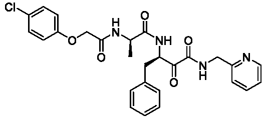 | [M+H]⁺=523.2 |
| **85** | 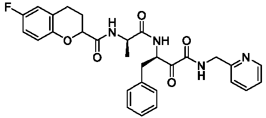 | [M+H]⁺=533.2 |
| **86** | 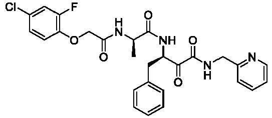 | [M+H]⁺=541.2 |
| **87** | 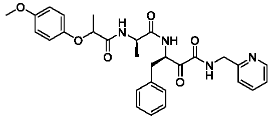 | [M+H]⁺=533.2 |
| **88** | 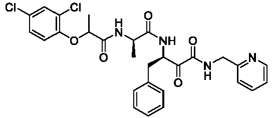 | [M+H]⁺=571.2 |
| **89** | 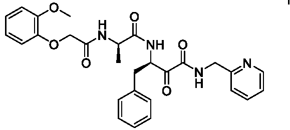 | [M+H]⁺=519.2 |
| **90** | 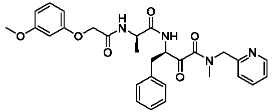 | [M+H]⁺=533.2 |
| **91** | 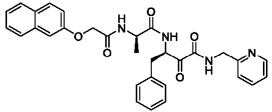 | [M+H]⁺=539.2 |
| **92** | 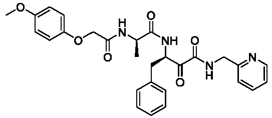 | [M+H]⁺=519.2 |
| **93** | 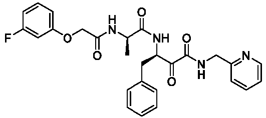 | [M+H]⁺=507.2 |
| **94** | 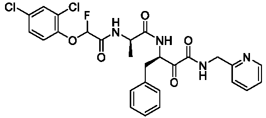 | [M+H]⁺=575.1 |
| **95** | 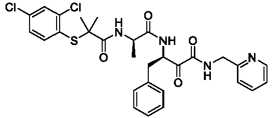 | [M+H]⁺=601.1 |
| **96** | 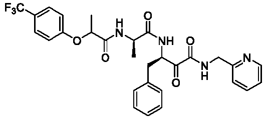 | [M+H]⁺=571.2 |
| **97** | 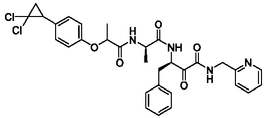 | [M+H]⁺=611.2 |
| **98** | 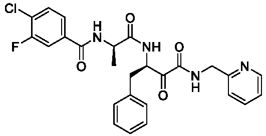 | [M+H]⁺=511.2 |
| **99** | 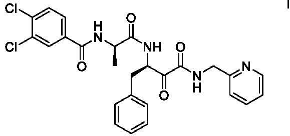 | [M+H]⁺=527.1 |
| **100** | 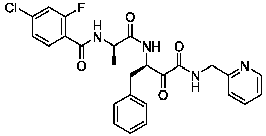 | [M+H]⁺=511.2 |
| **101** | 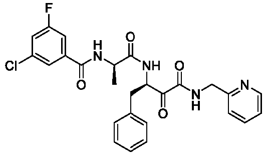 | [M+H]⁺=511.2 |
| **102** | 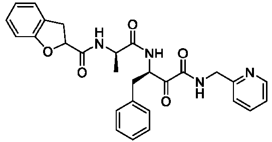 | [M+H]⁺=501.2 |
| **103** | 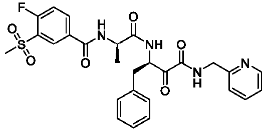 | [M+H]⁺=555.2 |
| **104** | 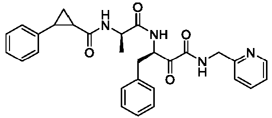 | [M+H]⁺=499.2 |
| **105** | 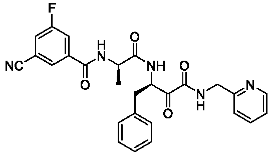 | [M+H]⁺=502.2 |
| **106** | 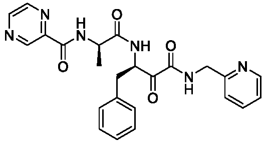 | [M+H]⁺=461.2 |
| **107** | 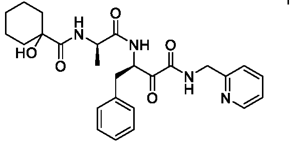 | [M+H]⁺=481.2 |
| **108** | 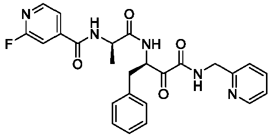 | [M+H]⁺=478.2 |
| **109** | 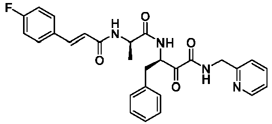 | [M+H]⁺=503.2 |
| **110** | 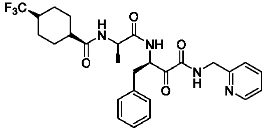 | [M+H]⁺=533.2 |
| **111** | 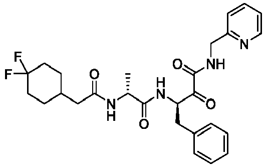 | ¹H NMR (400 MHz, DMSO-*d*₆) *δ* 9.28-9.19 (m, 1H), 8.52-8.49 (m, 1H), 8.35-8.29 (m, 1H), 7.94 (t, *J =* 8.2 Hz, 1H), 7.79-7.74 (m, 1H), 7.29-7.18 (m, 7H), 5.28-5.18 (m, 1H), 4.46-4.42 (m, 2H), 4.37-4.31 (m, 1H), 3.19-3.11(m, 1H), 2.89-2.76 (m, 1H), 2.05-2.03 (m, 2H), 2.02-1.98 (m, 2H), 1.89-1.67 (m, 5H), 1.15-1.00 (m, 3H). |
| | | [M+H]⁺=515.2458 |
| **112** | 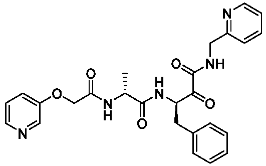 | [M+H]⁺=490.2 |
| **113** | 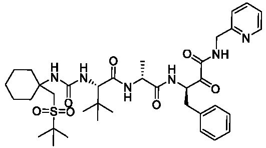 | [M+H]⁺ =727.4 |
| **114** | 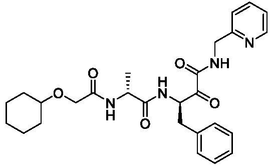 | [M+H]⁺ =495.3 |
| **115** | 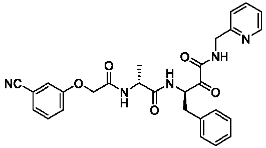 | [M+H]⁺ =514.2 |
| **116** | 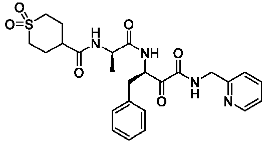 | [M+H]⁺ =515.2 |
| **117** | 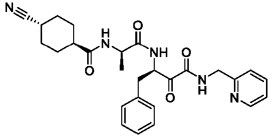 | [M+H]⁺ =490.2 |
| **118** | 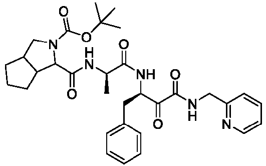 | [M+H]⁺ =592.3 |
| **119** | 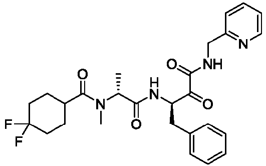 | [M+H]⁺ =515.2 |
| **120** | 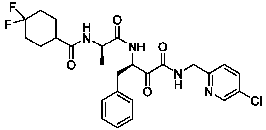 | [M+H]⁺ =535.2 |
| **121** | 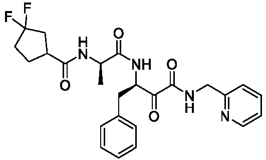 | [M+H]⁺ =487.2 |
| **122** | 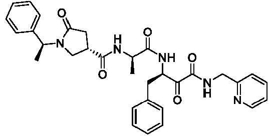 | [M+H]⁺ =570.3 |
| **123** | 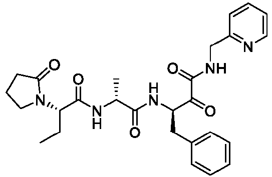 | [M+H]⁺ =508.3 |
| **124** | 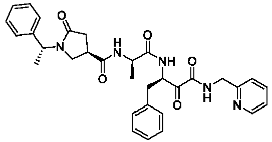 | [M+H]⁺ =570.3 |
| **125** | 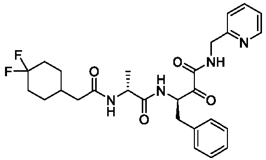 | [M+H]⁺ =515.2 |
| **126** | 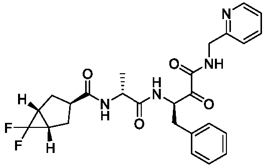 | ¹H NMR (400 MHz, DMSO-*d*₆) *δ* 9.21 (t, *J* = 6.2 Hz, 1H), 8.56-8.48 (m, 1H), 8.25 (d, *J* = 7.0 Hz, 1H), 8.06 (d, *J=* 7.8 Hz, 1H), 7.83-7.72 (m, 1H), 7.35-7.19 (m, 7H), 5.25-5.12 (m, 1H), 4.49-4.42 (m, 2H), 4.29 (t, *J =* 7.2 Hz, 1H), 3.19-3.10 (m, 1H), 2.93-2.84 (m, 1H), 2.70-2.57 (m, 1H), 2.19-1.97 (m, 6H), 1.14 (d, *J =* 7.1 Hz, 3H). |
| | | [M+H]⁺=499.2149 |
| **127** | 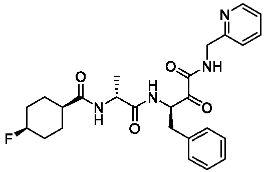 | [M+H]⁺ =483.2 |
| **128** | 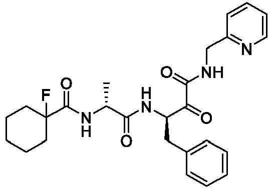 | [M+H]⁺ =483.2 |
| **129** | 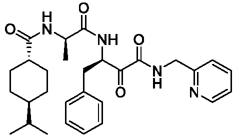 | [M+H]⁺ =507.3 |
| **130** | 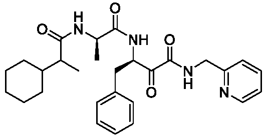 | [M+H]⁺ =493.3 |
| **131** | 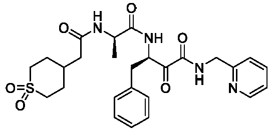 | [M+H]⁺ =529.2 |
| **132** | 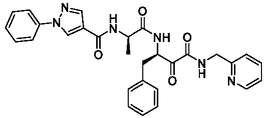 | [M+H]⁺ =525.2 |
| **133** | 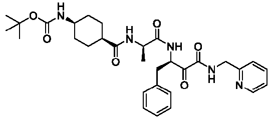 | [M+H]⁺ =580.3 |
| **134** | 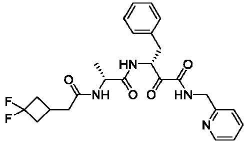 | [M+H]⁺ =487.2 |
| **135** | 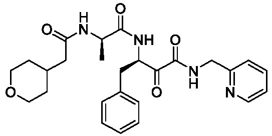 | [M+H]⁺ =481.2 |
| **136** | 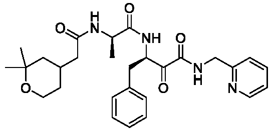 | [M+H]⁺ =509.3 |
| **137** | 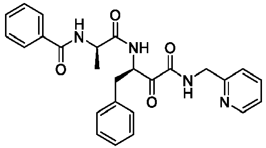 | ¹H NMR (400 MHz, DMSO-*d*₆) *δ* 9.23 (t, *J =* 27.6, 6.1 Hz, 1H), 8.53-8.47 (m, 1H), 8.46-8.32 (m, 2H), 7.93-7.82 (m, 2H), 7.77-7.70 (m, 1H), 7.57-7.50 (m, 1H), 7.49-7.41 (m, 2H), 7.31-7.14 (m, 7H), 5.28-5.17 (m, 1H), 4.58-4.48 (m, 1H), 4.43 (s, 2H), 3.22-3.09 (m, 1H), 2.94-2.79 (m, 1H), 1.23 (s, 3H). |
| | | [M+H]⁺=459.2 |
| **138** | 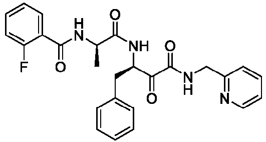 | [M+H]⁺ =477.2 |
| **139** | 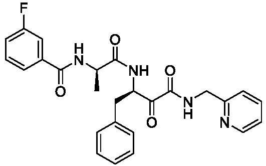 | [M+H]⁺ =477.2 |
| **140** | 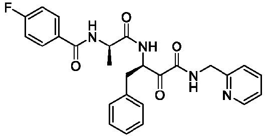 | [M+H]⁺ =477.2 |
| **141** | 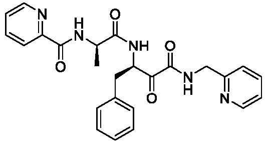 | [M+H]⁺ =460.2 |
| **142** | 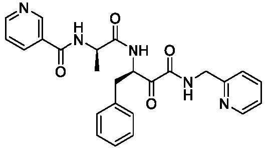 | [M+H]⁺ =460.2 |
| **143** | 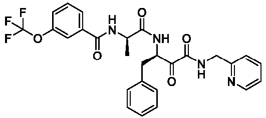 | [M+H]⁺ =543.2 |
| **144** | 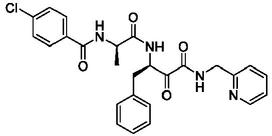 | [M+H]⁺ =493.2 |
| **145** | 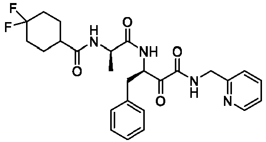 | ¹H NMR (400 MHz, DMSO-*d*₆) *δ* 9.21 (t, *J* = 6.2 Hz, 1H), 8.50 (d, *J =* 4.8 Hz, 1H), 8.25 (d, *J =* 6.9 Hz, 1H), 7.94 (d, *J* = 7.7 Hz, 1H), 7.76 (m, 1H), 7.40-7.11 (m, 7H), 5.26-5.15 (m, 1H), 4.43 (d, *J =* 6.2 Hz, 2H), 4.35-4.25 (m, 1H), 3.18-3.10 (m, 1H), 2.93-2.81 (m, 1H), 2.35-2.23 (m, 1H), 2.08-1.96 (m, 2H), 1.84-1.67 (m, 4H), 1.61-1.48 (m, 2H), 1.14 (d, *J =* 7.1 Hz, 3H). |
| | | [M+H]⁺=501.2305 |
| **146** | 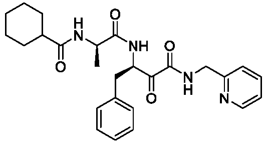 | [M+H]⁺ =465.2 |
| **147** | 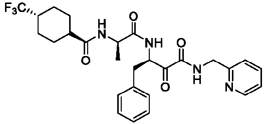 | [M+H]⁺ =533.2 |
| **148** | 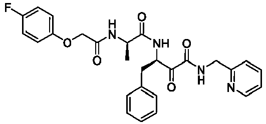 | [M+H]⁺ =507.2 |
| **149** | 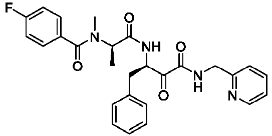 | [M+H]⁺ =491.2 |
| **150** | 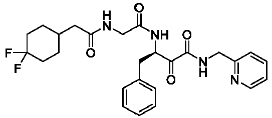 | ¹H NMR (400 MHz, DMSO-*d*₆) *δ* 9.25 (t, *J* = 6.2 Hz, 1H), 8.51 (dd, *J* = 4.9, 1.6 Hz, 1H), 8.32 (d, *J =* 7.1 Hz, 1H), 8.04 (t, *J =* 5.8 Hz, 1H), 7.77 (td, *J =* 7.7, 1.8 Hz, 1H), 7.31-7.25 (m, 3H), 7.25-7.20 (m, 4H), 5.34-5.22 (m, 1H), 4.45 (d, *J* = 6.1 Hz, 2H), 3.83-3.61 (m, 2H), 3.19-3.10 (m, 1H), 2.91-2.79 (m, 1H), 2.06 (d, *J =* 7.0 Hz, 2H), 2.01-1.88 (m, 2H), 1.86-1.76 (m, 2H), 1.76-1.63 (m, 3H), 1.21-1.10 (m, 2H). |
| | | [M+H]⁺=501.2309 |
| **151** | 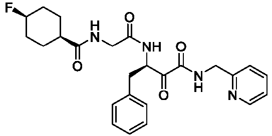 | [M+H]⁺ =469.2 |
| **152** | 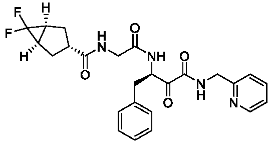 | ¹H NMR (400 MHz, DMSO-*d*₆) *δ* 9.26 (t, *J* = 6.2 Hz, 1H), 8.51 (d, *J* = 4.7 Hz, 1H), 8.30 (d, *J* = 7.1 Hz, 1H), 8.16 (t, *J =* 5.9 Hz, 1H), 7.77 (t, *J =* 7.9 Hz, 1H), 7.34-7.26 (m, 3H), 7.26-7.19 (m, 4H), 5.32-5.21 (m, 1H), 4.46 (d, *J =* 6.1 Hz, 2H), 3.80-3.61 (m, 2H), 3.20-3.10 (m, 1H), 2.93-2.79 (m, 1H), 2.73-2.59 (m, 1H), 2.19-2.02 (m, 6H). |
| | | [M+H]⁺=485.1996 |
| **153** | 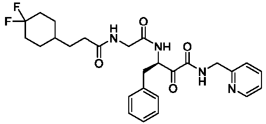 | [M+H]⁺ =515.2 |
| **154** | 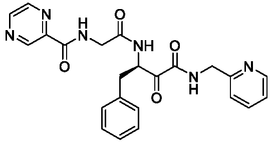 | [M+H]⁺ =447.2 |
| **155** | 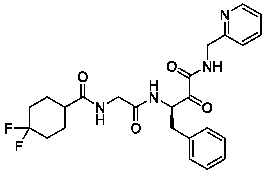 | ¹H NMR (400 MHz, DMSO-*d*₆) *δ* 9.25 (t, *J* = 6.3 Hz, 1H), 8.55-8.46 (m, 1H), 8.33-8.22 (m, 1H), 8.09-7.99 (m, 1H), 7.77 (t, *J* = 7.8 Hz, 1H), 7.34-7.25 (m, 3H), 7.25-7.15 (m, 4H), 5.32-5.21 (m, 1H), 4.44 (d, *J =* 6.1 Hz, 2H), 3.78-3.58 (m, 2H), 3.17-3.08 (m, 1H), 2.90-2.78 (m, 1H), 2.36-2.23 (m, 1H), 2.13-1.95 (m, 2H), 1.89-1.67 (m, 4H), 1.65-1.49 (m, 2H). |
| | | [M+H]⁺=487.2151 |
| **156** | 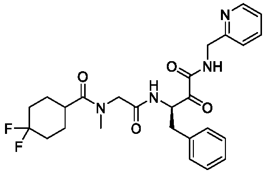 | ¹H NMR (400 MHz, DMSO-*d*₆) *δ* 9.29-9.23 (m, 1H), 8.64-8.33 (m, 2H), 7.78-7.75 (m, 1H), 7.32-7.17 (m, 7H), 5.32-5.21 (m, 1H), 4.46-4.39 (m, 2H), 4.03-3.83 (m, 2H), 3.22-3.12 (m, 1H), 2.91-2.68 (m, 4H), 2.10-1.90 (m, 3H), 1.80-1.65 (m, 3H), 1.56-1.45 (m, 3H). |
| | | [M+H]⁺=501.2304 |
| **157** | 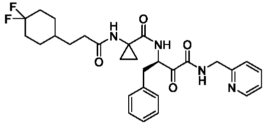 | [M+H]⁺ =541.3 |
| **158** | 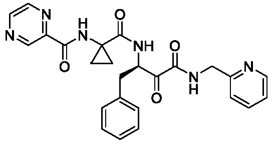 | [M+H]⁺ =473.2 |
| **159** | 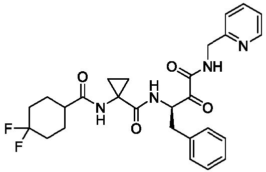 | ¹H NMR (400 MHz, DMSO-*d₆*) *δ* 9.22 (t, *J* = 6.2 Hz, 1H), 8.50 (d, *J* = 9.6 Hz, 1H), 8.40 (s, 1H), 7.77 (td, *J* = 7.7, 1.8 Hz, 1H), 7.61 (d, *J* = 7.1 Hz, 1H), 7.30-7.15 (m, 7H), 5.21-5.16 (m, 1H), 4.44 (d, *J* = 6.22H), 3.12 (dd, *J* = 13.9, 4.7 Hz, 1H), 2.98 (dd, *J* = 13.9, 7.8 Hz, 1H), 2.26-2.23 (m, 1H), 2.07-1.99 (m, 2H), 1.84-1.70 (m, 4H), 1.59-1.49 (m, 2H), 1.25-1.12 (m, 2H), 0.90-0.76 (m, 2H). |
| | | [M+H]⁺=513.2309 |
| **160** | 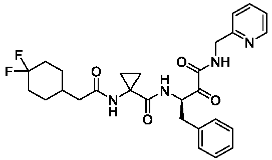 | [M+H]⁺ =527.2 |
| **161** | 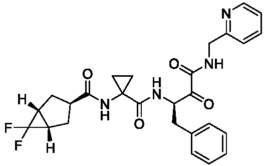 | ¹H NMR (400 MHz, DMSO-*d₆*) *δ* 9.20 (t, *J* = 6.3 Hz, 1H), 8.58-8.46 (m, 2H), 8.40 (s, 1H), 7.82-7.73 (m, 1H), 7.71-7.61 (m, 1H), 7.30-7.12 (m, 7H), 5.21-5.10 (m, 1H), 4.47-4.38 (m, 2H), 3.14-3.07 (m, 1H), 3.01-2.93 (m, 1H), 2.16-2.00 (m, 7H), 1.25-1.10 (m, 2H), 0.90-0.80 (m, 2H). |
| | | [M+H]⁺ =511.2 |
| **162** | 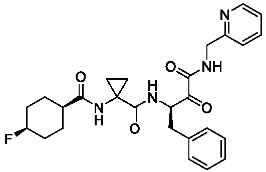 | [M+H]⁺ =495.2 |
| **163** | 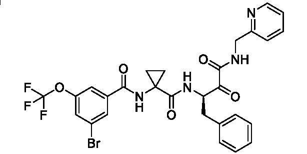 | [M+H]⁺ =633.1 |
| **164** | 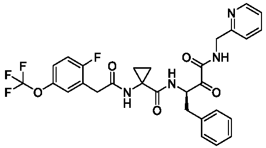 | [M+H]⁺ =587.2 |
| **165** | 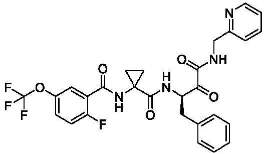 | [M+H]⁺ =573.2 |
| **166** | 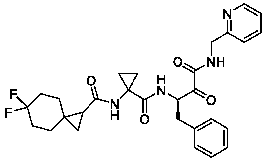 | [M+H]⁺ =539.2 |
| **167** | 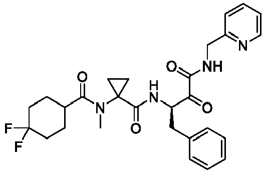 | [M+H]⁺ =527.2 |
| **168** | 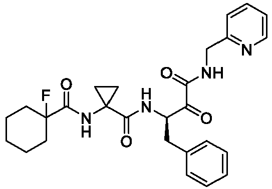 | [M+H]⁺ =495.2 |
| **169** | 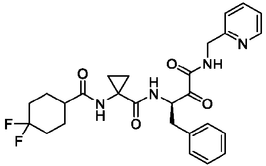 | [M+H]⁺ =513.2 |
| **170** | 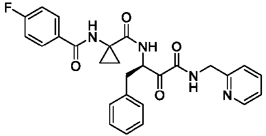 | [M+H]⁺ =489.2 |
| **171** | 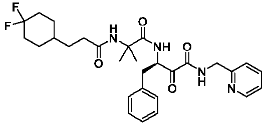 | [M+H]⁺ =543.3 |
| **172** | 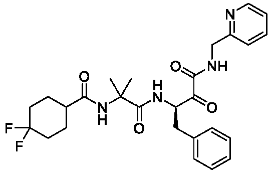 | ¹H NMR (400 MHz, DMSO-*d₆*) *δ* 9.19 (t, *J* = 6.2 Hz, 1H), 8.53-8.49 (m, 1H), 7.80 (s, 1H), 7.79-7.73 (m, 2H), 7.30-7.22 (m, 4H), 7.22-7.17 (m, 3H), 5.15-5.09 (m, 1H), 4.46-4.42 (m, 2H), 3.15-3.07 (m, 1H), 2.95-2.87 (m, 1H), 2.29-2.22 (m, 1H), 2.07-1.97 (m, 2H), 1.83-1.68 (m, 4H), 1.60-1.48 (m, 2H), 1.28 (s, 3H), 1.25 (s, 3H). |
| | | [M+H]⁺ =515.2 |
| **173** | 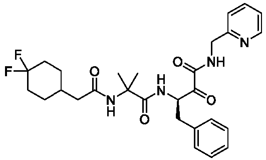 | [M+H]⁺ =529.3 |
| **174** | 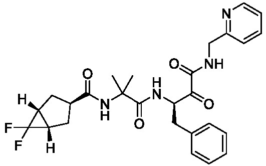 | [M+H]⁺ =513.2 |
| **175** | 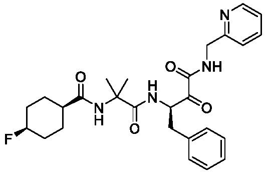 | [M+H]⁺ =497.3 |
| **176** | 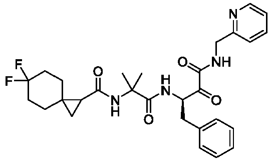 | [M+H]⁺ =541.3 |
| **177** | 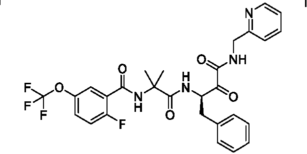 | [M+H]⁺ =575.2 |
| **178** | 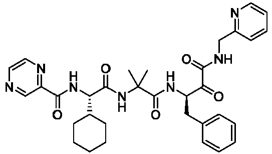 | [M+H]⁺ =614.3 |
| **179** | 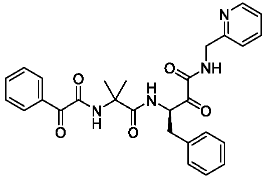 | [M+H]⁺ =501.2 |
| **180** | 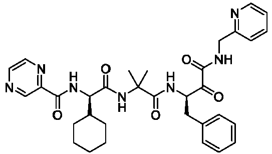 | [M+H]⁺ =614.3 |
| **181** | 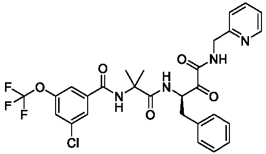 | [M+H]⁺ =591.2 |
| **182** | 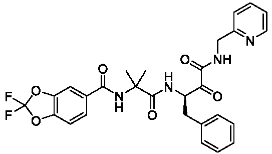 | [M+H]⁺ =553.2 |
| **183** | 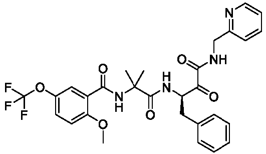 | [M+H]⁺ =587.2 |
| **184** | 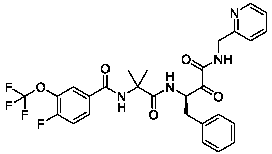 | [M+H]⁺ =575.2 |
| **185** | 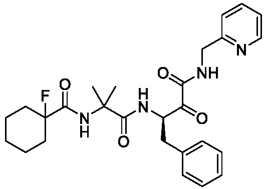 | [M+H]⁺ =497.3 |
| **186** | 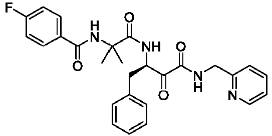 | [M+H]⁺ =491.2 |
| **187** | 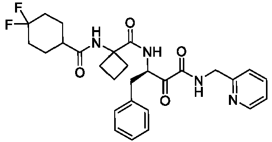 | [M+H]⁺ =527.2 |
| **188** | 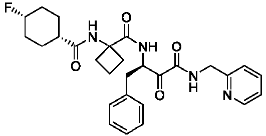 | [M+H]⁺ =509.3 |
| **189** | 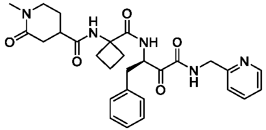 | [M+H]⁺ =520.3 |
| **190** | 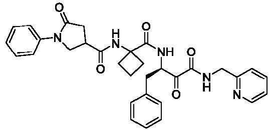 | [M+H]⁺ =568.3 |
| **191** | 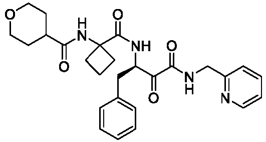 | [M+H]⁺ =493.2 |
| **192** | 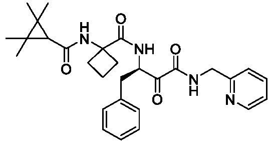 | [M+H]⁺ =505.3 |
| **193** | 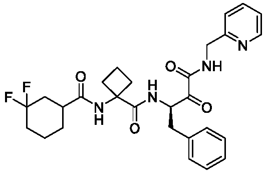 | [M+H]⁺ =527.2 |
| **194** | 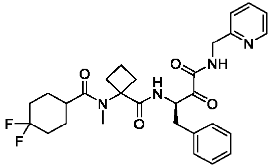 | [M+H]⁺ =541.3 |
| **195** | 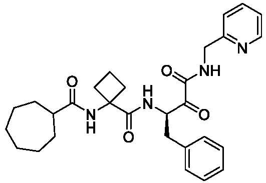 | [M+H]⁺ =505.3 |
| **196** | 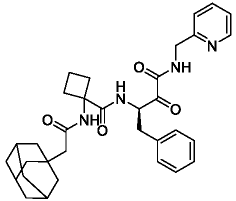 | [M+H]⁺ =557.3 |
| **197** | 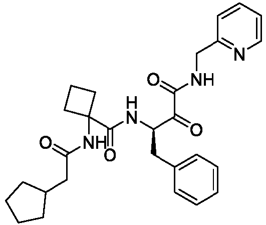 | [M+H]⁺ =491.3 |
| **198** | 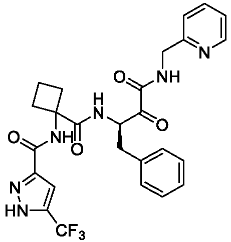 | [M+H]⁺ =543.2 |
| **199** | 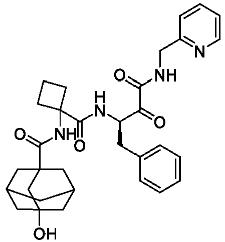 | [M+H]⁺ =559.3 |
| **200** | 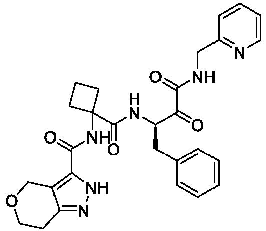 | [M+H]⁺ =531.2 |
| **201** | 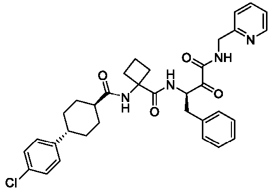 | [M+H]⁺ =601.3 |
| **202** | 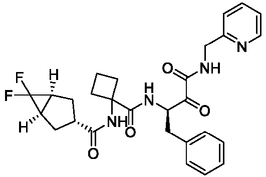 | [M+H]⁺ =525.2 |
| **203** | 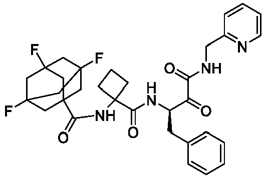 | [M+H]⁺ =597.3 |
| **204** | 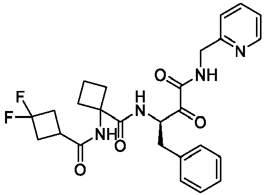 | [M+H]⁺ =499.2 |
| **205** | 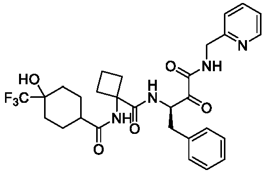 | [M+H]⁺ =575.2 |
| **206** | 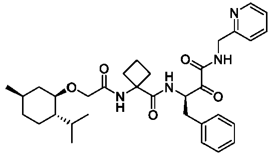 | [M+H]⁺ =577.3 |
| **207** | 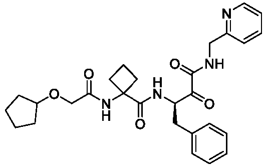 | [M+H]⁺ =507.3 |
| **208** | 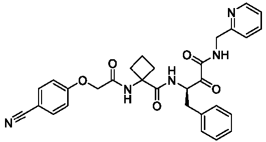 | [M+H]⁺ =540.2 |
| **209** | 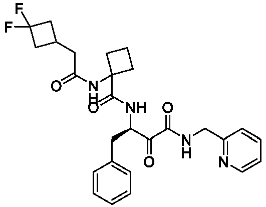 | [M+H]⁺ =513.2 |
| **210** | 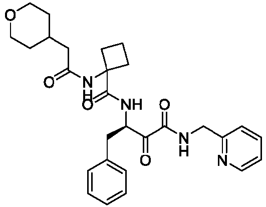 | [M+H]⁺ =507.3 |
| **211** | 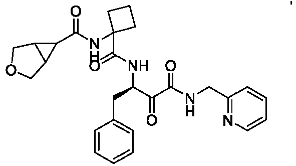 | [M+H]⁺ =491.2 |
| **212** | 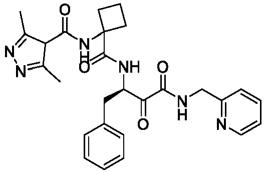 | [M+H]⁺ =503.2 |
| **213** | 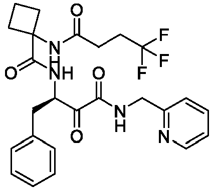 | [M+H]⁺ =505.2 |
| **214** | 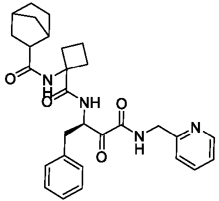 | [M+H]⁺ =503.3 |
| **215** | 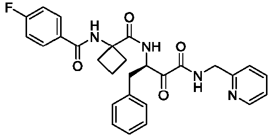 | [M+H]⁺ =503.2 |
| **216** | 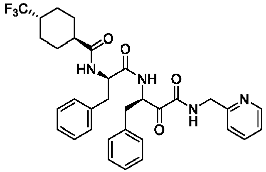 | [M+H]⁺ =609.3 |
| **217** | 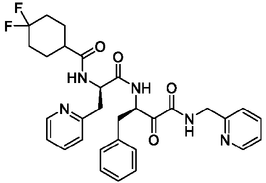 | [M+H]⁺ =578.3 |
| **218** | 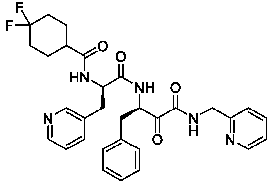 | [M+H]⁺ =578.3 |
| **219** | 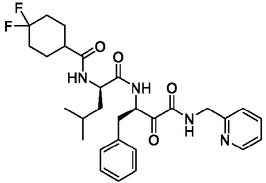 | [M+H]⁺ =543.3 |
| **220** | 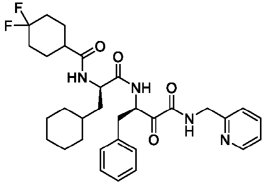 | [M+H]⁺ =583.3 |
| **221** | 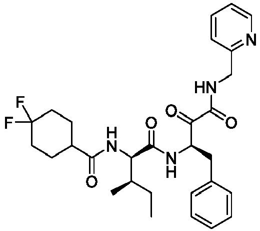 | [M+H]⁺ =543.3 |
| **222** | 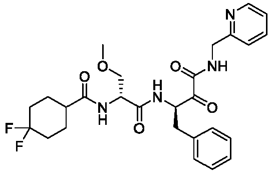 | ¹H NMR (400 MHz, DMSO-*d₆*) *δ* 9.23 (t, *J* = 6.2 Hz, 1H), 8.55-8.49 (m, 1H), 8.33 (d, *J* = 7.0 Hz, 1H), 7.99 (d, *J =* 8.2 Hz, 1H), 7.82-7.74 (m, 1H), 7.34-7.17 (m, 7H), 5.27-5.18 (m, 1H), 4.57-4.49 (m, 1H), 4.47-4.41 (m, 2H), 3.48-3.40 (m, 2H), 3.22 (s, 3H), 3.18-3.10 (m, 1H), 2.96-2.85 (m, 1H), 2.40-2.30 (m, 1H), 2.12-1.94 (m, 2H), 1.87-1.67 (m, 4H), 1.62-1.50 (m, 2H). |
| | | [M+H]⁺ =531.2416 |
| **223** | 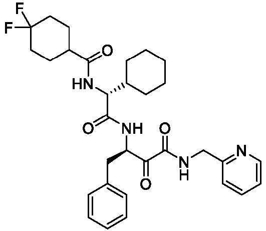 | [M+H]⁺ =569.3 |
| **224** | 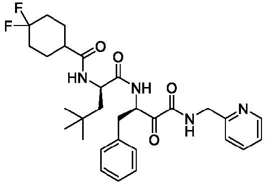 | [M+H]⁺ =557.3 |
| **225** | 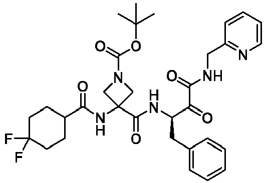 | [M+H]⁺ =628.3 |
| **226** | 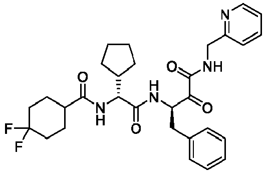 | [M+H]⁺ =555.3 |
| **227** | 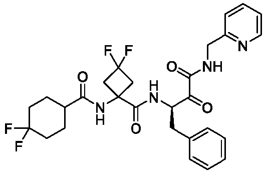 | [M+H]⁺ =563.2 |
| **228** | 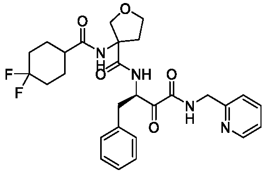 | [M+H]⁺ =543.2 |
| **229** | 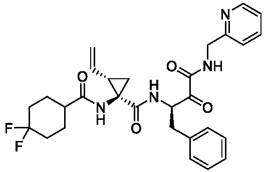 | [M+H]⁺ =539.2 |
| **230** | 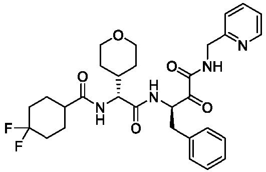 | [M+H]⁺ =571.3 |
| **231** | 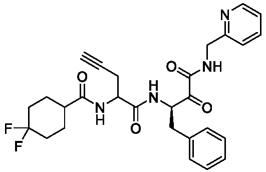 | [M+H]⁺ =525.2 |
| **232** | 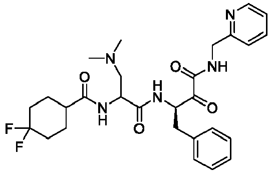 | [M+H]⁺ =544.3 |
| **233** | 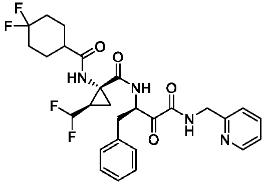 | [M+H]⁺ =563.2 |
| **234** | 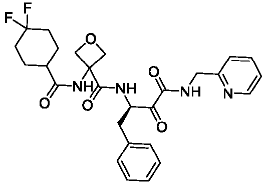 | [M+H]⁺ =529.2 |
| **235** | 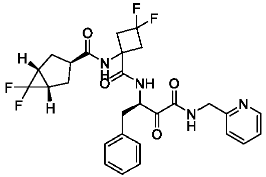 | [M+H]⁺ =561.2 |
| **236** | 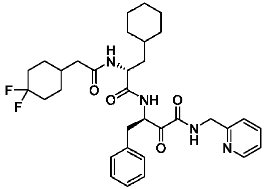 | [M+H]⁺ =597.3 |
| **237** | 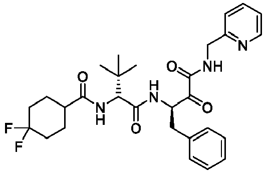 | [M+H]⁺ =543.3 |
| **238** | 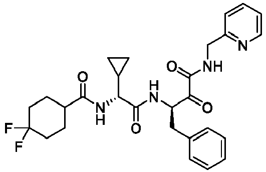 | [M+H]⁺ =527.2 |
| **239** | 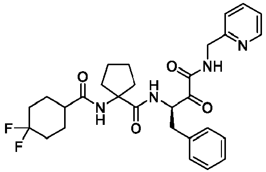 | [M+H]⁺ =541.3 |
| **240** | 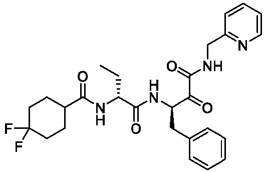 | ¹H NMR (400 MHz, DMSO-*d₆*) *δ* 9.22 (t, *J* = 6.2 Hz, 1H), 8.50 (d, *J* = 5.6 Hz, 1H), 8.32 (d, *J* = 6.8 Hz, 1H), 7.85 (d, *J* = 8.3 Hz, 1H), 7.78-7.74 (m, 1H), 7.29-7.19 (m, 7H), 5.26-5.21 (m, 1H), 4.46-4.41 (m, 2H), 4.35-4.19 (m, 2H), 3.16-3.11 (m, 1H), 2.88-2.82 (m, 1H), 2.36-2.30 (m, 1H), 2.03-1.98 (m, 2H), 1.79-1.69 (m, 4H), 1.59-1.47 (m, 3H), 0.80 (t, J = 7.4 Hz, 3H). |
| | | [M+H]⁺ =515.2466 |
| **241** | 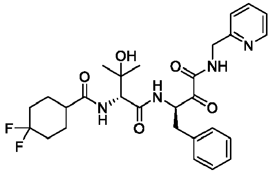 | [M+H]⁺ =545.3 |
| **242** | 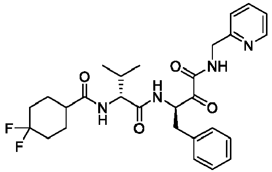 | ¹H NMR (400 MHz, DMSO-*d₆*) *δ* 9.22 (t, *J* = 6.2 Hz, 1H), 8.50 (d, *J =* 3.0 Hz, 1H), 8.43-8.40 (m, 1H), 7.77-7.73 (m, 2H), 7.29-7.14 (m, 7H), 5.30-5.25 (m, 1H), 4.46-4.41 (m, 2H), 4.25-4.19 (m, 1H), 3.19-3.10 (m, 1H), 2.87-2.72 (m, 1H), 2.41-2.35 (m, 1H), 2.08 -2.01 (m, 2H), 1.95-1.88 (m, 1H), 1.80-1.67 (m, 4H), 1.60-1.50 (m, 2H), 0.82-0.61 (m, 6H). |
| | | [M+H]⁺ =529.2621 |
| **243** | 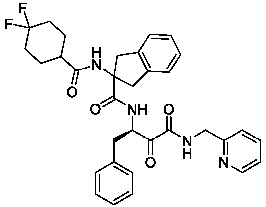 | [M+H]⁺ =589.3 |
| **244** | 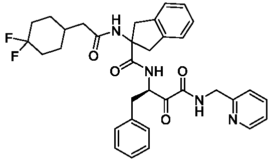 | [M+H]⁺ =603.3 |
| **245** | 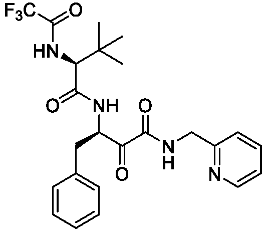 | [M+H]⁺ =493.2 |
| **246** | 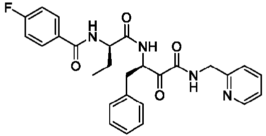 | [M+H]⁺ =491.2 |
| **247** | 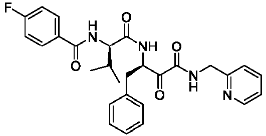 | [M+H]⁺ =505.2 |
| 248 | 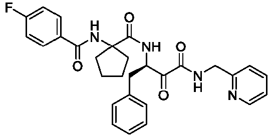 | [M+H]⁺ =517.2 |
| **249** | 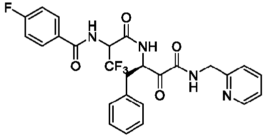 | [M+H]⁺ =531.2 |
| **250** | 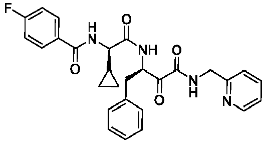 | [M+H]⁺ =503.2 |
| **251** | 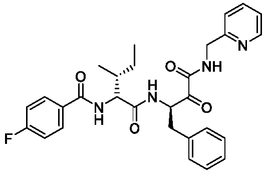 | [M+H]⁺ =519.2 |
| **252** | 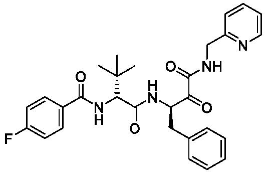 | [M+H]⁺ =519.2 |
| **253** | 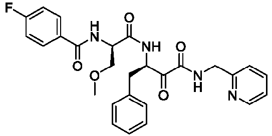 | [M+H]⁺ =507.2 |
| **254** | 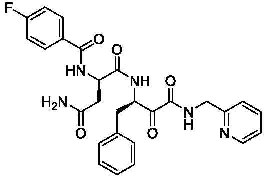 | [M+H]⁺ =520.2 |
| **255** | 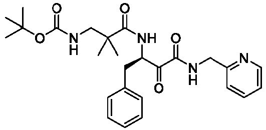 | [M+H]⁺ =483.3 |
| **256** | 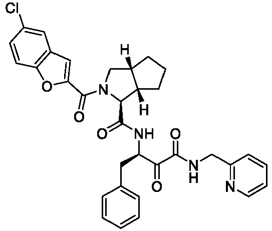 | [M+H]⁺ =599.2 |
| **257** | 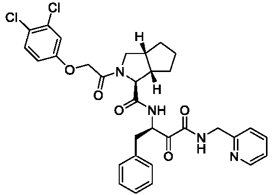 | [M+H]⁺ =623.2 |
| **258** | 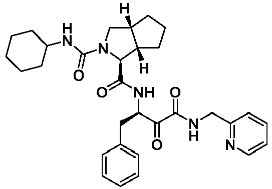 | ¹H NMR (400 MHz, DMSO-*d₆*) *δ* 9.22 (t, *J* = 6.2 Hz, 0.5H), 9.17 (t, *J* = 6.2 Hz, 0.5H), 8.55-8.47 (m, 1H), 8.27-8.15 (m, 1H), 7.80-7.70 (m, 1H), 7.30-7.12 (m, 8H), 5.71-5.59 (m, 1H), 5.24-5.13 (m, 1H), 4.50-4.37 (m, 2H), 4.11-4.01 (m, 1H), 3.49-3.42 (m, 1H), 3.18-3.02 (m, 2H), 2.92-2.78 (m, 1H), 2.59-2.51 (m, 0.5H), 2.47-2.42 (m, 0.5H), 2.41-2.34 (m, 0.5H), 2.18-2.09 (m, 0.5H), 1.75-1.27 (m, 12H), 1.22-1.09 (m, 4H). |
| | | [M+H]⁺ =546.3 |
| **259** | 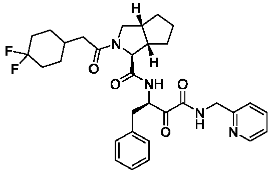 | [M+H]⁺ =581.3 |
| **260** | 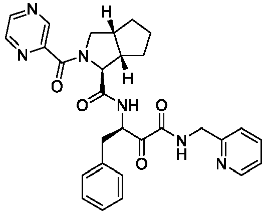 | [M+H]⁺ =527.2 |
| **261** | 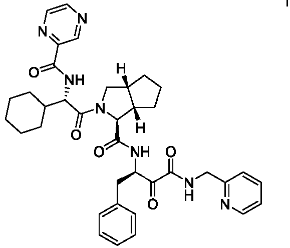 | [M+H]⁺ =666.3 |
| **262** | 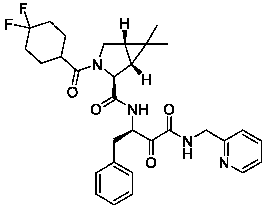 | [M+H]⁺ =567.3 |
| **263** | 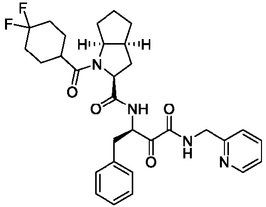 | [M+H]⁺ =567.3 |
| **264** | 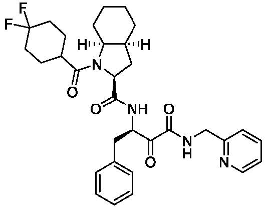 | [M+H]⁺ =581.3 |
| **265** | 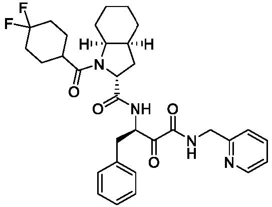 | [M+H]⁺ =581.3 |
| **266** | 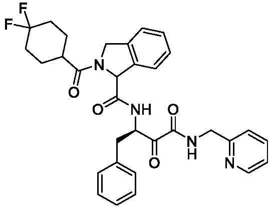 | [M+H]⁺ =575.2 |
| **267** | 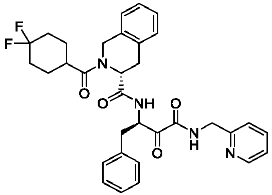 | [M+H]⁺ =589.3 |
| **268** | 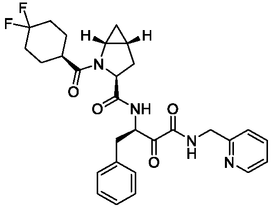 | [M+H]⁺ =539.2 |
| **269** | 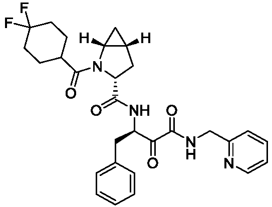 | [M+H]⁺ =539.2 |
| **270** | 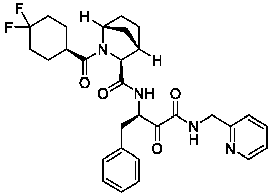 | [M+H]⁺ =553.3 |
| **271** | 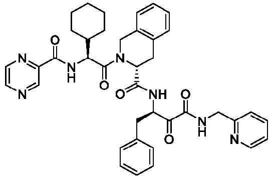 | [M+H]⁺ =688.3 |
| **272** | 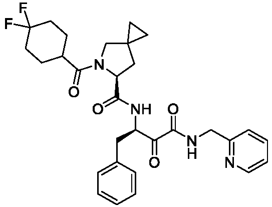 | [M+H]⁺ =553.3 |
| **273** | 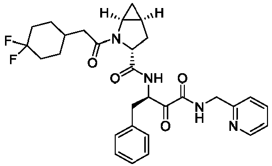 | [M+H]⁺ =553.3 |
| **274** | 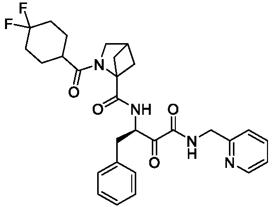 | [M+H]⁺ =539.2 |
| **275** | 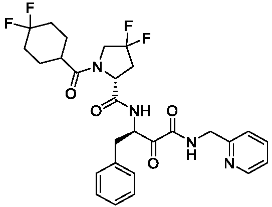 | ¹H NMR (400 MHz, DMSO-*d₆*) *δ* 9.32-9.18 (m, 1H), 8.77-8.39 (m, 2H), 7.78-7.74 (m, 1H), 7.29-7.22 (m, 7H), 5.28-5.16 (m, 1H), 4.75-4.57 (m, 1H), 4.46-4.42 (m, 2H), 4.18-3.63 (m, 2H), 3.24-3.11 (m, 1H), 2.92-2.55 (m, 3H), 2.34-2.22 (m, 1H), 2..11-1.98 (m, 2H), 1.88-1.75 (m, 3H), 1.58-1.4 (m, 3H). |
| | | [M+H]⁺ =563.2269 |
| **276** | 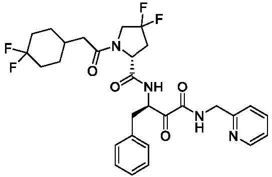 | ¹H NMR (400 MHz, DMSO-*d₆*) *δ* 9.31-9.18 (m, 1H), 8.75-8.42 (m, 2H), 8.03-7.69 (m, 2H), 7.29-7.20(m, 6H), 5.30-5.18 (m, 1H), 4.66-4.58 (m, 1H), 4.45-4.42 (m, 2H), 4.15-3.84 (m, 2H), 3.224-3.11 (m, 1H), 2.92-2.66 (m, 2H), 2.33-2.20 (m, 2H), 2.05-1.61 (m, 8H), 1.24-1.03 (m, 2H). |
| | | [M+H]⁺ =577.2428 |
| **277** | 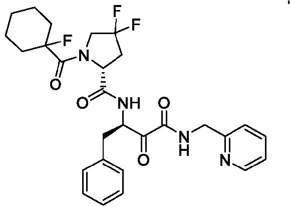 | [M+H]⁺ =545.2 |
| **278** | 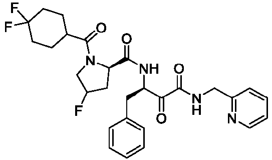 | [M+H]⁺ =545.2 |
| **279** | 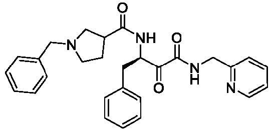 | [M+H]⁺ =471.2 |
| **280** | 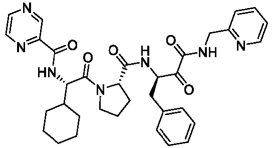 | [M+H]⁺ =626.3 |
| **281** | 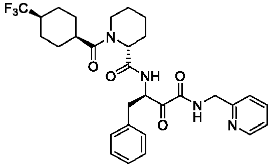 | [M+H]⁺ =573.3 |
| **282** | 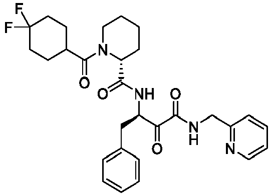 | [M+H]⁺ =541.3 |
| **283** | 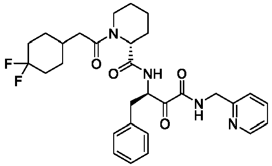 | [M+H]⁺ =555.3 |
| **284** | 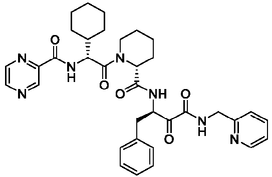 | [M+H]⁺ =640.3 |
| **285** | 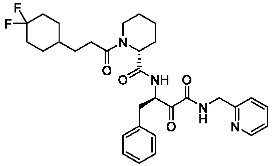 | [M+H]⁺ =569.3 |
| **286** | 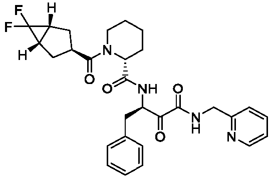 | [M+H]⁺ =539.2 |
| **287** | 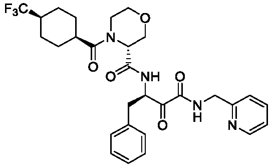 | [M+H]⁺ =575.2 |
| **288** | 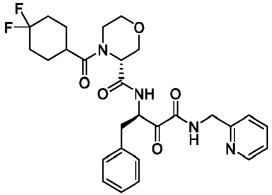 | ¹H NMR (400 MHz, DMSO-*d₆*) *δ* 9.29-9.17 (m, 1H), 8.50 (d, *J* = 3.9 Hz, 1H), 8.29 (t, *J* = 7.9 Hz, 1H), 7.79-7.73 (m, 1H), 7.31-7.20 (m, 7H), 5.27-5.16 (m, 1H), 4.80-4.76 (m, 1H), 4.48-4.38 (m, 2H), 4.24-4.10 (m, 2H), 3.78-3.69 (m, 2H), 3.55-3.42 (m, 2H), 3.18-3.13 (m, 1H), 2.97-2.90 (m, 1H), 2.83-2.78 (m, 1H), 2.06-1.73 (m, 5H), 1.62-1.47 (m, 3H). |
| | | [M+H]⁺ =543.2435 |
| **289** | 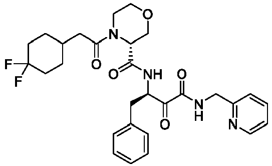 | ¹H NMR (400 MHz, DMSO-d6) *δ* 9.27-9.17 (m, 1H), 8.51-8.29 (m, 2H), 8.02-7.69 (m, 2H), 7.30-7.21 (m, 6H), 5.22-5.14 (m, 1H), 4.77-4.32 (m, 3H), 4.22-3.99 (m, 2H), 3.78-3.39 (m, 3H), 3.27-2.84 (m, 3H), 2.34-2.15 (m, 2H), 1.99-1.91 (m, 2H), 1.83-1.64 (m, 5H), 1.23-1.08 (m, 2H). |
| | | [M+H]⁺ =557.2567 |
| **290** | 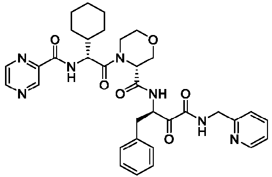 | [M+H]⁺ =642.3 |
| **291** | 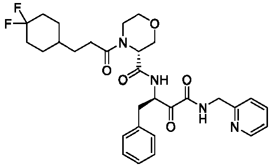 | [M+H]⁺ =571.3 |
| **292** | 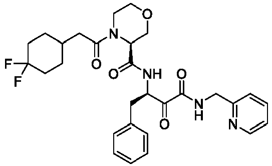 | [M+H]⁺ =557.3 |
| **293** | 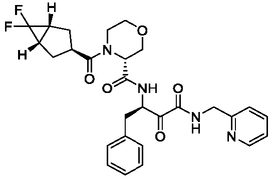 | ¹H NMR (400 MHz, DMSO-*d₆*) *δ* 9.31-9.16 (m, 1H), 8.55-8.50 (m, 1H), 8.32 (d, *J* = 7.0 Hz, 1H), 7.79-7.74 (m, 1H), 7.30-7.18 (m, 7H), 5.32-5.14 (m, 1H), 4.78-4.61 (m, 1H), 4.46-4.38 (m, 2H), 4.24-3.99 (m, 2H), 3.77-3.71 (m, 1H), 3.60-3.39 (m, 2H), 3.23-3.12 (m, 1H), 3.00-2.82 (m, 2H), 2.22-1.90 (m, 7H). |
| | | [M+H]⁺ =541.2261 |
| **294** | 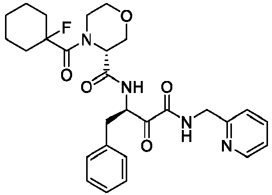 | [M+H]⁺ =525.3 |
| **295** | 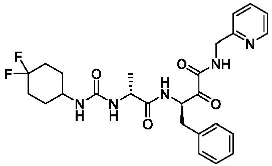 | [M+H]⁺ =516.2 |
| **296** | 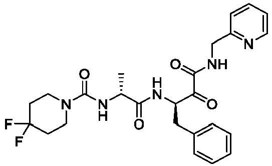 | ¹H NMR (400 MHz, DMSO-*d₆*) *δ* 9.30-9.16 (m, 1H), 8.55-8.49 (m, 1H), 8.50-8.43 (m, 1H), 7.79-7.73 (m, 1H), 7.30-7.17 (m, 7H), 6.76-6.66 (m, 1H), 4.46-4.42 (m, 1H), 4.40-4.35 (m, 1H), 4.22-4.15 (m, 1H), 3.94-3.87 (m, 1H), 3.43-3.41 (m, 4H), 3.16-3.09 (m, 1H), 2.94-2.83 (m, 1H), 1.92-1.83 (m, 4H), 1.17 (d, *J* = 7.2 Hz, 1.5H), 1.04 (d, *J* = 7.2 Hz, 1.5H). |
| | | [M+H]⁺ =502.2 |
| **297** | 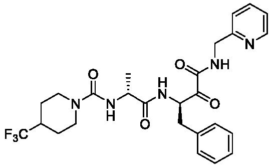 | [M+H]⁺ =534.2 |
| **298** | 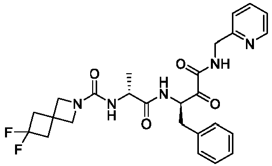 | [M+H]⁺ =514.2 |
| **299** | 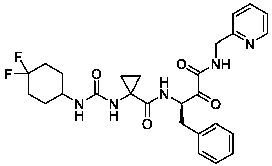 | [M+H]⁺ =528.2 |
| **300** | 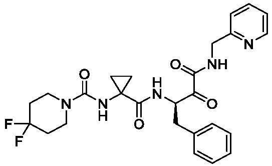 | [M+H]⁺ =514.2 |
| **301** | 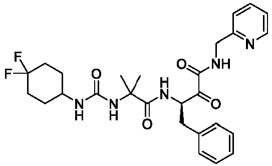 | [M+H]⁺ =530.3 |
| **302** | 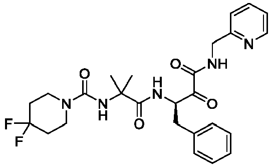 | [M+H]⁺ =516.2 |
| **303** | 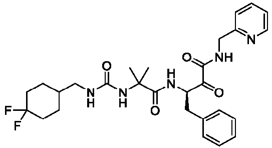 | [M+H]⁺ =544.3 |
| **304** | 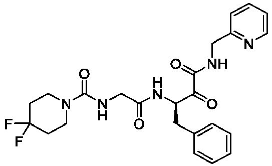 | [M+H]⁺ =488.2 |
| **305** | 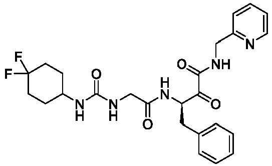 | [M+H]⁺ =502.2 |
| **306** | 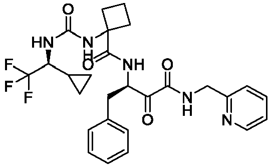 | [M+H]⁺ =546.2 |
| **307** | 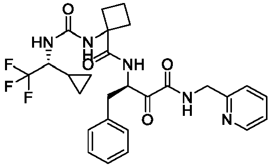 | [M+H]⁺ =546.2 |
| **308** | 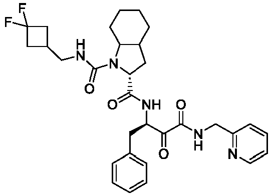 | [M+H]⁺ =582.3 |
| **309** | 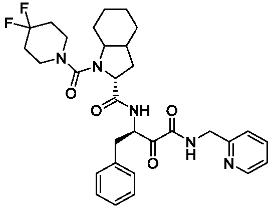 | [M+H]⁺ =582.3 |
| **310** | 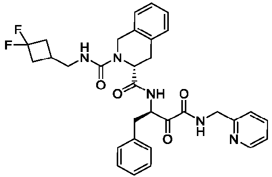 | [M+H]⁺ =590.3 |
| **311** | 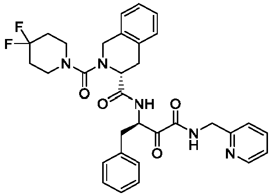 | [M+H]⁺ =590.3 |
| **312** | 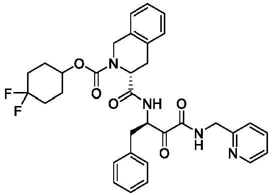 | [M+H]⁺ =605.3 |
| **313** | 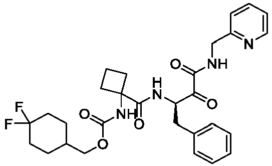 | ¹H NMR (400 MHz, DMSO-*d₆*) *δ* 9.29-9.18 (m, 1H), 8.51 (d, 1H), 7.82-7.73 (m, 1H), 7.75-7.61 (m, 1H), 7.42-7.11 (m, 8H), 5.27-5.10 (m, 1H), 4.46 (d, *J* = 6.2 Hz, 2H), 3.87-3.74 (m, 1H), 3.19-3.10 (m, 1H), 3.02-2.85 (m, 1H), 2.43-2.29 (m, 2H), 2.08-1.96 (m, 3H), 1.86-1.66 (m, 5H), 1.28-1.20 (m, 2H). |
| | | [M+H]⁺ =557.3 |
| **314** | 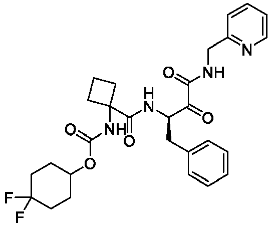 | [M+H]⁺ =543.2 |
| **315** | 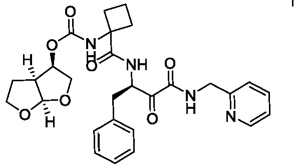 | [M+H]⁺ =537.2 |
| **316** | 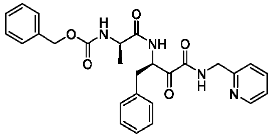 | [M+H]⁺ =489.2 |
| **317** | 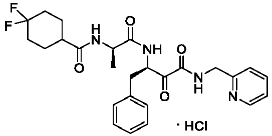 | [M+H]⁺ =501.2 |
| **318** | 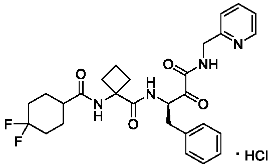 | [M+H]⁺ =527.2 |
| **\319** | 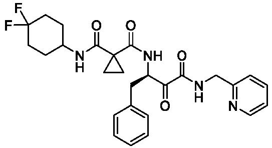 | [M+H]⁺ =513.2 |
| **320** | 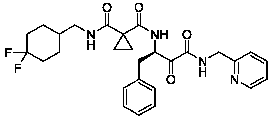 | [M+H]⁺ =527.2 |
| **321** | 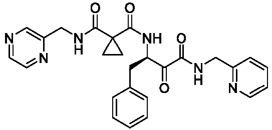 | ¹H NMR (400 MHz, Chloroform-*d*) *δ* 8.60-8.53 (m, 2H), 8.45 (d, *J* = 2.6 Hz, 1H), 8.37-8.31 (m, 1H), 8.07 (q, *J* = 5.8 Hz, 2H), 7.73-7.61 (m, 2H), 7.31-7.27 (m, 2H), 7.26-7.20 (m, 3H), 7.17-7.09 (m, 2H), 5.46-5.37 (m, 1H), 4.68-4.55 (m, 4H), 3.42-3.34 (m, 1H), 3.13-3.02 (m, 1H), 1.52-1.41 (m, 2H), 1.36-1.31 (m, 1H), 1.29-1.24 (m, 1H). |
| | | [M+H]⁺ =487.2 |
| **322** | 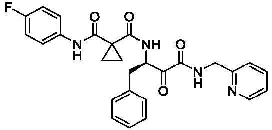 | [M+H]⁺ =489.2 |
| **323** | 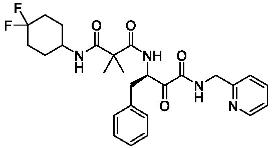 | [M+H]⁺ =515.2 |
| **324** | 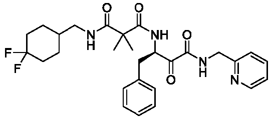 | ¹H NMR (400 MHz, Chloroform-*d*) *δ* 8.62-8.55 (m, 1H), 8.12-8.03 (m, 1H), 7.75-7.66 (m, 1H), 7.31-7.26 (m, 3H), 7.26-7.22 (m, 2H), 7.14-7.08 (m, 2H), 6.86-6.76 (m, 2H), 5.57-5.49 (m, 1H), 4.63 (d, *J* = 5.4 Hz, 2H), 3.41-3.33 (m, 1H), 3.20-3.11 (m, 1H), 3.09-2.98 (m, 2H), 2.07-2.04 (m, 1H), 1.78-1.72 (m, 2H), 1.67-1.49 (m, 4H), 1.40-1.37 (m, 3H), 1.35 (s, 3H), 1.26 (s, 2H). |
| | | [M+H]⁺ =529.3 |
| **325** | 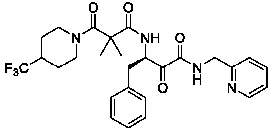 | [M+H]⁺ =533.2 |
| **326** | 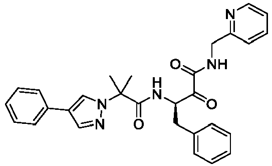 | ¹H NMR (400 MHz, DMSO-*d₆*) *δ* 9.23 (t, *J* = 6.3 Hz, 1H), 8.50 (d, *J* = 4.9 Hz, 1H), 8.26 (s, 1H), 7.97 (s, 1H), 7.76 (t, *J* = 7.9 Hz, 1H), 7.69-7.53 (m, 3H), 7.37 (t, *J* = 7.6 Hz, 2H), 7.32-7.04 (m, 8H), 5.23-5.10 (m, 1H), 4.51-4.37 (m, 2H), 3.18-3.06 (m, 1H), 2.98-2.85 (m, 1H), 1.70 (s, 6H). |
| | | [M+H]⁺ =496.2 |
| **327** | 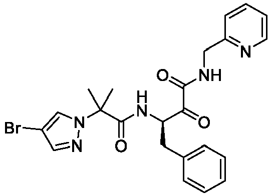 | [M+H]⁺ =498.1 |
| **328** | 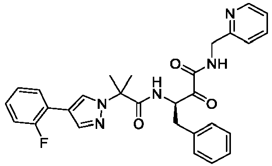 | [M+H]⁺ =514.2 |
| **329** | 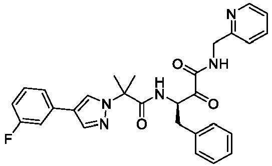 | [M+H]⁺ =514.2 |
| **330** | 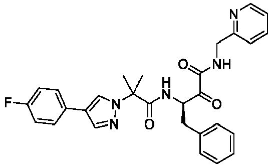 | [M+H]⁺ =514.2 |
| **331** | 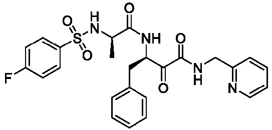 | ¹H NMR (400 MHz, DMSO-*d₆*) *δ* 9.34-9.22 (m, 1H), 8.54-8.47 (m, 1H), 8.45-8.34 (m, 2H), 8.08-7.99 (m, 1H), 7.83-7.73 (m, 2H), 7.72-7.62 (m, 2H), 7.29-7.16 (m, 7H), 5.20-4.92 (m, 1H), 4.44 (d, *J* = 18.7, 6.4 Hz, 2H), 3.94-3.84 (m, 1H), 3.16-3.05 (m, 1H), 2.82-2.65 (m, 1H), 0.97 (d, 3H). |
| | | [M+H]⁺ =513.2 |
| **332** | 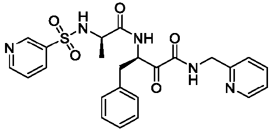 | [M+H]⁺ =496.2 |
| **333** | 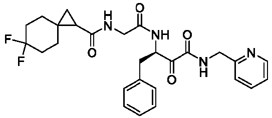 | [M+H]⁺ =513.2 |
| **334** | 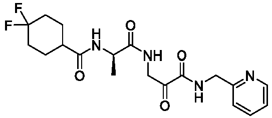 | ¹H NMR (400 MHz, DMSO-*d₆*) *δ* 8.49 (d, *J* = 4.8 Hz, 1H), 8.44 (t, *J =* 6.1 Hz, 1H), 8.17 (d, *J =* 7.7 Hz, 1H), 7.96 (d, *J* = 5.9 Hz, 1H), 7.75 (t, *J* = 7.9 Hz, 1H), 7.34-7.20 (m, 2H), 4.47-4.32 (m, 2H), 4.32-4.19 (m, 1H), 4.05 (dd, *J* = 7.6, 4.1 Hz, 1H), 3.22-3.13 (m, 1H),2.44-2.34 (m, 1H), 2.10-1.93 (m, 2H), 1.92-1.67 (m, 4H), 1.67-1.48 (m, 2H), 1.18 (d, *J* = 7.1 Hz, 3H). |
| | | [M+H]⁺ =411.2 |
| **335** | 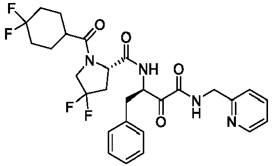 | [M+H]⁺ =563.2 |
| **336** | 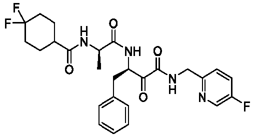 | [M+H]⁺ =519.2 |
| **337** | 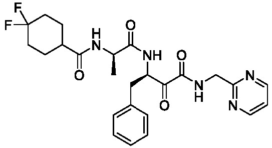 | [M+H]⁺ =502.2 |
| **338** | 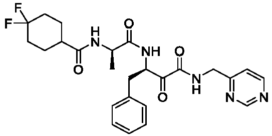 | [M+H]⁺ =502.2 |
| **339** | 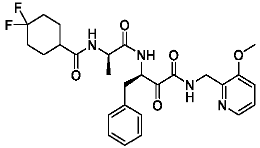 | [M+H]⁺ =531.2 |
| **340** | 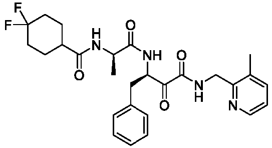 | [M+H]⁺ =515.2 |
| **341** | 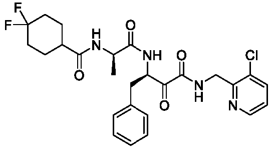 | [M+H]⁺ =535.2 |
| **342** | 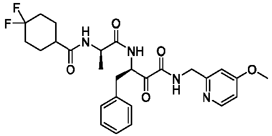 | [M+H]⁺ =531.2 |
| **343** | 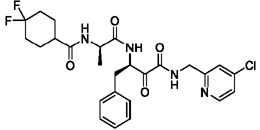 | [M+H]⁺ =535.2 |
| **344** | 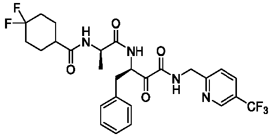 | [M+H]⁺ =569.2 |
| **345** | 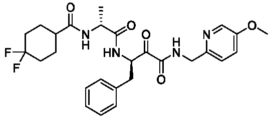 | [M+H]⁺ =531.2 |
| **346** | 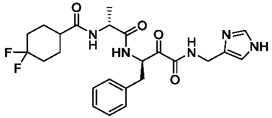 | [M+H]⁺ =490.2 |
| **347** | 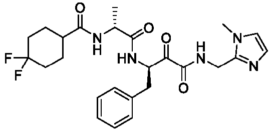 | [M+H]⁺ =504.2 |
| **348** | 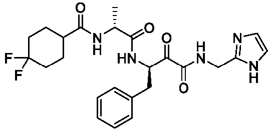 | [M+H]⁺ =490.2 |
| **349** | 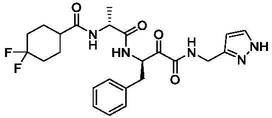 | [M+H]⁺ =490.2 |
| **350** | 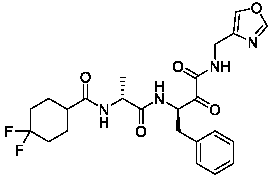 | [M+H]⁺ =491.2 |
| **351** | 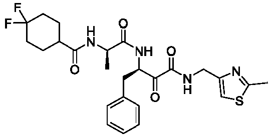 | [M+H]⁺ =521.2 |
| **352** | 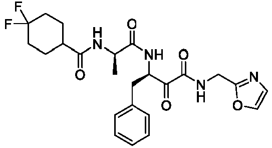 | [M+H]⁺ =491.2 |
| **353** | 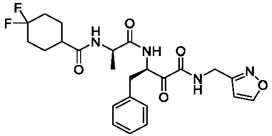 | [M+H]⁺ =491.2 |
| **354** | 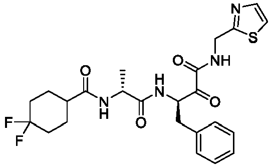 | ¹H NMR (400 MHz, DMSO-*d₆*) *δ* 9.54 (t, *J* = 6.2 Hz, 1H), 8.25 (d, *J* = 6.9 Hz, 1H), 7.94 (d, *J* = 7.7 Hz, 1H), 7.73 (d, *J* = 3.2 Hz, 1H), 7.64 (d, *J* = 3.3 Hz, 1H), 7.33-7.18 (m, 5H), 5.24-5.14 (m, 1H), 4.61 (d, *J =* 6.1 Hz, 2H), 4.37-4.24 (m, 1H), 3.18-3.08 (m, 1H), 2.93-2.81 (m, 1H), 2.35-2.24 (m, 1H), 2.09-1.95 (m, 2H), 1.86-1.66 (m, 4H), 1.62-1.47 (m, 2H), 1.15 (d, *J* = 7.0 Hz, 3H). |
| | | [M+H]⁺ =507.1983 |
| **355** | 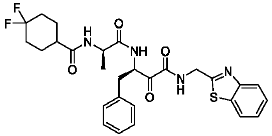 | [M+H]⁺ =557.2 |
| **356** | 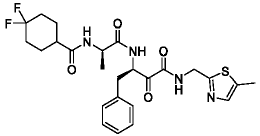 | [M+H]⁺ =521.2 |
| **357** | 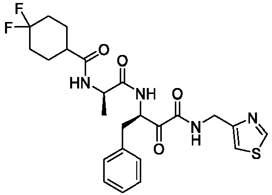 | [M+H]⁺ =507.2 |
| **358** | 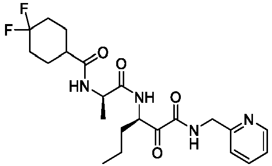 | [M+H]⁺ =453.2 |
| **359** | 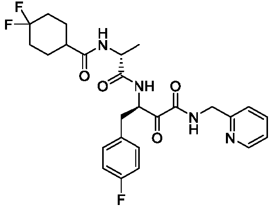 | [M+H]⁺ =519.2 |
| **360** | 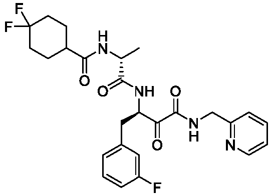 | [M+H]⁺ =519.2 |
| **361** | 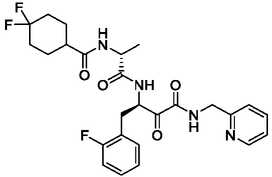 | [M+H]⁺ =519.2 |
| **362** | 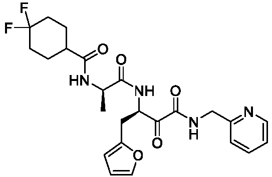 | [M+H]⁺ =491.2 |
| **363** | 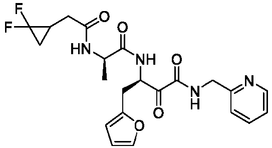 | [M+H]⁺ =463.2 |
| **364** | 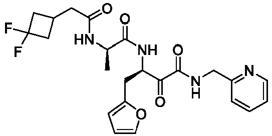 | [M+H]⁺ =477.2 |
| **365** | 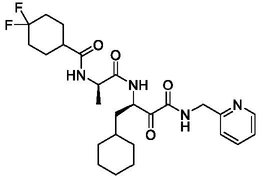 | [M+H]⁺ =507.3 |
| **366** | 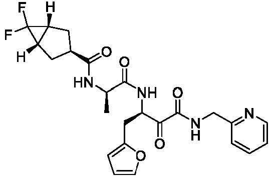 | [M+H]⁺ =489.2 |
| **367** | 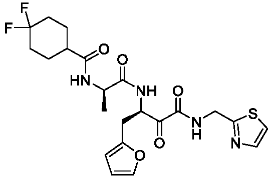 | [M+H]⁺ =497.2 |
| **368** | 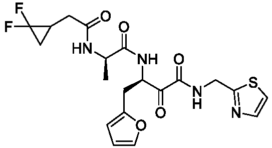 | [M+H]⁺ =469.1 |
| **369** | 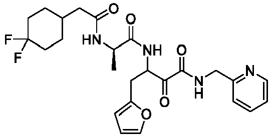 | [M+H]⁺ =457.2 |
| **370** | 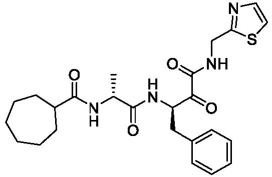 | [M+H]⁺ =485.2 |
| **371** | 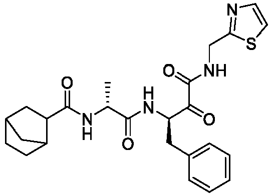 | [M+H]⁺ =483.2 |
| **372** | 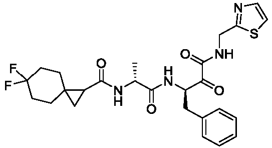 | [M+H]⁺ =533.2 |
| **373** | 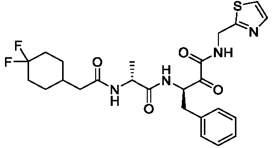 | [M+H]⁺ =521.2 |
| **374** | 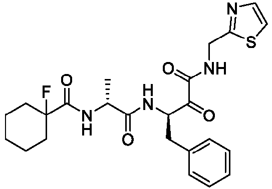 | [M+H]⁺ =489.2 |
| **375** | 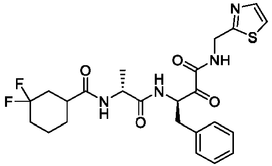 | ¹H NMR (400 MHz, DMSO-*d₆*) *δ* 9.54-9.52 (m, 1H), 8.28-8.23 (m, 1H), 8.05 (t, *J* = 6.1 Hz, 1H), 7.73 (d, *J* = 3.3 Hz, 1H), 7.64 (d, *J* = 3.6 Hz, 1H), 7.29-7.21 (m, 5H), 5.23-5.14 (m, 1H), 4.64-4.60 (m, 2H), 4.31-4.26 (m, 1H), 3.14-3.07 (s, 1H), 2.89-2.83 (m, 1H), 2.40-2.38 (m, 1H), 2.05-1.98 (m, 2H), 1.86-1.62 (m, 4H), 1.44-1.30 (m, 2H), 1.04-1.02 (m, 3H). |
| | | [M+H]⁺ =507.1875 |
| **376** | 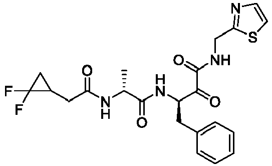 | [M+H]⁺ =479.2 |
| **377** | 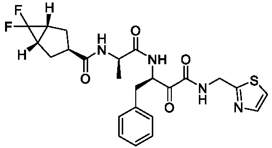 | [M+H]⁺ =505.2 |
| **378** | 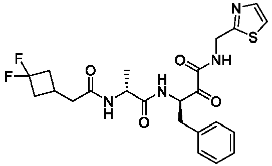 | ¹H NMR (400 MHz, DMSO-*d₆*) *δ* 9.54 (t, *J* = 6.3 Hz, 1H), 8.30 (d, *J* = 7.0 Hz, 1H), 8.01 (d, *J* = 7.5 Hz, 1H), 7.73 (d, *J* = 3.3 Hz, 1H), 7.64 (d, *J* = 3.2 Hz, 1H), 7.30-7.18 (m, 5H), 5.22-5.15 (m, 1H), 4.63-4.57 (m, 2H), 4.36-4.26 (m, 1H), 3.20-3.09 (m, 1H), 2.90-2.81 (m, 1H), 2.68-2.54 (m, 2H), 2.40-2.18 (m, 5H), 1.14 (d, *J* = 7.0 Hz, 3H). |
| | | [M+H]⁺ =493.1716 |
| **379** | 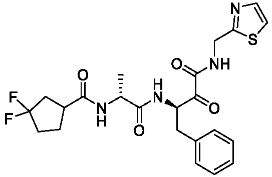 | ¹H NMR (400 MHz, DMSO-*d₆*) *δ* 9.57 (dt, J= 26.9, 5.8 Hz, 1H), 8.37-8.28 (m, 1H), 8.10-8.01 (m, 1H), 7.73 (dt, *J* = 4.0, 1.9 Hz, 1H), 7.64 (dt, *J* = 3.8, 2.0 Hz, 1H), 7.31-7.11 (m, 5H), 5.29-5.12 (m, 1H), 4.68-4.57 (m, 2H), 4.38-4.26 (m, 1H), 3.19-3.06 (m, 1H), 2.99-2.76 (m, 2H), 2.28-1.85 (m, 6H), 1.82-1.68 (m, 1H), 1.09 (dd, *J* = 49.8, 7.0 Hz, 3H). |
| | | [M+H]⁺ =493.1728 |
| **380** | 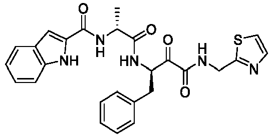 | [M+H]⁺ =504.2 |
| **381** | 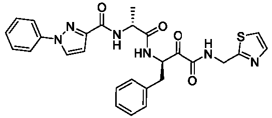 | [M+H]⁺ =531.2 |
| **382** | 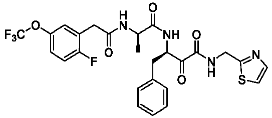 | [M+H]⁺ =581.1 |
| **383** | 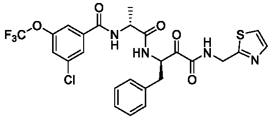 | [M+H]⁺ =583.1 |
| **384** | 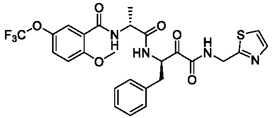 | [M+H]⁺ =579.2 |
| **385** | 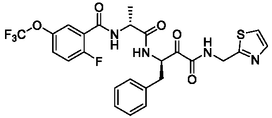 | [M+H]⁺ =567.1 |
| **386** | 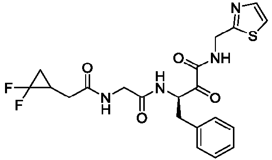 | [M+H]⁺ =465.1 |
| **387** | 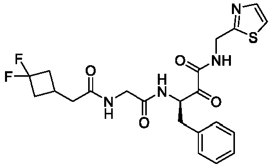 | ¹H NMR (400 MHz, DMSO-*d₆*) *δ* 9.56 (t, J = 6.2 Hz, 1H), 8.31 (d, *J* = 7.2 Hz, 1H), 8.09-8.06 (m, 1H), 7.73 (d, *J* = 3.3 Hz, 1H), 7.64 (d, *J* = 3.1 Hz, 1H), 7.30-7.20 (m, 5H), 5.27-5.23 (m, 1H), 4.67-4.56 (m, 2H), 3.76-3.62 (m, 2H), 3.15-3.10 (m 1H), 2.86-2.80 (m, 1H), 2.65-2.57 (m, 2H), 2.28-2.19 (m, 4H). |
| | | [M+H]⁺ =479.1557 |
| **388** | 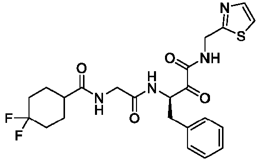 | ¹H NMR (400 MHz, DMSO-*d₆*) *δ* 9.59-9.56 (m, 1H), 8.27 (d, *J* = 7.0 Hz, 1H), 8.04-8.01(m, 1H), 7.73 (d, *J =* 3.4 Hz, 1H), 7.64 (d, *J* = 3.2 Hz, 1H), 7.29-7.20 (m, 5H), 5.26-5.18 (m, 1H), 4.65-4.55 (m, 2H), 3.75-3.63 (m, 2H), 3.15-3.10 (m, 1H), 2.86-2.80 (m, 1H), 2.33-2.30 (m, 1H), 2.02-2.01 (m, 2H), 1.84-1.70 (m, 4H), 1.66-1.56 (m, 2H). |
| | | [M+H]⁺ =493.1717 |
| **389** | 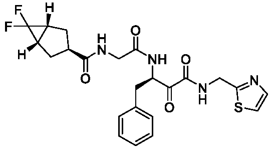 | [M+H]⁺ =491.2 |
| **390** | 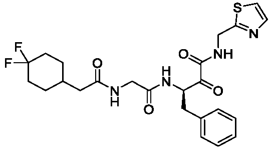 | [M+H]⁺ =507.2 |
| **391** | 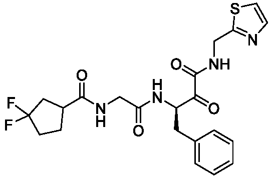 | ¹H NMR (400 MHz, DMSO-*d₆*) *δ* 9.58 (t, *J* = 6.2 Hz, 1H), 8.33 (d, *J =* 7.1 Hz, 1H), 8.16-8.13 (m, 1H), 7.73 (d, *J* = 3.3 Hz, 1H), 7.64 (d, *J* = 3.2 Hz, 1H), 7.30-7.20 (m, 5H), 5.26-5.22 (m, 1H), 4.68-4.57 (m, 2H), 3.77-3.64 (m, 2H), 3.14-3.11 (m, 1H), 2.96-2.80 (m, 2H), 2.26-1.97 (m, 5H), 1.83-1.74 (m, 1H). |
| | | [M+H]⁺ =479.1559 |
| **392** | 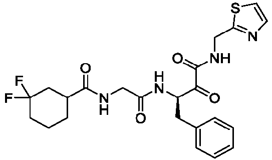 | ¹H NMR (400 MHz, DMSO-*d₆*) *δ* 9.63-9.51 (m, 1H), 8.33-8.24 (m, 1H), 8.20-8.09 (m, 1H), 7.76-7.68 (m, 1H), 7.67-7.58 (m, 1H), 7.34-7.12 (m, 5H), 5.28-5.17 (m, 1H), 4.67-4.55 (m, 2H), 3.77-3.59 (m, 2H), 3.18-3.08 (m, 1H), 2.85 (d, *J* = 11.6 Hz, 1H), 2.46-2.40 (m, 1H), 2.11-1.94 (m, 2H), 1.87-1.64 (m, 4H), 1.36-1.18 (m, 2H). |
| | | [M+H]⁺ =493.1718 |
| **393** | 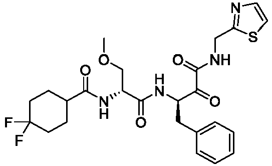 | [M+H]⁺ =537.2 |
| **394** | 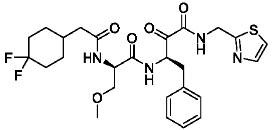 | [M+H]⁺ =551.2 |
| **395** | 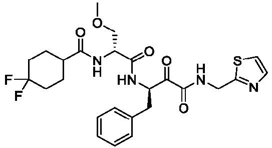 | ¹H NMR (400 MHz, DMSO-*d₆*) *δ* 9.56 (t, *J* = 6.3 Hz, 1H), 8.33 (d, *J* = 7.0 Hz, 1H), 7.99 (d, *J* = 8.2 Hz, 1H), 7.73 (d, *J* = 3.3 Hz, 1H), 7.64 (d, *J* = 3.3 Hz, 1H), 7.31-7.17 (m, 5H), 5.25-5.14 (m, 1H), 4.61 (d, *J* = 6.2 Hz, 2H), 4.55-4.48 (m, 1H), 3.48-3.36 (m, 2H), 3.22 (s, 3H), 3.17-3.09 (m, 1H), 2.93-2.85 (m, 1H), 2.40-2.31 (m, 1H), 2.09-1.97 (m, 2H), 1.84-1.69 (m, 4H), 1.64-1.51 (m, 2H). |
| | | [M+H]⁺ =537.1969 |
| **396** | 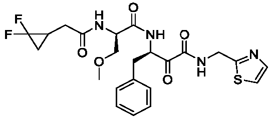 | [M+H]⁺ =509.2 |
| **397** | 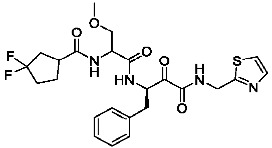 | ¹H NMR (400 MHz, DMSO-*d₆*) *δ* 9.56-9.51 (m, 1H), 8.39 (t, *J* = 7.3 Hz, 1H), 8.11 (d, *J* = 8.4 Hz, 1H), 7.73 (d, *J* = 3.1 Hz, 1H), 7.64 (d, *J* = 3.0 Hz, 1H), 7.27-7.20 (m, 5H), 5.25-5.14 (m, 1H), 4.62-4.51 (m, 3H), 3.45-3.39 (m, 2H), 3.22 (s, 3H), 3.14-3.10 (m, 1H), 3.01-2.84 (m, 2H), 2.26-2.11 (m, 3H), 2.02-1.90 (m, 2H), 1.78-171 (m, 1H). |
| | | [M+H]⁺ =523.1826 |
| **398** | 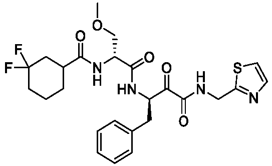 | ¹H NMR (400 MHz, DMSO-*d₆*) *δ* 9.60-9.48 (m, 1H), 8.38-8.28 (m, 1H), 8.12 (d, *J* = 8.1 Hz, 1H), 7.73 (d, *J* = 3.3 Hz, 1H), 7.68-7.62 (m, 1H), 7.30-7.24 (m, 2H), 7.24-7.18 (m, 3H), 5.26-5.13 (m, 1H), 4.67-4.56 (m, 2H), 4.55-4.45 (m, 1H), 3.48-3.37 (m, 2H), 3.22 (d, *J* = 3.1 Hz, 3H), 3.18-3.09 (m, 1H), 2.94-2.85 (m, 1H), 2.06-1.90 (m, 2H), 1.87-1.63 (m, 4H), 1.47-1.35 (m, 1H), 1.33-1.21 (m, 1H). |
| | | [M+H]⁺ =537.1969 |
| **399** | 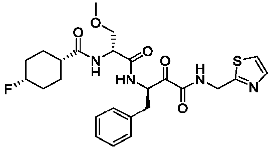 | [M+H]⁺ =519.2 |
| **400** | 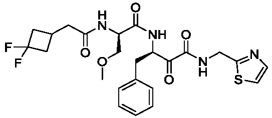 | ¹H NMR (400 MHz, DMSO-*d₆*) *δ* 9.55 (t, *J* = 6.4 Hz, 1H), 8.38 (d, *J* = 7.0 Hz, 1H), 8.07 (d, *J* = 8.2 Hz, 1H), 7.73 (d, *J* = 3.2 Hz, 1H), 7.64 (d, *J* = 3.2 Hz, 1H), 7.26 (d, *J* = 6.9 Hz, 2H), 7.21 (d, *J* = 7.5 Hz, 3H), 5.20 (q, *J* = 6.8, 5.9 Hz, 1H), 4.61 (d, *J* = 6.2 Hz, 2H), 4.53 (q, *J* = 6.7 Hz, 1H), 3.41 (d, *J =* 6.7 Hz, 2H), 3.22 (s, 3H), 3.18-3.09 (m, 1H), 2.92-2.84 (m, 1H), 2.67-2.55 (m, 2H), 2.41-2.20 (m, 5H). |
| | | [M+H]⁺ =523.1873 |
| **401** | 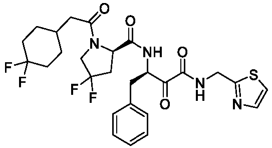 | [M+H]⁺ =583.2 |
| **402** | 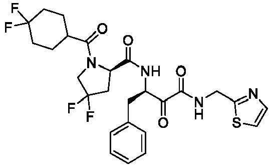 | [M+H]⁺ =569.2 |
| **403** | 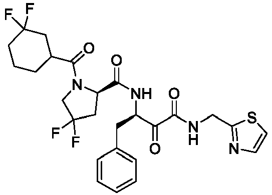 | [M+H]⁺ =569.2 |
| **404** | 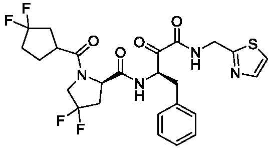 | [M+H]⁺ =555.2 |
| **405** | 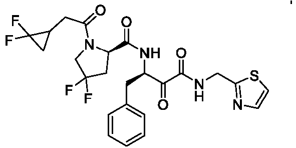 | [M+H]⁺ =541.2 |
| **406** | 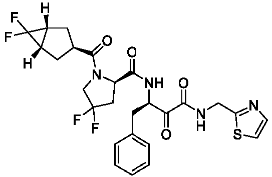 | [M+H]⁺ =567.2 |
| **407** | 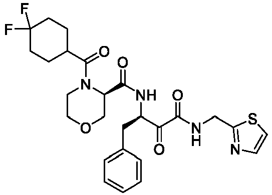 | [M+H]⁺ =549.2 |
| **408** | 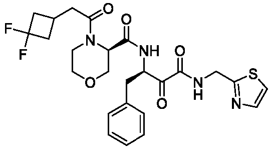 | [M+H]⁺ =535.2 |
| **409** | 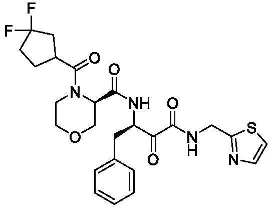 | [M+H]⁺ =535.2 |
| **410** | 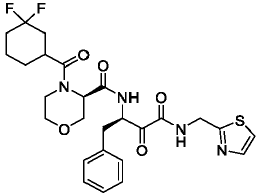 | [M+H]⁺ =549.2 |
| **411** | 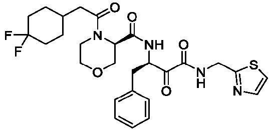 | [M+H]⁺ =563.2 |
| **412** | 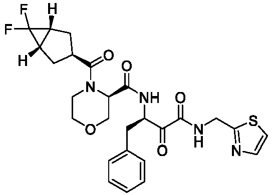 | [M+H]⁺ =547.2 |
| **413** | 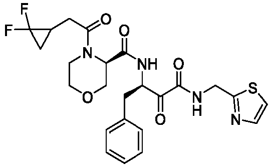 | [M+H]⁺ =521.2 |
| **414** | 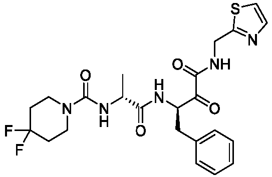 | [M+H]⁺ =508.2 |
| **415** | 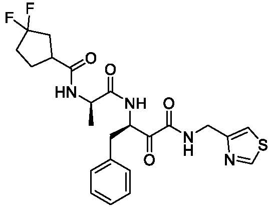 | [M+H]⁺ =493.2 |
| **416** | 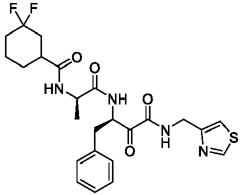 | [M+H]⁺ =507.2 |
| **417** | 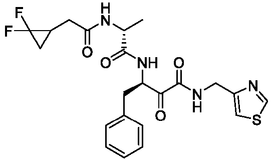 | [M+H]⁺ =479.2 |
| **418** | 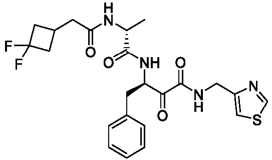 | [M+H]⁺ =493.2 |
| **419** | 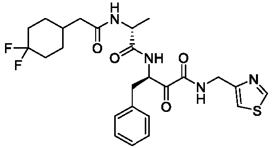 | [M+H]⁺ =521.2 |
| **420** | 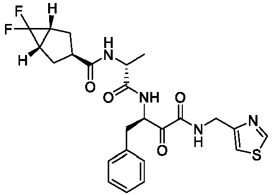 | [M+H]⁺ =505.2 |
| **421** | 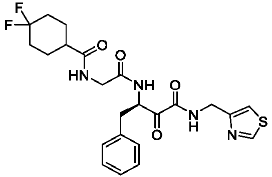 | [M+H]⁺ =493.2 |
| **422** | 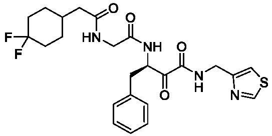 | [M+H]⁺ =507.2 |
| **423** | 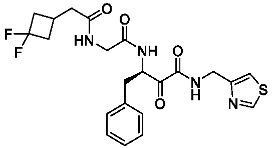 | [M+H]⁺ =479.2 |
| **424** | 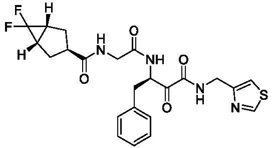 | [M+H]⁺ =491.2 |
| **425** | 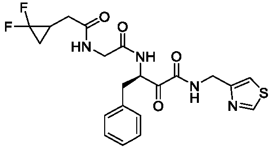 | [M+H]⁺ =465.1 |
| **426** | 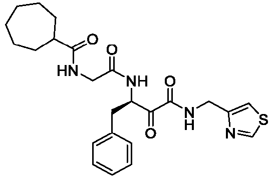 | [M+H]⁺ =471.2 |
| **427** | 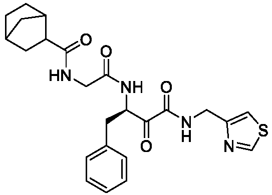 | [M+H]⁺ =469.2 |
| **428** | 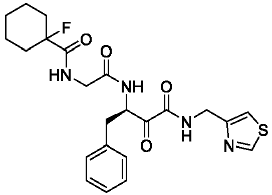 | [M+H]⁺ =475.2 |
| **429** | 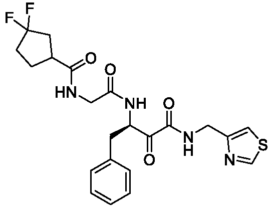 | [M+H]⁺ =479.2 |
| **430** | 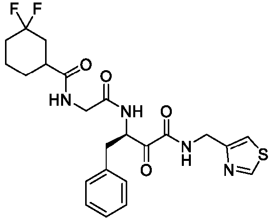 | [M+H]⁺ =493.2 |
| **431** | 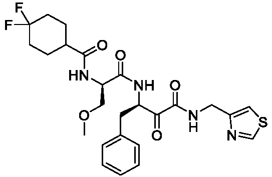 | [M+H]⁺ =537.2 |
| **432** | 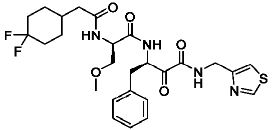 | [M+H]⁺ =551.2 |
| **433** | 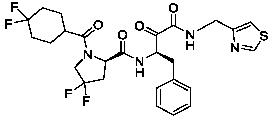 | [M+H]⁺ =569.2 |
| **434** | 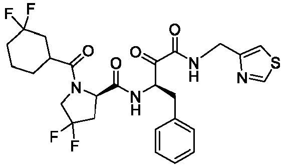 | [M+H]⁺ =569.2 |
| **435** | 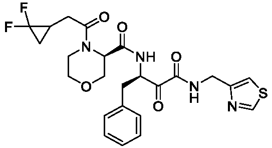 | [M+H]⁺ =521.2 |
| **436** | 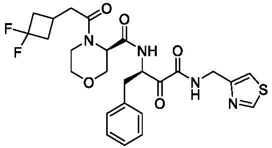 | [M+H]⁺ =535.2 |
| **437** | 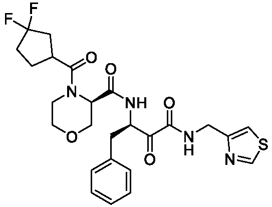 | [M+H]⁺ =535.2 |
| **438** | 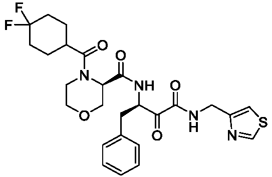 | [M+H]⁺ =549.2 |
| **439** | 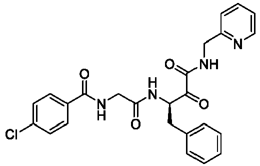 | ¹H NMR (400 MHz, DMSO-*d₆*) *δ* 9.25 (t, *J* = 6.2 Hz, 1H), 8.80 (t, *J* = 5.9 Hz, 1H), 8.54-8.47 (m, 1H), 8.44 (d, *J* = 7.1 Hz, 1H), 7.93-7.82 (m, 2H), 7.80-7.71 (m, 1H), 7.60-7.51 (m, 2H), 7.34-7.16 (m, 7H), 5.32-5.22 (m, 1H), 4.45 (d, *J* = 6.2 Hz, 2H), 3.99-3.81 (m, 2H), 3.20-3.09 (m, 1H), 2.92-2.81 (m, 1H). |
| | | [M+H] ⁺=479.1480 |
| **440** | 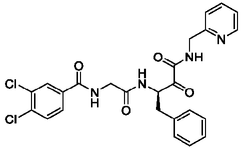 | ¹H NMR (400 MHz, DMSO-*d₆*) *δ* 9.25 (t, *J* = 6.2 Hz, 1H), 8.93 (t, *J* = 5.9 Hz, 1H), 8.50 (d, *J* = 4.8 Hz, 1H), 8.47 (d, *J* = 7.1 Hz, 1H), 8.10 (d, *J* = 1.9 Hz, 1H), 7.87-7.82 (m, 1H), 7.81-7.72 (m, 2H), 7.31-7.18 (m, 7H), 5.32-5.22 (m, 1H), 4.45 (d, *J =* 6.1 Hz, 2H), 3.98-3.83 (m, 2H), 3.20-3.10 (m, 1H), 2.91-2.82 (m, 1H). |
| | | [M+H] ⁺=513.1091 |
| **441** | 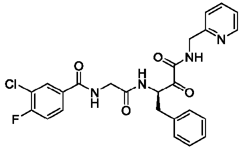 | ¹H NMR (400 MHz, DMSO-*d₆*) *δ* 9.25 (t, *J* = 6.2 Hz, 1H), 8.90 (t, *J* = 6.0 Hz, 1H), 8.49 (dd, *J* = 12.9, 6.0 Hz, 2H), 7.85 (d, *J* = 10.3 Hz, 1H), 7.80-7.71 (m, 3H), 7.32-7.17 (m, 7H), 5.31-5.23 (m, 1H), 4.49-4.39 (m, 2H), 3.99-3.85 (m, 2H), 3.19-3.11 (m, 1H), 2.91-2.82 (m, 1H). |
| | | [M+H] ⁺=497.1385 |
| **442** | 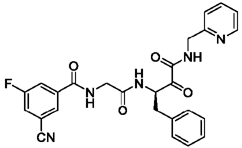 | ¹H NMR (400 MHz, DMSO-*d₆*) *δ* 9.25 (t, *J* = 6.2 Hz, 1H), 9.03 (t, *J* = 5.9 Hz, 1H), 8.55-8.47 (m, 2H), 8.15 (d, *J =* 1.5 Hz, 1H), 8.11-8.06 (m, 1H), 8.03-7.97 (m, 1H), 7.79-7.73 (m, 1H), 7.29-7.19 (m, 7H), 5.32-5.22 (m, 1H), 4.45 (d, *J* = 6.2 Hz, 2H), 4.00-3.86 (m, 2H), 3.20-3.12 (m, 1H), 2.91-2.80 (m, 1H). |
| | | [M+H] ⁺=488.1734 |
| **443** | 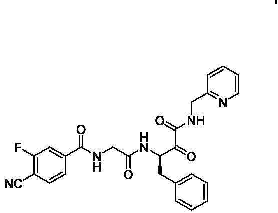 | ¹H NMR (400 MHz, DMSO-*d₆*) *δ* 9.25 (t, *J* = 6.2 Hz, 1H), 9.11 (t, *J* = 6.0 Hz, 1H), 8.59-8.46 (m, 2H), 8.08 (dd, *J* = 8.1, 6.6 Hz, 1H), 7.92 (dd, *J* = 10.3, 1.5 Hz, 1H), 7.86 (dd, *J* = 8.1, 1.5 Hz, 1H), 7.76 (td, *J* = 7.7, 1.9 Hz, 1H), 7.30-7.19 (m, 7H), 5.31-5.23 (m, 1H), 4.44 (d, *J* = 6.1 Hz, 2H), 4.01-3.85 (m, 2H), 3.19-3.09 (m, 1H), 2.90-2.80 (m, 1H). |
| | | [M+H] ⁺=488.1725 |
| **444** | 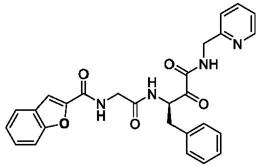 | ¹H NMR (400 MHz, DMSO-*d₆*) *δ* 9.26 (t, *J* = 6.2 Hz, 1H), 8.83 (t, *J* = 6.0 Hz, 1H), 8.54-8.47 (m, 2H), 7.82-7.72 (m, 2H), 7.67 (d, *J* = 8.3 Hz, 1H), 7.57 (s, 1H), 7.52-7.44 (m, 1H), 7.35 (t, *J* = 7.5 Hz, 1H), 7.31-7.18 (m, 7H), 5.35-5.25 (m, 1H), 4.45 (d, *J* = 6.2 Hz, 2H), 4.02-3.83 (m, 2H), 3.20-3.12 (m, 1H), 2.93-2.83 (m, 1H). |
| | | [M+H] ⁺=485.1816 |
| **445** | 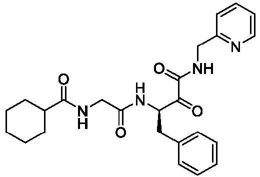 | ¹H NMR (400 MHz, DMSO-*d₆*) *δ* 9.25 (t, *J* = 6.2 Hz, 1H), 8.54-8.46 (m, 1H), 8.21 (d, *J* = 7.1 Hz, 1H), 7.88 (t, *J* = 5.8 Hz, 1H), 7.81-7.71 (m, 1H), 7.31-7.17 (m, 7H), 5.29-5.21 (m, 1H), 4.44 (d, *J* = 6.2 Hz, 2H), 3.74-3.58 (m, 2H), 3.17-3.10 (m, 1H), 2.90-2.80 (m, 1H), 2.19-2.09 (m, 1H), 1.72-1.64 (m, 4H), 1.62-1.57 (m, 1H), 1.34-1.11 (m, 6H). |
| | | [M+H] ⁺ = 451.2341 |
| **446** | 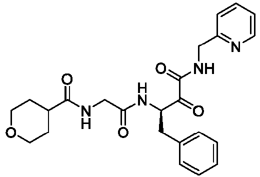 | ¹H NMR (400 MHz, DMSO-*d₆*) *δ* 9.31-9.21 (m, 8H), 8.50 (d, *J* = 4.9 Hz, 1H), 8.28 (d, *J* = 7.1 Hz, 1H), 7.99 (t, *J* = 5.9 Hz, 1H), 7.81-7.73 (m, 1H), 7.31-7.25 (m, 3H), 7.25-7.18 (m, 4H), 5.30-5.21 (m, 1H), 4.44 (d, *J* = 6.2 Hz, 2H), 3.83 (dt, *J* = 11.6, 3.3 Hz, 2H), 3.77-3.60 (m, 2H), 3.31-3.24 (m, 2H), 3.17-3.09 (m, 1H), 2.88-2.80 (m, 1H), 2.46-2.34 (m, 1H), 1.67-1.45 (m, 4H). |
| | | [M+H] ⁺=453.2135 |
| **447** | 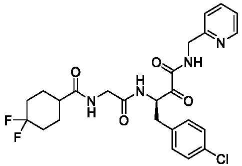 | ¹H NMR (400 MHz, DMSO-*d₆*) *δ* 9.25 (t, *J* = 6.2 Hz, 1H), 8.50 (d, *J* = 5.0 Hz, 1H), 8.32 (d, *J* = 7.2 Hz, 1H), 8.04 (t, *J* = 5.8 Hz, 1H), 7.81-7.70 (m, 1H), 7.37-7.31 (m, 2H), 7.29-7.09 (m, 4H), 5.30-5.14 (m, 1H), 4.50-4.38 (m, 2H), 3.78-3.59 (m, 2H), 3.17-3.07 (m, 1H), 2.87-2.78 (m, 1H), 2.35-2.26 (m, 1H), 2.06-1.95 (m, 2H), 1.86-1.70 (m, 4H), 1.62-1.53 (m, 2H). |
| | | [M+H] ⁺=521.1776 |
| **448** | 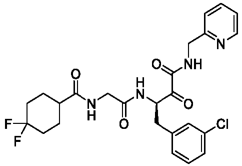 | ¹H NMR (400 MHz, DMSO-*d₆*) *δ* 9.26 (t, *J* = 6.1 Hz, 1H), 8.50 (d, *J* = 5.0 Hz, 1H), 8.36 (d, *J* = 7.2 Hz, 1H), 8.04 (t, *J* = 5.8 Hz, 1H), 7.81-7.72 (m, 1H), 7.34-7.18 (m, 6H), 5.26-5.18 (m, 1H), 4.44 (d, *J* = 6.1 Hz, 2H), 3.76-3.61 (m, 2H), 3.18-3.11 (m, 1H), 2.88-2.80 (m, 1H), 2.31 (t, *J* = 12.0 Hz, 1H), 2.09-1.95 (m, 2H), 1.87-1.67 (m, 4H), 1.64-1.49 (m, 2H). |
| | | [M+H] ⁺=521.1775 |
| **449** | 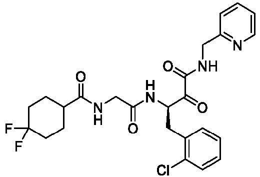 | ¹H NMR (400 MHz, DMSO-*d₆*) *δ* 9.26-9.17 (m, 1H), 8.50 (t, *J* = 3.8 Hz, 1H), 8.43-8.35 (m, 1H), 8.11-8.00 (m, 1H), 7.81-7.72 (m, 1H), 7.44-7.38 (m, 1H), 7.34-7.25 (m, 4H), 7.23-7.19 (m, 1H), 5.35-5.25 (m, 1H), 4.44-4.33 (m, 2H), 3.74-3.61 (m, 2H), 3.31-3.23 (m, 1H), 3.05-2.91 (m, 1H), 2.36-2.24 (m, 1H), 2.10-1.92 (m, 2H), 1.83-1.66 (m, 4H), 1.65-1.45 (m, 2H). |
| | | [M+H] ⁺=521.1767 |
| **450** | 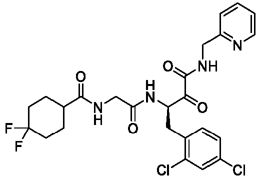 | ¹H NMR (400 MHz, DMSO-*d₆*) *δ* 9.21 (t, *J* = 6.2 Hz, 1H), 8.49 (d, *J* = 4.2 Hz, 1H), 8.39 (d, *J* = 7.3 Hz, 1H), 8.03 (t, *J* = 5.8 Hz, 1H), 7.76 (td, *J* = 7.7, 1.8 Hz, 1H), 7.57 (d, *J* = 1.5 Hz, 1H), 7.34-7.21 (m, 4H), 5.29-5.21 (m, 1H), 4.44-4.36 (m, 2H), 3.69-3.63 (m, 2H), 3.30-3.24 (m, 1H), 3.00-2.92 (m, 1H), 2.38-2.23 (m, 1H), 2.07-1.98 (m, 2H), 1.78 (d, *J* = 29.5 Hz, 4H), 1.64-1.53 (m, 2H). |
| | | [M+H] ⁺=555.1367 |
| **451** | 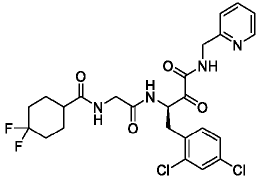 | ¹H NMR (400 MHz, DMSO-*d₆*) *δ* 9.26 (t, *J* = 6.2 Hz, 1H), 8.50 (d, *J* = 5.0 Hz, 1H), 8.36 (d, *J* = 7.2 Hz, 1H), 8.04 (t, *J* = 5.8 Hz, 1H), 7.80-7.73 (m, 1H), 7.53 (d, *J* = 8.3 Hz, 1H), 7.49 (d, *J* = 2.0 Hz, 1H), 7.30-7.26 (m, 1H), 7.25-7.19 (m, 2H), 5.26-5.14 (m, 1H), 4.49-4.39 (m, 2H), 3.75-3.62 (m, 2H), 3.18-3.10 (m, 1H), 2.90-2.79 (m, 1H), 2.35-2.27 (m, 1H), 2.07-1.97 (m, 2H), 1.82-1.71 (m, 4H), 1.62-1.55 (m, 2H). |
| | | [M+H] ⁺=555.1371 |
| **452** | 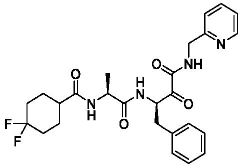 | ¹H NMR (400 MHz, DMSO-*d₆*) *δ* 9.28 (t, *J* = 6.2 Hz, 1H), 8.51 (d, *J* = 5.1 Hz, 1H), 8.29 (d, *J* = 7.6 Hz, 1H), 7.92 (d, *J* = 8.0 Hz, 1H), 7.79-7.75 (m, 1H), 7.32-7.16 (m, 7H), 5.31-5.22 (m, 1H), 4.46-4.45 (m, 2H), 4.32-4.27 (m, 1H), 3.19-3.14 (m, 1H), 2.89-2.75 (m, 1H), 2.32-2.28 (m, 1H), 2.09-1.95 (m, 2H), 1.86-1.64 (m, 4H), 1.60-1.48(m, 2H), 1.03 (d, J = 7.1 Hz, 3H). |
| | | [M+H] ⁺=501.2306 |
| **453** | 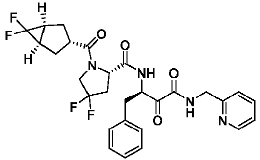 | ¹H NMR (400 MHz, DMSO-*d₆*) *δ* 9.37-9.15 (m, 1H), 8.75-8.40 (m, 2H), 8.08-7.68 (m, 2H), 7.40-7.15 (m, 6H), 5.37-5.11 (m, 1H), 4.80-4.52 (m, 1H), 4.50-4.38 (m, 2H), 4.19-3.60 (m, 2H), 3.23-3.08 (m, 1H), 2.99-2.61 (m, 3H), 2.35-1.77 (m, 7H). |
| | | [M+H] ⁺=561.2113 |
| **454** | 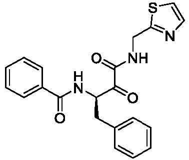 | ¹H NMR (400 MHz, DMSO-*d₆*) *δ* 9.61 (t, *J* = 6.2 Hz, 1H), 8.93 (d, *J* = 7.2 Hz, 1H), 7.80 (d, *J* = 7.5 Hz, 2H), 7.72 (t, *J* = 2.8 Hz, 1H), 7.61 (t, *J* = 2.7 Hz, 1H), 7.58-7.52 (m, 1H), 7.48 (d, *J =* 7.4 Hz, 2H), 7.38-7.27 (m, 4H), 7.22 (t, *J* = 7.8 Hz, 1H), 5.42-5.31 (m, 1H), 4.69-4.60 (m, 2H), 3.29-3.21 (m, 1H), 3.08-2.95 (m, 1H). |
| | | [M+H] ⁺=394.1212 |
| **455** | 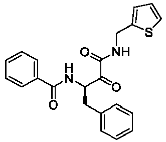 | ¹H NMR (400 MHz, DMSO-*d₆*) *δ* 9.38 (t, *J* = 6.4 Hz, 1H), 8.90 (d, *J* = 7.3 Hz, 1H), 7.78 (d, *J* = 7.6 Hz, 2H), 7.54 (t, *J* = 7.4 Hz, 1H), 7.46 (t, *J* = 7.6 Hz, 2H), 7.38-7.26 (m, 5H), 7.20 (t, *J* = 7.3 Hz, 1H), 6.99-6.96 (m, 1H), 6.93 (d, *J* = 4.4 Hz, 1H), 5.41-5.31 (m, 1H), 4.55-4.47 (m, 2H), 3.27-3.19 (m, 1H), 3.02-2.93 (m, 1H). |
| | | [M+H] ⁺=393.1262 |
| **456** | 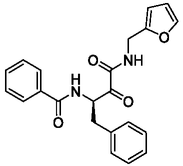 | ¹H NMR (400 MHz, DMSO-*d₆*) *δ* 9.21 (t, *J* = 6.1 Hz, 1H), 8.89 (d, *J* = 7.2 Hz, 1H), 7.78 (d, *J* = 7.6 Hz, 2H), 7.57-7.51 (m, 2H), 7.46 (t, *J* = 7.7 Hz, 2H), 7.36-7.27 (m, 4H), 7.23-7.18 (m, 1H), 6.39-6.35 (m, 1H), 6.23 (d, *J* = 3.1 Hz, 1H), 5.38-5.30 (m, 1H), 4.38-4.29 (m, 2H), 3.22 (dd, *J* = 14.3, 3.6 Hz, 1H), 3.01-2.92 (m, 1H). |
| | | [M+H] ⁺=377.1494 |
| **457** | 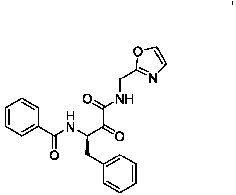 | ¹H NMR (400 MHz, DMSO-*d₆*) *δ* 9.39 (t, *J* = 6.2 Hz, 1H), 8.89 (d, *J* = 7.4 Hz, 1H), 8.02-7.97 (m, 1H), 7.78 (d, *J* = 7.7 Hz, 2H), 7.58-7.50 (m, 1H), 7.46 (t, *J* = 7.5 Hz, 2H), 7.37-7.25 (m, 4H), 7.20 (t, *J* = 7.1 Hz, 1H), 7.17-7.10 (m, 1H), 5.34 (d, *J* = 9.5 Hz, 1H), 4.52-4.42 (m, 2H), 3.28-3.19 (m, 1H), 3.03-2.90 (m, 1H). |
| | | [M+H] ⁺=378.1445 |
| **458** | 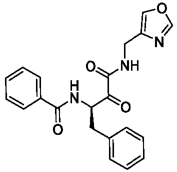 | ¹H NMR (400 MHz, DMSO-*d₆*) *δ* 9.15 (t, *J* = 6.0 Hz, 1H), 8.89 (d, *J* = 7.2 Hz, 1H), 8.30 (s, 1H), 7.87 (s, 1H), 7.78 (dt, *J* = 7.1, 1.4 Hz, 2H), 7.57-7.50 (m, 1H), 7.46 (dd, *J* = 8.4, 7.0 Hz, 2H), 7.36-7.24 (m, 4H), 7.24-7.17 (m, 1H), 5.41-5.30 (m, 1H), 4.31-4.22 (m, 2H), 3.26-3.20 (m, 1H), 3.01-2.91 (m, 1H). |
| | | [M+H] ⁺=378.1444 |
| **459** | 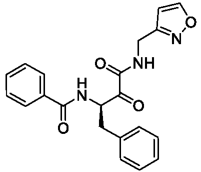 | ¹H NMR (400 MHz, DMSO-*d₆*) *δ* 9.34 (t, *J* = 6.2 Hz, 1H), 8.92 (d, *J* = 7.1 Hz, 1H), 8.81 (d, *J* = 1.8 Hz, 1H), 7.81-7.74 (m, 2H), 7.58-7.49 (m, 1H), 7.50-7.39 (m, 2H), 7.37-7.24 (m, 4H), 7.25-7.16 (m, 1H), 6.42 (t, *J* = 1.9 Hz, 1H), 5.38-5.26 (m, 1H), 4.45-4.37 (m, 2H), 3.27-3.17 (m, 1H), 3.03-2.91 (m, 1H). |
| | | [M+H] ⁺=378.1447 |
| **460** | 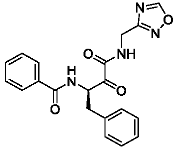 | ¹H NMR (400 MHz, DMSO-*d₆*) *δ* 9.56 (s, 1H), 9.43 (t, *J* = 5.9 Hz, 1H), 8.88 (d, *J* = 7.3 Hz, 1H), 7.79 (d, *J =* 7.6 Hz, 2H), 7.54 (t, *J* = 7.3 Hz, 1H), 7.46 (t, *J* = 7.5 Hz, 2H), 7.37-7.27 (m, 4H), 7.21 (t, *J* = 7.1 Hz, 1H), 5.42-5.34 (m, 1H), 4.56-4.52 (m, 5H), 3.28-3.20 (m, 1H), 3.01-2.92 (m, 1H). |
| | | [M+H] ⁺=379.1399 |
| **461** | 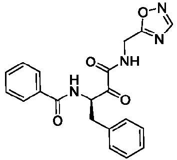 | ¹H NMR (400 MHz, DMSO-*d₆*) *δ* 9.55 (t, *J* = 6.0 Hz, 1H), 8.94 (s, 1H), 8.92 (d, *J* = 7.1 Hz, 1H), 7.81-7.76 (m, 2H), 7.54 (td, *J* = 7.2, 1.5 Hz, 1H), 7.49-7.44 (m, 2H), 7.36-7.26 (m, 4H), 7.23-7.18 (m, 1H), 5.37-5.29 (m, 1H), 4.72-4.65 (m, 2H), 3.27-3.20 (m, 1H), 3.02-2.94 (m, 1H). |
| | | [M+H] ⁺=379.1396 |
| **462** | 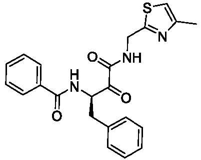 | ¹H NMR (400 MHz, DMSO-*d₆*) *δ* 9.22 (t, *J* = 6.2 Hz, 1H), 8.93 (d, *J* = 7.1 Hz, 1H), 7.85-7.76 (m, 2H), 7.54 (dd, *J* = 8.3, 6.4 Hz, 1H), 7.46 (t, *J* = 7.5 Hz, 2H), 7.31 (dt, *J* = 14.8, 7.4 Hz, 4H), 7.20 (t, *J* = 7.0 Hz, 1H), 7.10 (s, 1H), 5.38-5.28 (m, 1H), 4.48-4.31 (m, 2H), 3.29-3.21 (m, 1H), 3.04-2.93 (m, 1H), 2.60 (s, 3H). |
| | | [M+H] ⁺=408.1371 |
| **463** | 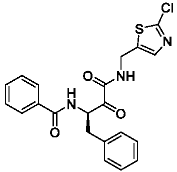 | ¹H NMR (400 MHz, DMSO-*d₆*) *δ* 9.42 (t, *J* = 6.1 Hz, 1H), 8.97 (d, *J* = 6.9 Hz, 1H), 7.76 (d, *J* = 7.6 Hz, 2H), 7.53 (d, *J* = 7.3 Hz, 2H), 7.45 (t, *J* = 7.5 Hz, 2H), 7.36-7.25 (m, 4H), 7.24-7.15 (m, 3H), 5.28-5.20 (m, 1H), 4.51-4.38 (m, 2H), 3.22 (dd, *J* = 13.8, 4.4 Hz, 1H), 3.07-2.95 (m, 1H). |
| | | [M+H] ⁺=428.0826 |
| **464** | 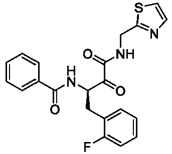 | ¹H NMR (400 MHz, DMSO-*d₆*) *δ* 9.53 (t, *J* = 6.3 Hz, 1H), 8.98 (d, *J* = 7.0 Hz, 1H), 7.76 (d, *J* = 7.6 Hz, 2H), 7.69 (d, *J* = 3.3 Hz, 1H), 7.58 (d, *J* = 3.3 Hz, 1H), 7.54 (t, *J* = 7.5 Hz, 1H), 7.45 (t, *J* = 7.6 Hz, 2H), 7.37 (t, *J =* 7.7 Hz, 1H), 7.26 (t, *J* = 6.9 Hz, 1H), 7.17-7.08 (m, 2H), 5.34-5.24 (m, 1H), 4.64-4.53 (m, 2H), 3.30-3.26 (m, 1H), 3.11-3.03 (m, 1H). |
| | | [M+H] ⁺=412.1132 |
| **465** | 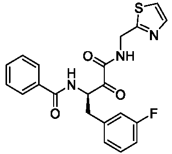 | ¹H NMR (400 MHz, DMSO-*d₆*) *δ* 9.59 (t, *J* = 6.2 Hz, 1H), 8.97 (d, *J =* 7.1 Hz, 1H), 7.82-7.75 (m, 2H), 7.71 (d, *J* = 3.2 Hz, 1H), 7.60 (d, *J* = 3.1 Hz, 1H), 7.57-7.52 (m, 1H), 7.50-7.44 (m, 2H), 7.37-7.30 (m, 1H), 7.19-7.12 (m, 2H), 7.08-7.01 (m, 1H), 5.37-5.26 (m, 1H), 4.68-4.59 (m, 2H), 3.31-3.23 (m, 1H), 3.09-2.98 (m, 1H). |
| | | [M+H] ⁺=412.1123 |
| **466** | 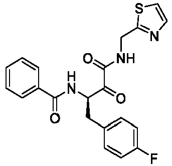 | ¹H NMR (400 MHz, DMSO-*d₆*) *δ* 9.58 (t, *J* = 6.2 Hz, 1H), 8.93 (d, *J* = 7.1 Hz, 1H), 7.80-7.76 (m, 2H), 7.71 (d, *J* = 3.3 Hz, 1H), 7.60 (d, *J* = 3.2 Hz, 1H), 7.56-7.52 (m, 1H), 7.46 (dd, *J =* 8.1, 6.6 Hz, 2H), 7.37-7.32 (m, 2H), 7.15-7.09 (m, 2H), 5.34-5.25 (m, 1H), 4.63 (d, *J* = 6.3 Hz, 2H), 3.26-3.18 (m, 1H), 3.02-2.93 (m, 1H). |
| | | [M+H] ⁺=412.1131 |
| **467** | 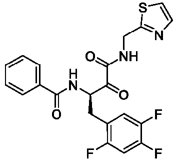 | ¹H NMR (400 MHz, DMSO-*d₆*) *δ* 9.56 (t, *J* = 6.2 Hz, 1H), 9.08 (d, *J* = 6.8 Hz, 1H), 7.83-7.77 (m, 2H), 7.74-7.72 (m, 1H), 7.63-7.57 (m, 2H), 7.54-7.48 (m, 4H), 5.29-5.21 (m, 1H), 4.67-4.58 (m, 2H), 3.33-3.21 (m, 1H), 3.17-3.07 (m, 1H). |
| | | [M+H] ⁺=448.0934 |
| **468** | 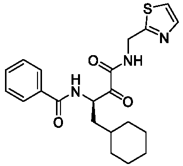 | ¹H NMR (400 MHz, DMSO-*d₆*) *δ* 9.55 (t, *J* = 6.1 Hz, 1H), 8.76 (d, *J* = 6.8 Hz, 1H), 7.88 (d, *J* = 7.6 Hz, 2H), 7.72 (d, *J* = 3.2 Hz, 1H), 7.63 (d, *J* = 3.2 Hz, 1H), 7.56 (t, *J* = 7.5 Hz, 1H), 7.48 (t, *J* = 7.5 Hz, 2H), 5.31-5.23 (m, 1H), 4.63 (d, *J* = 6.2 Hz, 2H), 1.85-1.77 (m, 1H), 1.72-1.59 (m, 6H), 1.50-1.44 (m, 1H), 1.18-1.08 (m, 2H), 1.01-0.84 (m, 3H). |
| | | [M+H] ⁺=400.1691 |
| **469** | 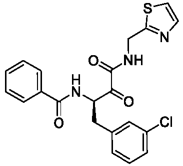 | ¹H NMR (400 MHz, DMSO-*d₆*) *δ* 9.59 (t, *J* = 6.2 Hz, 1H), 8.97 (d, *J =* 7.1 Hz, 1H), 7.81-7.75 (m, 2H), 7.71 (d, *J* = 3.3 Hz, 1H), 7.60 (d, *J* = 3.3 Hz, 1H), 7.57-7.52 (m, 1H), 7.47 (dd, *J* = 8.2, 6.7 Hz, 2H), 7.39 (t, *J* = 1.8 Hz, 1H), 7.35-7.30 (m, 1H), 7.29-7.24 (m, 2H), 5.32-5.26 (m, 1H), 4.62 (d, *J =* 6.3 Hz, 2H), 3.27-3.21 (m, 1H), 3.05-2.97 (m, 1H). |
| | | [M+H] ⁺=428.0833 |
| **470** | 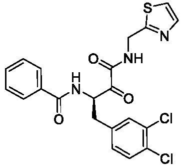 | ¹H NMR (400 MHz, DMSO-*d₆*) *δ* 9.57 (t, *J* = 6.3 Hz, 1H), 8.99 (d, *J* = 7.1 Hz, 1H), 7.81-7.75 (m, 2H), 7.72-7.69 (m, 1H), 7.61-7.52 (m, 4H), 7.47 (t, *J* = 8.0 Hz, 2H), 7.29 (dd, *J* = 8.3, 2.0 Hz, 1H), 5.30-5.22 (m, 1H), 4.61 (d, *J* = 6.2 Hz, 2H), 3.26-3.19 (m, 1H), 3.05-2.97 (m, 1H). |
| | | [M+H] ⁺=462.0449 |
| **471** | 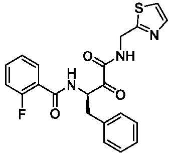 | ¹H NMR (400 MHz, DMSO-*d₆*) *δ* 9.64 (t, *J* = 6.3 Hz, 1H), 8.75 (dd, *J* = 7.3, 2.4 Hz, 1H), 7.74 (d, *J* = 3.3 Hz, 1H), 7.64 (d, *J =* 3.0 Hz, 1H), 7.58-7.47 (m, 2H), 7.34-7.20 (m, 7H), 5.44-5.34 (m, 1H), 4.66 (d, *J* = 6.3 Hz, 2H), 3.29-3.18 (m, 1H), 3.01-2.89 (m, 1H). |
| | | [M+H] ⁺=412.1119 |
| **472** | 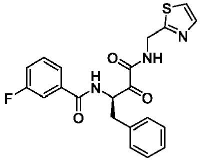 | ¹H NMR (400 MHz, DMSO-*d*₆) *δ* 9.61 (t, *J* = 6.4 Hz, 1H), 9.03 (d, *J =* 7.2 Hz, 1H), 7.72 (d, *J =* 3.3 Hz, 1H), 7.67-7.51 (m, 4H), 7.44-7.37 (m, 1H), 7.34-7.26 (m, 4H), 7.23-7.17 (m, 1H), 5.40-5.29 (m, 1H), 4.64 (d, *J =* 6.3 Hz, 2H), 3.27-3.19 (m, 1H), 3.05-2.92 (m, 1H). |
| | | [M+H] ⁺=412.1121 |
| **473** | 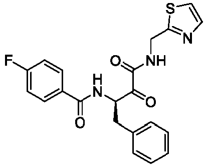 | ¹H NMR (400 MHz, DMSO-*d*₆) *δ* 9.60 (t, *J* = 6.2 Hz, 1H), 8.97 (d, *J =* 7.1 Hz, 1H), 7.90-7.83 (m, 2H), 7.71 (d, *J* = 3.3 Hz, 1H), 7.61 (d, *J* = 3.3 Hz, 1H), 7.36-7.26 (m, 6H), 7.24-7.18 (m, 1H), 5.37-5.29 (m, 1H), 4.70-4.60 (m, 2H), 3.27-3.20 (m, 1H), 3.04-2.92 (m, 1H). |
| | | [M+H] ⁺=412.1122 |
| **474** | 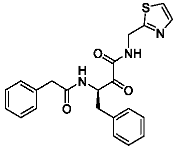 | ¹H NMR (400 MHz, DMSO-*d*₆) *δ* 9.55 (t, *J* = 6.2 Hz, 1H), 8.60 (d, *J =* 7.1 Hz, 1H), 7.73 (d, *J =* 3.3 Hz, 1H), 7.64 (d, *J* = 3.3 Hz, 1H), 7.36-7.11 (m, 10H), 5.22-5.11 (m, 1H), 4.64-4.52 (m, 2H), 3.43 (s, 2H), 3.20-3.10 (m, 1H), 2.92-2.74 (m, 1H). |
| | | [M+H] ⁺=408.1372 |
| **475** | 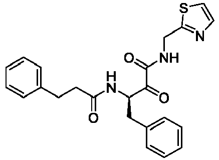 | ¹H NMR (400 MHz, DMSO-*d*₆) *δ* 9.54 (t, *J* = 6.3 Hz, 1H), 8.40 (d, *J =* 7.1 Hz, 1H), 7.73 (d, *J =* 3.2 Hz, 1H), 7.64 (d, *J =* 3.4 Hz, 1H), 7.32-7.08 (m, 10H), 5.24-5.11 (m, 1H), 4.62 (d, *J* = 6.2 Hz, 2H), 3.11 (dd, *J* = 14.0, 4.3 Hz, 1H), 2.83-2.76 (m, 1H), 2.72 (t, *J*= 8.1 Hz, 2H), 2.38 (t, *J =* 7.9 Hz, 2H). |
| | | [M+H] ⁺=422.1528 |
| **476** | 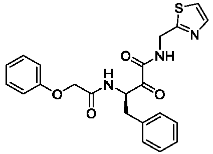 | ¹H NMR (400 MHz, DMSO-*d*₆) *δ* 9.62 (t, *J* = 6.3 Hz, 1H), 8.49 (d, *J =* 7.5 Hz, 1H), 7.72 (d, *J =* 3.3 Hz, 1H), 7.61 (d, *J =* 3.3 Hz, 1H), 7.39-7.09 (m, 7H), 6.96 (t, *J =* 7.3 Hz, 1H), 6.88 (d, *J* = 8.1 Hz, 2H), 5.35-5.20 (m, 1H), 4.64 (d, *J =* 6.3 Hz, 2H), 4.48 (s, 2H), 3.23-3.13 (m, 1H), 3.02-2.87 (m, 1H). |
| | | [M+H] ⁺=424.1321 |
| **477** | 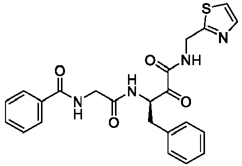 | ¹H NMR (400 MHz, DMSO-*d*₆) *δ* 9.57 (t, *J* = 6.0 Hz, 1H), 8.68 (t, *J =* 5.8 Hz, 1H), 8.40 (d, *J =* 7.1 Hz, 1H), 7.89-7.82 (m, 2H), 7.73 (d, *J* = 3.2 Hz, 1H), 7.63 (d, *J* = 3.2 Hz, 1H), 7.58-7.50 (m, 1H), 7.49-7.44 (m, 2H), 7.29-7.16 (m, 8H), 5.30-5.21 (m, 1H), 4.66-4.60 (m, 2H), 4.00-3.81 (m, 2H), 3.19-3.10 (m, 1H), 2.92-2.82 (m, 1H). |
| | | [M+H] ⁺=451.1432 |
| **478** | 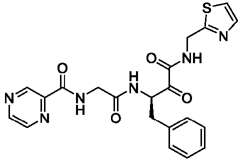 | ¹H NMR (400 MHz, DMSO-*d*₆) *δ* 9.58 (t, *J* = 5.9 Hz, 1H), 9.19 (s, 1H), 9.00-8.87 (m, 2H), 8.75 (s, 1H), 8.51 (d, *J* = 7.1 Hz, 1H), 7.72 (t, *J* = 2.3 Hz, 1H), 7.62 (dd, *J =* 3.2, 1.6 Hz, 1H), 7.32-7.16 (m, 5H), 5.30-5.20 (m, 1H), 4.62 (d, *J =* 6.2 Hz, 2H), 4.06-3.90 (m, 2H), 3.17-3.08 (m, 1H), 2.93-2.79 (m, 1H). |
| | | [M+H] ⁺=453.1338 |
| **479** | 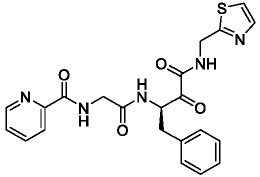 | ¹H NMR (400 MHz, DMSO-*d*₆) *δ* 9.58 (t, *J* = 5.9 Hz, 1H), 8.81 (d, *J =* 5.4 Hz, 1H), 8.65 (d, *J =* 4.3 Hz, 1H), 8.51 (d, *J =* 6.8 Hz, 1H), 8.09-7.95 (m, 2H), 7.72 (d, *J* = 3.0 Hz, 1H), 7.64-7.60 (m, 2H), 7.22 (q, *J =* 7.6, 5.7 Hz, 5H), 5.24 (d, *J =* 4.0 Hz, 1H), 4.62 (d, *J =* 6.1 Hz, 2H), 4.08-3.87 (m, 2H), 3.18-3.10 (m, 1H), 2.92-2.82 (m, 1H). |
| | | [M+H] ⁺=452.1384 |
| **480** | 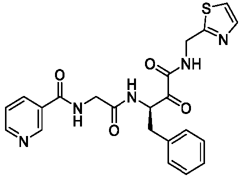 | ¹H NMR (400 MHz, DMSO-*d*₆) *δ* 9.58 (t, *J* = 5.6 Hz, 1H), 9.04-8.98 (m, 1H), 8.96-8.89 (m, 1H), 8.74-8.69 (m, 2H), 8.46 (d, *J =* 6.5 Hz, 1H), 8.19 (dd, *J =* 5.2, 2.7 Hz, 1H), 7.73 (t, *J* = 2.9 Hz, 1H), 7.63 (t, *J* = 2.9 Hz, 1H), 7.52 (dd, *J =* 7.8, 4.9 Hz, 2H), 7.30-7.14 (m, 5H), 5.31-5.21 (m, 1H), 4.68-4.61 (m, 2H), 3.96-3.89 (m, 2H), 3.18-3.09 (m, 1H), 2.92-2.79 (m, 1H). |
| | | [M+H] ⁺=452.1387 |
| **481** | 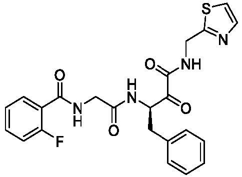 | ¹H NMR (400 MHz, DMSO-*d*₆) *δ* 9.59 (t, *J* = 5.5 Hz, 1H), 8.43 (d, *J =* 7.1 Hz, 1H), 8.41-8.32 (m, 1H), 7.73 (d, *J* = 2.8 Hz, 1H), 7.69 (t, *J* = 7.5 Hz, 1H), 7.63 (d, *J* = 2.8 Hz, 1H), 7.59-7.52 (m, 1H), 7.33-7.19 (m, 7H), 5.31-5.22 (m, 1H), 4.66-4.60 (m, 2H), 4.00-3.82 (m, 2H), 3.20-3.10 (m, 1H), 2.92-2.81 (m, 1H). |
| | | [M+H] ⁺=469.1341 |
| **482** | 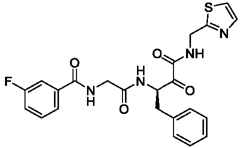 | ¹H NMR (400 MHz, DMSO-*d*₆) *δ* 9.57 (t, *J* = 6.0 Hz, 1H), 8.72 (t, *J* = 5.7 Hz, 1H), 8.41 (d, *J* = 7.0 Hz, 1H), 8.00-7.89 (m, 2H), 7.73 (d, *J* = 3.2 Hz, 1H), 7.63 (d, *J* = 3.2 Hz, 1H), 7.31 (t, *J* = 8.7 Hz, 2H), 7.27-7.11 (m, 5H), 5.30-5.16 (m, 1H), 4.62 (d, *J =* 6.3 Hz, 2H), 3.97-3.81 (m, 2H), 3.18-3.07 (m, 1H), 2.91-2.80 (m, 1H). |
| | | [M+H] ⁺=469.1342 |
| **483** | 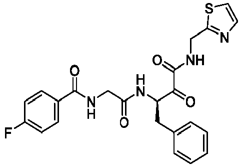 | ¹H NMR (400 MHz, DMSO-*d*₆) *δ* 9.58 (t, *J* = 5.7 Hz, 1H), 8.81 (t, *J =* 5.3 Hz, 1H), 8.43 (d, *J =* 6.9 Hz, 1H), 7.75-7.68 (m, 2H), 7.65 (t, 2H), 7.54 (q, *J* = 7.1 Hz, 1H), 7.40 (t, *J =* 8.0 Hz, 1H), 7.29-7.18 (m, 5H), 5.31-5.21 (m, 1H), 4.63 (d, *J* = 5.5 Hz, 2H), 4.00-3.83 (m, 2H), 3.18-3.11 (m, 1H), 2.93-2.79 (m, 1H). |
| | | [M+H] ⁺=469.1339 |
| **484** | 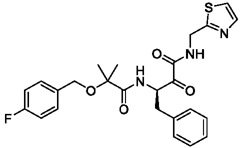 | ¹H NMR (400 MHz, DMSO-*d*₆) *δ* 9.61 (t, *J* = 6.2 Hz, 1H), 7.92 (d, *J =* 7.7 Hz, 1H), 7.72 (d, *J =* 1.9 Hz, 1H), 7.65-7.64 (m, 1H), 7.33-7.27 (m, 3H), 7.22-7.15 (m, 6H), 5.34-5.24 (m, 1H), 4.66-4.62 (m, 2H), 4.31-4.23 (m, 2H), 3.22-3.16 (m, 1H), 3.05-2.97 (m, 1H), 1.30 (s, 3H), 1.24 (s, 3H). |
| | | [M+H] ⁺=484.2 |
| **485** | 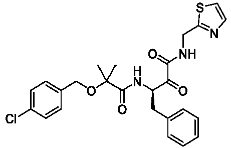 | ¹H NMR (400 MHz, DMSO-*d*₆) *δ* 9.61 (t, *J* = 6.2 Hz, 1H), 7.93 (d, *J =* 7.7 Hz, 1H), 7.73 (d, *J =* 3.3 Hz, 1H), 7.65 (d, *J* = 3.3 Hz, 1H), 7.41-7.35 (m, 2H), 7.32-7.27 (m, 2H), 7.25-7.16 (m, 5H), 5.32-5.23 (m, 1H), 4.66-4.61 (m, 2H), 4.31-4.22 (m, 2H), 3.23-3.17 (m, 1H), 3.05-2.97 (m, 1H), 1.27 (d, *J =* 24.3 Hz, 6H). |
| | | [M+H] ⁺=501.0 |
| **486** | 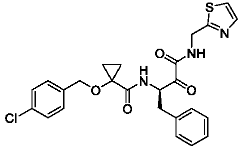 | ¹H NMR (400 MHz, DMSO-*d*₆) *δ* 9.61 (t, *J* = 6.2 Hz, 1H), 8.23 (d, *J =* 7.6 Hz, 1H), 7.75-7.70 (m, 1H), 7.66-7.60 (m, 1H), 7.44-7.38 (m, 3H), 7.33-7.19 (m, 6H), 6.01-5.85 (m, 1H), 4.71-4.60 (m, 2H), 4.57-4.45 (m, 2H), 3.26-3.14 (m, 1H), 3.03-2.89 (m, 1H), 1.58-1.43 (m, 4H). |
| | | [M+H] ⁺=514.1 |
| **487** | 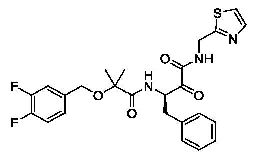 | ¹H NMR (400 MHz, DMSO-*d*₆) *δ* 9.60 (t, *J* = 6.2 Hz, 1H), 8.01 (d, *J =* 7.3 Hz, 2H), 7.72 (d, *J =* 2.7 Hz, 1H), 7.67-7.64 (m, 2H), 7.58 (d, *J =* 3.2 Hz, 1H), 7.40-7.33 (m, 2H), 7.24-7.15 (m, 5H), 5.31-5.20 (m, 1H), 4.64-4.58 (m, 2H), 4.32-4.19 (m, 2H), 3.25-3.16 (m, 1H), 3.06-2.98 (m, 1H), 1.23 (s, 3H), 1.23 (s, 3H). |
| | | [M+H] ⁺=518.6 |
| **488** | 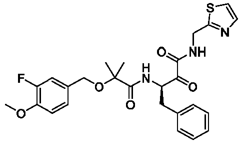 | ¹H NMR (400 MHz, DMSO-*d*₆) *δ* 9.61 (t, *J* = 6.3 Hz, 1H), 7.95 (d, *J =* 7.7 Hz, 1H), 7.73 (t, *J =* 3.4 Hz, 1H), 7.65 (d, *J =* 3.3 Hz, 1H), 7.22-7.16 (m, 8H), 5.36-5.23 (m, 1H), 4.64 (d, *J* = 6.3 Hz, 2H), 4.28-4.19 (m, 2H), 3.82 (s, 3H), 3.20-3.13 (m, 1H), 3.02 (dd, *J =* 13.9, 8.8 Hz, 1H), 1.31 (s, 3H), 1.25 (s, 3H). |
| | | [M+H] ⁺=530.6 |
| **489** | 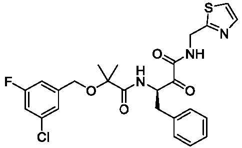 | ¹H NMR (400 MHz, DMSO-*d*₆) *δ* 9.60 (t, *J* = 6.2 Hz, 1H), 8.10-7.99 (m, 1H), 7.78-7.71 (m, 1H), 7.67-7.63 (m, 1H), 7.36-7.28 (m, 2H), 7.25-7.16 (m, 5H), 5.28-5.16 (m, 1H), 4.66-4.54 (m, 2H), 4.22-4.05 (m, 1H), 3.23-3.10 (m, 0H), 2.86-2.73 (m, 0H), 1.23 (d, *J =* 2.4 Hz, 4H). |
| | | [M+H] ⁺=551.1 |
| **490** | 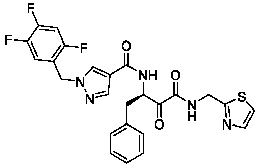 | ¹H NMR (400 MHz, DMSO-*d*₆) *δ* 9.56 (t, *J* = 6.2 Hz, 1H), 8.56 (d, *J* = 7.2 Hz, 1H), 8.25 (s, 1H), 7.88 (s, 1H), 7.71 (d, *J =* 3.3 Hz, 1H), 7.65-7.60 (m, 1H), 7.58 (d, *J* = 3.2 Hz, 1H), 7.52-7.45 (m, 1H), 7.31-7.14 (m, 5H), 5.38 (s, 2H), 5.32-5.25 (m, 1H), 4.62 (d, *J* = 6.2 Hz, 2H), 3.23-3.15 (m, 1H), 2.94-2.85 (m, 1H). |
| | | [M+H] ⁺=528.1 |
| **491** | 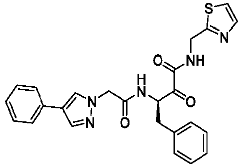 | ¹H NMR (400 MHz, DMSO-*d*₆) *δ* 9.61 (t, *J* = 6.2 Hz, 1H), 8.69 (d, *J =* 7.1 Hz, 1H), 8.05 (s, 1H), 7.88 (s, 1H), 7.73 (d, *J =* 3.2 Hz, 1H), 7.63 (d, *J =* 3.3 Hz, 1H), 7.56-7.54 (m, 2H), 7.39-7.33 (m, 3H), 7.31-7.27 (m, 2H), 7.25-7.21 (m, 3H), 5.33-5.24 (m, 1H), 4.86 (s, 2H), 4.65-4.59 (m, 2H), 3.19-3.15 (m, 1H), 2.91-2.84 (m, 1H). |
| | | [M+H] ⁺=474.5 |
| **492** | 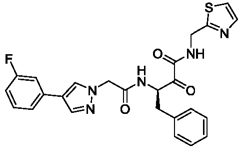 | ¹H NMR (400 MHz, DMSO-*d*₆) *δ* 9.59 (t, *J* = 6.2 Hz, 1H), 8.67 (d, *J =* 7.1 Hz, 1H), 8.12 (d, *J =* 0.8 Hz, 1H), 7.95 (d, *J* = 0.8 Hz, 1H), 7.72 (d, *J* = 3.2 Hz, 1H), 7.63 (d, *J =* 3.3 Hz, 1H), 7.46-7.36 (m, 3H), 7.33-7.26 (m, 2H), 7.25-7.18 (m, 3H), 7.04-6.97 (m, 1H), 5.34-5.25 (m, 1H), 4.85 (d, *J* = 1.8 Hz, 2H), 4.71-4.54 (m, 2H), 3.22-3.12 (m, 1H), 2.94-2.81 (m, 1H). |
| | | [M+H] ⁺=492.1 |
| **493** | 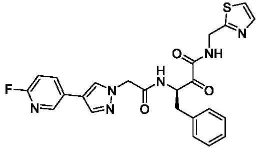 | ¹H NMR (400 MHz, DMSO-*d*₆) *δ* 9.60 (t, *J* = 6.3 Hz, 1H), 8.72 (d, *J =* 7.1 Hz, 1H), 8.47 (d, *J =* 2.4 Hz, 1H), 8.19-8.12 (m, 2H), 7.98 (s, 1H), 7.73 (d, *J* = 3.3 Hz, 1H), 7.63 (d, *J =* 3.3 Hz, 1H), 7.34-7.26 (m, 2H), 7.25-7.13 (m, 4H), 5.32-5.25 (m, 1H), 4.88 (s, 1H), 4.67-4.58 (m, 2H), 3.21-3.14 (m, 1H), 2.93-2.81 (m, 1H). |
| | | [M+H] ⁺=493.1 |
| **494** | 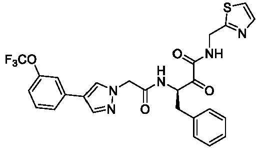 | ¹H NMR (400 MHz, DMSO-*d*₆) *δ* 9.61 (t, *J* = 6.3 Hz, 1H), 8.71 (d, *J =* 7.1 Hz, 1H), 8.19 (s, 1H), 7.99 (s, 1H), 7.73 (d, *J* = 3.3 Hz, 1H), 7.63 (d, *J* = 3.2 Hz, 1H), 7.62-7.54 (m, 2H), 7.48 (t, *J* = 8.0 Hz, 1H), 7.32-7.26 (m, 2H), 7.25-7.21 (m, 3H), 7.20-7.16 (m, 2H), 5.32-5.22 (m, 1H), 4.86 (d, *J =* 1.7 Hz, 2H), 4.62 (dd, *J =* 6.3, 2.3 Hz, 2H), 3.20-3.12 (m, 1H), 2.92-2.83 (m, 1H). |
| | | [M+H] ⁺=558.1 |
| **495** | 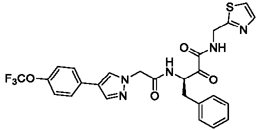 | ¹H NMR (400 MHz, DMSO-*d*₆) *δ* 9.61 (t, *J* = 6.2 Hz, 1H), 8.70 (d, *J =* 7.1 Hz, 1H), 8.10 (s, 1H), 7.92 (s, 1H), 7.73 (d, *J =* 3.3 Hz, 1H), 7.70-7.65 (m, 2H), 7.63 (d, *J* = 3.3 Hz, 1H), 7.38-7.33 (m, 2H), 7.31-7.26 (m, 2H), 7.26-7.19 (m, 3H), 5.33-5.25 (m, 1H), 4.86 (d, *J =* 1.8 Hz, 2H), 4.67-4.57 (m, 2H), 3.21-3.14 (m, 1H), 2.91-2.85 (m, 1H). |
| | | [M+H] ⁺=558.1 |
| **496** | 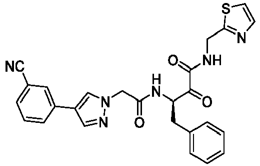 | ¹H NMR (400 MHz, DMSO-*d*₆) *δ* 9.61 (t, *J* = 6.2 Hz, 1H), 8.75 (d, *J =* 7.1 Hz, 1H), 8.21 (s, 1H), 8.10-8.07 (m, 1H), 8.03 (s, 1H), 7.93-7.89 (m, 1H), 7.73 (d, *J =* 3.2 Hz, 1H), 7.65-7.60 (m, 2H), 7.56 (t, *J* = 7.8 Hz, 1H), 7.32-7.27 (m, 2H), 7.26-7.19 (m, 3H), 5.33-5.25 (m, 1H), 4.87 (s, 2H), 4.63 (dd, *J* = 6.2, 2.2 Hz, 2H), 3.22-3.13 (m, 1H), 2.92-2.84 (m, 1H). |
| | | [M+H] ⁺=499.1 |
| **497** | 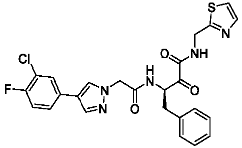 | ¹H NMR (400 MHz, DMSO-*d*₆) *δ* 9.61 (t, *J* = 6.3 Hz, 1H), 8.71 (d, *J =* 7.1 Hz, 1H), 8.13 (s, 1H), 7.99-7.94 (m, 1H), 7.81 (dd, *J* = 7.1, 2.2 Hz, 1H), 7.73 (d, *J* = 3.2 Hz, 1H), 7.64 (d, *J* = 3.3 Hz, 1H), 7.59-7.55 (m, 1H), 7.40 (t, *J =* 9.0 Hz, 1H), 7.32-7.26 (m, 2H), 7.25-7.19 (m, 3H), 5.32-5.24 (m, 1H), 4.85 (d, *J* = 1.9 Hz, 2H), 4.66-4.58 (m, 2H), 3.22-3.13 (m, 1H), 2.91-2.82 (m, 1H). |
| | | [M+H] ⁺=526.1 |
| **498** | 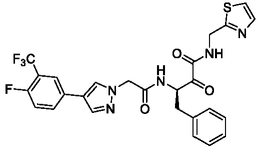 | ¹H NMR (400 MHz, DMSO-*d*₆) *δ* 9.61 (t, *J* = 6.3 Hz, 1H), 8.71 (d, *J =* 7.1 Hz, 1H), 8.22 (s, 1H), 8.02 (s, 1H), 7.96-7.90 (m, 2H), 7.73 (d, *J* = 3.2 Hz, 1H), 7.63 (d, *J* = 3.3 Hz, 1H), 7.54-7.47 (m, 1H), 7.33-7.26 (m, 2H), 7.24-7.19 (m, 3H), 5.33-5.24 (m, 1H), 4.86 (d, *J =* 2.0 Hz, 2H), 4.66-4.59 (m, 2H), 3.21-3.12 (m, 1H), 2.92-2.82 (m, 1H). |
| | | [M+H] ⁺=560.1 |
| **499** | 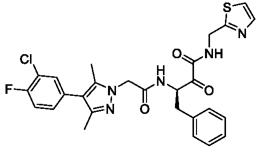 | ¹H NMR (400 MHz, DMSO-*d*₆) *δ* 9.61 (t, *J* = 6.2 Hz, 1H), 8.62 (d, *J* = 7.2 Hz, 1H), 7.75-7.72 (m, 1H), 7.64 (d, *J* = 3.3 Hz, 1H), 7.45 (t, *J* = 4.5 Hz, 1H), 7.40 (dd, *J* = 7.2, 2.1 Hz, 1H), 7.32-7.27 (m, 2H), 7.26-7.20 (m, 3H), 7.17-7.13 (m, 1H), 5.31-5.23 (m, 1H), 4.74 (s, 2H), 4.65-4.59 (m, 2H), 3.22-3.14 (m, 1H), 2.91-2.81 (m, 1H), 2.11 (s, 3H), 2.05 (s, 3H). |
| | | [M+H] ⁺=554.1 |
| **500** | 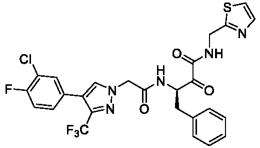 | ¹H NMR (400 MHz, DMSO-*d*₆) *δ* 9.66-9.59 (m, 1H), 8.91-8.83 (m, 1H), 8.14 (d, *J =* 5.8 Hz, 1H), 7.73 (d, *J* = 3.2 Hz, 1H), 7.63 (t, *J* = 2.5 Hz, 1H), 7.59-7.54 (m, 1H), 7.54-7.48 (m, 1H), 7.42-7.35 (m, 1H), 7.33-7.27 (m, 2H), 7.26-7.18 (m, 3H), 5.35-5.24 (m, 1H), 5.02-4.91 (m, 2H), 4.68-4.56 (m, 2H), 3.20-3.13 (m, 1H), 2.91-2.81 (m, 1H). |
| | | [M+H] ⁺=594.1 |
| **501** | 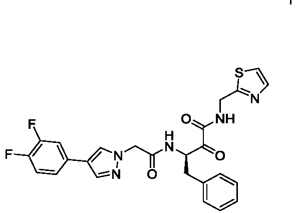 | ¹H NMR (400 MHz, DMSO-*d*₆) *δ* 9.60 (t, *J* = 6.2 Hz, 1H), 8.74-8.69 (m, 1H), 8.11 (s, 1H), 7.93 (s, 1H), 7.90-7.85 (m, 2H), 7.73 (d, *J* = 3.3 Hz, 1H), 7.63 (d, *J* = 3.2 Hz, 1H), 7.45-7.41 (m, 1H), 7.31-7.26 (m, 2H), 7.25-7.21 (m, 3H), 5.31-5.25 (m, 1H), 4.85 (d, *J =* 1.9 Hz, 2H), 4.64-4.60 (m, 2H), 3.19-3.13 (m, 1H), 2.91-2.80 (m, 1H). |
| | | [M+H] ⁺⁺=510.1 |
| **502** | 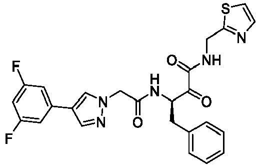 | ¹H NMR (400 MHz, DMSO-*d*₆) *δ* 9.61 (t, *J* = 6.2 Hz, 1H), 8.73 (d, *J =* 7.1 Hz, 1H), 8.21 (s, 1H), 8.02 (s, 1H), 7.73 (d, *J =* 3.3 Hz, 1H), 7.63 (d, *J =* 3.2 Hz, 1H), 7.37-7.27 (m, 4H), 7.25-7.20 (m, 3H), 7.05-6.99 (m, 1H), 5.33-5.25 (m, 1H), 4.87-4.83 (m, 2H), 4.68-4.58 (m, 2H), 3.21-3.15 (m, 1H), 2.91-2.83 (m, 1H). |
| | | [M+H] ⁺⁺=510.1 |
| **503** | 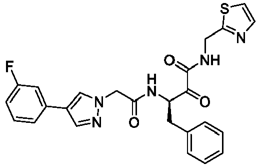 | ¹H NMR (400 MHz, DMSO-*d*₆) *δ* 9.59 (t, *J* = 6.2 Hz, 1H), 8.67 (d, *J =* 7.1 Hz, 1H), 8.12 (d, *J =* 0.8 Hz, 1H), 7.95 (d, *J* = 0.8 Hz, 1H), 7.72 (d, *J* = 3.2 Hz, 1H), 7.63 (d, *J =* 3.3 Hz, 1H), 7.46-7.36 (m, 3H), 7.33-7.26 (m, 2H), 7.25-7.18 (m, 3H), 7.04-6.97 (m, 1H), 5.34-5.25 (m, 1H), 4.85 (d, *J* = 1.8 Hz, 2H), 4.71-4.54 (m, 2H), 3.22-3.12 (m, 1H), 2.94-2.81 (m, 1H). |
| | | [M+H] ⁺=492.1 |
| **504** | 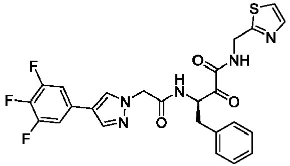 | ¹H NMR (400 MHz, DMSO-*d*₆) *δ* 9.60 (t, *J* = 6.2 Hz, 1H), 8.73 (d, *J =* 7.1 Hz, 1H), 8.18 (s, 1H), 7.99 (s, 1H), 7.73 (d, *J =* 3.3 Hz, 1H), 7.63 (d, *J =* 3.3 Hz, 1H), 7.61-7.54 (m, 2H), 7.33-7.26 (m, 2H), 7.26-7.16 (m, 3H), 5.34-5.24 (m, 1H), 4.85 (d, *J* = 2.3 Hz, 2H), 4.68-4.58 (m, 2H), 3.22-3.15 (m, 1H), 2.91-2.83 (m, 1H). |
| | | [M+H] ⁺=528.1 |
| **505** | 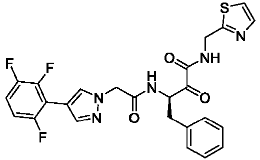 | ¹H NMR (400 MHz, DMSO-*d*₆) *δ* 9.60 (t, *J* = 6.3 Hz, 1H), 8.72 (dd, *J* = 15.4, 7.1 Hz, 1H), 8.20 (d, *J* = 2.4 Hz, 1H), 7.83 (s, 1H), 7.75-7.71 (m, 1H), 7.65-7.61 (m, 1H), 7.53 (s, 1H), 7.39-7.16 (m, 6H), 5.32-5.22 (m, 1H), 4.87-4.81 (m, 2H), 4.66-4.58 (m, 2H), 3.23-3.12 (m, 1H), 2.90-2.82 (m, 1H). |
| | | [M+H] ⁺=528.1 |
| **506** | 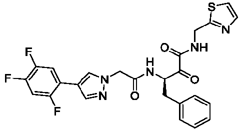 | ¹H NMR (400 MHz, DMSO-*d*₆) *δ* 9.60 (t, *J* = 6.2 Hz, 1H), 8.71 (d, *J* = 7.1 Hz, 1H), 8.11 (d, *J* = 2.6 Hz, 1H), 7.97 (s, 1H), 7.93-7.84 (m, 1H), 7.73 (d, *J* = 3.3 Hz, 1H), 7.65-7.56 (m, 2H), 7.32-7.27 (m, 2H), 7.25-7.18 (m, 3H), 5.34-5.20 (m, 1H), 4.93-4.87 (m, 2H), 4.68-4.57 (m, 2H), 3.20-3.13 (m, 1H), 2.90-2.84 (m, 1H). |
| | | [M+H] ⁺=528.1 |
| **507** | 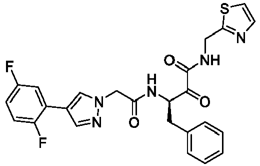 | ¹H NMR (400 MHz, DMSO-*d*₆) *δ* 9.61 (t, *J* = 6.2 Hz, 1H), 8.71 (d, *J =* 7.2 Hz, 1H), 7.78 (d, *J =* 2.3 Hz, 1H), 7.73 (d, *J =* 3.3 Hz, 1H), 7.65-7.54 (m, 2H), 7.41-7.31 (m, 1H), 7.30-7.25 (m, 2H), 7.25-7.10 (m, 4H), 6.70-6.62 (m, 1H), 5.33-5.26 (m, 1H), 4.93 (s, 2H), 4.69-4.58 (m, 2H), 3.23-3.13 (m, 1H), 2.97-2.84 (m, 1H). |
| | | [M+H] ⁺=510.1 |
| **508** | 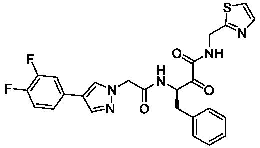 | ¹H NMR (400 MHz, DMSO-*d*₆) *δ* 9.67-9.56 (m, 1H), 8.68 (t, *J =* 5.9 Hz, 1H), 7.82-7.69 (m, 3H), 7.66-7.59 (m, 2H), 7.53-7.42 (m, 1H), 7.34-7.25 (m, 2H), 7.25-7.18 (m, 3H), 6.81-6.75 (m, 1H), 5.34-5.25 (m, 1H), 4.93-4.83 (m, 2H), 4.70-4.57 (m, 2H), 3.21-3.14 (m, 1H), 2.92-2.81 (m, 1H). |
| | | [M+H] ⁺=510.1 |
| **509** | 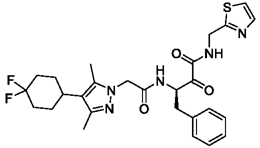 | ¹H NMR (400 MHz, DMSO-*d*₆) *δ* 9.68-9.54 (m, 1H), 8.43 (d, *J* = 7.2 Hz, 1H), 7.80-7.59 (m, 2H), 7.35-7.26 (m, 2H), 7.25-7.15 (m, 3H), 5.31-5.18 (m, 1H), 4.70-4.53 (m, 4H), 3.21-3.11 (m, 1H), 2.91-2.80 (m, 1H), 2.61-2.54 (m, 1H), 2.07 (s, 3H), 2.00 (s, 3H), 1.90-1.56 (m, 8H). |
| | | [M+H] ⁺=544.2 |
| **510** | 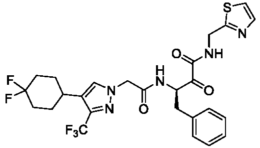 | ¹H NMR (400 MHz, DMSO-*d*₆) *δ* 9.61 (t, *J* = 6.2 Hz, 1H), 8.79 (d, *J* = 7.1 Hz, 1H), 7.77 (s, 1H), 7.73 (d, *J* = 3.3 Hz, 1H), 7.63 (d, *J* = 3.3 Hz, 1H), 7.32-7.26 (m, 2H), 7.26-7.18 (m, 3H), 5.32-5.24 (m, 1H), 4.87 (s, 2H), 4.66-4.58 (m, 2H), 3.21-3.12 (m, 1H), 2.90-2.81 (m, 1H), 2.77-2.67 (m, 1H), 2.10-1.84 (m, 6H), 1.58-1.49 (m, 2H). |
| | | [M+H] ⁺=584.2 |
| **511** | 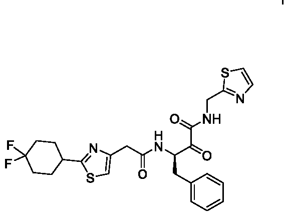 | ¹H NMR (400 MHz, DMSO-*d*₆) *δ* 9.55 (t, *J* = 6.2 Hz, 1H), 8.51 (d, *J =* 7.1 Hz, 1H), 7.73 (d, *J =* 3.3 Hz, 1H), 7.64 (d, *J* = 3.3 Hz, 1H), 7.30-7.16 (m, 5H), 7.13 (s, 1H), 5.30-5.20 (m, 1H), 4.62 (dd, *J* = 6.2, 2.8 Hz, 2H), 3.58 (s, 2H), 3.21-3.09 (m, 2H), 2.84 (dd, *J =* 14.0, 9.0 Hz, 1H), 2.14-2.03 (m, 4H), 2.04-1.88 (m, 2H), 1.79-1.62 (m, 2H). |
| | | [M+H] ⁺=533.1 |
| **512** | 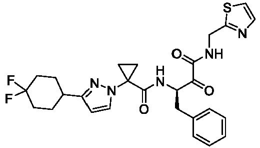 | ¹H NMR (400 MHz, DMSO-*d*₆) *δ* 9.58 (t, *J* = 6.2 Hz, 1H), 7.74 (d, *J =* 3.3 Hz, 1H), 7.71 (d, *J =* 2.4 Hz, 1H), 7.66 (d, *J =* 3.2 Hz, 1H), 7.26-7.20 (m, 3H), 6.99-6.91 (m, 2H), 6.55 (d, *J* = 7.6 Hz, 1H), 6.20 (d, *J* = 2.4 Hz, 1H), 5.36-5.25 (m, 1H), 4.70-4.56 (m, 2H), 3.13-3.05 (m, 1H), 3.00-2.93 (m, 1H), 2.77-2.67 (m, 1H), 2.10-1.81 (m, 8H), 1.63-1.51 (m, 4H). |
| | | [M+H] ⁺=542.2 |
| **513** | 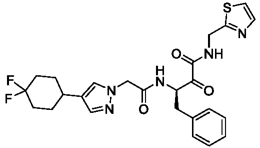 | ¹H NMR (400 MHz, DMSO-*d*₆) *δ* 9.60 (t, *J* = 6.3 Hz, 1H), 8.53 (d, *J =* 7.2 Hz, 1H), 7.73 (d, *J =* 3.3 Hz, 1H), 7.64 (d, *J* = 3.3 Hz, 1H), 7.44 (s, 1H), 7.35-7.33 (m, 1H), 7.30-7.18 (m, 5H), 5.33-5.24 (m, 1H), 4.75 (s, 2H), 4.67-4.62 (m, 2H), 3.19-3.12 (m, 1H), 2.92-2.81 (m, 1H), 2.69-2.56 (m, 1H), 2.07-1.87 (m, 6H), 1.56-1.45 (m, 2H). |
| | | [M+H] ⁺=516.2 |
| **514** | 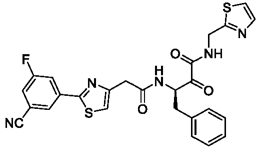 | ¹H NMR (400 MHz, DMSO-*d*₆) *δ* 9.57 (t, *J* = 6.2 Hz, 1H), 8.65 (t, *J =* 7.2 Hz, 1H), 8.29 (d, *J =* 1.6 Hz, 1H), 8.02-7.95 (m, 1H), 7.84-7.77 (m, 1H), 7.72 (d, *J =* 3.3 Hz, 1H), 7.63 (d, *J* = 3.3 Hz, 1H), 7.47 (s, 1H), 7.29-7.14 (m, 5H), 5.29-5.20 (m, 1H), 4.69-4.55 (m, 2H), 3.71 (d, *J =* 3.9 Hz, 2H), 3.23-3.14 (m, 1H), 2.92-2.81 (m, 1H). |
| | | [M+H] ⁺=534.1 |
| **515** | 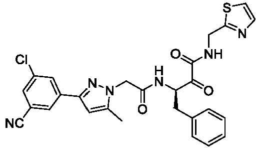 | ¹H NMR (400 MHz, DMSO-*d*₆) *δ* 9.61 (t, *J* = 6.2 Hz, 1H), 8.73 (d, *J =* 7.4 Hz, 1H), 8.27 (t, *J =* 1.7 Hz, 1H), 8.14-8.07 (m, 1H), 7.84-7.78 (m, 1H), 7.75-7.69 (m, 1H), 7.66-7.55 (m, 1H), 7.32-7.12 (m, 5H), 6.70 (s, 1H), 5.32-5.23 (m, 1H), 4.84 (s, 2H), 4.66-4.59 (m, 2H), 3.22-3.15 (m, 1H), 2.90-2.82 (m, 1H), 2.15 (s, 3H). |
| | | [M+H] ⁺=547.1 |
| **516** | 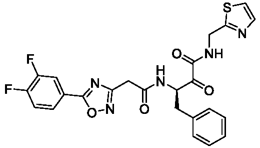 | ¹H NMR (400 MHz, DMSO-*d*₆) *δ* 9.60 (t, *J* = 6.3 Hz, 1H), 8.85 (d, *J =* 7.1 Hz, 1H), 8.21-8.10 (m, 1H), 8.00-7.92 (m, 1H), 7.78-7.67 (m, 2H), 7.65-7.62 (m, 1H), 7.33-7.18 (m, 5H), 5.32-5.22 (m, 1H), 4.68-4.55 (m, 2H), 3.79 (s, 2H), 3.21-3.12 (m, 1H), 2.91-2.80 (m, 1H). |
| | | [M+H] ⁺=512.1 |
| **517** | 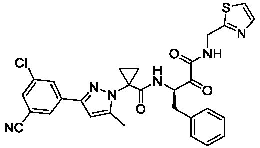 | ¹H NMR (400 MHz, DMSO-*d*₆) *δ* 9.58 (t, *J* = 6.2 Hz, 1H), 8.30 (d, *J =* 2.1 Hz, 1H), 8.15-8.05 (m, 1H), 7.79 (d, *J* = 8.6 Hz, 1H), 7.74 (d, *J* = 3.3 Hz, 1H), 7.65 (d, *J* = 3.3 Hz, 1H), 7.33-7.15 (m, 3H), 7.05 (d, *J =* 6.8 Hz, 2H), 6.85 (d, *J =* 7.3 Hz, 1H), 6.73 (s, 1H), 5.24-5.15 (m, 1H), 4.66-4.56 (m, 2H), 3.16-3.07 (m, 1H), 2.98-2.85 (m, 1H), 2.09 (s, 3H), 1.64-1.48 (m, 4H). |
| | | [M+H] ⁺=**573.1** |
| **518** | 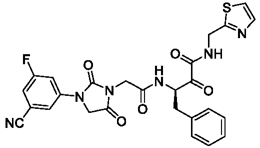 | ¹H NMR (400 MHz, DMSO-*d*₆) *δ* 9.60 (t, *J* = 6.3 Hz, 1H), 8.78 (d, *J =* 7.1 Hz, 1H), 8.21 (d, *J =* 1.9 Hz, 1H), 7.73 (d, *J =* 3.2 Hz, 1H), 7.63 (d, *J =* 3.3 Hz, 1H), 7.50-7.44 (m, 1H), 7.34-7.15 (m, 6H), 5.29-5.21 (m, 1H), 4.65-4.60 (m, 2H), 4.12 (s, 2H), 3.89 (s, 2H), 3.21-3.11 (m, 1H), 2.92-2.83 (m, 1H). |
| | | [M+H] ⁺=549.1 |
| **519** | 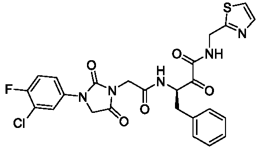 | ¹H NMR (400 MHz, DMSO-*d*₆) *δ* 9.59 (t, *J* = 6.3 Hz, 1H), 8.76 (d, *J =* 7.1 Hz, 1H), 7.87 (dd, *J =* 6.6, 2.9 Hz, 1H), 7.73 (d, *J* = 3.3 Hz, 1H), 7.66-7.59 (m, 2H), 7.49 (t, *J =* 9.1 Hz, 1H), 7.33-7.26 (m, 2H), 7.23 (q, *J =* 3.7, 3.2 Hz, 3H), 5.29-5.17 (m, 1H), 4.70-4.47 (m, 4H), 4.11 (s, 2H), 3.18-3.10 (m, 1H), 2.92-2.83 (m, 1H). |
| | | [M+H] ⁺=558.1 |
| **520** | 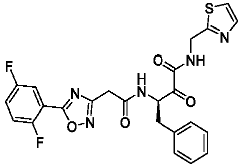 | ¹H NMR (400 MHz, DMSO-*d*₆) *δ* 9.59 (t, *J* = 6.2 Hz, 1H), 8.84 (d, *J =* 7.1 Hz, 1H), 7.92-7.85 (m, 1H), 7.72 (d, *J =* 3.2 Hz, 1H), 7.69-7.58 (m, 3H), 7.31-7.16 (m, 5H), 5.30-5.21 (m, 1H), 4.67-4.59 (m, 2H), 3.81 (s, 2H), 3.20-3.12 (m, 1H), 2.92-2.83 (m, 1H). |
| | | [M+H] ⁺=**512.1** |
| **521** | 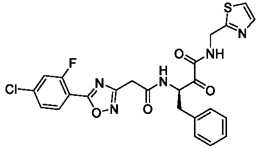 | ¹H NMR (400 MHz, DMSO-*d*₆) *δ* 9.59 (t, *J* = 6.2 Hz, 1H), 8.85 (d, *J* = 7.1 Hz, 1H), 8.11 (t, *J* = 8.2 Hz, 1H), 7.82 (dd, *J* = 10.7, 2.0 Hz, 1H), 7.73 (d, *J* = 3.3 Hz, 1H), 7.63 (d, *J =* 3.3 Hz, 1H), 7.58 (dd, *J =* 8.5, 2.0 Hz, 1H), 7.31-7.16 (m, 5H), 5.30-5.21 (m, 1H), 4.68-4.56 (m, 2H), 3.81 (s, 2H), 3.21-3.14 (m, 1H), 2.93-2.83 (m, 1H). |
| | | [M+H] **⁺=528.1** |
| **522** | 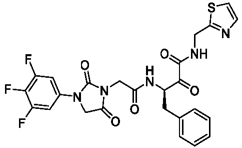 | ¹H NMR (400 MHz, DMSO-*d*₆) *δ* 9.59 (t, *J* = 6.2 Hz, 1H), 8.77 (d, *J =* 7.1 Hz, 1H), 7.73 (d, *J =* 3.3 Hz, 1H), 7.62 (dd, *J* = 14.0, 4.6 Hz, 3H), 7.35-7.16 (m, 5H), 5.30-5.19 (m, 1H), 4.62 (dd, *J =* 6.2, 3.2 Hz, 2H), 4.57 (s, 2H), 4.10 (s, 2H), 3.20-3.10 (m, 1H), 2.91-2.81 (m, 1H). |
| | | [M+H] ⁺=560.1 |
| **523** | 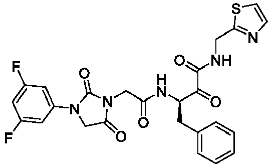 | ¹H NMR (400 MHz, DMSO-*d*₆) *δ* 9.59 (t, *J* = 6.2 Hz, 1H), 8.76 (d, *J =* 7.1 Hz, 1H), 7.73 (d, *J =* 3.3 Hz, 1H), 7.63 (d, *J =* 3.3 Hz, 1H), 7.47-7.36 (m, 2H), 7.35-7.15 (m, 5H), 7.07-6.98 (m, 1H), 5.29-5.19 (m, 1H), 4.68-4.61 (m, 2H), 4.58 (s, 2H), 4.11 (s, 2H), 3.19-3.12 (m, 1H), 2.90-2.83 (m, 1H). |
| | | [M+H] ⁺=**542.1** |
| **524** | 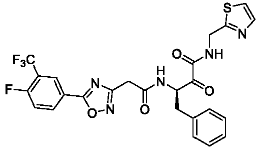 | ¹H NMR (400 MHz, DMSO-*d*₆) *δ* 9.59 (t, *J* = 6.3 Hz, 1H), 8.86 (d, *J* = 7.1 Hz, 1H), 8.50-8.43 (m, 1H), 8.41-8.32 (m, 1H), 7.88-7.79 (m, 1H), 7.72 (d, *J =* 3.2 Hz, 1H), 7.63 (d, *J =* 3.2 Hz, 1H), 7.31-7.15 (m, 6H), 5.32-5.22 (m, 1H), 4.67-4.59 (m, 2H), 3.80 (s, 2H), 3.20-3.14 (m, 1H), 2.90-2.83 (m, 1H). |
| | | [M+H] ⁺=562.1 |
| **525** | 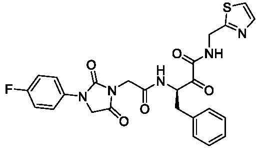 | ¹H NMR (400 MHz, DMSO-*d*₆) *δ* 9.59 (t, *J* = 6.3 Hz, 1H), 8.76 (d, *J =* 7.0 Hz, 1H), 7.73 (d, *J =* 3.3 Hz, 1H), 7.68-7.62 (m, 3H), 7.31-7.21 (m, 7H), 5.28-5.20 (m, 1H), 4.65-4.60 (m, 2H), 4.57 (s, 2H), 4.10 (s, 2H), 3.18-3.12 (m, 1H), 2.90-2.83 (m, 1H). |
| | | [M+H] ⁺=524.1 |
| **526** | 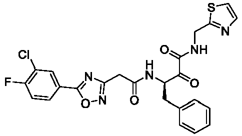 | ¹H NMR (400 MHz, DMSO-*d*₆) *δ* 9.60 (t, *J* = 6.2 Hz, 1H), 8.83 (d, *J =* 7.1 Hz, 1H), 8.26 (dd, *J =* 7.0, 2.2 Hz, 1H), 8.14-8.08 (m, 1H), 7.79-7.67 (m, 3H), 7.64 (d, *J =* 3.3 Hz, 1H), 7.32-7.19 (m, 5H), 5.30-5.23 (m, 1H), 4.69-4.58 (m, 2H), 3.78 (s, 2H), 3.21-3.12 (m, 1H), 2.91-2.82 (m, 1H). |
| | | [M+H] ⁺=528.1 |
| **527** | 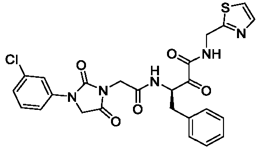 | ¹H NMR (400 MHz, DMSO-*d*₆) *δ* 9.59 (t, *J* = 6.3 Hz, 1H), 8.75 (d, *J =* 7.0 Hz, 1H), 7.73 (d, *J =* 3.3 Hz, 1H), 7.64-7.58 (m, 2H), 7.46-7.41 (m, 1H), 7.30-7.20 (m, 7H), 5.30-5.20 (m, 1H), 4.65-4.55 (m, 4H), 4.11 (s, 2H), 3.18-3.11 (m, 1H), 2.91-2.83 (m, 1H). |
| | | [M+H] ⁺=540.1 |
| **528** | 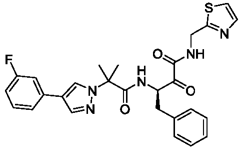 | ¹H NMR (400 MHz, DMSO-*d*₆) *δ* 9.56 (t, *J* = 6.2 Hz, 1H), 8.36 (s, 1H), 8.03 (s, 1H), 7.73 (d, *J =* 3.3 Hz, 1H), 7.66-7.60 (m, 2H), 7.53-7.47 (m, 2H), 7.42-7.36 (m, 1H), 7.21-7.14 (m, 3H), 7.10-7.07 (m, 2H), 7.03-6.98 (m, 1H), 5.16-5.09 (m, 1H), 4.66-4.58 (m, 2H), 3.15-3.06 (m, 1H), 2.95-2.85 (m, 1H), 1.74 (s, 2H), 1.69 (s, 3H). |
| | | [M+H] ⁺=520.2 |
| **529** | 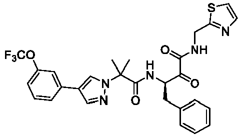 | ¹H NMR (400 MHz, DMSO-*d*₆) *δ* 9.20 (t, *J* = 6.3 Hz, 1H), 8.44 (d, *J =* 18.2 Hz, 1H), 8.35-8.25 (m, 2H), 8.14-7.99 (m, 3H), 7.54-7.43 (m, 2H), 7.19-7.10 (m, 3H), 7.11-7.04 (m, 2H), 6.88-6.80 (m, 1H), 5.20-5.08 (m, 1H), 4.62-4.49 (m, 2H), 3.15-3.06 (m, 1H), 2.96-2.84 (m, 1H), 1.72 (d, *J =* 16.0 Hz, 6H). |
| | | [M+H] ⁺=586.2 |
| **530** | 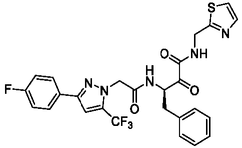 | ¹H NMR (400 MHz, DMSO-*d*₆) *δ* 9.62 (t, *J* = 6.2 Hz, 1H), 8.81 (d, *J =* 7.2 Hz, 1H), 7.95-7.83 (m, 2H), 7.73 (d, *J* = 3.3 Hz, 1H), 7.63 (d, *J* = 3.3 Hz, 1H), 7.44 (s, 1H), 7.35-7.18 (m, 7H), 5.35-5.23 (m, 1H), 5.07-4.94 (m, 2H), 4.68-4.54 (m, 2H), 3.23-3.13 (m, 1H), 2.93-2.81 (m, 1H). |
| | | [M+H] ⁺=560.5 |
| **531** | 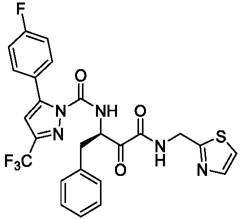 | ¹H NMR (400 MHz, DMSO-*d*₆) *δ* 9.63 (t, *J* = 6.2 Hz, 1H), 8.86 (d, *J =* 7.2 Hz, 1H), 7.72 (d, *J =* 3.2 Hz, 1H), 7.62 (d, *J =* 3.2 Hz, 1H), 7.56-7.47 (m, 2H), 7.34-7.21 (m, 5H), 7.20-7.14 (m, 2H), 6.90 (s, 1H), 5.31-5.23 (m, 1H), 4.85 (s, 2H), 4.67-4.60 (m, 2H), 3.19-3.11 (m, 1H), 2.88-2.78 (m, 1H). |
| | | [M+H] ⁺=546.1 |
| **532** | 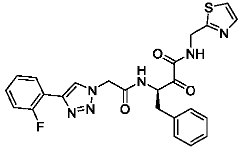 | ¹H NMR (400 MHz, DMSO-*d*₆) *δ* 9.63 (t, *J* = 6.2 Hz, 1H), 8.97 (d, *J =* 7.1 Hz, 1H), 8.31 (d, *J =* 3.8 Hz, 1H), 8.13 (td, *J =* 7.6, 1.7 Hz, 1H), 7.73 (d, *J =* 3.3 Hz, 1H), 7.62 (d, *J =* 3.3 Hz, 1H), 7.44-7.39 (m, 1H), 7.38-7.27 (m, 4H), 7.27-7.19 (m, 3H), 5.30 (dq, *J =* 11.7, 4.0 Hz, 1H), 5.24 (d, *J =* 3.7 Hz, 2H), 4.62 (dd, *J =* 6.2, 2.1 Hz, 2H), 3.25-3.13 (m, 1H), 2.96-2.82 (m, 1H). |
| | | [M+H] ⁺=493.1 |
| **533** | 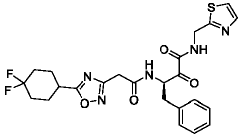 | ¹H NMR (400 MHz, DMSO-*d*₆) *δ* 9.59 (t, *J* = 6.2 Hz, 1H), 8.75 (d, *J =* 7.1 Hz, 1H), 7.73 (d, *J =* 3.3 Hz, 1H), 7.64 (d, *J* = 3.3 Hz, 1H), 7.28 (d, *J* = 6.9 Hz, 2H), 7.23 (d, *J =* 7.3 Hz, 3H), 5.31-5.18 (m, 1H), 4.69-4.56 (m, 2H), 3.67 (s, 2H), 3.19-3.11 (m, 1H), 2.91-2.82 (m, 1H), 2.17-2.01 (m, 5H), 2.00-1.86 (m, 1H), 1.83-1.70 (m, 2H). |
| | | [M+H] ⁺=518.2 |
| **534** | 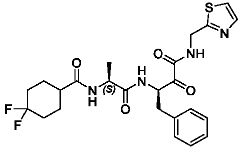 | ¹H NMR (400 MHz, DMSO-*d*₆) *δ* 9.60 (t, *J* = 6.2 Hz, 1H), 8.28 (d, *J* = 7.6 Hz, 1H), 7.86 (t, *J* = 7.4 Hz, 1H), 7.73 (d, *J =* 3.3 Hz, 1H), 7.64 (d, *J =* 3.2 Hz, 1H), 7.30-7.23 (m, 2H), 7.24-7.18 (m, 3H), 5.28-5.18 (m, 1H), 4.67-4.62 (m, 2H), 3.16-3.13 (m, 1H), 2.83-2.75 (m, 1H), 2.33-2.27 (m, 1H), 2.07-1.99 (m, 6H), 1.82-1.71 (m, 4H), 1.58-1.53 (m, 2H), 1.03 (d, *J =* 7.1 Hz, 3H). |
| | | [M+H] ⁺=507.2 |
| **535** | 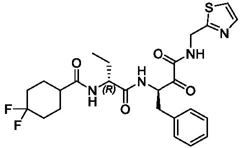 | ¹H NMR (400 MHz, DMSO-*d*₆) *δ* 9.55 (t, *J* = 6.2 Hz, 1H), 8.32 (d, *J =* 6.9 Hz, 1H), 7.85 (d, *J =* 8.2 Hz, 1H), 7.73 (d, *J =* 3.3 Hz, 1H), 7.64 (d, *J =* 3.2 Hz, 1H), 7.32-7.16 (m, 5H), 5.28-5.15 (m, 1H), 4.68-4.53 (m, 2H), 4.25-4.18 (m, 1H), 3.16-3.06 (m, 1H), 2.90-2.79 (m, 1H), 2.37-2.27 (m, 1H), 2.07-2.00 (m, 2H), 1.87-1.67 (m, 4H), 1.65-1.42 (m, 4H), 0.80 (t, *J =* 7.4 Hz, 3H). |
| | | [M+H] ⁺=521.2 |
| **536** | 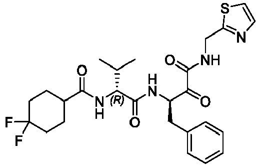 | ¹H NMR (400 MHz, DMSO-*d*₆) *δ* 9.55 (t, *J* = 6.2 Hz, 1H), 8.41 (d, *J =* 6.8 Hz, 1H), 7.79-7.71 (m, 2H), 7.64 (d, *J* = 3.3 Hz, 1H), 7.28-7.16 (m, 5H), 5.30-5.21 (m, 1H), 4.65-4.58 (m, 2H), 4.25-4.17 (m, 1H), 3.14-3.09 (m, 1H), 2.89-2.78 (m, 1H), 2.37 (d, *J =* 11.7 Hz, 2H), 2.06-1.99 (m, 2H), 1.83-1.68 (m, 4H), 1.62-1.50 (m, 3H), 0.80 (dd, *J =* 12.5, 6.7 Hz, 6H). |
| | | [M+H] ⁺=535.2 |
| **537** | 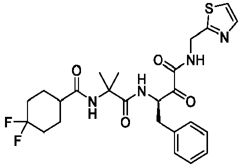 | ¹H NMR (400 MHz, DMSO-*d*₆) *δ* 9.52 (t, *J* = 6.3 Hz, 1H), 7.80 (s, 1H), 7.78-7.71 (m, 2H), 7.65 (d, *J =* 3.3 Hz, 1H), 7.42-7.07 (m, 6H), 5.21-5.00 (m, 1H), 4.72-4.52 (m, 2H), 3.17-3.03 (m, 1H), 2.95-2.81 (m, 1H), 2.34-2.19 (m, 1H), 2.14-1.95 (m, 2H), 1.88-1.67 (m, 4H), 1.62-1.45 (m, 2H), 1.27 (d, *J =* 8.0 Hz, 6H). |
| | | [M+H] ⁺=521.2 |
| **538** | 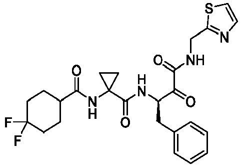 | ¹H NMR (400 MHz, DMSO-*d*₆) *δ* 9.54 (t, *J* = 6.2 Hz, 1H), 8.39 (s, 1H), 7.74 (d, *J =* 3.3 Hz, 1H), 7.66 (d, *J =* 3.2 Hz, 1H), 7.59 (d, *J* = 7.2 Hz, 1H), 7.28-7.18 (m, 3H), 7.17-7.11 (m, 2H), 5.21-5.14 (m, 1H), 4.69-4.54 (m, 2H), 3.15-3.08 (m, 1H), 3.02-2.94 (m, 1H), 2.28-2.18 (m, 1H), 2.09-1.96 (m, 2H), 1.84-1.68 (m, 4H), 1.60-1.45 (m, 2H), 1.26-1.10 (m, 2H), 0.90-0.78 (m, 2H). |
| | | [M+H] ⁺=519.2 |
| **539** | 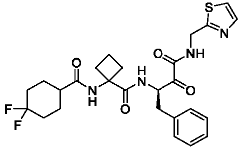 | ¹H NMR (400 MHz, DMSO-*d*₆) *δ* 9.56 (t, *J* = 6.2 Hz, 1H), 8.32 (s, 1H), 7.73 (d, *J* = 3.3 Hz, 1H), 7.67-7.62 (m, 2H), 7.24-7.19 (m, 3H), 7.17-7.13 (m, 2H), 5.20-5.08 (m, 1H), 4.70-4.53 (m, 2H), 3.18-3.10 (m, 1H), 2.94-2.87 (m, 1H), 2.41-2.35 (m, 1H), 2.29-2.18 (m, 2H), 2.07-1.97 (m, 4H), 1.86-1.70 (m, 6H), 1.61-1.51 (m, 2H). |
| | | [M+H] ⁺=533.6 |
| **540** | 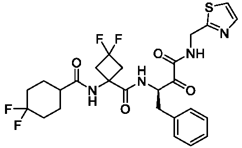 | ¹H NMR (400 MHz, DMSO-*d*₆) *δ* 9.58 (t, *J* = 6.2 Hz, 1H), 8.64 (s, 1H), 7.93 (d, *J =* 7.3 Hz, 1H), 7.73 (d, *J =* 3.3 Hz, 1H), 7.65 (d, *J* = 3.3 Hz, 1H), 7.28-7.18 (m, 3H), 7.17-7.11 (m, 2H), 5.20-5.07 (m, 1H), 4.71-4.54 (m, 2H), 3.18-3.12 (m, 1H), 3.10-2.95 (m, 2H), 2.93-2.84 (m, 1H), 2.77-2.58 (m, 2H), 2.36-2.21 (m, 1H), 2.10-1.97 (m, 2H), 1.89-1.70 (m, 4H), 1.63-1.43 (m, 2H). |
| | | [M+H] ⁺=569.2 |
| **541** | 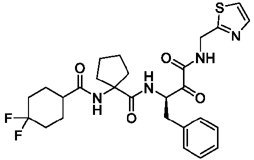 | ¹H NMR (400 MHz, DMSO-*d*₆) *δ* 9.52 (t, *J* = 6.2 Hz, 1H), 7.91 (s, 1H), 7.73 (d, *J* = 3.2 Hz, 1H), 7.71-7.67 (m, 1H), 7.65 (d, *J* = 3.3 Hz, 1H), 7.27-7.20 (m, 3H), 7.19-7.14 (m, 2H), 5.15-5.08 (m, 1H), 4.64-4.58 (m, 2H), 3.16-3.07 (m, 1H), 2.93-2.84 (m, 1H), 2.33-2.24 (m, 1H), 2.11-1.92 (m, 4H), 1.85-1.71 (m, 6H), 1.62-1.46 (m, 6H). |
| | | [M+H] ⁺=547.2 |
| **542** | 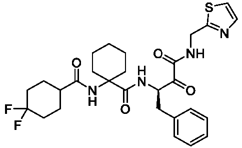 | ¹H NMR (400 MHz, DMSO-*d*₆) *δ* 9.52 (t, *J* = 6.2 Hz, 1H), 7.73 (d, *J* = 3.3 Hz, 1H), 7.70-7.62 (m, 2H), 7.56 (s, 1H), 7.28-7.08 (m, 5H), 5.18-5.06 (m, 1H), 4.69-4.52 (m, 2H), 3.17-3.07 (m, 1H), 2.95-2.85 (m, 1H), 2.45-2.33 (m, 1H), 2.11-1.92 (m, 4H), 1.86-1.66 (m, 4H), 1.61-1.34 (m, 10H). |
| | | [M+H] ⁺⁺=561.2 |
| **543** | 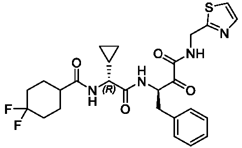 | ¹H NMR (400 MHz, DMSO-*d*₆) *δ* 9.58 (dt, *J* = 25.4, 6.2 Hz, 1H), 8.34 (dd, *J* = 31.5, 7.2 Hz, 1H), 7.97 (dd, *J =* 8.3, 4.2 Hz, 1H), 7.73 (t, *J* = 3.1 Hz, 1H), 7.64 (t, *J =* 3.3 Hz, 1H), 7.31-7.14 (m, 5H), 5.29-5.18 (m, 1H), 4.68-4.54 (m, 2H), 3.19-3.09 (m, 1H), 2.93-2.75 (m, 1H), 2.41-2.27 (m, 1H), 2.12-1.91 (m, 2H), 1.87-1.67 (m, 4H), 1.55 (m, 2H), 1.03-0.93 (m, 1H), 0.91-0.75 (m, 1H), 0.27-0.12 (m, 2H). |
| | | [M+H] ⁺=533.2 |
| **544** | 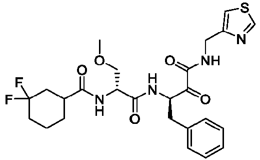 | ¹H NMR (400 MHz, DMSO-*d*₆) *δ* 9.35-9.17 (m, 1H), 9.05 (s, 1H), 8.45-8.28 (m, 1H), 8.16-8.06 (m, 1H), 7.38 (d, *J* = 16.5 Hz, 1H), 7.30-7.10 (m, 5H), 5.28-5.14 (m, 1H), 4.56-4.40 (m, 3H), 3.48-3.36 (m, 2H), 3.23-3.07 (m, 4H), 2.91-2.78 (m, 1H), 2.63-2.53 (m, 1H), 2.08-1.93 (m, 2H), 1.87-1.57 (m, 4H), 1.49-1.32 (m, 2H). |
| | | [M+H] ⁺=537.2 |
| **545** | 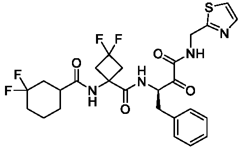 | ¹H NMR (400 MHz, DMSO-*d*₆) *δ* 9.66-9.47 (m, 1H), 8.71 (d, *J* = 2.1 Hz, 1H), 8.04 (dd, *J* = 10.7, 7.4 Hz, 1H), 7.73 (d, *J* = 3.3 Hz, 1H), 7.65 (dd, *J =* 3.3, 0.9 Hz, 1H), 7.30-7.19 (m, 3H), 7.19-7.11 (m, 2H), 5.19-5.03 (m, 1H), 4.71-4.53 (m, 2H), 3.21-3.02 (m, 2H), 2.88 (td, *J* = 13.8, 8.9 Hz, 2H), 2.72-2.54 (m, 3H), 2.18-2.07 (m, 1H), 2.03-1.94 (m, 1H), 1.86-1.63 (m, 4H), 1.49-1.34 (m, 1H), 1.33-1.17 (m, 1H). |
| | | [M+H] ⁺=569.2 |
| **546** | 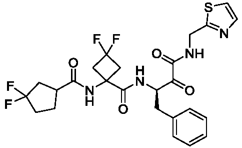 | ¹H NMR (400 MHz, DMSO-*d*₆) *δ* 9.58 (t, *J* = 6.2 Hz, 1H), 8.82 (s, 1H), 8.05 (t, *J* = 7.7 Hz, 1H), 7.73 (d, *J =* 3.3 Hz, 1H), 7.65 (d, *J* = 3.3 Hz, 1H), 7.30-7.20 (m, 3H), 7.19-7.14 (m, 2H), 5.17-5.09 (m, 1H), 4.67-4.59 (m, 2H), 3.18-3.11 (m, 1H), 2.94-2.88 (m, 2H), 2.81-2.56 (m, 4H), 2.26-2.17 (m, 2H), 2.07-1.93 (m, 3H), 1.82-1.69 (m, 1H). |
| | | [M+H] ⁺=555.2 |
| **547** | 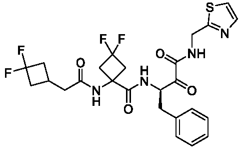 | ¹H NMR (400 MHz, DMSO-*d*₆) *δ* 9.56 (t, *J* = 6.2 Hz, 1H), 8.70 (s, 1H), 8.02 (d, *J =* 7.4 Hz, 1H), 7.73 (d, *J =* 3.2 Hz, 1H), 7.65 (d, *J* = 3.3 Hz, 1H), 7.29-7.20 (m, 3H), 7.17-7.13 (m, 2H), 5.20-5.07 (m, 1H), 4.71-4.54 (m, 2H), 3.20-3.11 (m, 1H), 3.07-2.95 (m, 2H), 2.93-2.84 (m, 1H), 2.77-2.54 (m, 5H), 2.37-2.22 (m, 4H). |
| | | [M+H] ⁺=555.2 |
| **548** | 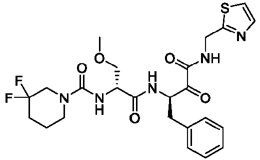 | H NMR (400 MHz, DMSO-*d*₆) *δ* 9.69-9.50 (m, 1H), 8.38-8.25 (m, 1H), 7.77-7.67 (m, 1H), 7.67-7.56 (m, 1H), 7.32-7.23 (m, 2H), 7.25-7.08 (m, 3H), 6.70-6.54 (m, 1H), 5.28-5.14 (m, 1H), 4.69-4.55 (m, 2H), 4.47-4.35 (m, 1H), 3.64 (t, *J =* 12.1 Hz, 2H), 3.51-3.43 (m, 1H), 3.42-3.37 (m, 1H), 3.32-3.24 (m, 1H), 3.18 (d, *J =* 26.5 Hz, 3H), 3.15-3.05 (m, 1H), 2.95-2.86 (m, 1H), 2.87-2.77 (m, 1H), 2.06-1.92 (m, 2H), 1.67-1.56 (m, 2H). |
| | | [M+H] ⁺=538.2 |
| **549** | 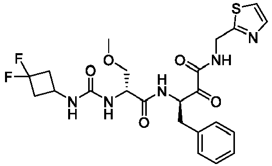 | ¹H NMR (400 MHz, DMSO-*d*₆) *δ* 9.65-9.50 (m, 1H), 8.40 (dd, *J* = 26.9, 7.2 Hz, 1H), 7.73 (t, *J =* 3.2 Hz, 1H), 7.64 (dd, *J =* 5.3, 3.2 Hz, 1H), 7.32-7.23 (m, 2H), 7.21 (dd, *J* = 7.4, 4.8 Hz, 3H), 6.70 (d, *J* = 6.9 Hz, 1H), 6.07 (dd, *J =* 8.3, 5.0 Hz, 1H), 5.29-5.14 (m, 1H), 4.67-4.58 (m, 2H), 4.43-4.29 (m, 1H), 3.97-3.85 (m, 1H), 3.47-3.37 (m, 1H), 3.31-3.22 (m, 1H), 3.18-3.09 (m, 1H), 3.08 (s, 3H), 2.93-2.79 (m, 3H), 2.46-2.31 (m, 2H). |
| | | [M+H] ⁺=524.2 |
| **550** | 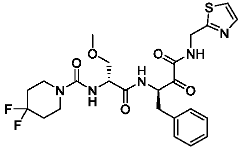 | ¹H NMR (400 MHz, DMSO-*d*₆) *δ* 9.54 (t, *J* = 6.2 Hz, 1H), 8.28 (d, *J =* 7.0 Hz, 1H), 7.73 (dd, *J =* 3.3, 1.1 Hz, 1H), 7.64 (dd, *J* = 3.2, 1.2 Hz, 2H), 7.29-7.24 (m, 2H), 7.23-7.14 (m, 3H), 6.69 (d, *J =* 7.9 Hz, 1H), 5.23-5.11 (m, 1H), 4.66-4.56 (m, 2H), 4.46-4.34 (m, 1H), 3.50-3.46 (m, 2H), 3.45-3.38 (m, 5H), 3.22 (s, 3H), 3.14-3.08 (m, 1H), 2.94-2.85 (m, 1H), 1.93-1.81 (m, 4H). |
| | | [M+H] ⁺=538.2 |
| **551** | 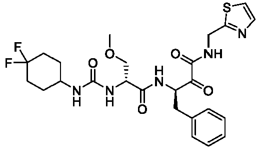 | ¹H NMR (400 MHz, DMSO-*d*₆) *δ* 9.56 (t, *J* = 6.2 Hz, 1H), 8.33 (d, *J =* 7.1 Hz, 1H), 7.73 (d, *J =* 3.3 Hz, 1H), 7.63 (d, *J =* 3.3 Hz, 1H), 7.30-7.25 (m, 2H), 7.23-7.18 (m, 3H), 6.32 (d, *J* = 7.6 Hz, 1H), 5.94 (d, *J* = 8.3 Hz, 1H), 5.25-5.16 (m, 1H), 4.62 (d, *J* = 6.3 Hz, 2H), 4.38-4.30 (m, 1H), 3.63-3.56 (m, 1H), 3.48-3.42 (m, 1H), 3.40-3.36 (m, 1H), 3.17-3.11 (m, 1H), 2.93-2.84 (m, 1H), 2.00-1.85 (m, 4H), 1.80-1.74 (m, 2H), 1.44-1.33 (m, 2H), 1.25 (d, *J =* 6.4 Hz, 3H). |
| | | [M+H] ⁺=552.6 |
| **552** | 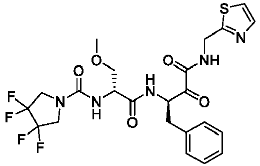 | ¹H NMR (400 MHz, DMSO-*d*₆) *δ* 9.54 (t, *J* = 6.2 Hz, 1H), 8.43 (d, *J =* 7.0 Hz, 1H), 7.73 (d, *J =* 3.3 Hz, 1H), 7.64 (d, *J =* 3.3 Hz, 1H), 7.29-7.25 (m, 2H), 7.24-7.16 (m, 3H), 6.71 (d, *J* = 8.2 Hz, 1H), 5.21-5.12 (m, 1H), 4.67-4.56 (m, 2H), 4.48-4.37 (m, 1H), 4.08-3.89 (m, 4H), 3.52-3.40 (m, 2H), 3.22 (s, 3H), 3.18-3.08 (m, 1H), 2.94-2.83 (m, 1H). |
| | | [M+H] ⁺=560.2 |
| **553** | 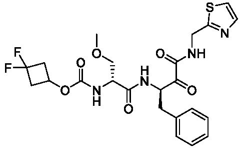 | ¹H NMR (400 MHz, DMSO-*d*₆) *δ* 9.66-9.51 (m, 1H), 8.53-8.37 (m, 1H), 7.79-7.71 (m, 1H), 7.68-7.60 (m, 1H), 7.50-7.37 (m, 1H), 7.31-7.25 (m, 2H), 7.25-7.14 (m, 3H), 5.33-5.14 (m, 1H), 4.87-4.73 (m, 1H), 4.67-4.57 (m, 2H), 4.30-4.18 (m, 1H), 3.48-3.37 (m, 1H), 3.28 (d, *J* = 6.1 Hz, 1H), 3.21 (s, 3H), 3.13-3.10 (m, 1H), 3.07-2.95 (m, 2H), 2.90-2.79 (m, 1H), 2.69-2.53 (m, 2H). |
| | | [M+H] ⁺=525.2 |
| **554** | 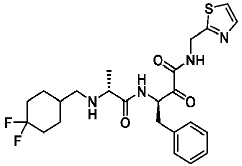 | ¹H NMR (400 MHz, DMSO-*d*₆) *δ* 9.73-9.61 (m, 1H), 9.27-9.17 (m, 1H), 7.79-7.72 (m, 1H), 7.68-7.64 (m, 1H), 7.35-7.17 (m, 5H), 5.43-5.30 (m, 1H), 4.70-4.60 (m, 2H), 3.89-3.75 (m, 1H), 3.28-3.17 (m, 1H), 2.84-2.72 (m, 1H), 2.65-2.54 (m, 1H), 2.31-2.19 (m, 1H), 2.08-1.90 (m, 3H), 1.85-1.61 (m, 7H), 1.39 (d, *J =* 6.9, 2.4 Hz, 3H). |
| | | [M+H] ⁺=493.2 |
| **555** | 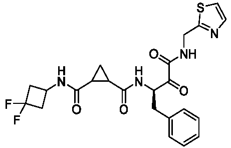 | ¹H NMR (400 MHz, DMSO-*d*₆) *δ* 9.53-9.44 (m, 1H), 8.54-8.45 (m, 1H), 8.35-8.22 (m, 1H), 7.76-7.71 (m, 1H), 7.66-7.63 (m, 1H), 7.31-7.25 (m, 2H), 7.23-7.19 (m, 3H), 5.19-5.10 (m, 1H), 4.63-4.56 (m, 2H), 3.99-3.89 (m, 2H), 3.12-3.04 (m, 1H), 2.87-2.73 (m, 4H), 2.63-2.53 (m, 1H), 1.96-1.79 (m, 2H), 1.35-1.27 (m, 1H), 1.10-1.03 (m, 1H). |
| | | [M+H] ⁺⁺=491.1 |
| **556** | 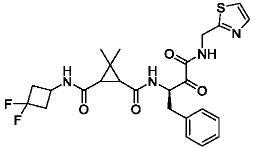 | ¹H NMR (400 MHz, DMSO-*d*₆) *δ* 9.56-9.48 (m, 1H), 8.78-8.72 (m, 1H), 7.73 (dd, *J =* 3.3, 0.8 Hz, 1H), 7.64 (dd, *J =* 3.3, 2.0 Hz, 1H), 7.28-7.19 (m, 5H), 7.17-7.13 (m, 1H), 5.25-5.16 (m, 1H), 4.66-4.58 (m, 2H), 4.01-3.96 (m, 1H), 3.18-3.10 (m, 1H), 2.96-2.78 (m, 4H), 2.80-2.73 (m, 1H), 1.75-1.70 (m, 1H), 1.68-1.64 (m, 1H), 1.18 (s, 6H). |
| | | [M+H] ⁺=519.2 |
| **557** | 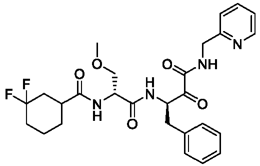 | ¹H NMR (400 MHz, DMSO-*d*₆) *δ* 9.21 (t, *J* = 6.1 Hz, 1H), 8.55-8.47 (m, 2H), 8.33 (dd, *J* = 14.6, 7.0 Hz, 1H), 8.17-8.06 (m, 1H), 7.81-7.73 (m, 1H), 7.32-7.25 (m, 3H), 7.23-7.17 (m, 4H), 5.29-5.16 (m, 1H), 4.58-4.49 (m, 1H), 4.46-4.40 (m, 2H), 3.47-3.38 (m, 1H), 3.31-3.28 (m, 1H), 3.21 (s, 3H), 3.13-3.09 (m, 1H), 2.92-2.83 (m, 1H), 2.59-2.52 (m, 1H), 2.03-1.94 (m, 1H), 1.84-1.66 (m, 4H), 1.47-1.27 (m, 2H). |
| | | [M+H] ⁺=531.2 |
| **558** | 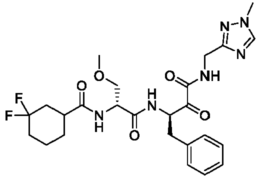 | ¹H NMR (400 MHz, DMSO-*d*₆) *δ* 9.18-9.03 (m, 1H), 8.37 (s, 1H), 8.33-8.17 (m, 1H), 8.15-8.02 (m, 1H), 7.32-7.11 (m, 5H), 5.34-5.20 (m, 1H), 4.58-4.46 (m, 1H), 4.41-4.29 (m, 2H), 3.81 (s, 3H), 3.51-3.37 (m, 1H), 3.27-3.10 (m, 5H), 2.90-2.73 (m, 1H), 2.60-2.52 (m, 1H). |
| | | [M+H] ⁺=535.6 |
| **559** | 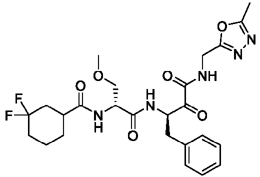 | ¹H NMR (400 MHz, DMSO-*d*₆) *δ* 9.42 (d, *J =* 27.0 Hz, 3H), 8.33 (dd, *J =* 33.5, 7.2 Hz, 1H), 8.16-7.98 (m, 1H), 7.30-7.10 (m, 5H), 5.34-5.10 (m, 1H), 4.58-4.41 (m, 3H), 3.42 (q, *J* = 6.5, 5.4 Hz, 1H), 3.22 (d, *J* = 3.3 Hz, 1H), 3.23-3.15 (m, 1H), 3.13 (d, *J* = 6.4 Hz, 3H), 2.92-2.74 (m, 1H), 2.59-2.50 (m, 1H), 2.45 (d, *J =* 17.8 Hz, 3H), 2.06-1.92 (m, 2H), 1.89-1.63 (m, 4H), 1.48-1.34 (m, 1H), 1.35-1.25 (m, 1H). |
| | | [M+H] ⁺=536.2 |
| **560** | 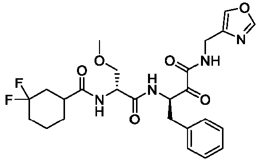 | ¹H NMR (400 MHz, DMSO-*d*₆) *δ* 9.20-9.08 (m, 1H), 8.32 (s, 1H), 8.30-8.26 (m, 1H), 8.14-8.08 (m, 1H), 7.91 (s, 1H), 7.29-7.24 (m, 2H), 7.23-7.15 (m, 3H), 5.29-5.15 (m, 1H), 4.54-4.43 (m, 2H), 4.21-4.14 (m, 1H), 3.45-3.40 (m, 1H), 3.30-3.27 (m, 1H), 3.21 (s, 3H), 3.16-3.09 (m, 2H), 2.84-2.74 (m, 1H), 2.03-1.93 (m, 2H), 1.83-1.66 (m, 4H), 1.48-1.35 (m, 2H). |
| | | [M+H] ⁺=521.2 |
| **561** | 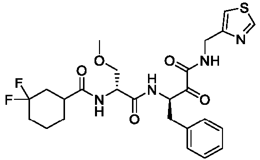 | ¹H NMR (400 MHz, DMSO-*d*₆) *δ* 9.35-9.17 (m, 1H), 9.05 (s, 1H), 8.45-8.28 (m, 1H), 8.16-8.06 (m, 1H), 7.38 (d, *J* = 16.5 Hz, 1H), 7.30-7.10 (m, 5H), 5.28-5.14 (m, 1H), 4.56-4.40 (m, 3H), 3.48-3.36 (m, 2H), 3.23-3.07 (m, 4H), 2.91-2.78 (m, 1H), 2.63-2.53 (m, 1H), 2.08-1.93 (m, 2H), 1.87-1.57 (m, 4H), 1.49-1.32 (m, 2H). |
| | | [M+H] ⁺=537.2 |
| **562** | 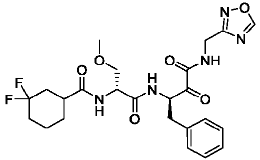 | ¹H NMR (400 MHz, DMSO-*d*₆) *δ* 9.60-9.54 (m, 1H), 9.47-9.34 (m, 1H), 8.39-8.25 (m, 1H), 8.15-8.06 (m, 1H), 7.30-7.25 (m, 2H), 7.24-7.18 (m, 3H), 5.29-5.16 (m, 1H), 4.55-4.43 (m, 3H), 3.48-3.38 (m, 1H), 3.31-3.27 (m, 1H), 3.22 (s, 3H), 3.16-3.10 (m, 2H), 2.90-2.76 (m, 1H), 2.60-2.51 (m, 1H), 2.04-1.92 (m, 2H), 1.86-1.63 (m, 4H), 1.46-1.34 (m, 1H), 1.32-1.25 (m, 1H). |
| | | [M+H] ⁺=522.2 |
| **563** | 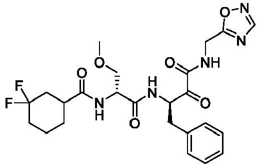 | ¹H NMR (400 MHz, DMSO-*d*₆) *δ* 9.59-9.47 (m, 1H), 8.98-8.93 (m, 1H), 8.43-8.27 (m, 1H), 8.16-8.07 (m, 1H), 7.30-7.25 (m, 2H), 7.24-7.18 (m, 4H), 5.25-5.14 (m, 1H), 4.74-4.61 (m, 2H), 4.57-4.45 (m, 1H), 3.46-3.41 (m, 1H), 3.32-3.29 (m, 1H), 3.23 (d, *J =* 3.3 Hz, 3H), 3.14-3.09 (m, 1H), 2.91-2.79 (m, 1H), 2.59-2.52 (m, 1H), 2.05-1.94 (m, 2H), 1.87-1.65 (m, 4H), 1.47-1.34 (m, 1H), 1.30-1.22 (m, 1H). |
| | | [M+H] ⁺=522.2 |
| **564** | 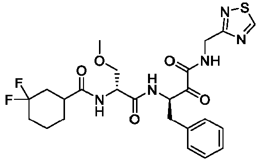 | ¹H NMR (400 MHz, DMSO-*d*₆) *δ* 10.25 (d, *J =* 1.6 Hz, 1H), 9.46-9.32 (m, 1H), 8.38-8.22 (m, 1H), 8.15-8.06 (m, 1H), 7.30-7.24 (m, 2H), 7.24-7.19 (m, 3H), 5.31-5.21 (m, 1H), 4.69-4.62 (m, 2H), 4.56-4.47 (m, 1H), 3.48-3.40 (m, 1H), 3.31-3.27 (m, 1H), 3.23 (d, *J =* 3.4 Hz, 3H), 3.18-3.15 (m, 1H), 2.90-2.77 (m, 1H), 2.60-2.52 (m, 1H), 2.03-1.94 (m, 2H), 1.83-1.66 (m, 4H), 1.48-1.34 (m, 1H), 1.33-1.25 (m, 1H). |
| | | [M+H] ⁺=538.2 |
| **565** | 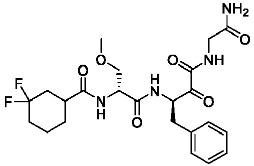 | ¹H NMR (400 MHz, DMSO-*d*₆) *δ* 8.72-8.67 (m, 1H), 8.25-8.21 (m, 1H), 8.12 (d, *J* = 8.1 Hz, 1H), 7.38 (s, 2H), 7.27-7.24 (m, 2H), 7.20 (d, 3H), 5.26-5.19 (m, 1H), 4.55-4.45 (m, 2H), 4.38-4.29 (m, 1H), 3.23 (d, *J =* 3.4 Hz, 3H), 3.14-3.13 (m, 2H), 3.06-3.04 (m, 1H), 2.84-2.79 (m, 1H), 2.59-2.53 (m, 1H), 1.99-1.94 (m, 2H), 1.79-1.72 (m, 4H), 1.33-1.23 (m, 2H). |
| | | [M+H] ⁺=497.2 |
| **566** | 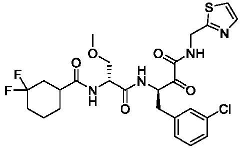 | ¹H NMR (400 MHz, DMSO-*d*₆) *δ* 9.66-9.52 (m, 1H), 8.54-8.38 (m, 1H), 8.14-8.05 (m, 1H), 7.73 (t, *J =* 3.1 Hz, 1H), 7.66-7.60 (m, 1H), 7.34-7.25 (m, 3H), 7.20-7.15 (m, 1H), 5.27-5.10 (m, 1H), 4.68-4.59 (m, 2H), 4.56-4.46 (m, 1H), 3.44-3.38 (m, 1H), 3.31-3.26 (m, 1H), 3.22 (d, *J =* 3.0 Hz, 3H), 3.19-3.16 (m, 1H), 3.14-3.11 (m, 1H), 2.92-2.78 (m, 1H), 2.09-1.92 (m, 2H), 1.87-1.61 (m, 4H), 1.48-1.34 (m, 1H), 1.32-1.25 (m, 1H). |
| | | [M+H] ⁺=571.2 |
| **567** | 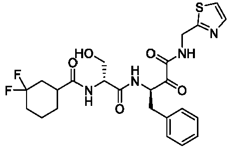 | ¹H NMR (400 MHz, DMSO-*d*₆) *δ* 9.61-9.45 (m, 1H), 8.53-8.38 (m, 1H), 8.15-8.03 (m, 1H), 7.77-7.68 (m, 1H), 7.65-7.58 (m, 1H), 7.51-7.42 (m, 1H), 7.40-7.31 (m, 1H), 5.31-5.11 (m, 1H), 4.64-4.55 (m, 2H), 4.51-4.42 (m, 1H), 3.41-3.36 (m, 1H), 3.31-3.26 (m, 1H), 3.20 (d, *J* = 3.0 Hz, 2H), 3.17 (s, 3H), 2.96-2.80 (m, 1H), 1.98 (s, 2H), 1.86-1.61 (m, 4H), 1.49-1.30 (m, 2H). |
| | | [M+H] ⁺=523.2 |
| **568** | 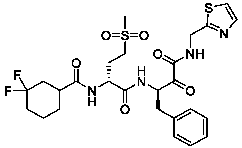 | ¹H NMR (400 MHz, DMSO-*d*₆) *δ* 9.67-9.57 (m, 1H), 8.48-8.39 (m, 1H), 8.20-8.08 (m, 1H), 7.73 (d, 1H), 7.64 (d, *J =* 3.2 Hz, 1H), 7.32-7.23 (m, 2H), 7.26-7.17 (m, 3H), 5.28-5.19 (m, 1H), 4.70-4.56 (m, 2H), 4.47-4.35 (m, 1H), 3.15-3.12 (m, 1H), 3.09-3.03 (m, 1H), 2.97-2.92 (m, 3H), 2.89-2.78 (m, 2H), 2.11-1.88 (m, 4H), 1.85-1.70 (m, 4H), 1.68-1.62 (m, 1H), 1.46-1.37 (m, 1H), 1.34-1.26 (m, 1H). |
| | | [M+H] ⁺=599.2 |
| **569** | 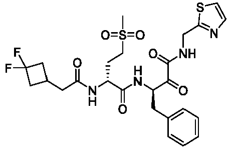 | ¹H NMR (400 MHz, DMSO-*d*₆) *δ* 9.65-9.56 (m, 1H), 8.49 (t, *J =* 6.6 Hz, 1H), 8.17-8.06 (m, 1H), 7.73 (d, *J =* 3.3 Hz, 1H), 7.64 (d, *J* = 3.3 Hz, 1H), 7.30-7.25 (m, 2H), 7.24-7.19 (m, 3H), 5.27-5.21 (m, 1H), 4.68-4.57 (m, 2H), 4.47-4.38 (m, 1H), 3.19-3.12 (m, 1H), 3.08-3.00 (m, 1H), 2.94 (s, 3H), 2.89-2.77 (m, 1H), 2.69-2.57 (m, 3H), 2.33-2.17 (m, 4H), 2.08-1.98 (m, 1H), 1.96-1.86 (m, 1H). |
| | | [M+H] ⁺=585.7 |
| **570** | 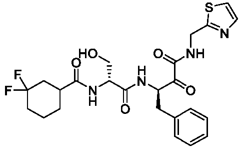 | ¹H NMR (400 MHz, DMSO-*d*₆) *δ* 9.64-9.51 (m, 1H), 8.29-8.14 (m, 1H), 8.01-7.91 (m, 1H), 7.76-7.70 (m, 1H), 7.67-7.57 (m, 1H), 7.29-7.23 (m, 2H), 7.23-7.17 (m, 3H), 5.29-5.15 (m, 1H), 4.88-4.77 (m, 1H), 4.69-4.55 (m, 2H), 4.39-4.25 (m, 1H), 3.60-3.51 (m, 1H), 3.52-3.39 (m, 2H), 3.18-3.07 (m, 1H), 2.92-2.83 (m, 1H), 2.10-1.95 (m, 2H), 1.87-1.62 (m, 4H), 1.47-1.36 (m, 1H), 1.32-1.26 (m, 1H). |
| | | [M+H] ⁺=523.2 |
| **571** | 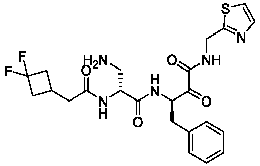 | ¹H NMR (400 MHz, DMSO-*d*₆) *δ* 9.62-9.49 (m, 1H), 8.30-8.21 (m, 1H), 7.95-7.89 (m, 1H), 7.75-7.72 (m, 1H), 7.66-7.62 (m, 1H), 7.29-7.24 (m, 2H), 7.23-7.17 (m, 3H), 5.28-5.20 (m, 1H), 4.85-4.78 (m, 1H), 4.65-4.59 (m, 2H), 4.39-4.28 (m, 1H), 3.58-3.51 (m, 1H), 3.49-3.40 (m, 2H), 3.16-3.08 (m, 1H), 2.93-2.84 (m, 1H), 2.70-2.54 (m, 3H), 2.33-2.20 (m, 3H). |
| | | [M+H] ⁺=508.2 |
| **572** | 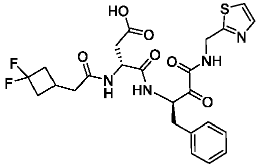 | ¹H NMR (400 MHz, DMSO-*d*₆) *δ* 12.29 (s, 1H), 9.68-9.48 (m, 1H), 8.21 (t, *J* = 7.7 Hz, 1H), 8.15 (dd, *J* = 8.1, 2.8 Hz, 1H), 7.73 (d, *J* = 3.2 Hz, 1H), 7.64 (d, *J* = 3.2 Hz, 1H), 7.34-7.24 (m, 2H), 7.24-7.16 (m, 3H), 5.25-5.15 (m, 1H), 4.69-4.52 (m, 3H), 3.16-3.06 (m, 1H), 2.94-2.82 (m, 1H), 2.71-2.54 (m, 3H), 2.45-2.37 (m, 1H), 2.35-2.18 (m, 5H). |
| | | [M+H] ⁺=537.5 |
| **573** | 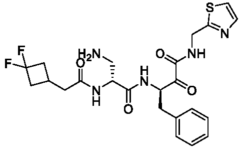 | H NMR (400 MHz, DMSO-*d*₆) *δ* 9.69-9.57 (m, 1H), 8.61-8.42 (m, 2H), 8.22-8.11 (m, 3H), 7.75 (d, *J =* 3.3 Hz, 1H), 7.67 (d, *J* = 3.3 Hz, 1H), 7.30-7.25 (m, 2H), 7.24-7.18 (m, 3H), 5.28-5.18 (m, 1H), 4.67-4.61 (m, 2H), 4.59-4.51 (m, 1H), 3.19-3.11 (m, 1H), 3.09-3.00 (m, 1H), 2.96-2.82 (m, 2H), 2.71-2.58 (m, 2H), 2.46-2.38 (m, 1H), 2.37-2.24 (m, 1H), 2.04-1.93 (m, 1H), 1.89-1.71 (m, 2H). |
| | | [M+H] ⁺=508.2 |
| **574** | 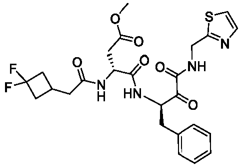 | ¹H NMR (400 MHz, DMSO-*d*₆) *δ* 9.67-9.51 (m, 1H), 8.38-8.25 (m, 1H), 8.22-8.08 (m, 1H), 7.74 (d, 1H), 7.65 (d, *J =* 3.3 Hz, 1H), 7.32-7.25 (m, 2H), 7.24-7.13 (m, 3H), 5.26-5.16 (m, 2H), 4.69-4.59 (m, 3H), 3.58 (s, 3H), 3.18-3.08 (m, 1H), 2.94-2.79 (m, 1H), 2.68-2.55 (m, 3H), 2.38-2.20 (m, 5H). |
| | | [M+H] ⁺=551.2 |
| **575** | 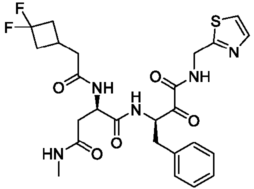 | ¹H NMR (400 MHz, DMSO-*d*₆) *δ* 9.70-9.36 (m, 1H), 8.08-7.88 (m, 1H), 7.80-7.54 (m, 3H), 7.34-7.05 (m, 6H), 5.34-5.09 (m, 1H), 4.69-4.42 (m, 3H), 3.20-3.06 (m, 1H), 2.95-2.76 (m, 1H), 2.66-2.52 (m, 6H), 2.37-2.21 (m, 6H). |
| | | [M+H] ⁺=550.2 |

The beneficial effects of the compound according to the present invention were demonstrated by reference to Experimental examples.

### Experimental example 1: Assay of the inhibitory level of the compound according to the present invention on M^{pro} enzyme activity

### (1) Experimental methods

The recombinant SARS-CoV-2 M^{pro} (with a final concentration of 750 nM) was mixed with a series of dilutions for each compound in 25 µL assay buffer (20 mM Tris-HCl, pH 7.5, 150 mM NaCl, 1 mM EDTA, 2 mM DTT) and incubated for 10 min. The reaction was initiated by adding 25 µL of fluorescent substrate (MCA-A VLQtSGFR-Lys (Dnp)-Lys-NH₂), with a final concentration of 20 µM, and the fluorescence signal at 320 nm (excitation)/405 nm (emission) was measured using the microplate reader (BMG). The Vmax of the reaction with the addition of compounds at different concentrations was calculated, together with the Vmax of the reaction with the addition of DMSO, that were used to generate an IC₅₀ curve. For each compound, the IC₅₀ value of anti-SARS-CoV-2 M^{pro} was measured using 9 concentrations, with 3 independent repeated experiments. All experimental data were analyzed using GraphPad Prism software.

### (2) Experimental results

**Table 2. The inhibitory effects of compounds on SARS-COV-2 M^{pro} enzyme activity.**

| **Compounds** | **IC₅₀** | **Compounds** | **IC₅₀** |
|---|---|---|---|
| **1** | 14.6±1.3 nM | **294** | 14.5±0.2 nM |
| **2** | 17.6±0.4 nM | **295** | 12.2±0.0 nM |
| **3** | 410.6±28.2 nM | **296** | 14.8±0.1 nM |
| **4** | 20.4±1.8 nM | **297** | 18.1±0.3 nM |
| **5** | 15.4±0.7 nM | **298** | 14.9±0.1 nM |
| **6** | 48.3±1.2 nM | **299** | 15.7±0.1 nM |
| **7** | 28.5±0.9 nM | **300** | 2.43±0.04 µM |
| **9** | 12.1±2.5 nM | **301** | 9.2±0.4 nM |
| **10** | 113.5±32.1 nM | **302** | 41.5±0.9 nM |
| **12** | 12.3±0.8 nM | **303** | 10.7±1.0 nM |
| **13** | 14.3±0.4 nM | **304** | 53.8±5.8 nM |
| **14** | 19.3±2.8 nM | **305** | 24.3±0.4 nM |
| **15** | 9.8±0.1 nM | **306** | 19.3±1.0 nM |
| **16** | 18.9±1.7 nM | **307** | 26.0±2.1 nM |
| **17** | 19.8±0.2 nM | **308** | 13.9±1.2 nM |
| **18** | 13.7±2.0 nM | **309** | 19.4±0.0 nM |
| **19** | 45.2±1.2 nM | **310** | 12.4±0.0 nM |
| **20** | 15.2±0.2 nM | **311** | 16.7±0.6 nM |
| **21** | 26.9±3.9 nM | **312** | 14.5±0.1 nM |
| **22** | 18.6±1.7 nM | **313** | 16.0±2.0 nM |
| **25** | 24.9±0.3 nM | **314** | 12.4±0.3 nM |
| **26** | 13.9±0.2 nM | **315** | 20.2±3.3 nM |
| **27** | 885.8±0.2 nM | **316** | 15.9±0.4 nM |
| **28** | >5 µM | **317** | 16.0±0.3 nM |
| **29** | >5 µM | **318** | 10.7±0.3 nM |
| **30** | >5 µM | **319** | 37.1±2.9 nM |
| **31** | >5 µM | **320** | 16.7±0.3 nM |
| **32** | >5 µM | **321** | 165.8±2.6 nM |
| **34** | >5 µM | **322** | 125.2±6.0 nM |
| **36** | 30.9±0.2 nM | **323** | 30.7±1.9 nM |
| **38** | 796.3±1.1 nM | **324** | 9.6±0.1 nM |
| **39** | >5 µM | **325** | 177.4±2.5 nM |
| **41** | >2.5 µM | **326** | 20.7±0.5 nM |
| **42** | 17.6±0.4 nM | **327** | 245.4±2.7 nM |
| **43** | 20.2±2.5 nM | **328** | 31.7±0.1 nM |
| **44** | 21.7±2.1 nM | **329** | 19.4±1.8 nM |
| **45** | 14.7±1.0 nM | **330** | 194.8±14.5 nM |
| **46** | 13.3±0.1 nM | **331** | 100.8±2.8 nM |
| **47** | 14.1±0.1 nM | **332** | 888.3±1.2 nM |
| **48** | 30.9±0.3 nM | **333** | 13.4±0.2 nM |
| **49** | 14.7±0.4 nM | **334** | >5 µM |
| **52** | 44.2±3.4 nM | **336** | 15.0±0.8 nM |
| **53** | 187.8±0.7 nM | **337** | 87.9±14.6 nM |
| **54** | 298.4±2.9 nM | **338** | 20.0±0.8 nM |
| **57** | 351.5±6.8 nM | **339** | 6.13±0.22 µM |
| **58** | 100.9±0.1 nM | **340** | 76.2±1.7 nM |
| **59** | 26.6±1.8 nM | **341** | 11.53±3.79 µM |
| **60** | 24.9±0.1 nM | **342** | 22.2±0.2 nM |
| **61** | 11.8±0.1 nM | **343** | 18.3±0.7 nM |
| **62** | 27.8±0.9 nM | **344** | 857.2±3.1 nM |
| **63** | 19.6±0.3 nM | **345** | 60.9±4.7 nM |
| **64** | 16.1±0.2 nM | **346** | >2.5 µM |
| **65** | 21.2±0.8 nM | **347** | >2.5 µM |
| **66** | 11.7±0.1 nM | **350** | 42.0±3.7 nM |
| **67** | 17.3±1.6 nM | **351** | 1.16±0.32 µM |
| **68** | 12.4±1.1 nM | **352** | 2.50±0.29 µM |
| **69** | 17.7±1.5 nM | **353** | 845.8±2.1 nM |
| **70** | 22.9±0.3 nM | **354** | 27.5±0.7 nM |
| **71** | 10.9±0.5 nM | **355** | >2.5 µM |
| **72** | 27.7±4.1 nM | **356** | 43.1±0.1 nM |
| **73** | 25.8±0.5 nM | **357** | 22.3±0.9 nM |
| **74** | 23.3±0.4 nM | **358** | 93.2±1.1 nM |
| **75** | 19.3±0.7 nM | **359** | 36.4±1.9 nM |
| **76** | 23.0±1.1 nM | **360** | 31.0±3.9 nM |
| **77** | 24.1±0.0 nM | **361** | 27.2±2.8 nM |
| **78** | 53.2±0.5 nM | **362** | 34.0±0.6 nM |
| **79** | 10.9±0.1 nM | **363** | 133.2±10.0 nM |
| **80** | 58.6±3.2 nM | **364** | 75.1±0.1 nM |
| **81** | 27.9±5.5 nM | **365** | 23.1±1.0 nM |
| **82** | 5.8±0.1 nM | **366** | 64.6±0.7 nM |
| **83** | 6.7±0.1 nM | **367** | 105.4±10.5 nM |
| **84** | 40.0±2.4 nM | **368** | 196.8±14.0 nM |
| **85** | 56.7±2.7 nM | **369** | >2.5 µM |
| **86** | 70.5±2.5 nM | **370** | 14.8±0.7 nM |
| **87** | 42.1±1.0 nM | **371** | 31.7±2.6 nM |
| **88** | 24.4±0.0 nM | **372** | 10.6±0.4 nM |
| **89** | 40.9±1.2 nM | **373** | 16.0±0.8 nM |
| **90** | 44.2±0.7 nM | **374** | 20.8±1.6 nM |
| **91** | 48.4±2.0 nM | **375** | 13.8±1.2 nM |
| **92** | 26.6±0.4 nM | **376** | 35.2±3.6 nM |
| **93** | 28.8±1.0 nM | **377** | 80.0±3.6 nM |
| **94** | 13.5±1.3 nM | **378** | 21.2±0.3 nM |
| **95** | 8.2±1.1 nM | **379** | 22.5±0.7 nM |
| **96** | 20.2±0.7 nM | **380** | 55.8±7.2 nM |
| **97** | 37.2±4.8 nM | **381** | 416.8±4.0 nM |
| **98** | 40.7±1.9 nM | **382** | 15.0±1.3 nM |
| **99** | 27.8±1.1 nM | **383** | 21.5±0.8 nM |
| **100** | 13.0±1.4 nM | **384** | 15.5±0.3 nM |
| **101** | 21.6±0.8 nM | **385** | 55.8±7.2 nM |
| **102** | 26.6±0.4 nM | **386** | 166.5±2.1 nM |
| **103** | 28.8±1.0 nM | **387** | 12.5±1.2 nM |
| **104** | 16.8±1.1 nM | **388** | 62.9±8.1 nM |
| **105** | 25.3±1.7 nM | **389** | 109.9±1.6 nM |
| **106** | 160.8±4.1 nM | **390** | 21.8±1.1 nM |
| **107** | 52.0±0.2 nM | **391** | 108.8±8.6 nM |
| **108** | 66.4±1.2 nM | **392** | 62.8±3.4 nM |
| **109** | 168.9±0.1 nM | **393** | 88.2±5.6 nM |
| **110** | 31.3±0.1 nM | **394** | 12.4±1.4 nM |
| **111** | 25.3±0.2 nM | **395** | 18.4±1.3 nM |
| **112** | 112.6±0.5 nM | **396** | 48.8±0.4 nM |
| **113** | 69.1±8.1 nM | **397** | 54.9±1.8 nM |
| **114** | 34.9±1.0 nM | **398** | 19.3±2.9 nM |
| **115** | 37.8±3.0 nM | **399** | 32.8±1.5 nM |
| **116** | 130.1±0.4 nM | **400** | 10.2±0.1 nM |
| **117** | 46.1±0.3 nM | **401** | 19.0±0.1 nM |
| **118** | 40.2±3.4 nM | **402** | 19.9±0.3 nM |
| **119** | 16.5±1.5 nM | **403** | 20.4±2.4 nM |
| **120** | 113.3±1.0 nM | **404** | 47.7±1.0 nM |
| **121** | 50.7±0.4 nM | **405** | 104.2±1.7 nM |
| **122** | 280.9±2.3 nM | **406** | 52.7±1.1 nM |
| **123** | 162.9±2.0 nM | **407** | 21.4±0.3 nM |
| **124** | 45.1±0.6 nM | **408** | 14.9±0.5 nM |
| **125** | 19.7±1.2 nM | **409** | 36.6±0.4 nM |
| **126** | 11.1±0.6 nM | **410** | 25.3±1.7 nM |
| **127** | 40.8±0.6 nM | **411** | 15.5±0.1 nM |
| **128** | 9.4±1.3 nM | **412** | 24.8±0.8 nM |
| **129** | 36.8±1.9 nM | **413** | 29.1±2.5 nM |
| **130** | 98.6±6.8 nM | **414** | 95.9±9.7 nM |
| **131** | 36.8±0.1 nM | **415** | 19.4±2.2 nM |
| **132** | 157.6±26.2 nM | **416** | 19.0±0.4 nM |
| **133** | 197.9±9.6 nM | **417** | 22.1±3.0 nM |
| **134** | 24.5±0.0 nM | **418** | 10.6±0.3 nM |
| **135** | 57.3±5.3 nM | **419** | 9.8±0.1 nM |
| **136** | 96.5±2.2 nM | **420** | 10.8±0.7 nM |
| **137** | 22.1±7.7 nM | **421** | 20.7±0.5 nM |
| **138** | 48.6±0.4 nM | **422** | 17.1±0.1 nM |
| **139** | 24.3±2.3 nM | **423** | 13.2±0.2 nM |
| **140** | 15.5±1.0 nM | **424** | 20.7±0.2 nM |
| **141** | 1.16±0.24 µM | **425** | 36.5±0.3 nM |
| **142** | 1.21±0.89 µM | **426** | 9.4±0.1 nM |
| **143** | 16.4±1.5 nM | **427** | 20.9±0.4 nM |
| **144** | 19.0±0.3 nM | **428** | 17.1±0.1 nM |
| **145** | 17.1±2.5 nM | **429** | 33.8±1.0 nM |
| **146** | 12.3±0.2 nM | **430** | 20.0±0.5 nM |
| **147** | 13.0±0.7 nM | **431** | 233.7±1.8 nM |
| **148** | 11.7±2.4 nM | **432** | 11.5±0.1 nM |
| **149** | 36.4±7.4 nM | **433** | 749.6±1.1 nM |
| **150** | 12.2±0.1 nM | **434** | 2.05±0.02 µM |
| **151** | 25.7±0.6 nM | **435** | 58.2±1.3 nM |
| **152** | 18.7±0.6 nM | **436** | 171.0±2.4 nM |
| **153** | 12.0±0.6 nM | **437** | 61.1±1.2 nM |
| **154** | 193.5±2.0 nM | **438** | 23.4±1.8 nM |
| **155** | 14.1±0.1 nM | **439** | 46.2±0.3 nM |
| **156** | 33.4±0.9 nM | **440** | 20.4±0.4 nM |
| **157** | 12.5±0.5 nM | **441** | 18.5±0.3 nM |
| **158** | 252.3±9.0 nM | **442** | 20.6±1.0 nM |
| **159** | 21.5±5.7 nM | **443** | 167.9±1.9 nM |
| **160** | 13.5±3.5 nM | **444** | 214.3±1.2 nM |
| **161** | 15.3±1.6 nM | **445** | 21.8±0.3 nM |
| **162** | 17.5±0.8 nM | **446** | 414.1±1.8 nM |
| **163** | 20.8±0.7 nM | **447** | 187.3±1.1 nM |
| **164** | 13.2±0.5 nM | **448** | 59.9±0.5 nM |
| **165** | 20.3±0.2 nM | **449** | 45.3±1.2 nM |
| **166** | 23.3±0.8 nM | **450** | 45.4±0.2 nM |
| **167** | 3.81±0.17 µM | **451** | 34.4±1.8 nM |
| **168** | 10.7±0.4 nM | **452** | 66.2±0.9 nM |
| **169** | 22.6±3.7 nM | **453** | 37.2±0.2 nM |
| **170** | 19.4±0.7 nM | **454** | 1.30±0.07 µM |
| **171** | 8.9±0.4 nM | **455** | 77.28±2.31 µM |
| **172** | 14.9±0.7 nM | **456** | 37.24±0.51 µM |
| **173** | 10.3±0.1 nM | **457** | 51.51±0.94 µM |
| **174** | 13.2±1.0 nM | **458** | 11.40±0.08 µM |
| **175** | 47.3±2.8 nM | **459** | 54.74±0.35 µM |
| **176** | 15.9±0.0 nM | **460** | 53.05±3.34 µM |
| **177** | 10.5±0.2 nM | **461** | 62.28±2.07 µM |
| **178** | 17.5±0.5 nM | **462** | 40.68±1.50 µM |
| **179** | 14.5±1.9 nM | **463** | 95.17±5.99 µM |
| **180** | 31.8±0.9 nM | **464** | 3.50±0.23 µM |
| **181** | 16.6±0.3 nM | **465** | 2.55±0.02 µM |
| **182** | 22.4±0.5 nM | **466** | 1.95±0.29 µM |
| **183** | 21.9±0.3 nM | **467** | 3.37±0.18 µM |
| **184** | 13.3±0.2 nM | **468** | 10.48±0.63 µM |
| **185** | 9.7±0.7 nM | **469** | 1.43±0.15 µM |
| **186** | 18.6±1.1 nM | **470** | 2.03±0.07 µM |
| **187** | 10.7±0.5 nM | **471** | 263.5±4.0 nM |
| **188** | 79.0±8.8 nM | **472** | 478.3±4.8 nM |
| **189** | >2.5 µM | **473** | 1.31±0.1 µM |
| **190** | 29.6±0.0 nM | **474** | 948.8±39.9 nM |
| **191** | 73.0±4.5 nM | **475** | 1.22±0.10 µM |
| **192** | 25.4±0.9 nM | **476** | 880.8±1.2 nM |
| **193** | 16.7±0.4 nM | **477** | 1.86±0.04 µM |
| **194** | 386.8±30.0 nM | **478** | 3.62±0.27 µM |
| **195** | 30.8±0.1 nM | **479** | 2.24±0.10 µM |
| **196** | 14.5±0.1 nM | **480** | 2.23±0.37 µM |
| **197** | 13.8±0.8 nM | **481** | 998.3±0.20 nM |
| **198** | 18.1±2.7 nM | **482** | 538.2±4.3 nM |
| **199** | 52.7±1.0 nM | **483** | 338.4±3.0 nM |
| **200** | 219.3±0.6 nM | **484** | 95.7±1.5 nM |
| **201** | 653.4±4.1 nM | **485** | 33.3±1.7 nM |
| **202** | 17.4±1.2 nM | **486** | 45.2±5.2 nM |
| **203** | 19.6±0.6 nM | **487** | 29.3±1.1 nM |
| **204** | 36.7±0.9 nM | **488** | 118.3±1.6 nM |
| **205** | 23.9±0.9 nM | **489** | 10.2±1.7 nM |
| **206** | 18.0±0.5 nM | **490** | 5.21±0.72 µM |
| **207** | 46.1±0.9 nM | **491** | 215.8±7.3 nM |
| **208** | 37.1±1.7 nM | **492** | 66.3±6.1 nM |
| **209** | 12.2±0.2 nM | **493** | 451.5±2.3 nM |
| **210** | 20.7±1.1 nM | **494** | 82.5±3.7 nM |
| **211** | 57.7±3.0 nM | **495** | 2.27±0.09 µM |
| **212** | 346.2±9.9 nM | **496** | 77.0±4.1 nM |
| **213** | 41.4±0.8 nM | **497** | 36.0±2.4 nM |
| **214** | 17.4±0.5 nM | **498** | 102.7±5.3 nM |
| **215** | 13.7±0.9 nM | **499** | 32.9±1.5 nM |
| **216** | 16.3±0.4 nM | **500** | 38.9±0.5 nM |
| **217** | 27.3±1.2 nM | **501** | 63.8±6.0 nM |
| **218** | 23.4±0.8 nM | **502** | 47.0±0.5 nM |
| **219** | 13.4±1.7 nM | **503** | 66.3±6.1 nM |
| **220** | 13.9±0.1 nM | **504** | 47.7±0.9 nM |
| **221** | 15.6±1.2 nM | **505** | 239.5±4.0 nM |
| **222** | 16.5±1.5 nM | **506** | 105.0±2.9 nM |
| **223** | 11.1±0.3 nM | **507** | 12.6±0.4 nM |
| **224** | 25.8±1.3 nM | **508** | 10.6±0.8 nM |
| **225** | 38.2±3.0 nM | **509** | 150.5±6.6 nM |
| **226** | 16.0±0.8 nM | **510** | 136.7±1.6 nM |
| **227** | 19.0±0.3 nM | **511** | 30.4±2.1 nM |
| **228** | 18.1±1.1 nM | **512** | 22.1±0.5 nM |
| **229** | 33.8±1.0 nM | **513** | 30.4±0.8 nM |
| **230** | 64.5±0.7 nM | **514** | 56.8±0.4 nM |
| **231** | 35.4±0.2 nM | **515** | 26.2±0.3 nM |
| **232** | 60.7±0.1 nM | **516** | 33.0±1.9 nM |
| **233** | 10.3±0.3 nM | **517** | 56.2±3.2 nM |
| **234** | 18.8±2.7 nM | **518** | 248.5±1.4 nM |
| **235** | 28.6±2.1 nM | **519** | 32.7±3.1 nM |
| **236** | 41.4±0.1 nM | **520** | 161.3±1.2 nM |
| **237** | 131.7±4.4 nM | **521** | 440.7±4.5 nM |
| **239** | 32.2±1.0 nM | **522** | 74.7±4.2 nM |
| **241** | 42.1±1.6 nM | **523** | 122.7±5.1 nM |
| **242** | 26.8±1.8 nM | **524** | 32.8±3.2 nM |
| **243** | 18.4±0.1 nM | **525** | 84.5±1.8 nM |
| **244** | 15.1±0.1 nM | **526** | 28.4±1.5 nM |
| **245** | >5 µM | **527** | 44.3±1.1 nM |
| **246** | 23.6±0.3 nM | **528** | 50.8±1.0 nM |
| **247** | 38.2±2.8 nM | **529** | 224.5±1.3 nM |
| **248** | 24.6±0.6 nM | **530** | 32.5±1.1 nM |
| **249** | 255.6±6.4 nM | **531** | 598.0±2.8 nM |
| **250** | 42.4±2.4 nM | **532** | 624.5±2.6 nM |
| **251** | 34.2±0.5 nM | **533** | 69.0±6.9 nM |
| **252** | 94.9±2.7 nM | **534** | 1.14±0.15 µM |
| **253** | 22.4±1.4 nM | **535** | 35.7±0.17 nM |
| **254** | 45.5±3.1 nM | **536** | 24.8±0.3 nM |
| **255** | 110.7±4.1 nM | **537** | 89.5±1.5 nM |
| **256** | 20.5±1.7 nM | **538** | 33.5±1.5 nM |
| **257** | 71.8±0.1 nM | **539** | 21.8±0.5 nM |
| **258** | 93.2±0.6 nM | **540** | 26.9±0.1 nM |
| **259** | 21.4±3.5 nM | **541** | 51.8±1.4 nM |
| **260** | 742.0±6.3 nM | **542** | 52.4±7.0 nM |
| **261** | 863.6±0.1 nM | **543** | 106.8±1.3 nM |
| **262** | 933.7±5.0 nM | **544** | 22.5±0.9 nM |
| **263** | 1.56±0.05 µM | **545** | 26.9±0.2 nM |
| **264** | >2.5 µM | **546** | 71.7±7.1 nM |
| **265** | 15.6±0.1 nM | **547** | 25.1±1.0 nM |
| **266** | 34.4±2.3 nM | **548** | 410.4±1.0 nM |
| **267** | 11.2±0.3 nM | **549** | 21.2±0.6 nM |
| **268** | 295.8±3.8 nM | **550** | 66.1±2.9 nM |
| **269** | 19.6±0.4 nM | **551** | 23.3±3.1 nM |
| **270** | >2.5 µM | **552** | 200.9±1.6 nM |
| **271** | 11.5±0.1 nM | **553** | 18.8±4.6 nM |
| **272** | >2.5 µM | **554** | 890.1±1.3 nM |
| **273** | 17.0±0.1 nM | **555** | 477.3±4.1 nM |
| **274** | 910.8±6.5 nM | **556** | 203.5±0.6 nM |
| **275** | 10.9±0.4 nM | **557** | 26.1±0.4 nM |
| **276** | 13.0±0.8 nM | **558** | 1.18±0.01 µM |
| **277** | 30.7±0.6 nM | **559** | > 2.5 µM |
| **278** | 33.7±0.5 nM | **560** | 22.1±0.7 nM |
| **279** | 272.1±6.2 nM | **561** | 22.5±0.9 nM |
| **280** | 13.2±1.2 nM | **562** | > 2.5 µM |
| **281** | 14.8±0.1 nM | **563** | > 2.5 µM |
| **282** | 13.4±0.4 nM | **564** | 774.4±8.8 nM |
| **283** | 14.6±0.0 nM | **565** | 2.07±0.05 µM |
| **284** | 14.4±0.2 nM | **566** | 33.2±2.3 nM |
| **285** | 17.1±0.2 nM | **567** | 63.6±2.9 nM |
| **286** | 16.3±1.5 nM | **568** | 70.7±1.8 nM |
| **287** | 24.1±0.0 nM | **569** | 18.8±0.9 nM |
| **288** | 14.6±0.4 nM | **570** | 40.6±2.0 nM |
| **289** | 10.6±0.1 nM | **571** | 19.8±0.2 nM |
| **290** | 11.3±0.2 nM | **572** | 33.8±2.5 nM |
| **291** | 13.1±2.8 nM | **573** | 68.3±2.7 nM |
| **292** | 11.4±0.2 nM | **574** | 14.4±0.8 nM |
| **293** | 16.4±1.1 nM | **575** | 23.5±0.9 nM |

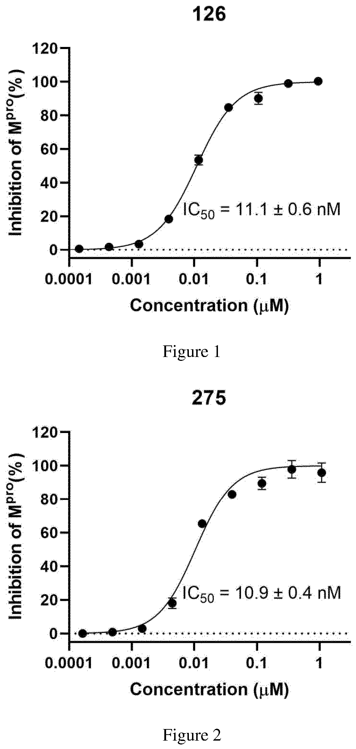

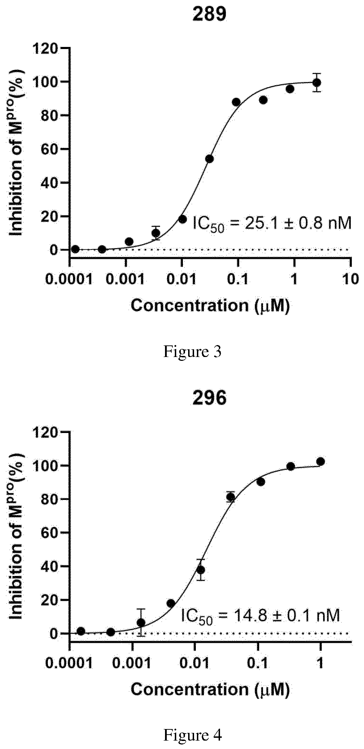

Based on the results of Table 2 and Figures 1 to 5, the compound of the present invention could effectively inhibit the activity of SARS-CoV-2 M^{pro}. Thereby, the compound of the present invention could be used in the manufacturer of SARS-CoV-2 M^{pro} inhibitors, medicaments against novel coronavirus, and medicaments for preventing and/or treating COVID-19.

### Experimental example 2: Cytotoxic assay of compounds against Vero E6 cells

### (1) Experimental methods

The cytotoxicity assessment of compounds was performed against Vero E6 cells. The specific experimental procedures: Vero E6 cells were seeded into a 96-well plate at a cell density of 2 × 10⁴ cells/well, with 100 µL/well, and then incubated overnight at 37 °C in a 5% CO₂ incubator. The next day, 100 µL of drug-containing medium was added to each well. The compound was diluted in a 2-fold serial dilution with a top concentration of 500 µM, for a total of 6 gradients. Three repeated wells were set for each concentration, and negative controls and blank controls without drugs were included for each experiment. After 72 hours of drug treatment, MTT assay was used to detect cell viability and calculate the 50% cytotoxic concentration (CC₅₀) of the compound against Vero E6 cells. All experimental data were analyzed using GraphPad Prism software.

### (2) Experimental results

**Table 3. The cytotoxicity of compounds according to the present invention against Vero E6 cells.**

| **Compounds** | **CC₅₀ (µM)** |
|---|---|
| **275** | 362.97 |
| **172** | 340.73 |
| **177** | 156.57 |
| **126** | >500 |
| **174** | >500 |
| **289** | >500 |
| **317** | >500 |
| **150** | >500 |
| **324** | >500 |
| **155** | 280.87 |
| **354** | >500 |
| **378** | >500 |
| **417** | >500 |
| **418** | >500 |
| **387** | >500 |
| **379** | >500 |
| **375** | >500 |
| **415** | >500 |
| **423** | >500 |
| **395** | 131.5 |
| **398** | >500 |
| **400** | >500 |
| **402** | 409.73 |
| **362** | >500 |

From Table 3, the compound of the present invention had low cytotoxicity against Vero E6 cells.

### Experimental example 3: Evaluation of in vivo pharmacokinetic properties of compounds in rats

### (1) Dosage regimens

Male Sprague-Dawley (SD) rats, weighing 200-230 g, were randomly divided into groups, with three rats for each group, and a series of test compounds were administered intragastrically (p.o.) or intravenously (i.v.) according to the regimens of following Table 4. The rats were fasted for 12 hours before the experiment and could drink water freely. 4 hours after administration, rats were fed uniformly.

The solution for gavage and intravenous administration was prepared with DMSO/HS15/PEG400/NaCl (5/3/40/52, v/v/v). The medicament was given according to the dosage shown in Table 4. The administration time was recorded, and approximately 0.20 mL of blood was collected for each sample from jugular vein or by other suitable methods at the set time points. The sample was anticoagulated using heparin sodium, and after collection, the blood sample was placed on ice, followed by centrifuging to separate the plasma within 1 h (centrifugation conditions: 6800 g, 6 min, 2-8 °C). Plasma samples were stored in a -80 °C freezer before analysis. The grouping and blood collection time points are shown in Table 4, with 3 animals at each time point.

**Table 4. Experimental protocol for evaluating the in vivo pharmacokinetic properties of compounds in rats.**

| **Test compounds** | **Species** | **Administration route** | **Dosage (mg/kg)** | **Sampling time (h)** |
|---|---|---|---|---|
| **275** | SD | i.v. | 1 | |
| | | p.o. | 10 | |
| **150** | SD | i.v. | 1 | |
| | | p.o. | 10 | |
| **126** | SD | i.v. | 1 | |
| | | p.o. | 10 | |
| **289** | SD | p.o. | 10 | |
| **145** | SD | i.v. | 1 | |
| | | p.o. | 10 | |
| **153** | SD | p.o. | 10 | |
| **333** | SD | p.o. | 10 | |
| **296** | SD | i.v. | 1 | 0.0833 h, 0.25 h, 0.5 h, 1 h, 2 h, 4 h, 6 h, 8 h, 24 h |
| | | p.o. | 10 | |
| **290** | SD | p.o. | 10 | |
| **155** | SD | i.v. | 1 | |
| | | p.o. | 10 | |
| **288** | SD | p.o. | 10 | |
| **417** | SD | p.o. | 10 | |
| **398** | SD | i.v. | 1 | |
| | | p.o. | 10 | |
| **395** | SD | i.v. | 1 | |
| | | p.o. | 10 | |
| **400** | SD | i.v. | 1 | |
| | | p.o. | 10 | |
| **379** | SD | i.v. | 1 | |
| | | p.o. | 10 | |
| **378** | SD | i.v. | 1 | |
| | | p.o. | 10 | |
| **354** | SD | i.v. | 1 | |
| | | p.o. | 10 | |

**Table 5. The main pharmacokinetic parameters of compounds.**

| **NO.** | **Admin** | **T_{1/2}** | **Tₘₐₓ** | **Cₘₐₓ** | **AUCₗₐₛₜ** | **AUC_{INF_obs}** | **MRT_{INF_obs}** | **CL** | **Vss_{obs}** | **F** |
|---|---|---|---|---|---|---|---|---|---|---|
| | | **(h)** | **(h)** | **(ng/mL)** | **(h*ng/mL)** | **(h*ng/mL)** | **(h)** | **(mL/min/kg)** | **(mL/kg)** | **%** |
| **275** | i.v. (1 mg/kg) | 2.55±0.22 | 0.19±0.10 | 1719.76±2 35.66 | 2613.19±5 62.73 | 2797.03±7 45.94 | 1.49±0.53 | 1.36±0.23 | 6.22±1.5 0 | - |
| | p.o. (10 mg/kg) | 2.83±0.61 | 0.50±0.00 | 1262.04±6 19.82 | 7932.82±3 923.78 | 8525.92±3 689.41 | 4.22±1.00 | - | - | 4.22 ±1.00 |
| **150** | i.v. (1 mg/kg) | 3.59±0.80 | 0.08±0.00 | 2005.79±3 21.68 | 6420.93±2 817.27 | 6633.76±2 645.21 | 3.94±1.26 | 0.83±0.21 | 2.91±1.4 8 | - |
| | p.o. (10 mg/kg) | 4.26±0.63 | 0.42±0.14 | 1301.30±3 06.83 | 12635.32± 3080.59 | 12913.36± 3231.97 | 5.95±0.09 | - | - | 19.68 ±4.80 |
| **126** | i.v. (1 mg/kg) | 3.65±3.48 | 0.08±0.00 | 1855.38±1 38.39 | 1054.47±2 85.03 | 1070.92±2 95.21 | 1.06±0.56 | 4.63±3.62 | 16.56±5. 41 | - |
| | p.o. (10 mg/kg) | 2.46±0.15 | 0.50±0.00 | 1558.56±1 1.56 | 5265.82±3 24.91 | 5270.77±3 26.97 | 4.02±0.17 | - | - | 49.94± 3.08 |
| **289** | p.o. (10 mg/kg) | 3.28±0.19 | 0.50±0.00 | 1039.07±3 49.02 | 3242.97±4 60.03 | 3251.20±4 62.49 | 2.98±0.33 | - | - | - |
| **145** | i.v. (1 mg/kg) | 1.37±0.05 | 0.08±0.00 | 1636.49±8 6.57 | 1161.84±2 05.24 | 1177.45±2 07.81 | 1.09±0.13 | 1.73±0.37 | 14.46±2. 62 | - |
| | p.o. (10 mg/kg) | 3.19±0.06 | 0.33±0.14 | 1165.92±2 22.56 | 3826.70±8 72.24 | 3845.58±8 74.63 | 3.75±0.56 | - | - | 32.94 ±7.51 |
| **153** | p.o. (10 mg/kg) | 7.14±0.46 | 1.67±0.58 | 1982.19±9 7.72 | 23508.61± 676.35 | 25984.59± 265.33 | 7.08±0.25 | - | - | - |
| **333** | p.o. (10 mg/kg) | 9.74±0.38 | 0.92±0.95 | 1586.56±5 73.41 | 18733.19± 9062.50 | 22358.97± 10828.66 | 7.47±0.38 | - | - | - |
| **296** | i.v. (1 mg/kg) | 3.55±3.39 | 0.08±0.00 | 1401.02±2 2.87 | 1022.92±1 55.71 | 1039.01±1 62.09 | 1.17±0.44 | 4.65±3.87 | 16.32±2. 68 | - |
| | p.o. (10 mg/kg) | 2.80±0.09 | 0.25±0.00 | 1043.31±2 09.46 | 5177.04±1 006.75 | 5187.95±1 010.79 | 4.43±0.87 | - | - | 50.61 ±9.84 |
| **290** | p.o. (10 mg/kg) | 2.66±0.11 | 0.50±0.00 | 722.02±23 5.04 | 3111.10±8 94.24 | 3115.25±8 94.44 | 4.16±0.30 | - | - | - |
| **155** | i.v. (1 mg/kg) | 3.12±0.35 | 0.08±0.00 | 1569.98±7. 88 | 5097.98±6 15.08 | 5120.50±6 27.56 | 3.99±0.42 | 0.88±0.10 | 3.29±0.4 2 | - |
| | p.o. (10 mg/kg) | 3.75±0.62 | 0.33±0.14 | 1147.38±2 89.40 | 12276.77± 2268.09 | 12440.00± 2226.87 | 5.98±0.36 | - | - | - |
| **288** | p.o. (10 mg/kg) | 3.03±0.36 | 0.75±0.43 | 1137.43±2 06.67 | 3494.02±1 247.65 | 3502.38±1 250.32 | 3.59±0.11 | - | - | - |
| **417** | p.o. (10 mg/kg) | 5.07±1.78 | 3.33±1.15 | 441.26±22 4.85 | 4888.00±2 319.99 | 5065.36±2 285.60 | 6.55±0.39 | - | - | - |
| **398** | i.v. (1 mg/kg) | 4.67±0.19 | 0.08±0.00 | 1741.92±2 85.19 | 2298.27±5 87.40 | 2320.99±5 91.73 | 2.34±0.22 | 7.47±1.67 | 3.03±0.7 1 | - |
| | p.o. (10 mg/kg) | 5.16±2.94 | 4.00±2.00 | 1430.18±9 8.51 | 19018.08± 2192.99 | 20197.31± 2602.34 | 6.60±0.89 | - | - | 82.75± 9.54 |
| **395** | i.v. (1 mg/kg) | 9.49±2.16 | 0.08±0.00 | 1697.71±2 37.45 | 2574.49±8 19.96 | 2829.72±9 58.55 | 3.55±0.52 | 6.48±2.63 | 5.25±2.1 3 | - |
| | p.o. (10 mg/kg) | 6.64±1.17 | 4.00±0.00 | 1006.49±1 14.61 | 12675.30± 1265.92 | 13950.66± 782.95 | 7.63±0.57 | - | - | 49.23± 4.92 |
| **400** | i.v. (1 mg/kg) | 5.09±0.57 | 0.08±0.00 | 1352.62±1 16.10 | 2357.97±6 38.69 | 2394.12±6 44.94 | 3.18±0.52 | 7.30±1.90 | 3.28±1.1 7 | - |
| | p.o. (10 mg/kg) | 4.67±1.04 | 2.00±0.00 | 805.87±75. 43 | 10791.37± 123.59 | 11166.54± 364.00 | 6.47±0.50 | - | - | 45.77± 0.52 |
| **379** | i.v. (1 mg/kg) | 1.90±0.27 | 0.08±0.00 | 1317.59±1 68.03 | 1292.28±5 30.92 | 1329.00±5 55.62 | 1.26±0.42 | 14.52±7.25 | 2.49±1.6 1 | - |
| | p.o. (10 mg/kg) | 4.15±1.48 | 1.67±0.58 | 634.54±16 1.39 | 6721.57±2 461.95 | 6903.48±2 619.87 | 5.90±0.79 | - | - | 52.01± 19.05 |
| **378** | i.v. (1 mg/kg) | 5.47±0.77 | 0.08±0.00 | 1568.34±2 01.27 | 2196.61±6 34.20 | 2254.80±6 72.40 | 3.19±0.31 | 7.95±2.80 | 3.65±0.7 4 | - |
| | p.o. (10 mg/kg) | 5.84±1.30 | 1.33±0.58 | 734.80±10 1.62 | 8180.11±7 95.05 | 8696.48±1 124.53 | 6.57±0.38 | - | | 7.95±2 .80 |
| **354** | i.v. (1 mg/kg) | 2.42±2.24 | 0.08±0.00 | 1105.29±6 9.99 | 1011.17±2 58.49 | 1020.76±2 61.50 | 0.93±0.18 | 1011.17±258 .49 | 1.31±0.5 4 | - |
| | p.o. (10 mg/kg) | 3.01±0.91 | 2.67±1.15 | 306.27±32. 12 | 2567.31±6 78.74 | 2844.61 ±3 26.65 | 4.89±1.14 | - | - | 25.39± 6.71 |

According to the experimental results, the compound of the present invention had good pharmacokinetics in SD rats.

### Experimental example 4: Cytotoxic assay of compounds against human BEAS-2B cells and HUVEC cells

### (1) Experimental methods

BEAS-2B cells (normal human bronchial epithelial cells) and HUVEC cells (human umbilical vein endothelial cells) were used to evaluate the cytotoxicity of compounds against human-derived cells. The detailed experimental procedures: BEAS-2B cells or HUVEC cells were seeded into a 96-well plate at a cell density of 2 × 10⁴ cells/well, that is, 100 µL/well, and then incubated overnight at 37 °C in a 5% CO₂ incubator. The next day, 100 µL of drug-containing medium was added to each well. The compound was diluted in a 2-fold serial dilution with a top concentration of 500 µM, for a total of 6 gradients. Three repeated wells were set for each concentration, and negative controls and blank controls without drugs were included for each experiment. After 72 hours of drug treatment, MTT assay was used to investigate cell viability and calculate the 50% cytotoxic concentration (CC₅₀) of compounds against BEAS-2B cells or HUVEC cells. All experimental data were analyzed using GraphPad Prism software.

### (2) Experimental results

**Table 6. The cytotoxicity of compounds against human BEAS-2B cells and HUVEC cells.**

| **Compounds** | **BEAS-2B cells** | **HUVEC cells** |
|---|---|---|
| | **CC₅₀ (µM)** | |
| **354** | >500 | >500 |
| **378** | >500 | >500 |
| **417** | >500 | >500 |
| **418** | >500 | >500 |
| **387** | >500 | >500 |
| **379** | >500 | >500 |
| **375** | 325.3 | 384.3 |
| **415** | >500 | 468.43 |
| **423** | >500 | >500 |
| **395** | 158.6 | 129.2 |
| **398** | >500 | >500 |
| **400** | >500 | >500 |
| **402** | >500 | 327.33 |
| **362** | >500 | >500 |

As shown in Table 6, the compounds of the present invention had low cytotoxicities against the tested human BEAS-2B cells and HUVEC cells.

### Experimental example 5: The assay of metabolic stability of compounds using human liver microsomes

### (1) Experimental methods

5 µL stock solution of each test compound in DMSO (10 mM) was added to 95 µL of methanol to prepare a 500 µM test compound solution. 0.1 M potassium phosphate buffer (K-buffer), with a pH value of 7.40 ± 0.01, was preheated in a 37 °C water bath. 1.5 µL solution of the test compound (500 µM) and 18.75 µL of 20 mg/mL human liver microsomes were added to 479.75 µL of preheated K-buffer, to obtain a 1.5 µM solution of the test compound containing 0.75 mg/mL microsomal solution. NADPH was dissolved in NADPH buffer to prepare a 3 × NADPH stock solution (6 mM, 5 mg/mL). 30 µL of 1.5 µM solution containing 0.75 mg/mL of microsomal solution was assigned into test plates pre-determined for different time points (0, 5, 15, 30, 45, 60 minutes). For 0 min, 150 µL of methanol containing internal standard was pre-added to the wells of the 0 min plate. All other test plates were pre-incubated at 37 °C for 5 min. 15 µL of NADPH stock solution (6 mM) was added to all test plates, to start the reaction and timing. At 5 min, 15 min, 30 min, 45 min, and 60 min, 150 µL of methanol containing internal standard was added to the wells of the corresponding plates to stop the reaction. After the reaction was quenched, the test plate was shaken on the vibrator for 10 minutes (600 rpm), and then centrifuged at 4000 rpm for 15 min. 80 µL of supernatant from each well was transferred to a 96-well sample plate containing 120 µL of water for LC/MS analysis.

### (2) Experimental results

**Table 7. Metabolic stability of compounds according to the present invention using human liver microsomes.**

| **Compounds** | **Human liver microsomes** |
|---|---|
| | **T_{1/2} (min)** |
| **354** | 35.61 |
| **378** | 35.08 |
| **417** | 32.04 |
| **379** | 42.21 |
| **395** | 38.99 |
| **398** | 35.34 |
| **400** | 30.40 |
| **362** | 33.64 |
| **PF-07321332** | 21.18 |

Based on the experimental results in Table 7, the compounds of the present invention had good stability for human liver microsome metabolism. Using human liver microsomes, the metabolic elimination half-life (T_{1/2}) of **PF-07321332,** an inhibitor of 2019-nCoV main protease (M^{pro}) developed by Pfizer, which has been listed, was measured to be 21.18 min under the same conditions as our experiment. The compound of the present invention was superior to **PF-07321332** in the metabolic stability assay with human liver microsomes.

### Experimental example 6: Biochemical activity assay of compounds using human proteases

### (1) Experimental methods

The biochemical activity of compounds against human proteases was determined using Cathepsin K inhibitor screening kit (K150-100), Cathepsin B inhibitor screening kit (K147-100), Thrombin activity testing kit (K373-100), Caspase-2 inhibitor screening kit (K152-100), and Cathepsin D inhibitor screening kit (K148-100) according to the instructions of the kits (Biovision, USA). All experiments were carried out in triplicate. All experimental data were analyzed using GraphPad Prism software.

### (2) Experimental results

**Table 8. The biochemical activities of the compounds according the present invention on human proteases.**

| **Compounds** | **Thrombin** | **Cathepsin K** | **Cathepsin B** | **Caspase-2** | **Cathepsin D** |
|---|---|---|---|---|---|
| | **IC₅₀ (µM)** | | | | |
| **378** | >100 | >100 | >100 | >100 | >100 |
| **417** | >100 | >100 | >100 | >100 | >100 |
| **379** | >100 | >100 | >100 | >100 | >100 |
| **395** | >100 | >100 | >100 | >100 | >100 |
| **398** | >100 | >100 | >100 | >100 | >100 |
| **400** | >100 | >100 | >100 | >100 | >100 |

According to the experimental results in Table 8, the compound of the present invention had almost no activity to several common human proteases with similar structures: human cathepsin K, human cathepsin B, human thrombin, human caspase 2 and human cathepsin D, indicating that the compounds of the present invention had good selectivity to the main protease of COVID-19.

### Experimental example 7: Effect of compound 398 on hERG potassium ion channels

### (1) Experimental objective

The rapid activation of delayed rectifier outward potassium current (IKr) in humans was mainly mediated by hERG ion channels and participated in human myocardial cell repolarization. Drug blockade of this current would lead to the appearance of QT prolongation syndrome in clinical practice, which could easily induce acute arrhythmia and even sudden death. In the present experimental example, the patch clamp technique was used to test the effect of compound **398** on hERG potassium current in stable cell lines transfected with hERG potassium channels, in order to determine whether the test compound had inhibitory effects on hERG ion channels.

### (2) Experimental methods

HEK-293-hERG cells were passaged and cultured to a suitable state, and then digested with trypsin, followed by separation and placing in a centrifuge tube. After centrifugation, the supernatant was discarded, and the cells were resuspended with extracellular fluid for later use. Before recording with patch clamp, the cells were dropped onto a petri dish, to ensure that they reached a certain density and were in a single isolated state.

hERG current was recorded using whole cell patch clamp technique. The cell suspension was added to a small petri dish, and then placed on an inverted microscope stage. After the cells adhered to the wall, they were perfused with extracellular fluid at a flow rate of 1-2 mL/min. The glass microelectrode was drawn in two steps by a micropipette puller, and its water resistance value was 2-5 MQ after filling the electrode with intracellular fluid.

After establishing a whole cell recording mode, the clamp potential was maintained at -80 mV. Depolarization voltage was applied to +60 mV for 850 ms, and then the cells was repolarized to -50 mV for 1275 ms, to extract hERG tail current. This set of pulse programs was repeated every 15 seconds throughout the entire experiment.

After the current stabilized, the drug was administered by continuous extracellular perfusion from low to high concentrations. Starting from low concentration, continual perfusion was given until obtaining stable drug effect, that is, the change in the current value of the last 5 stimulation bars at each concentration was less than 10% of the mean (when the current was ≥200 pA) or less than 30% of the mean (when the current was < 200 pA), and then proceeded to the next concentration of perfusion. The blocking effect of the test agents (0.3 µM, 1 µM, 3 µM, 10 µM, 30 µM) and the positive control Cisapride on hERG tail current was respectively determined. The stimulus distribution and signal acquisition was carried out using PatchMaster or Clampex 10.6 software; the signal was amplified with a patch clamp amplifier. Further data analysis and curve fitting were performed using FitMaster or Clampfit 10.6, EXCEL, Graphpad Prism, and SPSS 21.0. The data were expressed as the mean ± SD (standard deviation).

### (3) Experimental results

Under the conditions of this experiment, the positive control Cisapride had a concentration-dependent inhibitory effect on hERG current, and its inhibition rate on hERG current at a concentration of 0.1 µM was 86.36%, suggesting the reliability of the experimental results. In this experiment, the average inhibition rate on hERG current at a maximum concentration of 30 µM was 1.28%, that is, compound 398 had an IC₅₀ value of >30 µM for hERG current, indicating a low cardiotoxicity, and thus the compound was less likely to induce acute arrhythmia or even sudden death after administration.

**Table 9. The inhibitory effect of the compound according to the present invention on hERG current.**

| **Groups** | **Concentration (µM)** | **The average tail current before administration (pA)** | **The average tail current after administration (pA)** | **The inhition rate (%)** | **The average inhibition rate (%)** | **Standard deviation of inhibition rate** |
|---|---|---|---|---|---|---|
| **Cisapride** | 0.1 | 938.45 | 128.04 | 86.36 | / | / |
| **398** | 30 | 380.53 | 394.39 | -3.64 | 1.28 | 7.2 |
| | | 1447.54 | 1420.51 | 1.87 | | |
| | | 867.11 | 904.87 | -4.35 | | |
| | | 299.6 | 265.92 | 11.24 | | |

### Experimental example 8: Determination of the antiviral activities of the compounds according to the present invention at the cellular level using RT-qPCR method

### (1) Experimental methods

Vero E6-TMPRSS2 cells were infected with SARS-CoV-2 (B.1.1.529) BA.1 mutant strains at a MOI value of 0.1. The compound of the present invention was diluted from 20 µM to 0.0013 µM in a ratio of 1:5, and then the cells were treated, while PF-07321332 was used as the positive control. Cell lysates were collected at 24 hpi for RT-qPCR analysis. All experiments were performed in triplicate. All experimental data were analyzed using GraphPad Prism software

### (2) Experimental results

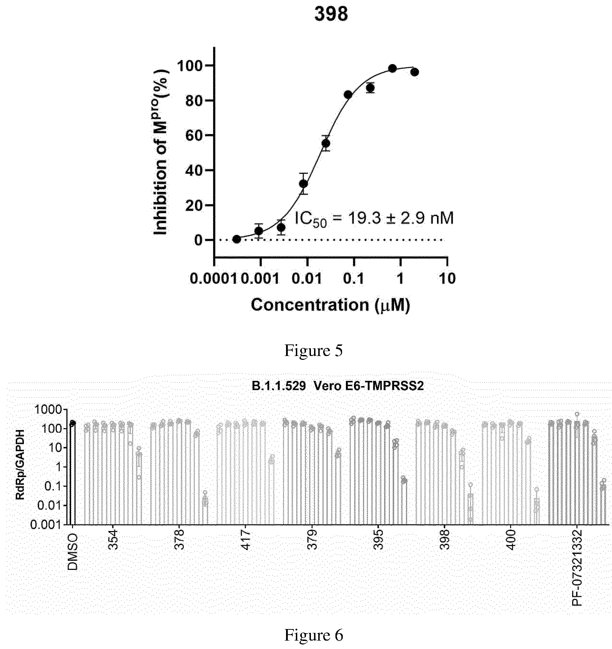

The experimental results are shown in Figure 6. The results indicated that the compound of the present invention could effectively inhibit the replication of mutant SARS-CoV-2 (B.1.1.529) in Vero E6-TMPRSS2 cells. Among them, compounds **398** and **395** showed better inhibitory activity on the replication of SARS-CoV-2 mutant strains (B.1.1.529) under the experimental conditions than the positive control compound **PF-07321332.**

### Experimental example 9: Determination of anti-viral activity of compound 398 at the cellular level using the plaque method

### (1) Experimental methods

Viro E6-TMPRSS2 cells were infected with MERS CoV, SARS-CoV-1, SARS-CoV-2 Alpha (B.1.1.7) mutant strains, Beta (B.1.351) mutant strains, Omicron (B.1.1.529) BA.2 and BA.5 mutant strains at 50-70 PFU/well in a 12-well plate. The cells were washed with PBS and then covered with 2% agarose/PBS, followed by mixing with 2×DMEM/2% FBS in a 1:1 ratio. At 2 hpi, compound **398,** and the positive compound Nirmatrelvir **(PF-07321332)** were diluted in a ratio of 1:5 from 0.0013 µM to 20 µM, with which the cells were treated. At 72 hpi, the cells were fixed and stained with 0.5% crystal violet in 25% ethanol/distilled water for 10 min, and the stained plaques were quantified (n=3). The EC₅₀ (median effective concentration) value was calculated using the dose-response model in GraphPad Prism 8.0 software.

### (2) Experimental results

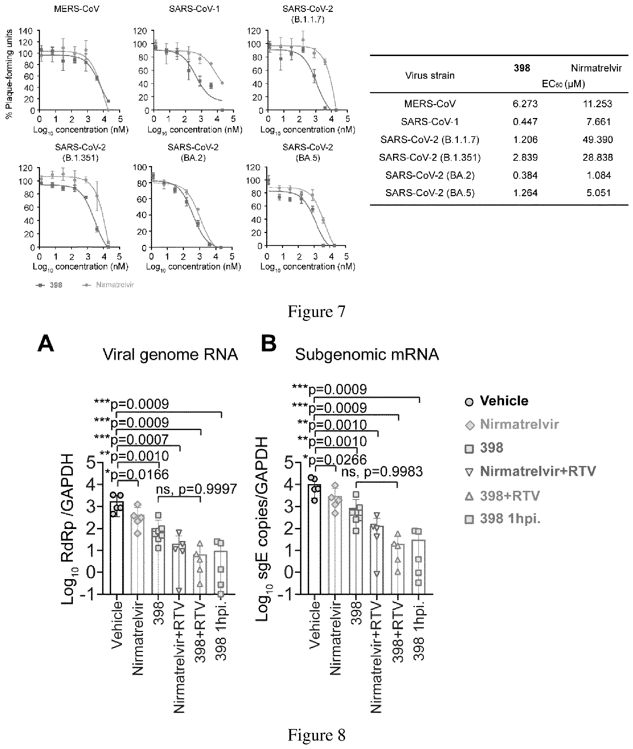

The experimental results are shown in Figure 7. The results showed that compound **398** could reduce the cytopathic effects caused by MERS-CoV, SARS-CoV-1, SARS-CoV-2 B.1.1.7 (Alpha), SARS-CoV-2 B.1.351 (Beta), SARS-CoV-2 Omicron BA.2, and BA.5 strains infecting Vero E6-TMPRSS2 cells, and its anti-viral activity was 1.79, 17.14, 40.95, 10.16, 2.82, and 4.00 times higher than the positive control compound Nirmatrelvir **(PF-07321332),** respectively, indicating that compared to Nirmatrelvir, compound **398** exhibited higher anti-viral activity against variants such as SARS-CoV-2 Omicron and other coronaviruses.

### Experimental example 10: Pharmacodynamic evaluation of in vivo anti-viral activity of compound 398 in K18-hACE2 transgenic mice

### (1) Experimental methods

K18-hACE2 transgenic mice (6-8 weeks old) were purchased from the Jackson Laboratory, and their use complied with all relevant ethical regulations and had been approved by the Committee on the Use of Living Animals in Teaching and Research at the University of Hong Kong. 2000 PFU SARS-CoV-2 Omicron (B.1.1.529) BA.2 mutant strains were inoculated into the nasal cavity (i.n.) of female or male K18-hACE2 transgenic mice. For early treatment, mice were orally administered 150 mg/kg of compound **398** twice a day from 1 hpi on the day of infection to the 4th day (4 dpi). For late treatment, mice were orally administered compound **398** (150 mg/kg) or compound **398** (150 mg/kg)/ritonavir (RTV, 10 mg/kg), Nirmatrelvir (150 mg/kg) or Nirmatrelvir (150 mg/kg)/ritonavir (RTV, 10 mg/kg) twice a day from 1 dpi to 4 dpi. Mice treated with the solvent (5% DMSO/3% Solutol HS-15/40% PEG400/physiological saline) were used as the control group. The survival of the mice was monitored daily during the experiment, and they were euthanized at 4 dpi. Organ tissue samples were taken for virological and histopathological analysis.

Determination of virus genome copy numbers: Animals were euthanized at 4 dpi when a K18-hACE2 transgenic mouse model infected with SARS-CoV-2 Omicron (B.1.1.529) BA.2 was treated, and lung tissue samples were obtained from each group of mice. RLT buffer (Qiogen) was used to lyse the tissue samples of transgenic mice, and then extracted using RNeasy Mini kit. After extracting RNA, the Transcriptor First Strand cDNA Synthesis Kit, QuantiNova SYBR Green RT-PCR Kit, or QuantiNova Probe RT-PCR Kit were used for RT-qPCR analysis.

Determination of viral titer: One day before infection, Vero E6-TMPRSS2 cells were inoculated in a 12-well plate. Animals were euthanized at 4 dpi when a K18-hACE2 transgenic mouse model infected with SARS-CoV-2 Omicron (B.1.1.529) BA.2 was treated, and lung tissue samples were obtained from each group of mice. The supernatant of the collected tissue samples was diluted in a continuous gradient, and then inoculated into the cells at 37 °C for 1 hour. After inoculation, the cells were washed with PBS for three times and mixed with 2% agarose/PBS and 2×DMEM/2% FBS in a 1:1 ratio. After 48 hours, the cells were fixed and stained with 0.5% crystal violet in 25% ethanol/distilled water for 10 min, that was used for plaque quantification.

Histopathological study: the nasal turbinates and lung tissues of transgenic mice, soaked in formic acid, were fixed overnight in 10% formalin. Then, the fixed samples were embedded in paraffin using TP1020 Leica semi-enclosed benchtop tissue processor and sliced at 5 µm. At 37 °C, the tissue slices were removed, dried, and fixed overnight onto anti-off slides. The slices were diluted sequentially with xylene, ethanol, and double distilled water, dewaxed and dehydrated, and then treated with antigen blocking buffer. The slices were heated at 85 °C for 90 s for antigen exposure, and then blocked with 0.3% hydrogen peroxide for 30 min, followed by blocking with 1% BSA for 30 min. Internal rabbit anti-SARS-CoV-2-N immune serum and goat anti-rabbit IgG antibody were used as primary and secondary antibodies, respectively. DAB (3,3'-diaminobenzidine) substrate kit was used to generate signals. The nucleus was labeled with Gill hematoxylin. The slide was sealed with an anti-fading sealant with DAPI. For H&E staining, infected tissue sections were stained with Gill hematoxylin and Eosin Y. Olympus BX53 optical microscope was used to capture images for analysis.

### (2) Experimental results

As shown in Figure 8, in the experiment of establishing a K18-hACE2 transgenic mouse model infected with SARS-CoV-2 Omicron (B. 1.1.529) BA.2, early administration of compound **398** significantly reduced the viral genomic RNA (vRNA) in the lungs of infected mice by 179 times at 4 dpi. In the same treatment regimen, the production of viral subgenome mRNA (sgRNA) was inhibited by 337 times. Even if the treatment with compound **398** was postponed to 24 hpi, the vRNA and sgRNA copies in the lungs of infected mice were reduced at 4 dpi by 16 times and 12 times, respectively. Compared with mice treated with Nirmatrelvir, the viral vRNA copy and sgRNA copy in the lungs of mice treated with compound **398** were reduced by four times.

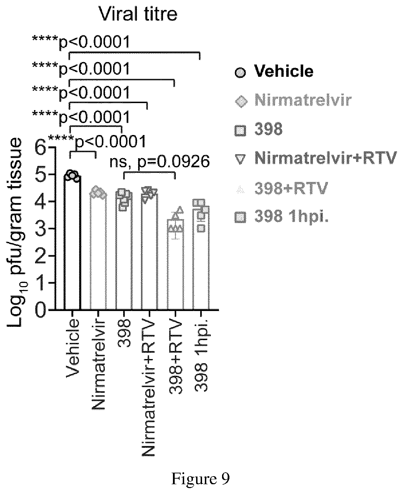

As shown in Figure 9, the infectious viral load in the lungs was measured using plaque assay, and the results showed that the treatment with compound **398** effectively inhibited the production of infectious viral particles in the lungs. According to the research reported, co-administration with RTV could delay the clearance of compounds by liver microsomes, thereby enhancing pharmacokinetics and antiviral efficacy *in vivo.* Therefore, this study also investigated the synergistic therapeutic effect of compound **398** in combination with RTV. It was worth noting that compound **398**/RTV treatment began at 24 hpi. Compared with single treatment, the viral load in the lungs of infected mice was further reduced by 15 times (vRNA), 43 times (sgRNA), and 6 times (infectious virus titer). Importantly, compared to mice treated with Nirmatrelvir/RTV, the infectious virus titers in the lungs of mice treated with compound **398**/RTV were significantly reduced by about 90%.

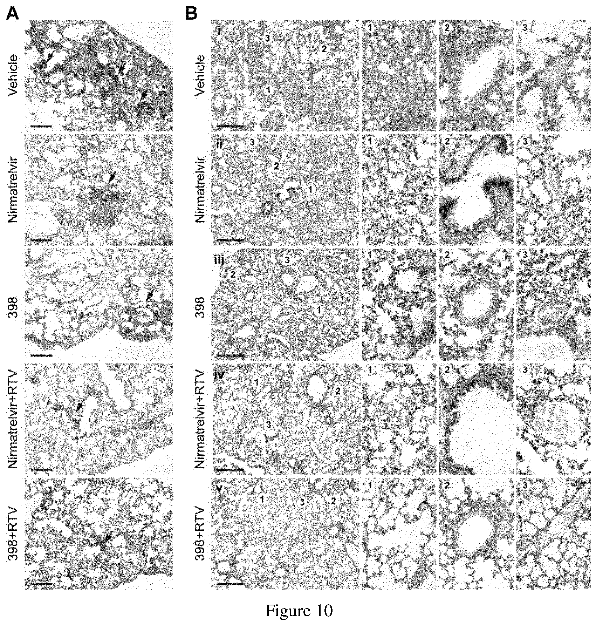

As shown in Figure 10, the immunohistochemical staining results for SARS-CoV-2 nucleocapsid (N) protein showed (Figure 10A) that the viral antigen was most expressed in the lungs of vehicle control mice (black arrow), followed by treatment with Nirmatrelvir or compound **398** alone. The combination therapy of Nirmatrelvir/RTV and compound **398**/RTV also limited the expression of N protein in the lung tissue of infected mice to very low levels. In addition, the results of H&E staining for tissues (Figure 10B) showed that the most prominent pulmonary pathological feature in the vehicle control group was multifocal inflammatory infiltration in the alveolar septa, peribronchiolar areas, and perivascular areas. In contrast, scattered inflammatory cell infiltration was occasionally observed in the alveolar interstitium of mice treated with compound **398.** Compared with single therapy, compound **398**/RTV combined administration further improved the lung tissue structure.

### Experimental example 11: In vivo pharmacokinetic evaluation of compound 398 in mice, beagle dogs, and cynomolgus monkeys

### (1) Dosing regimen

Male ICR mice (6-8 weeks old, weighing 16-25 g), male beagle dogs (1-2 years old, weighing 9-12 kg), or male cynomolgus monkeys (6-7 years old, weighing 6-8 kg) were randomly divided into groups, with three animals for each group, and a series of test compounds were administered orally (p.o.) or intravenously (i.v.) according to the protocol in following Table 10.

The solution for gavage and intravenous administration was prepared with DMSO/HS15/PEG400/NaCl (5/3/40/52, v/v/v). The test compound was administered according to the dosage shown in Table 4. The administration time was recorded, and for each sample, approximately 0.20 mL of blood was collected by jugular vein blood collection or other appropriate methods at the pre-determined time points. The blood sample was anticoagulated with heparin sodium, and after collection, the blood was placed on ice. The sample was centrifuged to separate the plasma within 1 h (centrifugation conditions: 6800 g, 6 min, 2-8 °C). Plasma samples were stored in a -80 °C freezer before analysis. The grouping and blood collection time points are shown in Table 4, with 3 animals at each time point.

**Table 10. Experimental protocol for evaluating the in vivo pharmacokinetic properties of compound 398 in mice, beagle dogs, and cynomolgus monkeys.**

| **Test compound** | **Species** | **Administration route** | **Dosage (mg/kg)** | **Collection time (h)** |
|---|---|---|---|---|
| **398** | ICR Mice | i.v. | 1 | 0.0833 h, 0.25 h, 0.5 h, 1 h, 2 h, 4 h, 6 h, 8 h, 24 h |
| | | p.o. | 150 | |
| | Beagle Dog | i.v. | 1 | |
| | | p.o. | 10 | |
| | Monkey | i.v. | 1 | |
| | | p.o. | 10 | |

**Table 11. The main pharmacokinetic parameters of the compound.**

| **Anim al** | **Admin** | **T_{1/2}** | **Tₘₐₓ** | **Cₘₐₓ** | **AUCₗₐₛₜ** | **AUC_{INF_obs}** | **MRT_{INF_obs}** | **CL** | **Vss_{obs}** | **F** |
|---|---|---|---|---|---|---|---|---|---|---|
| | | **(h)** | **(h)** | **(ng/mL)** | **(h*ng/mL)** | **(h*ng/mL)** | **(h)** | **(mL/min/kg)** | **(mL/kg)** | **%** |
| **ICR Mice** | i.v. (1 mg/kg) | 0.27±0.17 | 0.08±0.00 | 285.09±16. 35 | 85.90±7.60 | 87.12±7.81 | 0.17±0.03 | 192.28±16.4 5 | 4.27±2.2 9 | - |
| | p.o. (150 mg/kg) | 3.25±1.03 | 0.33±0.14 | 4078.10±6 36.18 | 15134.02± 5136.94 | 15692.57± 4424.87 | 3.62±1.18 | - | - | 67.42± 22.88 |
| **Beagl e Dog** | i.v. (1 mg/kg) | 0.61±0.05 | 0.083±0.0 0 | 1270.63±3 32.60 | 623.96±11 3.01 | 627.67±11 3.38 | 0.47±0.05 | 1625.21±265 .88 | 1419.64± 160.26 | - |
| | p.o. (10 mg/kg) | 5.59±1.24 | 1.00±0.00 | 3061.33±1 36.00 | 8235.92±3 70.33 | 8290.10±3 77.59 | 2.14±0.18 | - | - | 131.99 ±5.94 |
| **Monk ey** | i.v. (1 mg/kg) | 0.73±0.20 | 0.08±0.00 | 567.18±38. 24 | 501.29±59. 91 | 505.62±60. 72 | 0.90±0.18 | 1998.35±257 .17 | 2065.80± 374.39 | - |
| | p.o. (10 mg/kg) | 5.57±1.05 | 2.33±1.53 | 242.48±54. 21 | 1221.18±5 6.84 | 1264.93±7 2.03 | 4.71±0.89 | | | 24.36± 1.13 |

As shown in Table 11, compound **398** of the present invention exhibited good pharmacokinetic properties in mice, beagle dogs, and cynomolgus monkeys.

### Experimental example 12: Preliminary evaluation of the in vivo safety of compound 398 in mice

### (1) Experimental methods

The compound was dissolved in DMSO/HS15/PEG400/NaCl (5/3/40/52, v/v/v). ICR rats (age: 6-8 weeks) included half females (weighing 16-22 g) and half males (weighing 17-25 g). Compound **398** was tested according to the dosing regimen in Table 12, and clinical observations were performed for all animals. At the end of the experiment, the samples of the heart, liver, spleen, lungs, kidneys, and administration site were collected. The experimental results are shown in Table 12.

### (2) Experimental results

**Table 12. Preliminary evaluation of the in vivo safety of compound 398 in rats.**

| | Acute toxicity test | | General toxicity test | |
|---|---|---|---|---|
| Administration route | p.o. | | | |
| Dosage (mg/kg) | 1000 | 800 | 600 | 400 |
| Animal grouping (animal/group) | 6 (half male and half female) | | | |
| Dosing frequency | Single-dose | | Twice a day (with a 12 hour interval), for 14 consecutive days | |
| Experimental results | 1. During the administration period, no abnormal body weight or general condition was observed in any group. | | | |
| | 2. At the end of administration, histological examinations of the heart, liver, spleen, lungs, and kidneys were performed, and no abnormalities were found in any group. | | | |

The experimental results showed that compound **398** of the present invention had good *in vivo* safety in mice.

## Claims

1. A compound represented by formula I, or a pharmaceutically acceptable salt thereof, or a stereoisomer thereof, or an optical isomer thereof, or a deuterated compound thereof: 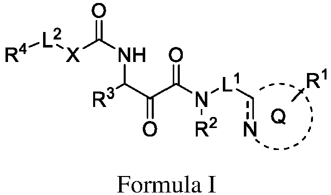
wherein, Q is a nitrogen-containing heteroaromatic ring or a nitrogen-containing fused heteroaromatic ring;
R¹ is selected from the group consisting of H, unsubstituted or halogenated C₁₋₈ alkyl, unsubstituted or halogenated C₁₋₈ alkoxy, halogen, 5-6-membered aryl, 5-6-membered heteroaryl, 3-8-membered saturated heterocyclyl, 3-8-membered saturated cycloalkyl, and NHCOR^{1a}; R^{1a} is selected from C₁₋₈ alkyl, and unsubstituted or halogenated following groups: 3-8-membered saturated heterocyclyl, 3-8-membered saturated cycloalkyl, 5-6-membered aryl, 5-6-membered heteroaryl;
R² is selected from the group consisting of H, C₁₋₈ alkyl, C₁₋₈ alkoxy, halogen, 5-6-membered aryl, 5-6-membered heteroaryl, 3-8-membered saturated heterocyclyl, and 3-8-membered saturated cycloalkyl;
R³ is selected from the group consisting of H, C₁₋₈ alkyl, C₁₋₈ alkoxy, halogen, 5-6-membered aryl, 5-6-membered heteroaryl, 3-8-membered saturated heterocyclyl, 3-8-membered saturated cycloalkyl, and CH₂R^{3a}; R^{3a} is selected from unsubstituted or halogenated following groups: 3-8-membered saturated heterocyclyl, 3-8-membered saturated cycloalkyl, 5-6-membered aryl, 5-6-membered heteroaryl, and fused aryl;
R⁴ is selected from the group consisting of H, and any one of the substituted or unsubstituted following groups: C₁₋₈ alkyl, C₁₋₈ alkoxy, 3-8-membered saturated cycloalkyl, 3-8-membered saturated heterocyclyl, 5-6-membered aryl, 5-6-membered heteroaryl, bridged group, fused aryl, fused heteroaryl, or (5-6-membered saturated heterocycle)-fused 5-6-membered aryl;
The substituents of the substituent group are R^{4a}, R^{4b}, R^{4c}, and R^{4d}, and each independently selected from the group consisting of H, halogen, phenyl, cyano, hydroxyl, ester group, trimethylsilyl, -(CH₂)ₘ-SO₂R', and -COOR"; alternatively, selected from any one of the substituted (with one or more of halogen, cyano, haloalkyl, and haloalkoxy) or unsubstituted following groups: C₁₋₈ alkyl, C₁₋₈ alkoxy, benzyl, pyridyl or 3-6-membered saturated cycloalkyl; said R' and R" are each independently selected from C₁₋₈ alkyl; M is an integer from 0 to 3;
or, said R^{4a} and R^{4b} are linked to form halogenated or unsubstituted 3-6-membered saturated carboncycle or carbon heterocycle;
L¹ is selected from the group consisting of absence, substituted or unsubstituted - (CH₂)ₙ- or -O-(CH₂)ₙ-, -O-, -NHCO-, -NHSO₂-, -CONH-, -NHCOO-, -NHCONH-, and - NH-; n is any integer from 1 to 3; the substituent of said L¹ is selected from the group consisting of C₁₋₃ alkyl, C₁₋₃ alkoxy or phenyl;
X is selected from the group consisting of absence, CR⁵R⁶, NR⁵R⁶, O or SO₂; R⁵ and R⁶ are each independently selected from the group consisting of:
H, halogen, and R^{5a}-, R^{5b}-, R^{5c}-substituted or unsubstituted C₁₋₈ alkyl; or R⁵ and R⁶ are linked to form R^{6a}-, R^{6b}-substituted or unsubstituted 3-8-membered saturated cycloalkyl or 3-8-membered saturated heterocyclyl;
R^{5a}, R^{5b}, and R^{5c} are each independently selected from the group consisting of H, alkynyl, hydroxyl, -CONH₂, -N(CH₃)₂, halogen, C₁₋₈ alkoxy, 5-6-membered aryl, and 5-6-membered heteroaryl; or any two of R^{5a}, R^{5b}, and R^{5c} are linked to form 3-8-membered saturated cycloalkyl or 3-8-membered saturated heterocyclyl;
R^{6a} and R^{6b} are each independently selected from the group consisting of H, alkenyl, halogen, and halogen-substituted or unsubstituted C₁₋₈ alkyl; or R^{6a} and R^{6b} are linked to form 5-6-membered aryl;
L² is selected from the group consisting of absence, 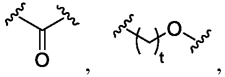 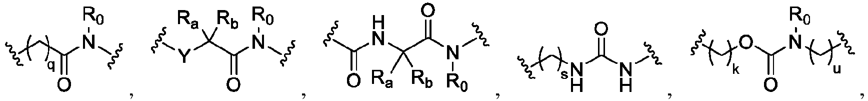 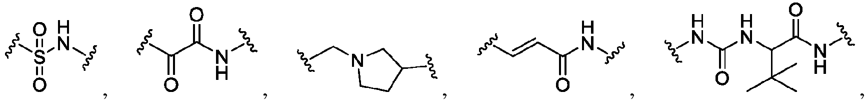 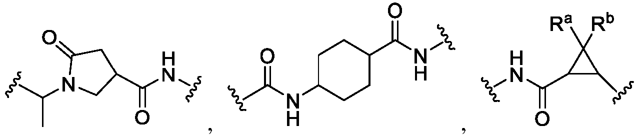 or Rₐ-, R_{b}-substituted or unsubstituted 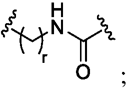
wherein, R₀ is selected from H and C₁₋₃ alkyl; or R₀ and R₅ are linked to form the substituted or unsubstituted following structure: bridged ring, 3-6-membered saturated ring or (3-6-membered saturated ring)-fused benzene; the substituent of said substituted structure is C₁₋₃ alkyl or halogen;
Rₐ and R_{b} are each independently selected from the group consisting of H, cyano, C_{1~5} alkyl, and 3-6-membered cycloalkyl; or Rₐ and R_{b} are linked to form 3-6-membered saturated carboncycle;
Y is O or S;
t is any integer from 1 to 3, q is any integer from 0 to 4, r is any integer from 0 to 3, s is any integer from 0 to 3, k is any integer from 0 to 3, and u is any integer from 0 to 3.

2. The compound according to claim 1, or a pharmaceutically acceptable salt thereof, or a stereoisomer thereof, or an optical isomer thereof, or a deuterated compound thereof, **characterized in that** Q is selected from the group consisting of 5-6-membered N-containing heteroaromatic ring, (5-membered N-containing heteroaromatic ring)-fused (6-membered N-containing heteroaromatic ring) or (6-membered N-containing heteroaromatic ring)-fused (6-membered N-containing heteroaromatic ring).

3. The compound according to claim 2, or a pharmaceutically acceptable salt thereof, or a stereoisomer thereof, or an optical isomer thereof, or a deuterated compound thereof, **characterized in that** Q is selected from the group consisting of

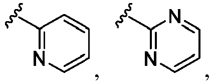

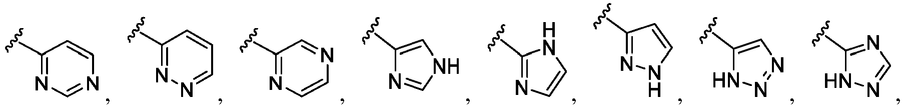

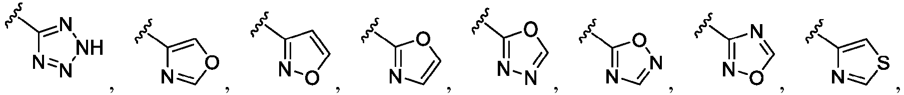

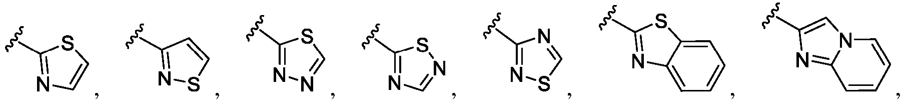

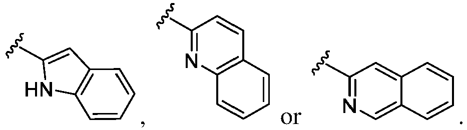

4. The compound according to claim 3, or a pharmaceutically acceptable salt thereof, or a stereoisomer thereof, or an optical isomer thereof, or a deuterated compound thereof, **characterized in that** Q is

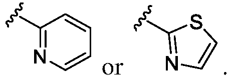

5. The compound according to claim 1, or a pharmaceutically acceptable salt thereof, or a stereoisomer thereof, or an optical isomer thereof, or a deuterated compound thereof, **characterized in that** R¹ is selected from the group consisting of H, halogen, halogenated or unsubstituted C₁₋₈ alkyl or unsubstituted C₁₋₈ alkoxy.

6. The compound according to claim 5, or a pharmaceutically acceptable salt thereof, or a stereoisomer thereof, or an optical isomer thereof, or a deuterated compound thereof, **characterized in that** R¹ is selected from the group consisting of H, halogen, halogenated or unsubstituted C₁₋₃ alkyl or unsubstituted C₁₋₃ alkoxy.

7. The compound according to claim 6, or a pharmaceutically acceptable salt thereof, or a stereoisomer thereof, or an optical isomer thereof, or a deuterated compound thereof, **characterized in that** R¹ is selected from the group consisting of H, F, Cl, CH₃, CHsO or - CF₃.

8. The compound according to claim 1, or a pharmaceutically acceptable salt thereof, or a stereoisomer thereof, or an optical isomer thereof, or a deuterated compound thereof, **characterized in that** R² is selected from the group consisting of H or C₁₋₈ alkyl.

9. The compound according to claim 8, or a pharmaceutically acceptable salt thereof, or a stereoisomer thereof, or an optical isomer thereof, or a deuterated compound thereof, **characterized in that** R² is H or CH₃.

10. The compound according to claim 1, or a pharmaceutically acceptable salt thereof, or a stereoisomer thereof, or an optical isomer thereof, or a deuterated compound thereof, **characterized in that** R³ is selected from the group consisting of H or CH₂R^{3a}; R^{3a} is selected from the unsubstituted or halogenated following groups: C₁₋₄ alkyl, 5-6-membered cycloalkyl, 5-6-membered aryl, 5-6-membered heteroaryl or fused aryl.

11. The compound according to claim 10, or a pharmaceutically acceptable salt thereof, or a stereoisomer thereof, or an optical isomer thereof, or a deuterated compound thereof, **characterized in that** R³ is selected from the group consisting of H or CH₂R^{3a}; R^{3a} is selected from the unsubstituted or halogenated following groups: C₁₋₂ alkyl, 5-6-membered cycloalkyl, 5-6-membered aryl, 5-6-membered heteroaryl or naphthyl.

12. The compound according to claim 11, or a pharmaceutically acceptable salt thereof, or a stereoisomer thereof, or an optical isomer thereof, or a deuterated compound thereof, **characterized in that** R³ is selected from the group consisting of H or CH₂R^{3a}; R^{3a} is selected from the group consisting of phenyl, ethyl, cyclohexyl, furyl, naphthyl or F-substituted phenyl.

13. The compound according to claim 1, or a pharmaceutically acceptable salt thereof, or a stereoisomer thereof, or an optical isomer thereof, or a deuterated compound thereof, **characterized in that** R⁴ is selected from any one of the substituted or unsubstituted following groups: C₁₋₄ alkyl, C₁₋₄ alkoxy, 3-7-membered saturated cycloalkyl, 4-6-membered saturated heterocyclyl, 5-6-membered aryl, 5-6-membered heteroaryl, bridged group, naphthyl, (5-6-membered aromatic heterocyclyl)-fused phenyl or (5-6-membered saturated heterocyclyl)-fused phenyl.
The substituents of the substituent group are R^{4a}, R^{4b}, R^{4c}, and R^{4d}, and each independently selected from the group consisting of H, halogen, phenyl, cyano, hydroxyl, ester group, trimethylsilyl, -(CH₂)ₘ-SO₂R', and -COOR"; alternatively, selected from any one of the halogen-substituted or unsubstituted following groups: C₁₋₃ alkyl, C₁₋₃ alkoxy or 3-4-membered saturated cycloalkyl; said R' and R" are each independently selected from C₁₋₄ alkyl; M is an integer from 0 to 2;
or, any two of said R^{4a}, R^{4b}, R^{4c} and R^{4d} are linked to form halogenated or unsubstituted 3-6-membered saturated carboncycle or heterocycle.

14. The compound according to claim 13, or a pharmaceutically acceptable salt thereof, or a stereoisomer thereof, or an optical isomer thereof, or a deuterated compound thereof, **characterized in that** R⁴ is selected from any one of the substituted or unsubstituted following groups: C₁₋₄ alkyl, C₁₋₄ alkoxy, 3-7-membered saturated cycloalkyl, 4-6-membered saturated heterocyclyl, 5-6-membered aryl, 5-6-membered N-containing heteroaryl, bridged group, naphthyl, benzofuranyl, benzopyridyl or (5-6-membered saturated O-containing heterocyclyl)-fused phenyl;
The substituents of the substituent group are R^{4a}, R^{4b}, R^{4c}, and R^{4d}, and each independently selected from the group consisting of H, halogen, phenyl, cyano, hydroxyl, ester group, trimethylsilyl, -(CH₂)ₘ-SO₂R', and -COOR"; alternatively, selected from any one of the halogen-substituted or unsubstituted following groups: C₁₋₃ alkyl, C₁₋₃ alkoxy or 3-membered saturated cycloalkyl; said R' and R" are each independently selected from C₁₋₄ alkyl; M is 0 or 1;
or, any two of said R^{4a}, R^{4b}, R^{4c} and R^{4d} are linked to form halogenated or unsubstituted 3-6-membered saturated carboncycle or O-containing heterocycle.

15. The compound according to claim 14, or a pharmaceutically acceptable salt thereof, or a stereoisomer thereof, or an optical isomer thereof, or a deuterated compound thereof, **characterized in that** R⁴ is 4-6-membered saturated cycloalkyl substituted with F.

16. The compound according to claim 15, or a pharmaceutically acceptable salt thereof, or a stereoisomer thereof, or an optical isomer thereof, or a deuterated compound thereof, **characterized in that** R⁴ is 4-6-membered saturated cycloalkyl substituted with two fluorines.

17. The compound according to claim 16, or a pharmaceutically acceptable salt thereof, or a stereoisomer thereof, or an optical isomer thereof, or a deuterated compound thereof, **characterized in that** R⁴ is 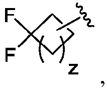 and z is an integer from 1 to 3.

18. The compound according to claim 14, or a pharmaceutically acceptable salt thereof, or a stereoisomer thereof, or an optical isomer thereof, or a deuterated compound thereof, **characterized in that** R⁴ is selected from any one of the substituted or unsubstituted following groups: -CH₃, -OCH₃,

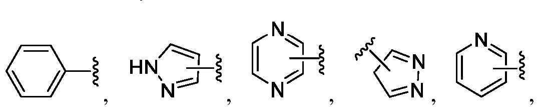

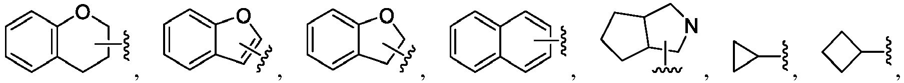

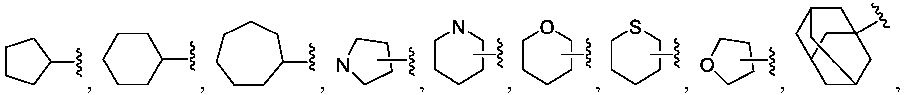

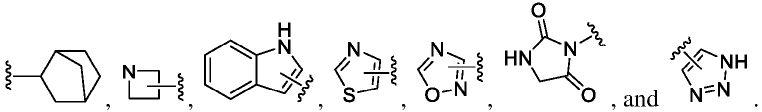

19. The compound according to claim 18, or a pharmaceutically acceptable salt thereof, or a stereoisomer thereof, or an optical isomer thereof, or a deuterated compound thereof, **characterized in that** R⁴ is selected from any one of the substituted or unsubstituted following groups: -CH₃, CF₃, -OCH₃, -OCF₃, -OC(CH₃)₃,

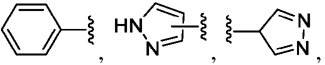

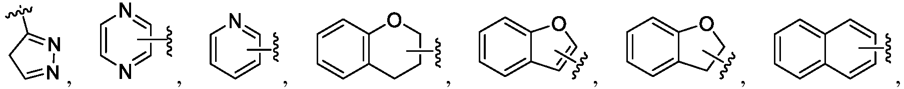

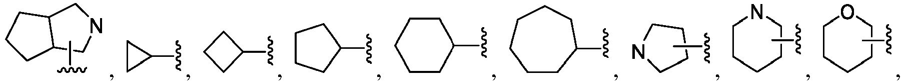

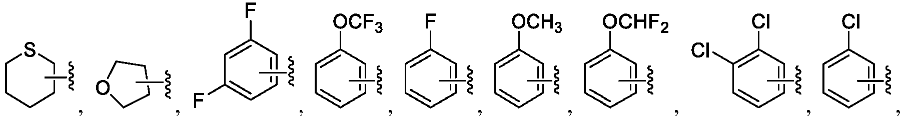

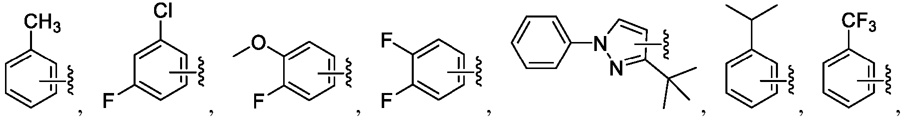

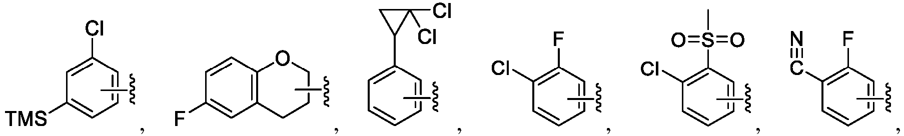

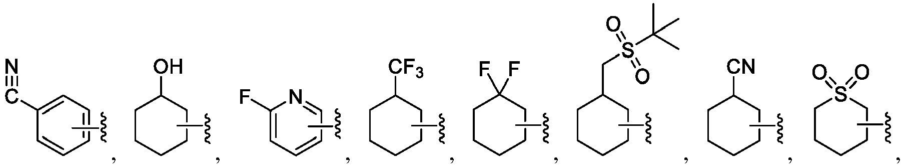

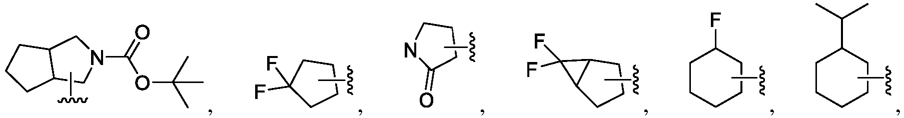

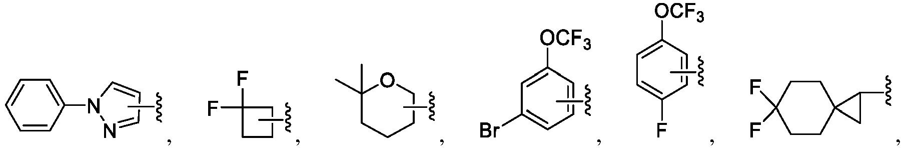

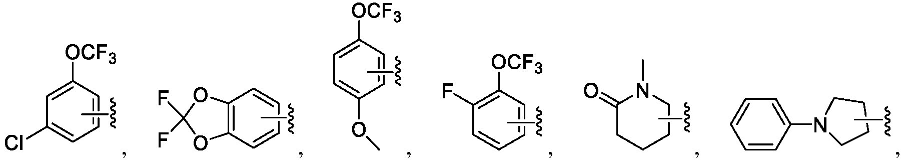

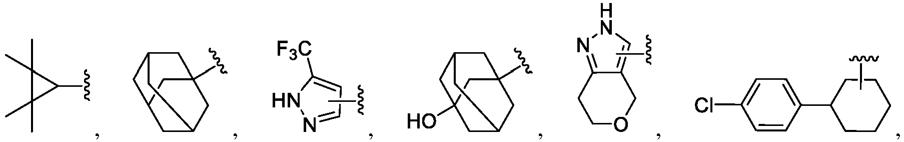

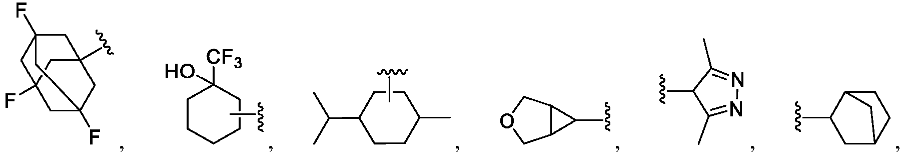

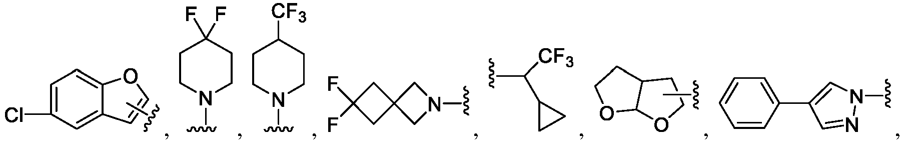

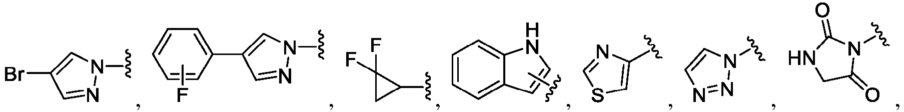

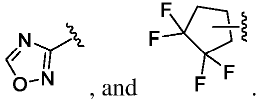

20. The compound according to claim 1, or a pharmaceutically acceptable salt thereof, or a stereoisomer thereof, or an optical isomer thereof, or a deuterated compound thereof, **characterized in that** L¹ is selected from substituted or unsubstituted -(CH₂)ₙ-, and n is any integer from 1 to 3; said substituted substituent is C₁₋₃ alkyl or phenyl.

21. The compound according to claim 20, or a pharmaceutically acceptable salt thereof, or a stereoisomer thereof, or an optical isomer thereof, or a deuterated compound thereof, **characterized in that** L¹ is selected from substituted or unsubstituted -CH₂-; said substituted substituent is methyl or phenyl.

22. The compound according to claim 1, or a pharmaceutically acceptable salt thereof, or a stereoisomer thereof, or an optical isomer thereof, or a deuterated compound thereof, **characterized in that** X is selected from the group consisting of absence, CR⁵R⁶ or NR⁵R⁶; R⁵ and R⁶ are each independently selected from the group consisting of:
H, F, and R^{5a}-, R^{5b}-, R^{5c}-substituted or unsubstituted C₁₋₅ alkyl; or R⁵ and R⁶ are linked to form R^{6a}-, R^{6b}-substituted or unsubstituted 3-6-membered saturated cycloalkyl or 4-membered saturated O-containing heterocyclyl;
R^{5a}, R^{5b}, and R^{5c} are each independently selected from the group consisting of H, ethynyl, hydroxyl, -CONH₂, -CONHCH₃, -N(CH₃)₂, F, methoxy, phenyl, methylsulfonyl, amino, carboxyl, methoxycarbonyl, and azaphenyl; or any two of R^{5a}, R^{5b} , and R^{5c} are linked to form (3-6)-membered saturated cycloalkyl or (5-6)-membered saturated O-containing heterocyclyl;
R^{6a} and R^{6b} are each independently selected from the group consisting of H, vinyl, F, F-substituted methyl; or R^{6a} and R^{6b} are linked to form phenyl;
L² is selected from the group consisting of 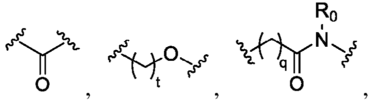 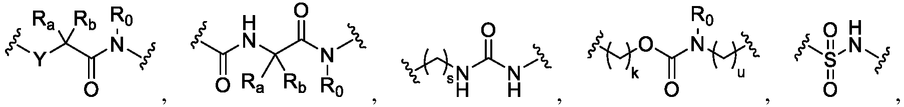 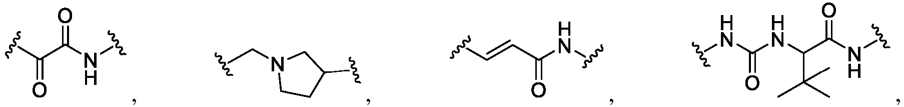 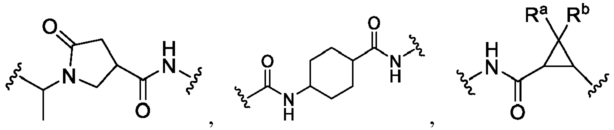 or Rₐ-, R_{b}-substituted or unsubstituted 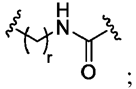
wherein, R₀ is H and C₁₋₃ alkyl; or, R₀ and R₅ are linked to form the following substituted or unsubstituted structures: 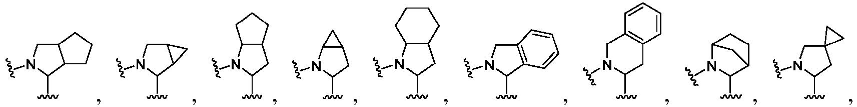 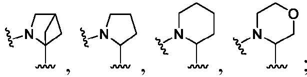 the substituent of said substituted structure is methyl or F;
Rₐ and R_{b} are each independently selected from the group consisting of H, cyano, butyl, and 6-membered cycloalkyl; or Rₐ and R_{b} are linked to form 3-membered carboncycle;
Y is O or S;
t is 0 or 1, q is any integer from 0 to 4, r is 0 or 1, s is 0 or 1, k is 0 or 1, and u is 0 or 1.

23. The compound according to claim 22, or a pharmaceutically acceptable salt thereof, or a stereoisomer thereof, or an optical isomer thereof, or a deuterated compound thereof, **characterized in that** X is CR⁵R⁶, L² is 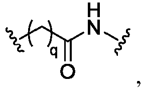 and q is 0 or 1.

24. The compound according to claim 22, or a pharmaceutically acceptable salt thereof, or a stereoisomer thereof, or an optical isomer thereof, or a deuterated compound thereof, **characterized in that** X is selected from the group consisting of absence, 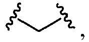 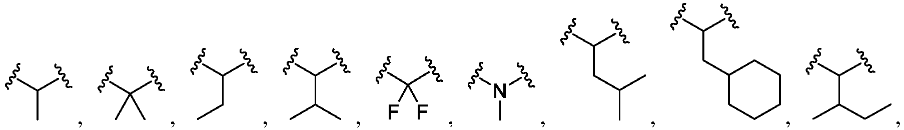 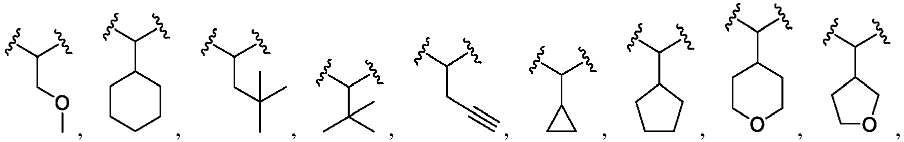 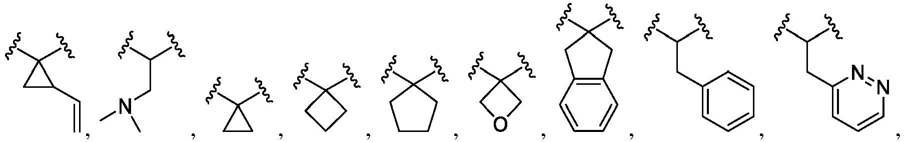 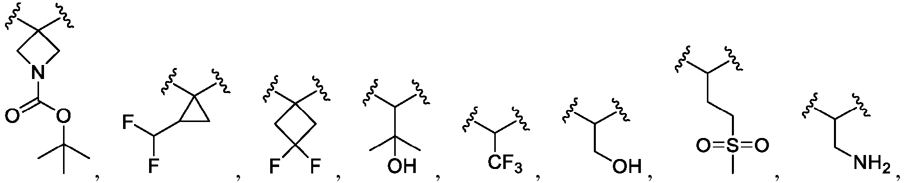 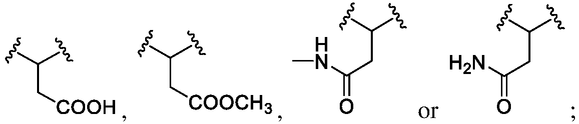
L² is selected from the group consisting of absence, 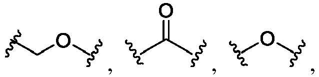 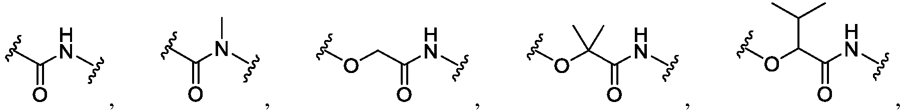 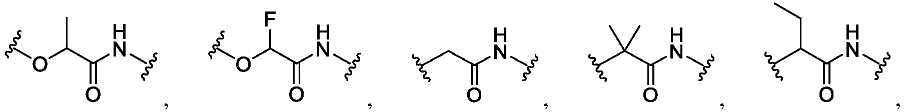 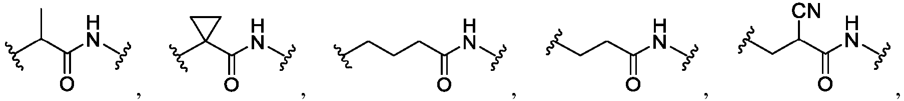 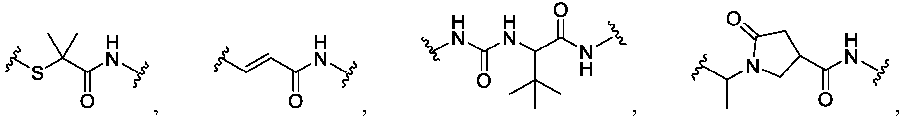 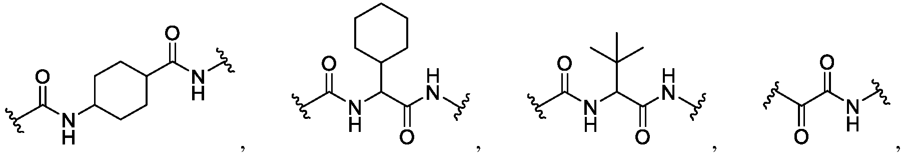 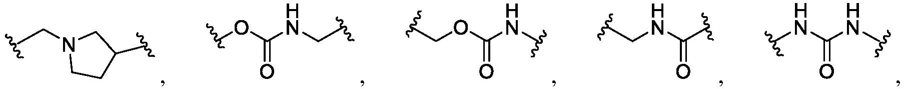 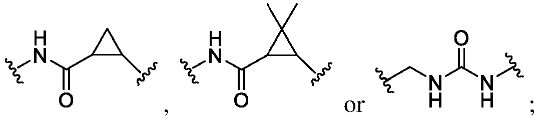
or, L² and X are linked to form any one of the following structures:

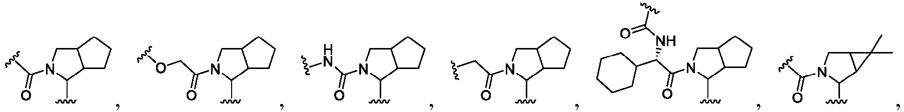

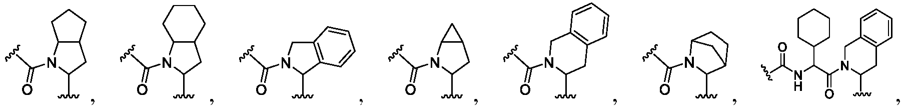

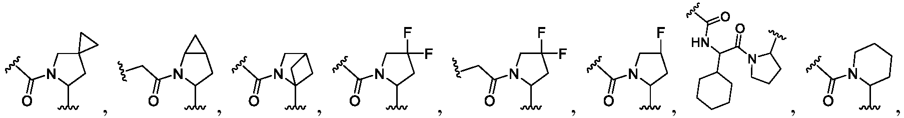

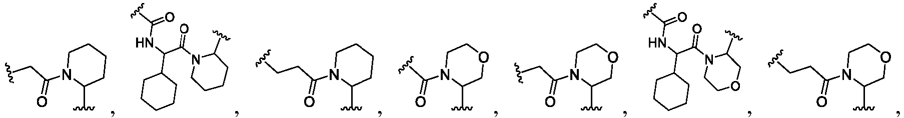

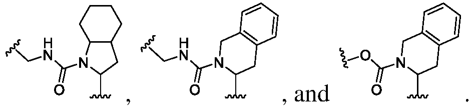

25. The compound according to claim 24, or a pharmaceutically acceptable salt thereof, or a stereoisomer thereof, or an optical isomer thereof, or a deuterated compound thereof, **characterized in that** X is

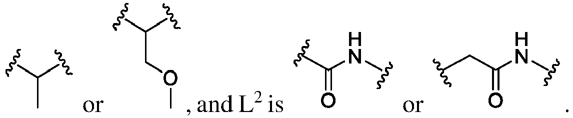

26. The compound according to any one of claims 1 to 25, or a pharmaceutically acceptable salt thereof, or a stereoisomer thereof, or an optical isomer thereof, or a deuterated compound thereof, **characterized in that** the structure of the compound is as represented by formula II or formula III:

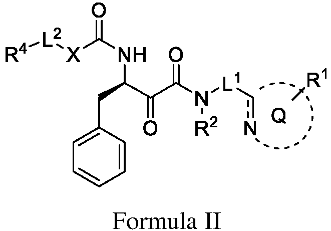

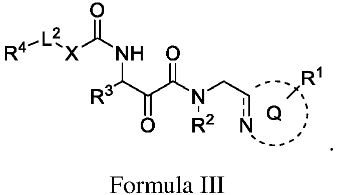

27. The compound according to claim 26, or a pharmaceutically acceptable salt thereof, or a stereoisomer thereof, or an optical isomer thereof, or a deuterated compound thereof, **characterized in that** the structure of the compound is as represented by formula II-A:

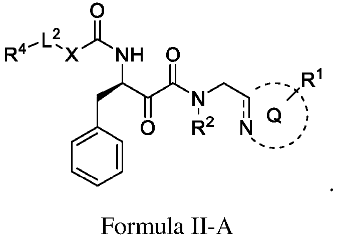

28. The compound according to claim 27, or a pharmaceutically acceptable salt thereof, or a stereoisomer thereof, or an optical isomer thereof, or a deuterated compound thereof, **characterized in that** the structure of the compound is as represented by formula II-A-a, formula II-A-b, formula II-A-c or formula II-A-d:

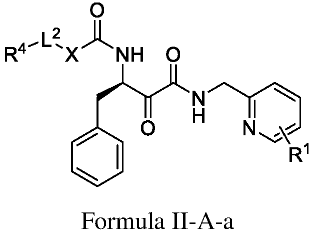

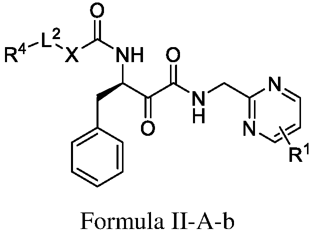

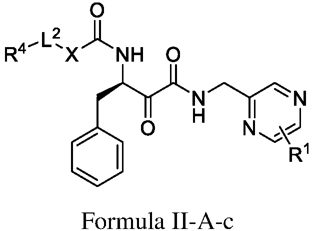

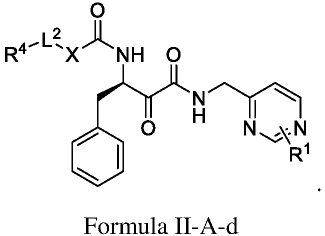

29. The compound according to claim 28, or a pharmaceutically acceptable salt thereof, or a stereoisomer thereof, or an optical isomer thereof, or a deuterated compound thereof, **characterized in that** the structure of the compound is as represented by formula II-A-a-1:

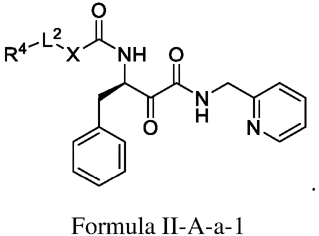

30. The compound according to claim 29, or a pharmaceutically acceptable salt thereof, or a stereoisomer thereof, or an optical isomer thereof, or a deuterated compound thereof, **characterized in that** the structure of the compound is selected from the group consisting of:

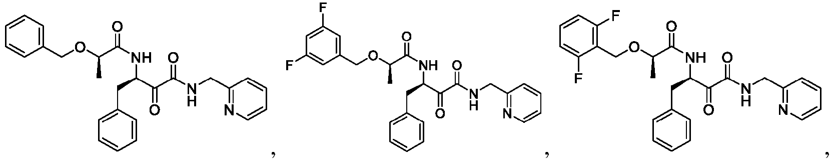

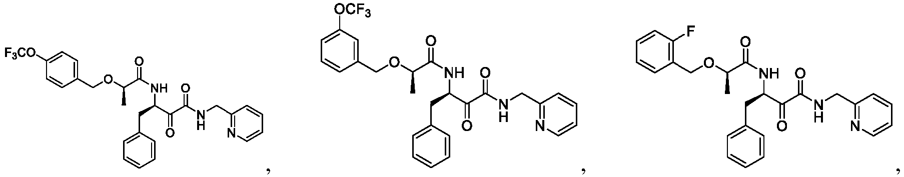

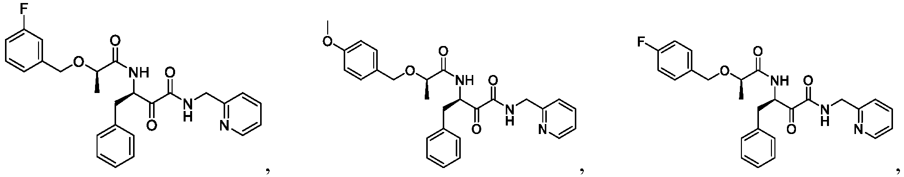

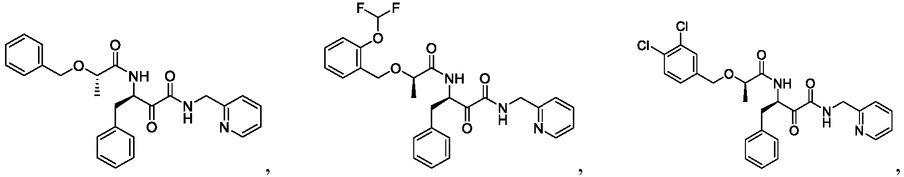

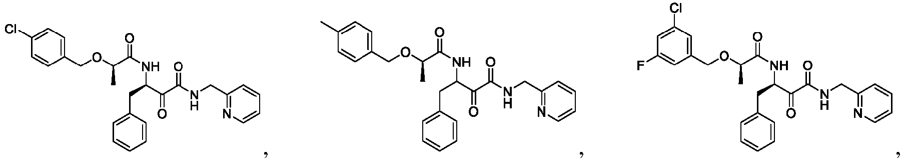

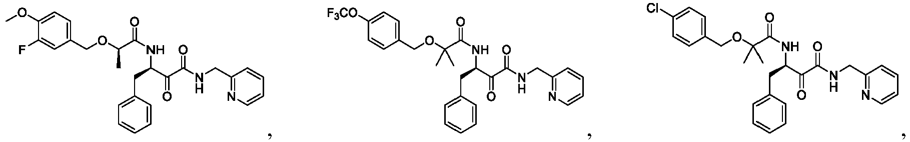

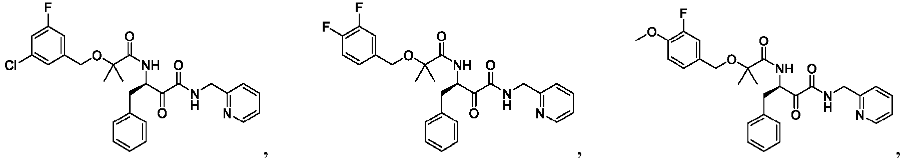

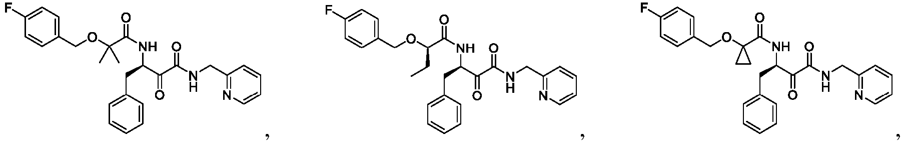

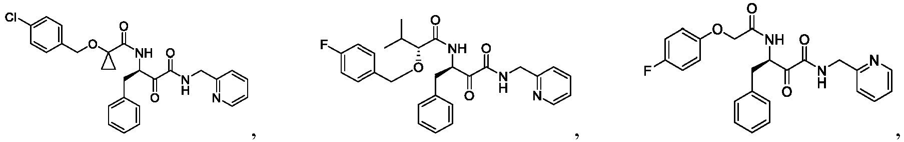

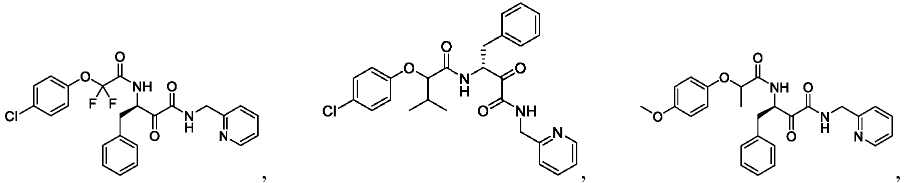

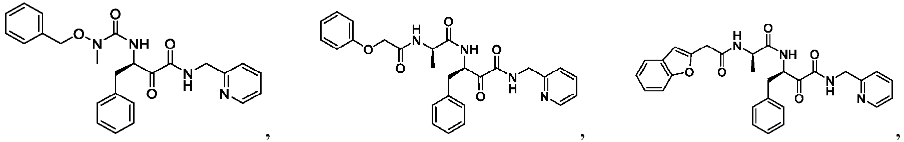

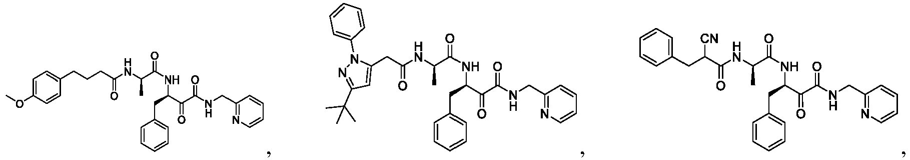

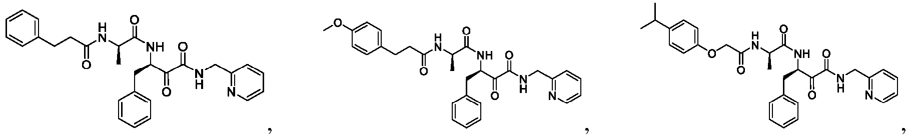

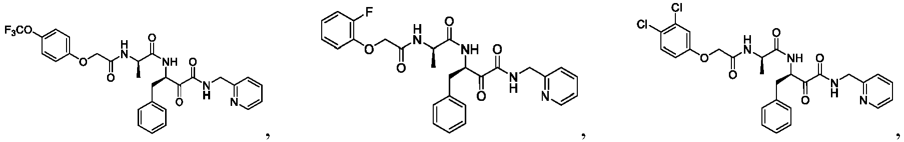

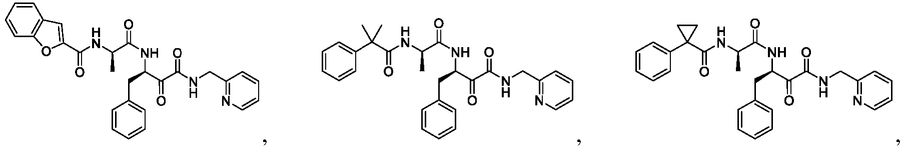

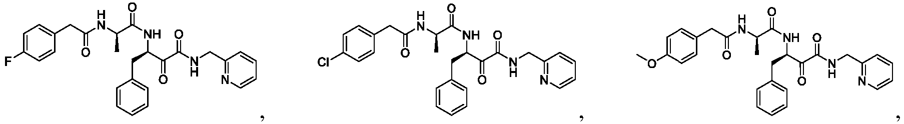

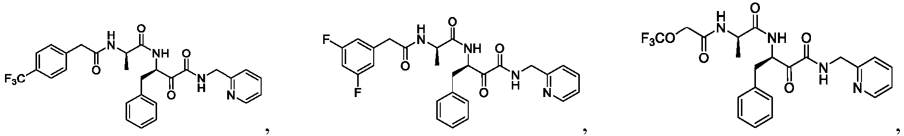

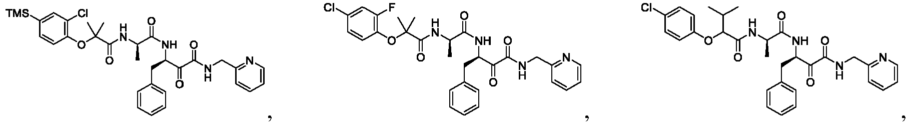

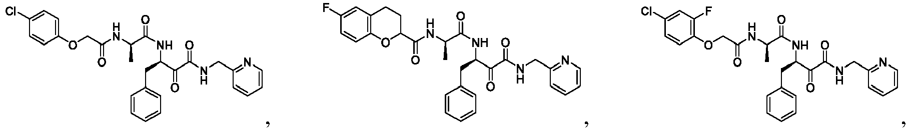

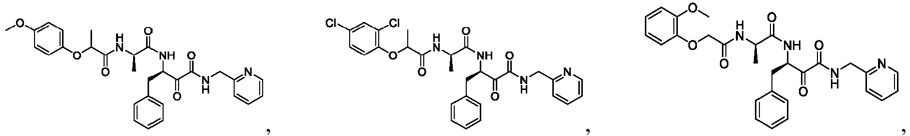

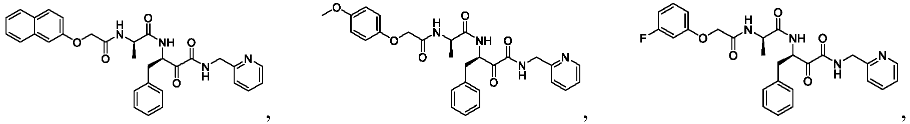

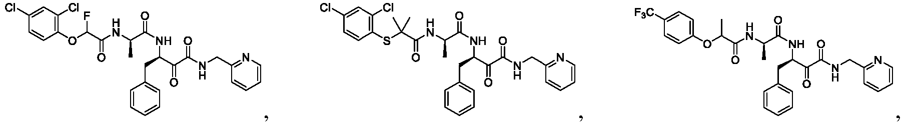

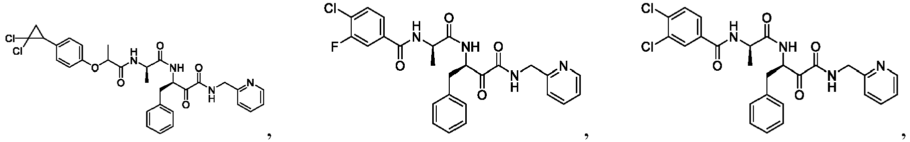

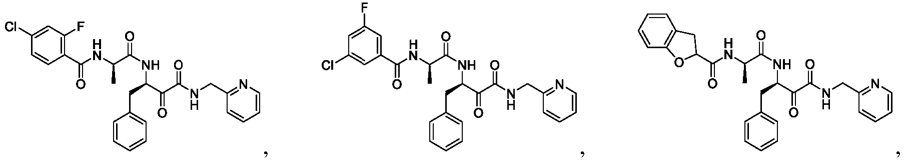

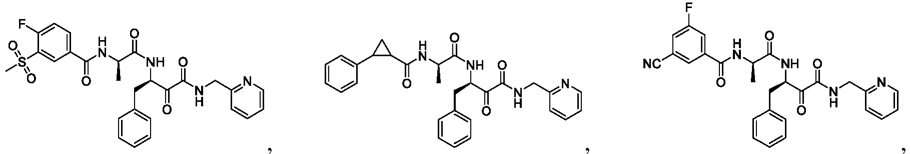

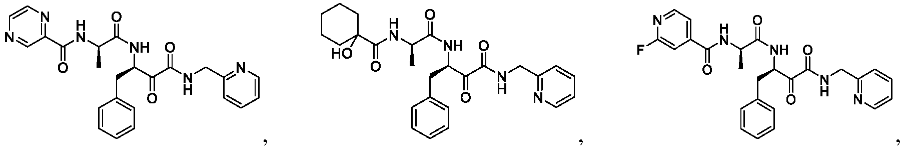

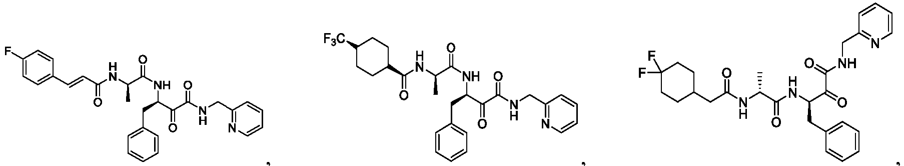

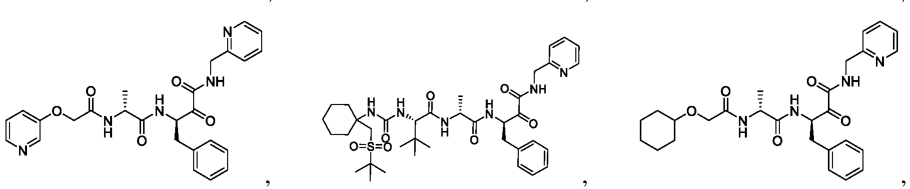

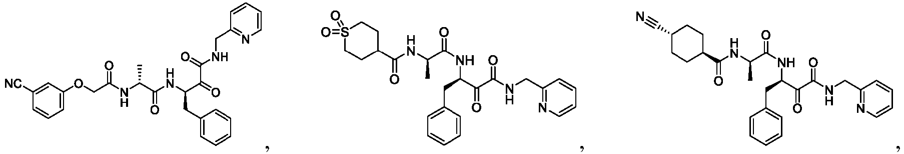

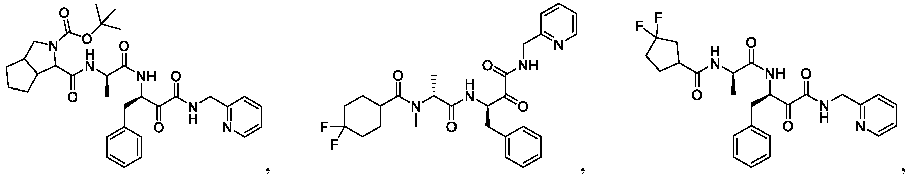

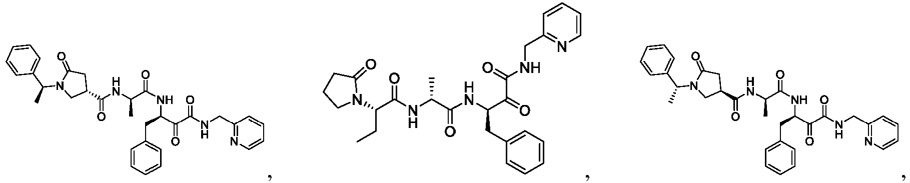

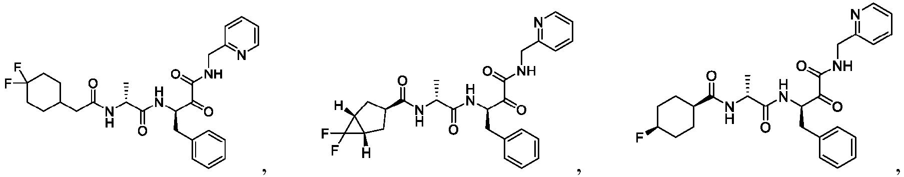

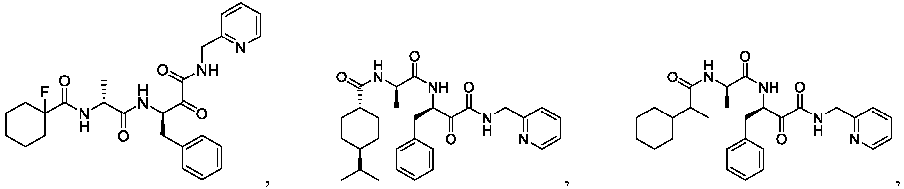

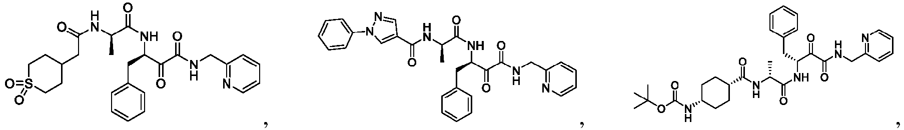

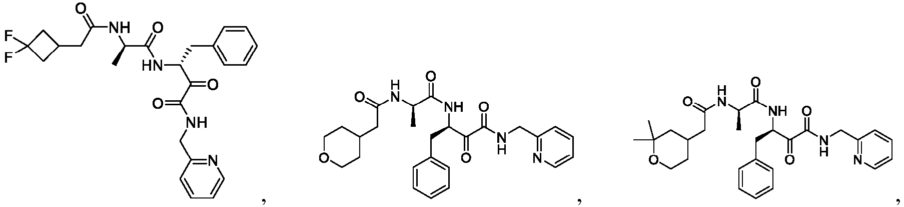

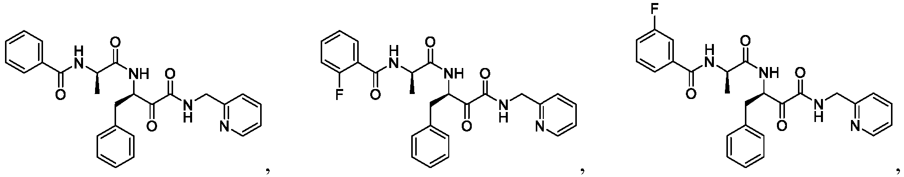

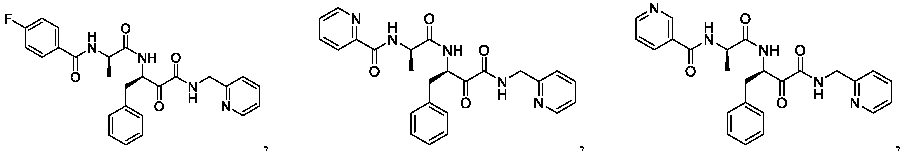

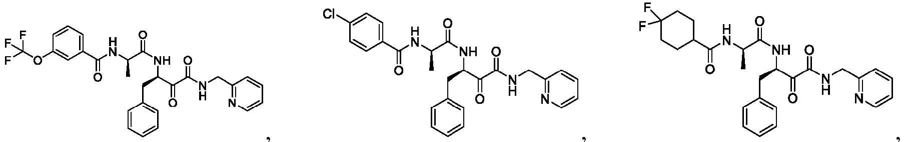

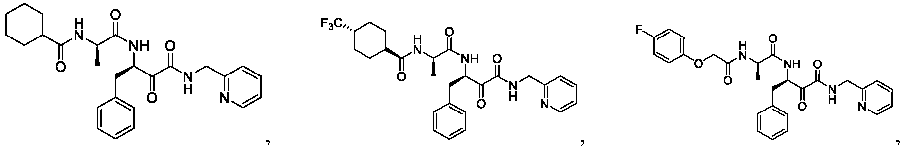

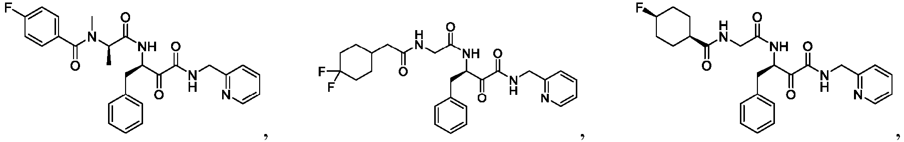

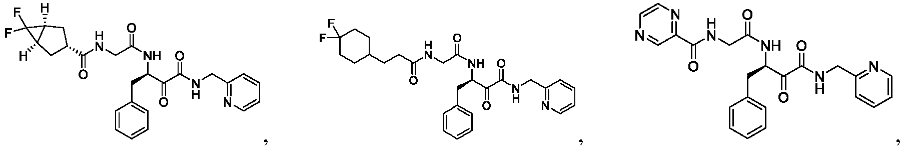

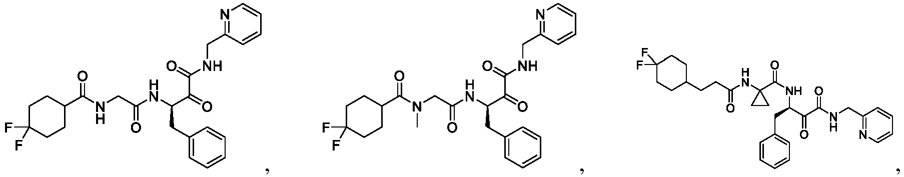

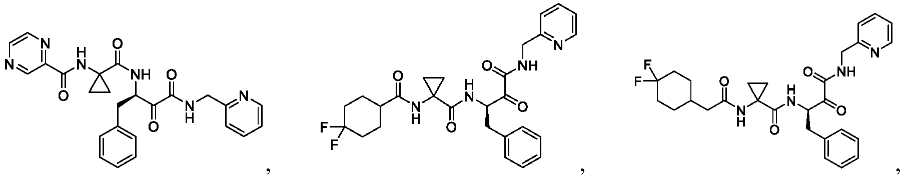

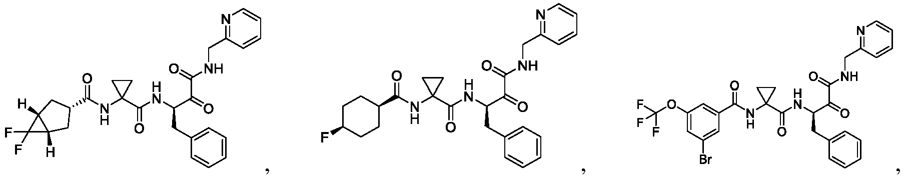

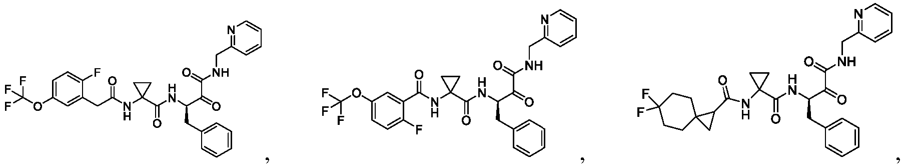

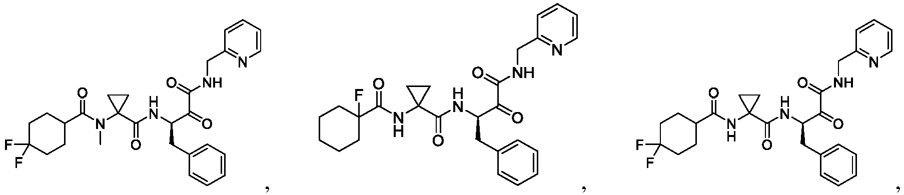

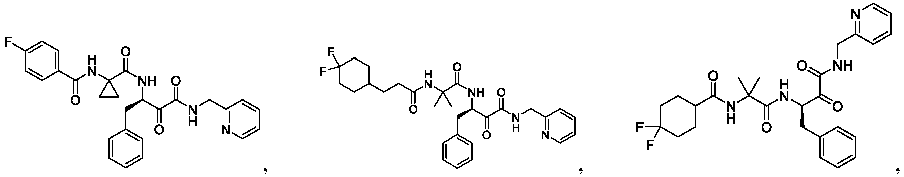

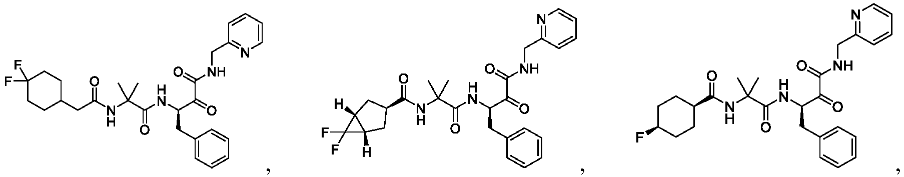

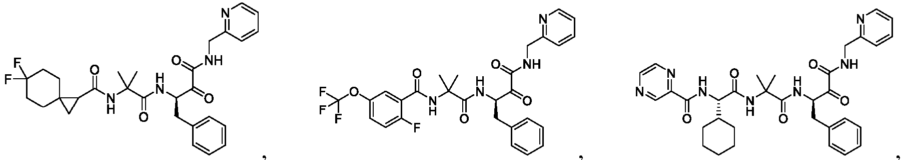

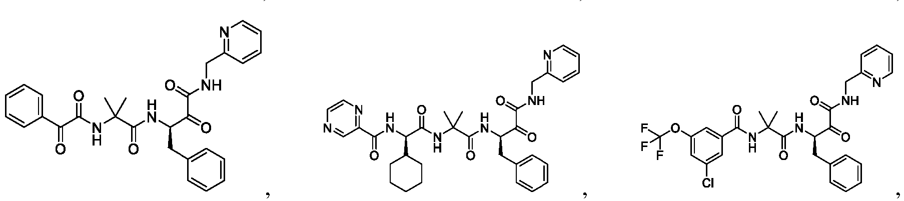

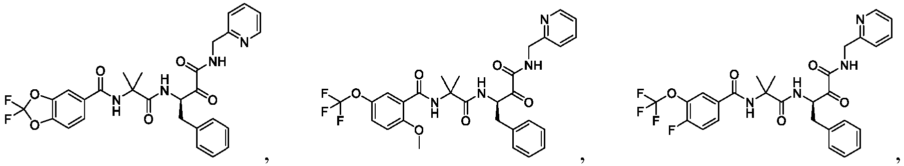

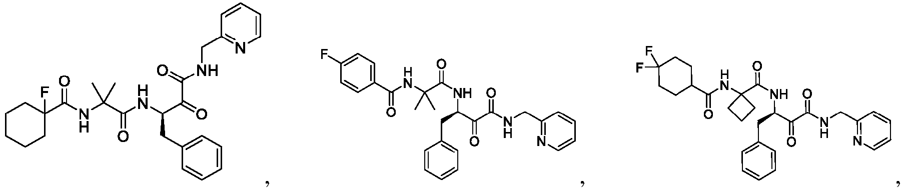

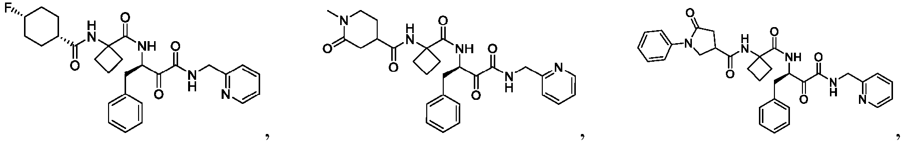

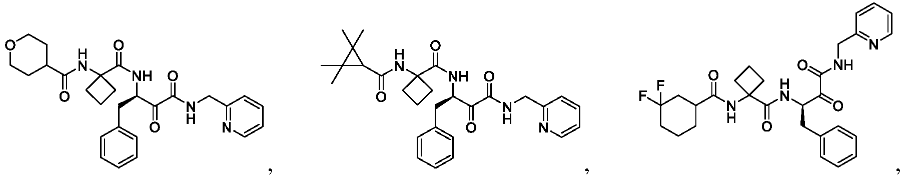

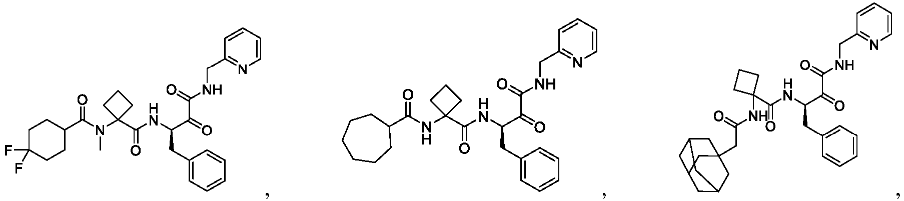

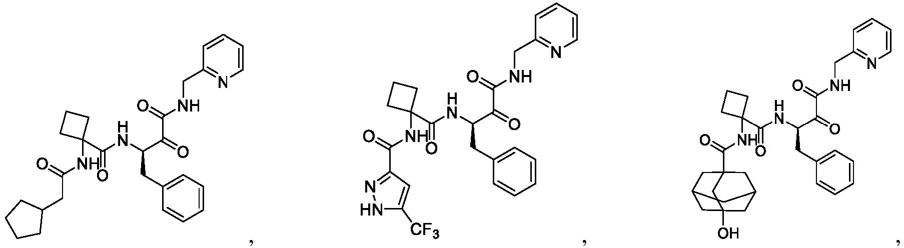

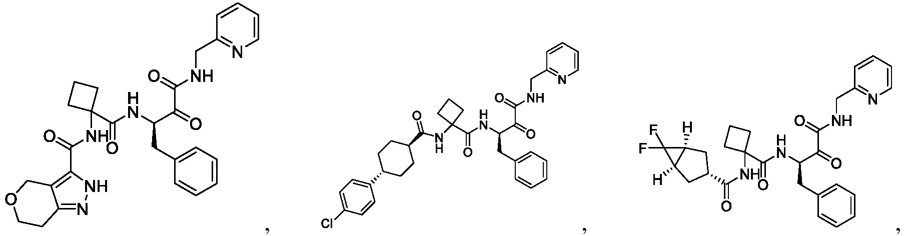

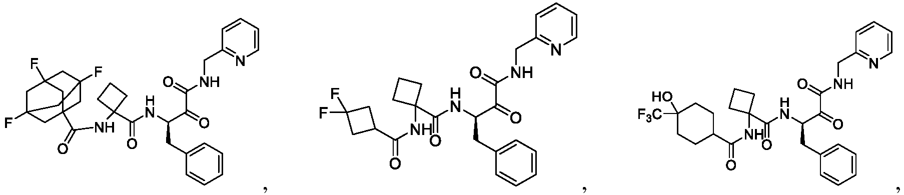

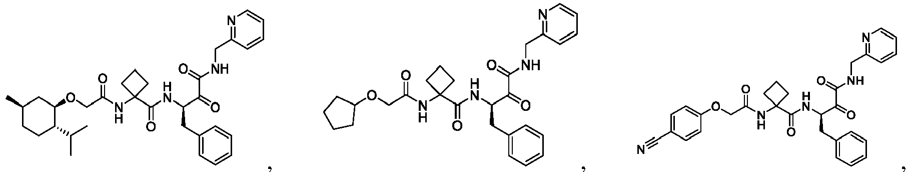

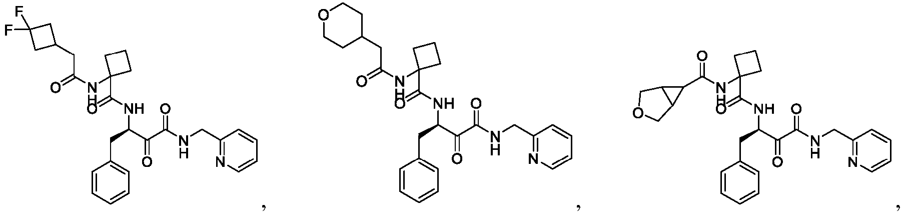

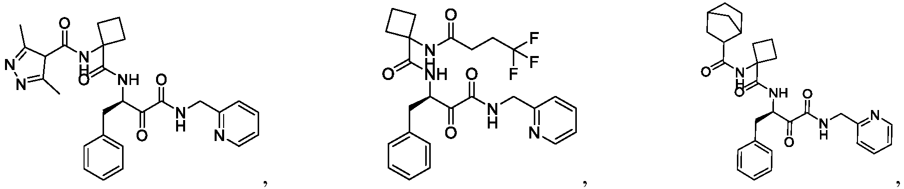

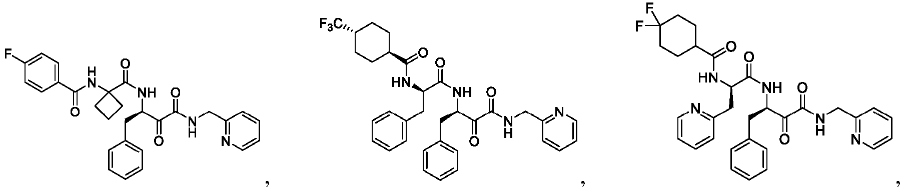

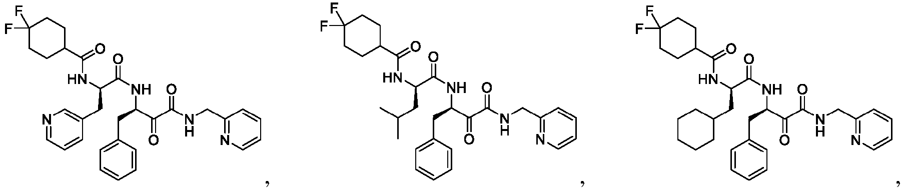

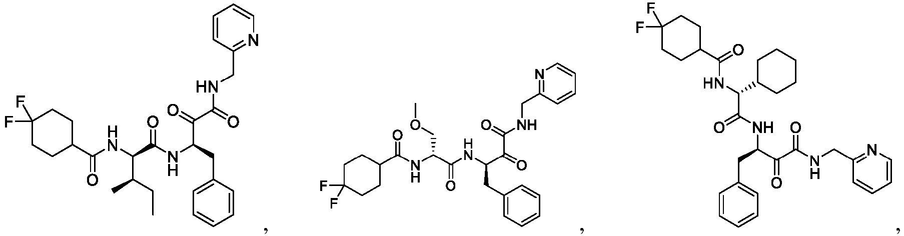

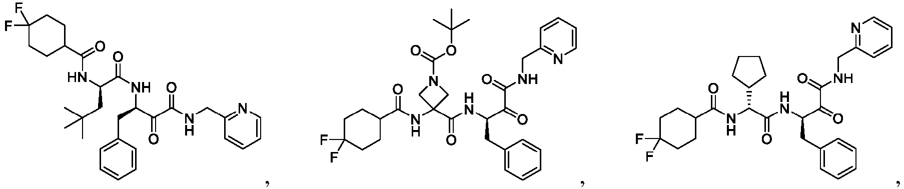

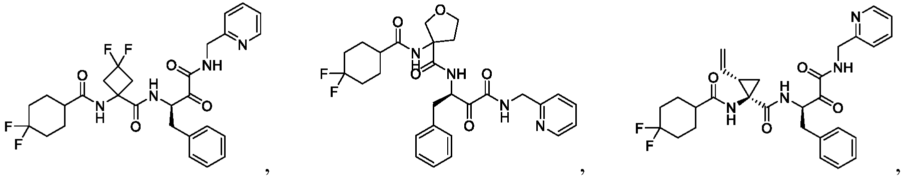

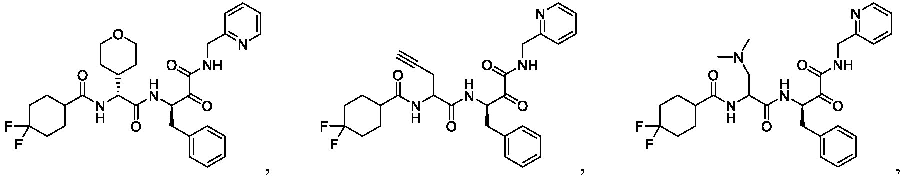

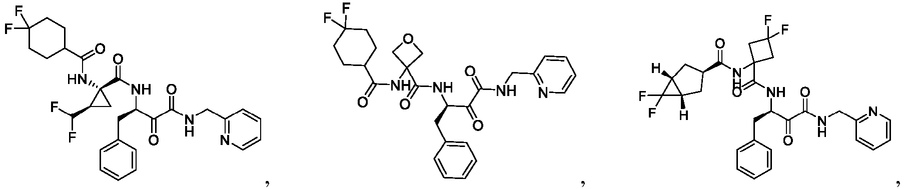

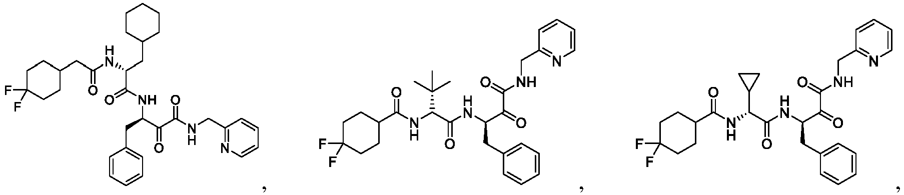

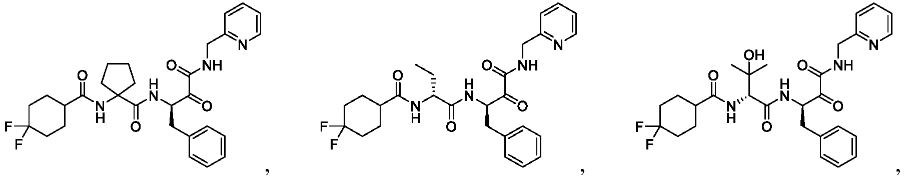

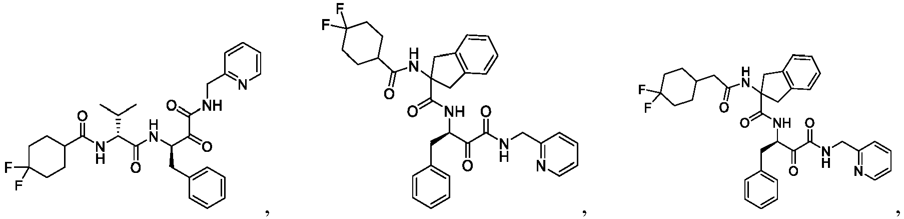

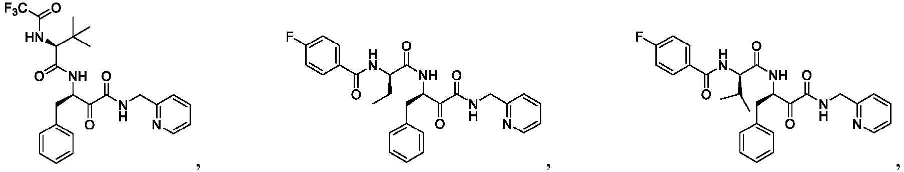

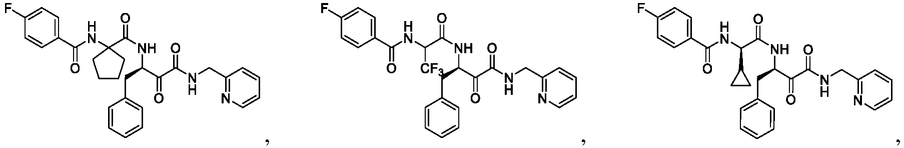

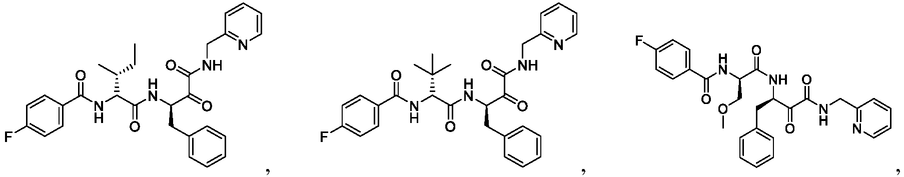

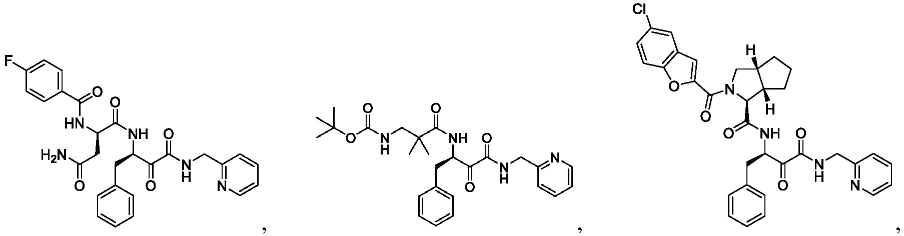

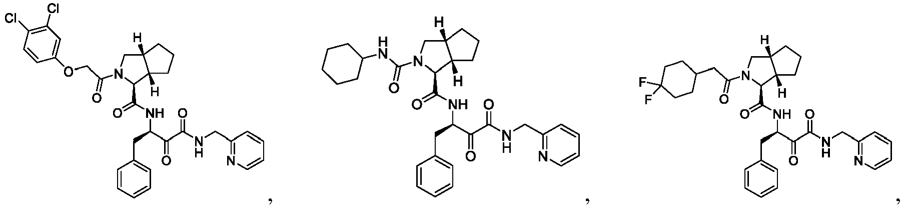

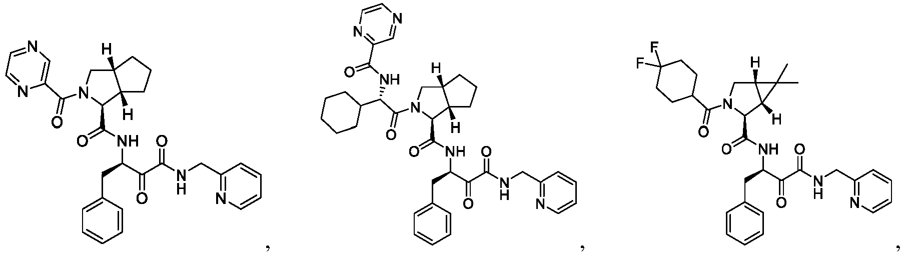

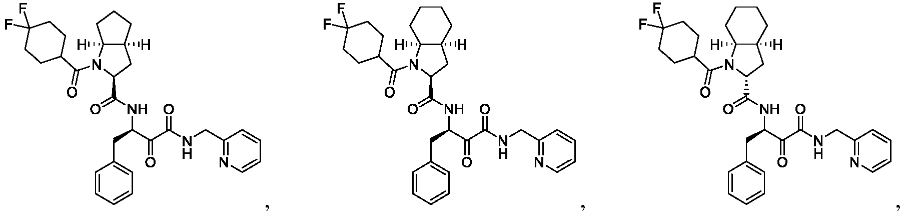

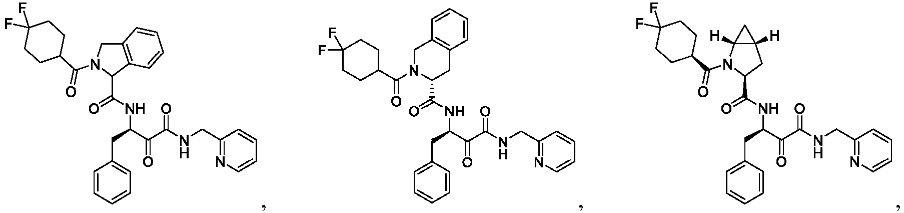

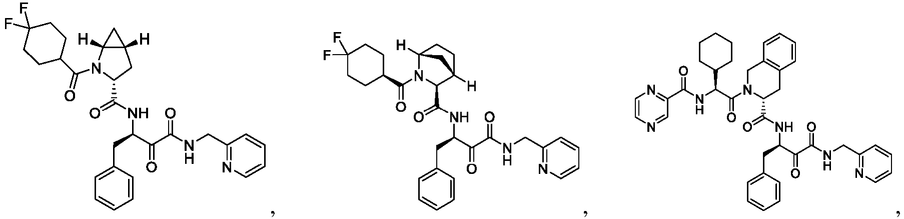

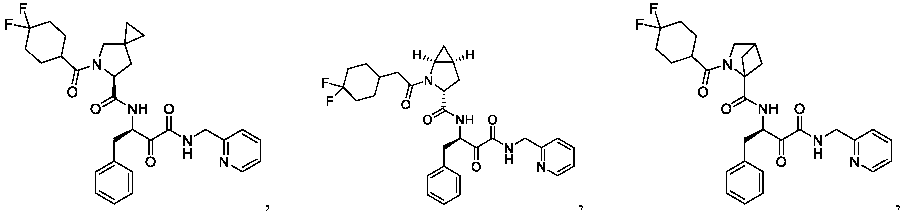

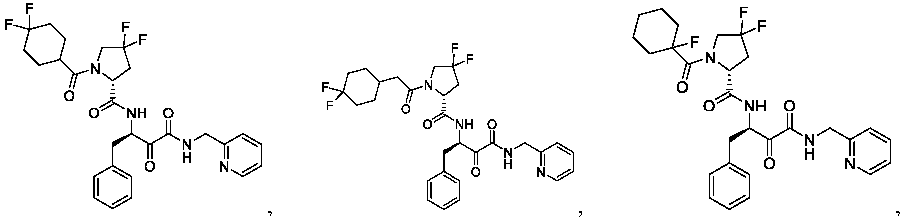

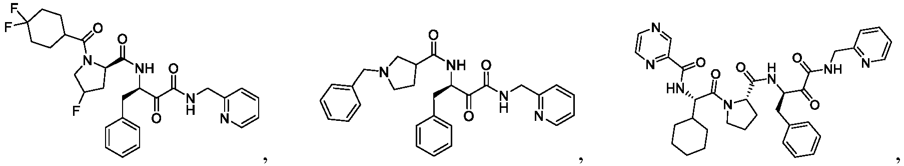

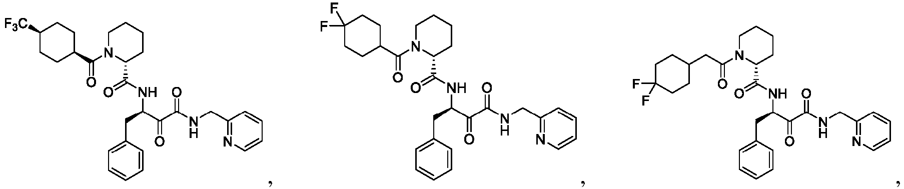

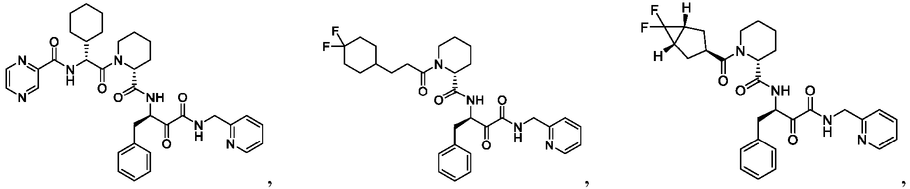

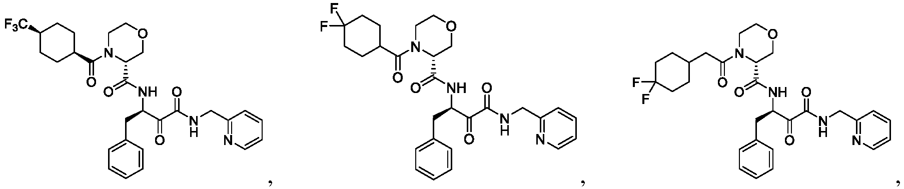

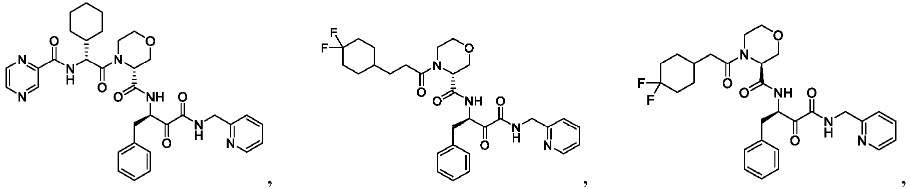

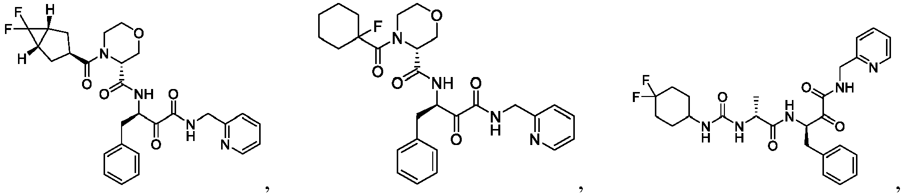

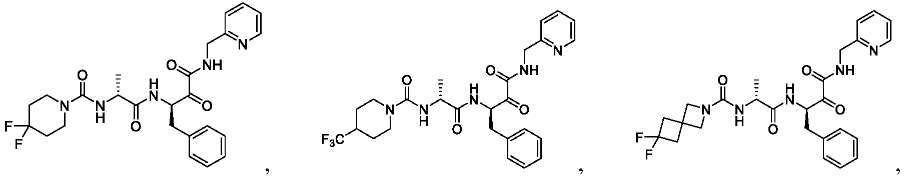

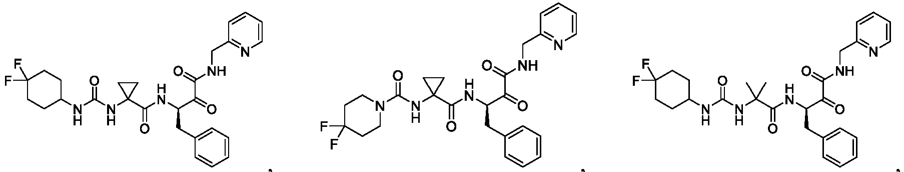

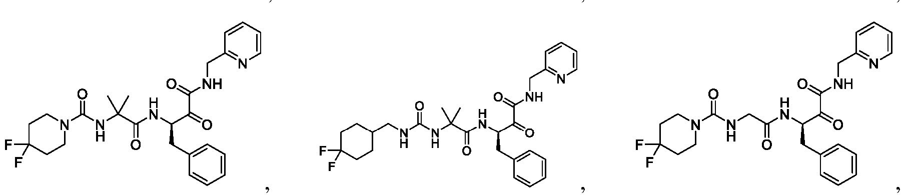

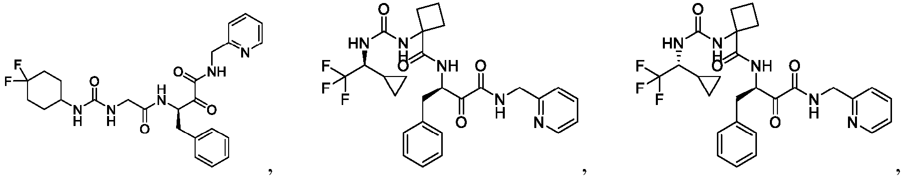

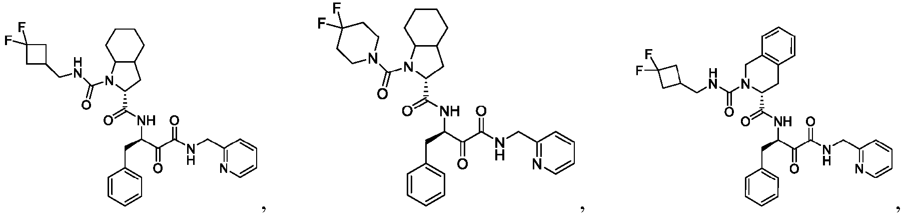

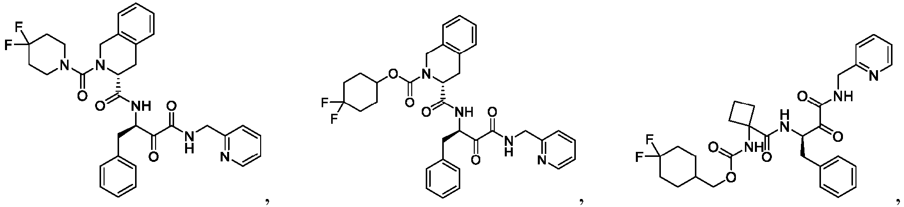

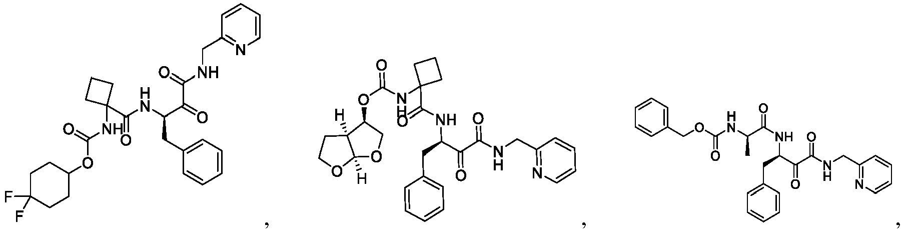

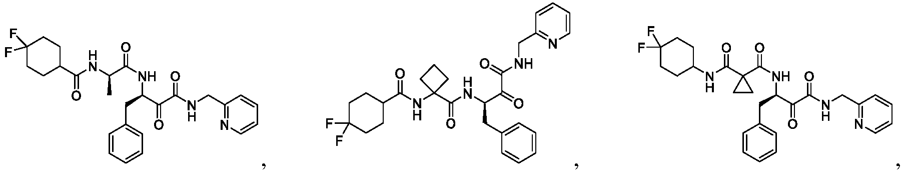

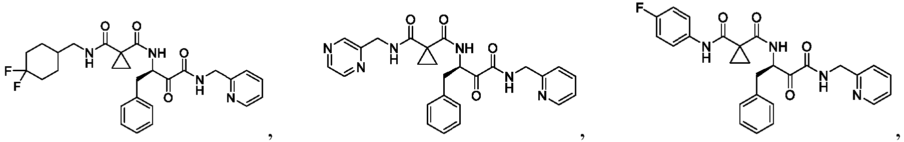

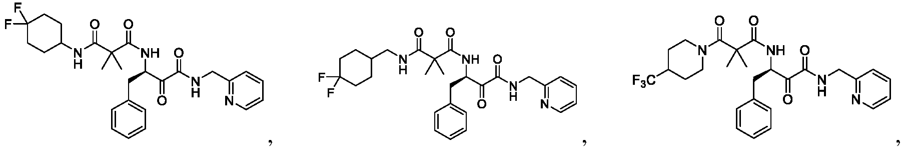

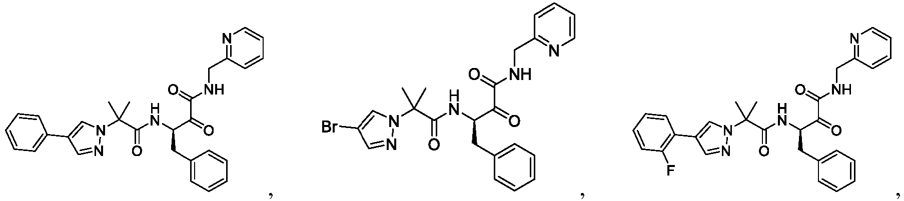

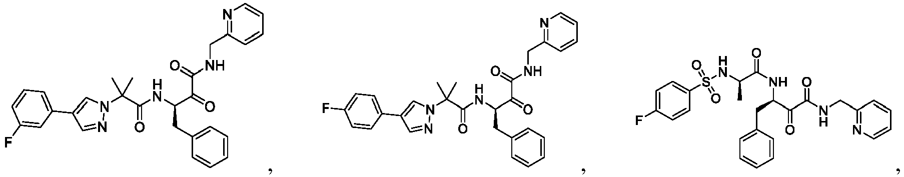

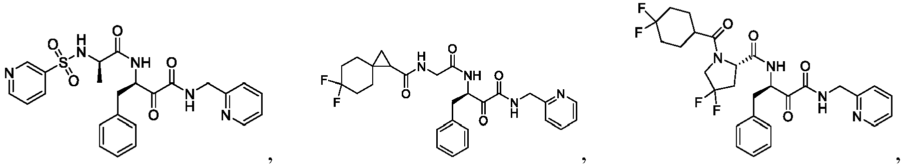

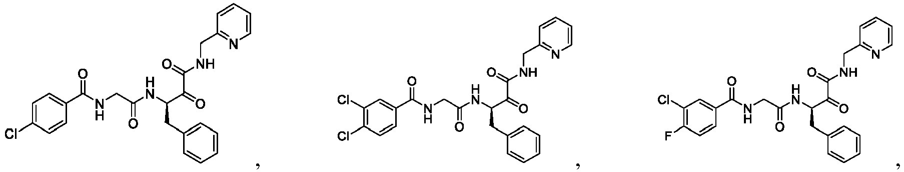

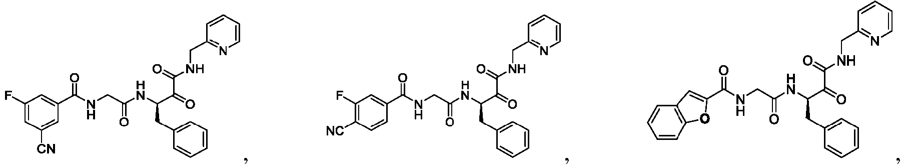

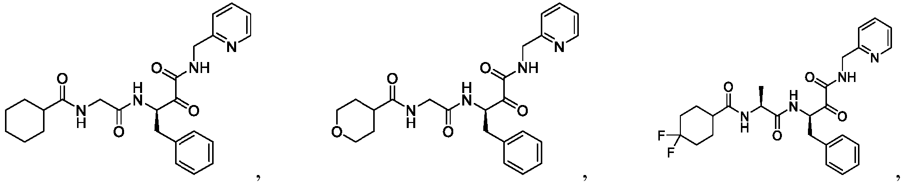

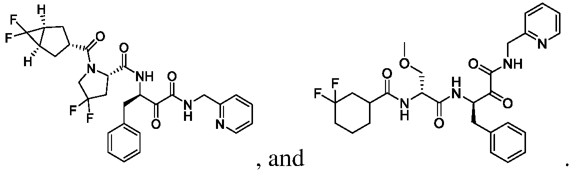

31. The compound according to claim 28, or a pharmaceutically acceptable salt thereof, or a stereoisomer thereof, or an optical isomer thereof, or a deuterated compound thereof, **characterized in that** the structure of the compound is as represented by formula II-A-a-2: 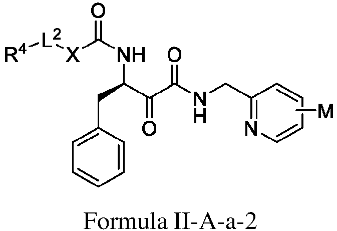 M is selected from the group consisting of F, Cl, CH₃, CH₃O or -CF₃.

32. The compound according to claim 31, or a pharmaceutically acceptable salt thereof, or a stereoisomer thereof, or an optical isomer thereof, or a deuterated compound thereof, **characterized in that** the structure of the compound is selected from the group consisting of: 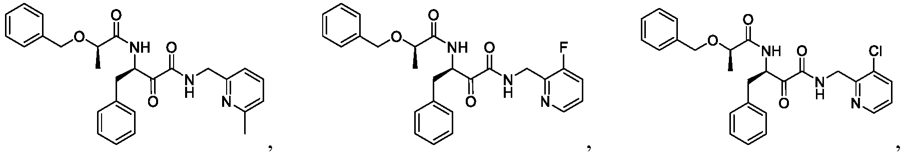 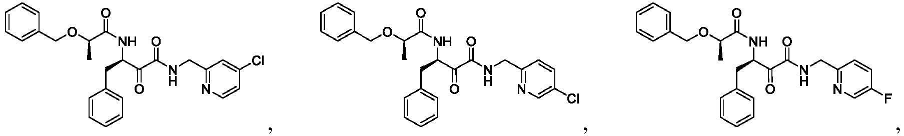 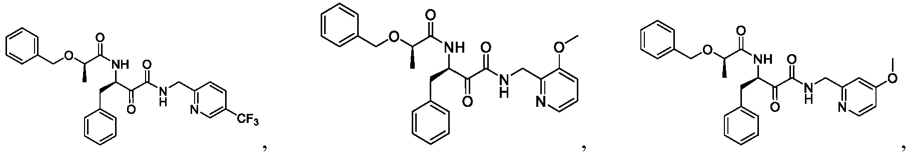 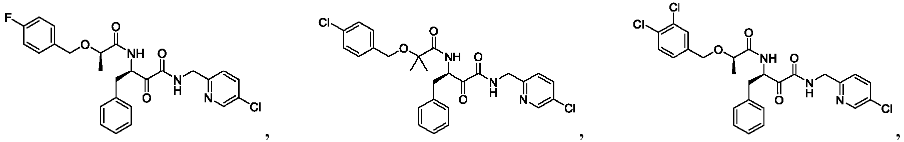 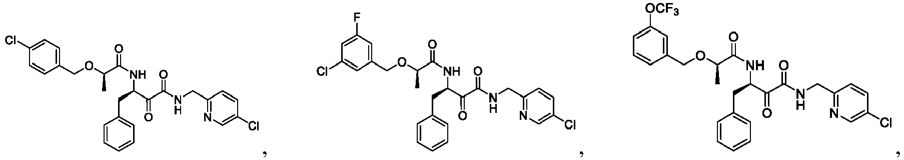 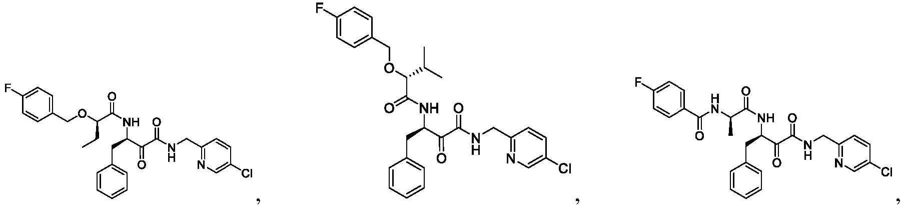 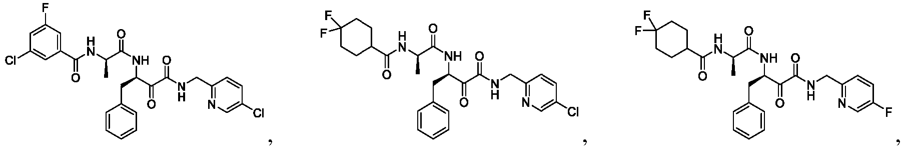 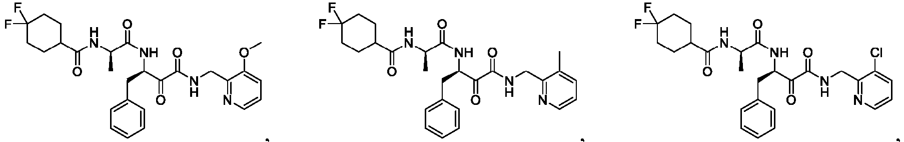 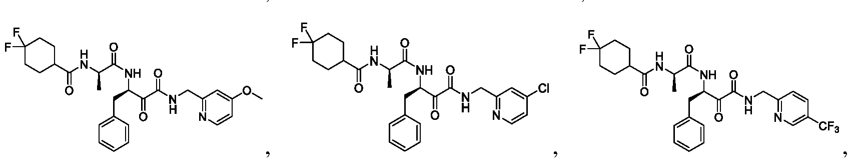 and

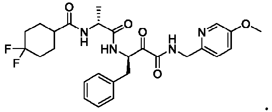

33. The compound according to claim 27, or a pharmaceutically acceptable salt thereof, or a stereoisomer thereof, or an optical isomer thereof, or a deuterated compound thereof, **characterized in that** the structure of the compound is as represented by formula I-A-b-1:

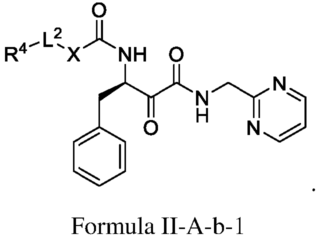

34. The compound according to claim 33, or a pharmaceutically acceptable salt thereof, or a stereoisomer thereof, or an optical isomer thereof, or a deuterated compound thereof, **characterized in that** the compound has the following structure:

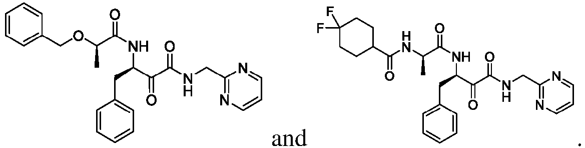

35. The compound according to claim 27, or a pharmaceutically acceptable salt thereof, or a stereoisomer thereof, or an optical isomer thereof, or a deuterated compound thereof, **characterized in that** the structure of the compound is as represented by formula II-A-c-1:

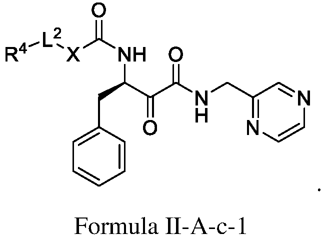

36. The compound according to claim 35, or a pharmaceutically acceptable salt thereof, or a stereoisomer thereof, or an optical isomer thereof, or a deuterated compound thereof, **characterized in that** the compound has the following structure:

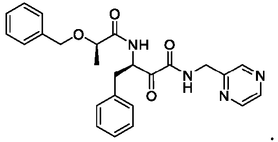

37. The compound according to claim 27, or a pharmaceutically acceptable salt thereof, or a stereoisomer thereof, or an optical isomer thereof, or a deuterated compound thereof, **characterized in that** the structure of the compound is as represented by formula II-A-d-1:

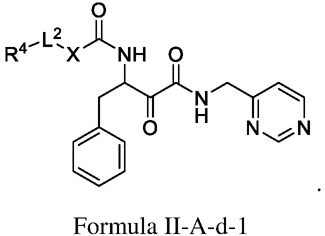

38. The compound according to claim 37, or a pharmaceutically acceptable salt thereof, or a stereoisomer thereof, or an optical isomer thereof, or a deuterated compound thereof, **characterized in that** the compound has the following structures:

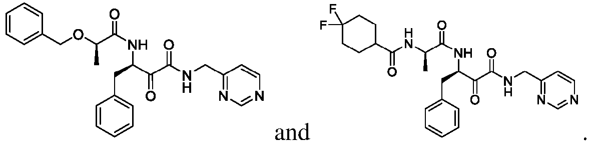

39. The compound according to claim 27, or a pharmaceutically acceptable salt thereof, or a stereoisomer thereof, or an optical isomer thereof, or a deuterated compound thereof, **characterized in that** the structure of the compound is as represented by formula II-A-e, formula II-A-f, formula II-A-g, formula II-A-h, formula II-A-i, formula II-A-i1, formula II-A-i2, formula II-A-i3, formula II-A-i4 or formula II-A-i5: 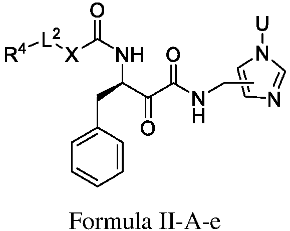 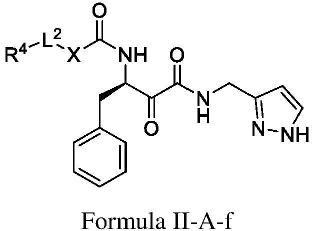 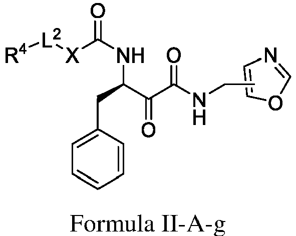 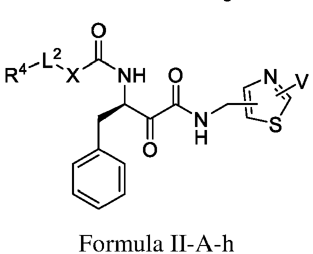 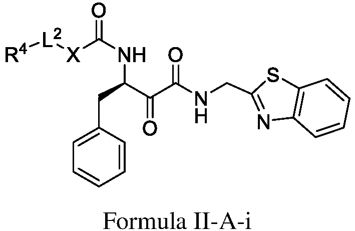 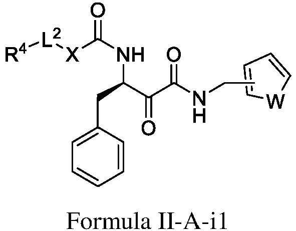 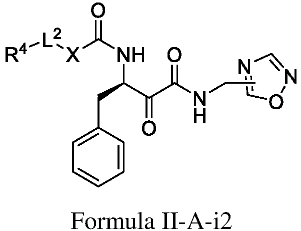 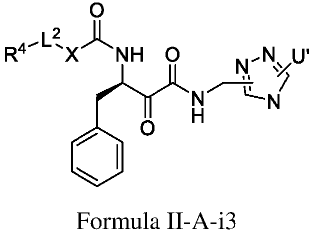 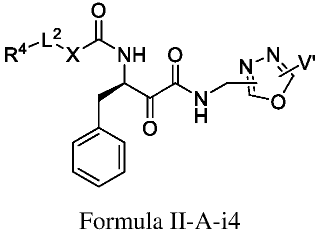 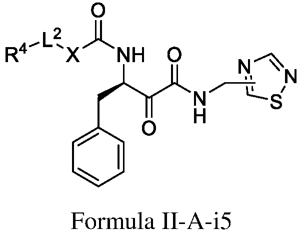 wherein, U, V, U', and V' are each independently selected from the group consisting of H, C₁₋₃ alkyl or halogen, and preferably are H, CH₃ or Cl; W is O or S.

40. The compound according to claim 39, or a pharmaceutically acceptable salt thereof, or a stereoisomer thereof, or an optical isomer thereof, or a deuterated compound thereof, **characterized in that** the structure of the compound is as represented by formula II-A-e-1:

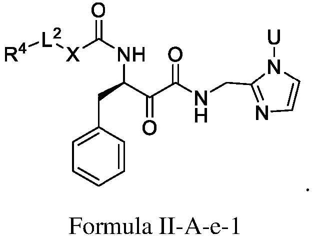

41. The compound according to claim 40, or a pharmaceutically acceptable salt thereof, or a stereoisomer thereof, or an optical isomer thereof, or a deuterated compound thereof, **characterized in that** the compound has the following structures:

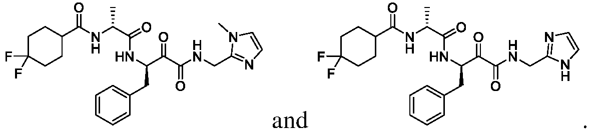

42. The compound according to claim 39, or a pharmaceutically acceptable salt thereof, or a stereoisomer thereof, or an optical isomer thereof, or a deuterated compound thereof, **characterized in that** the structure of the compound is as represented by formula II-A-e-2:

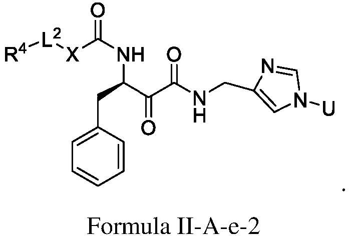

43. The compound according to claim 42, or a pharmaceutically acceptable salt thereof, or a stereoisomer thereof, or an optical isomer thereof, or a deuterated compound thereof, **characterized in that** the compound has the following structure:

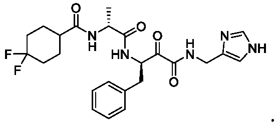

44. The compound according to claim 39, or a pharmaceutically acceptable salt thereof, or a stereoisomer thereof, or an optical isomer thereof, or a deuterated compound thereof, **characterized in that** the structure of the compound is as represented by any one of the following structures:

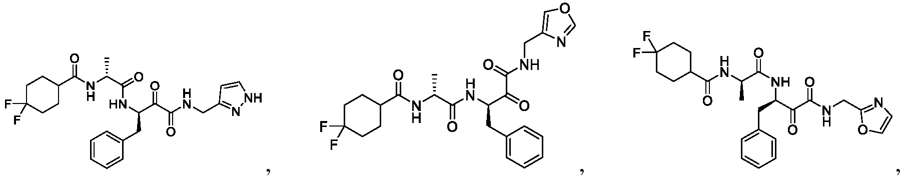

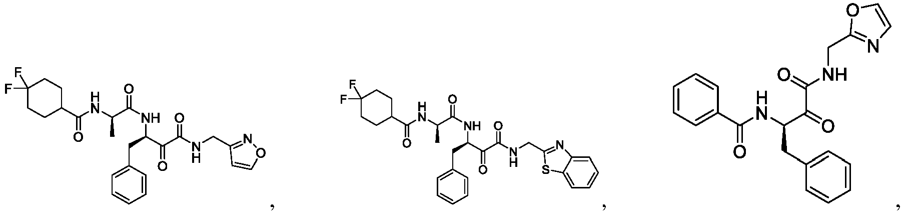

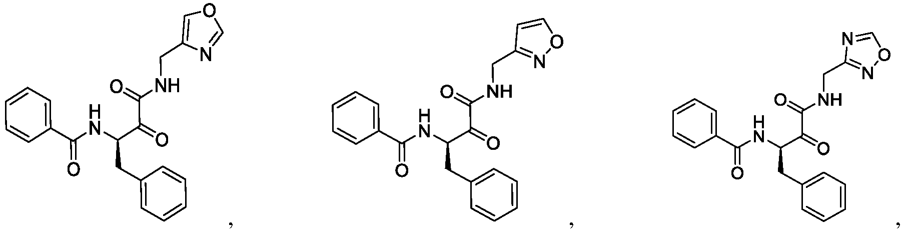

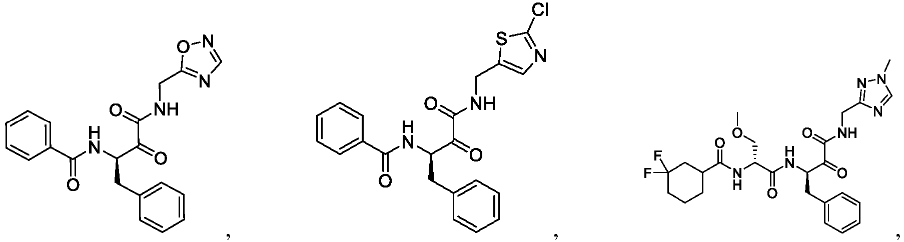

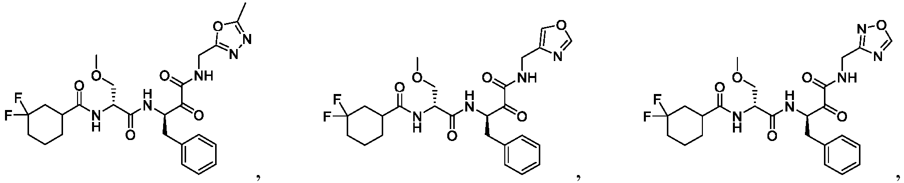

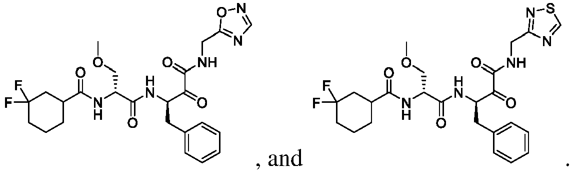

45. The compound according to claim 39, or a pharmaceutically acceptable salt thereof, or a stereoisomer thereof, or an optical isomer thereof, or a deuterated compound thereof, **characterized in that** the structure of the compound is as represented by formula II-A-h-1:

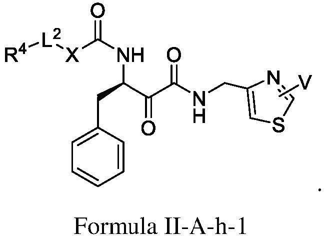

46. The compound according to claim 45, or a pharmaceutically acceptable salt thereof, or a stereoisomer thereof, or an optical isomer thereof, or a deuterated compound thereof, **characterized in that** the structure of the compound is as represented by any one of the following structures:

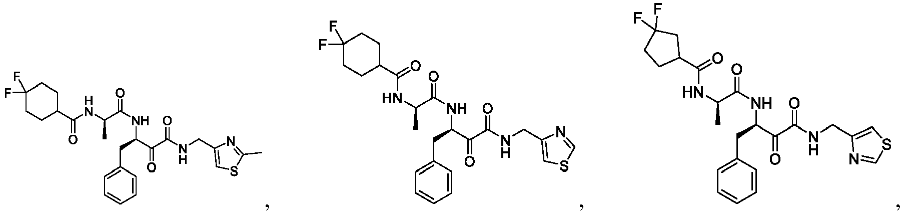

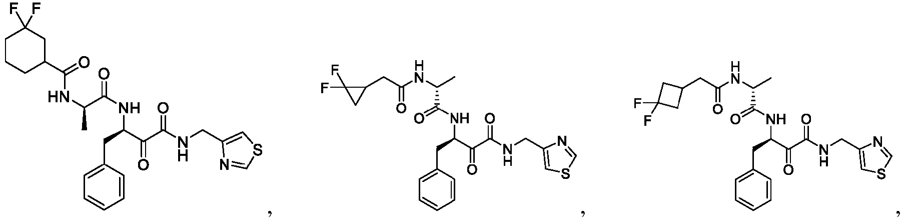

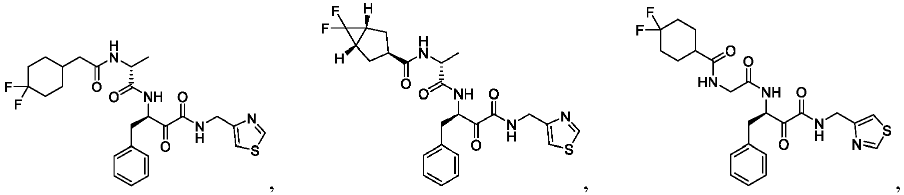

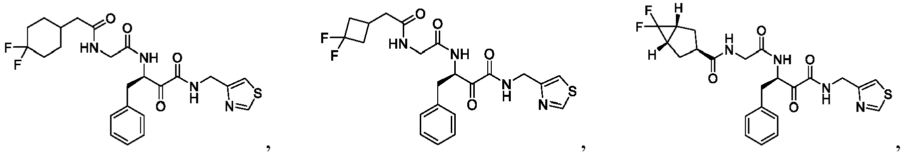

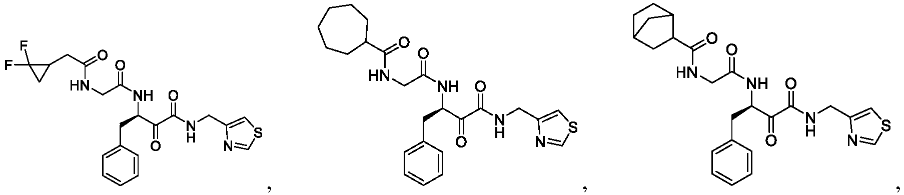

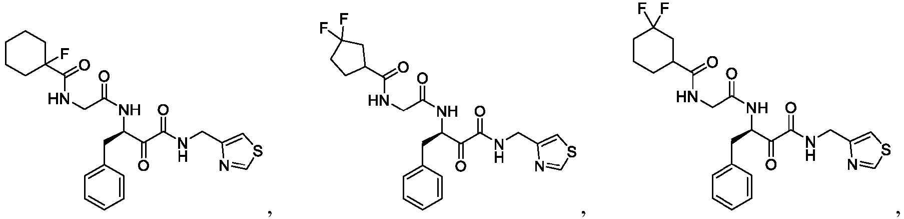

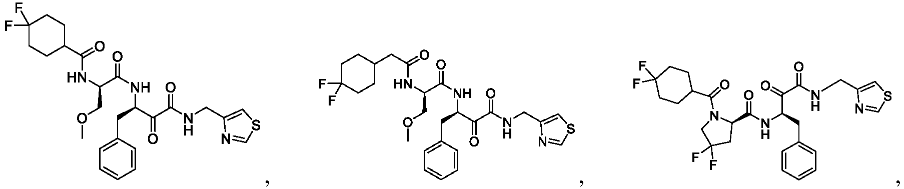

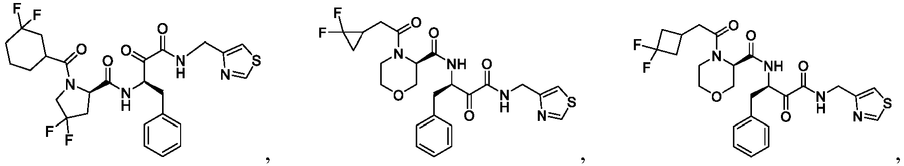

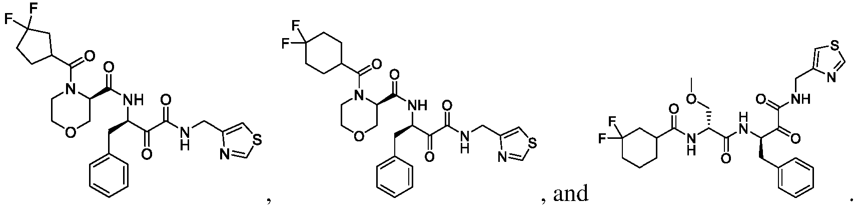

47. The compound according to claim 39, or a pharmaceutically acceptable salt thereof, or a stereoisomer thereof, or an optical isomer thereof, or a deuterated compound thereof, **characterized in that** the structure of the compound is as represented by formula II-A-h-2:

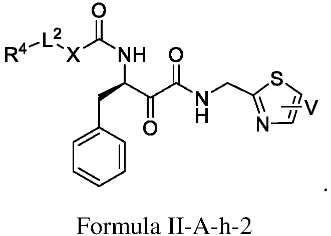

48. The compound according to claim 47, or a pharmaceutically acceptable salt thereof, or a stereoisomer thereof, or an optical isomer thereof, or a deuterated compound thereof, **characterized in that** the structure of the compound is as represented by any one of the following structures:

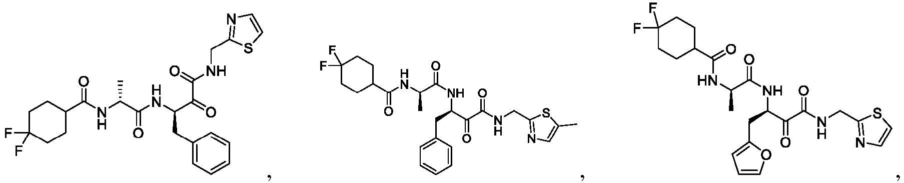

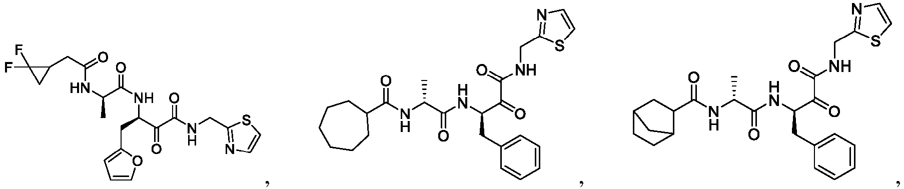

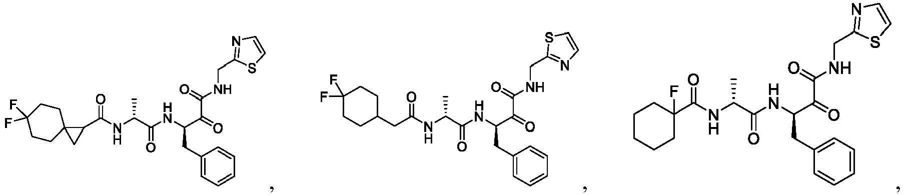

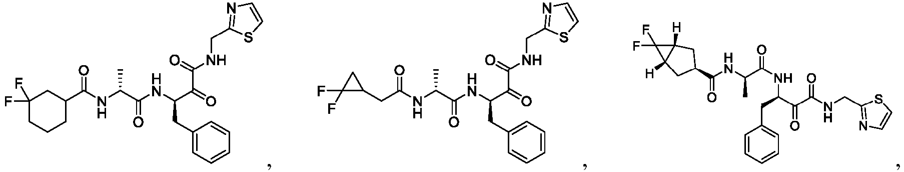

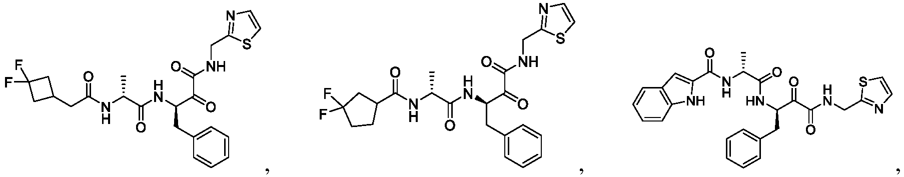

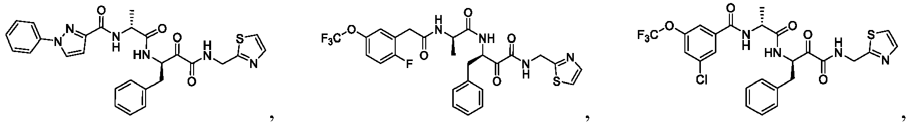

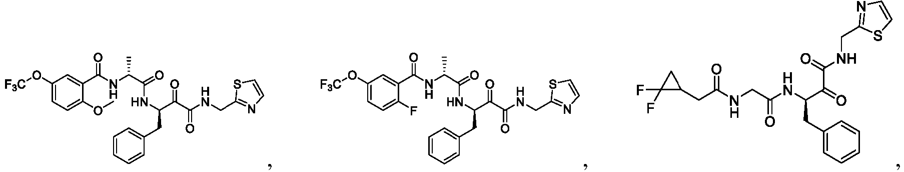

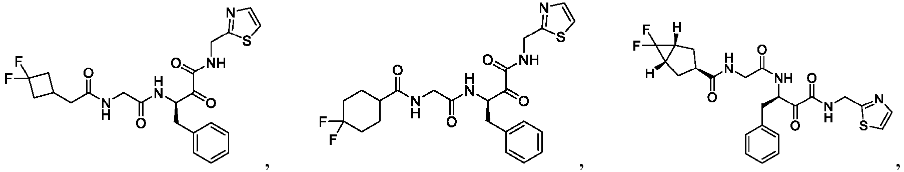

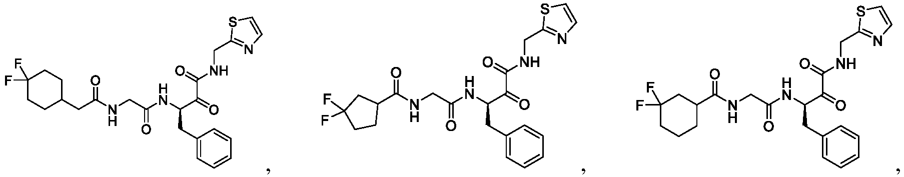

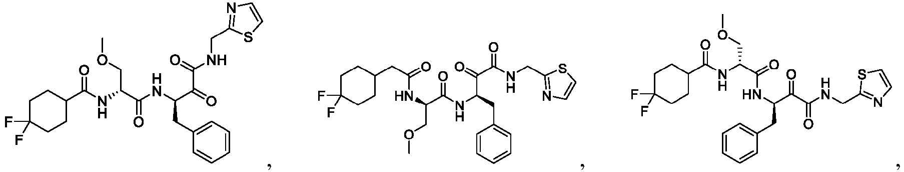

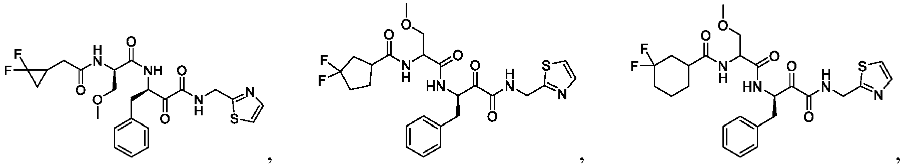

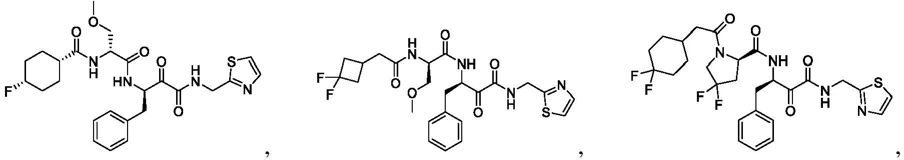

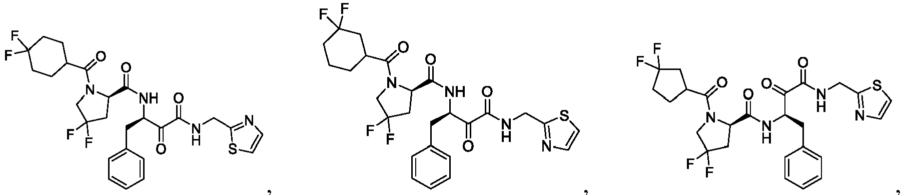

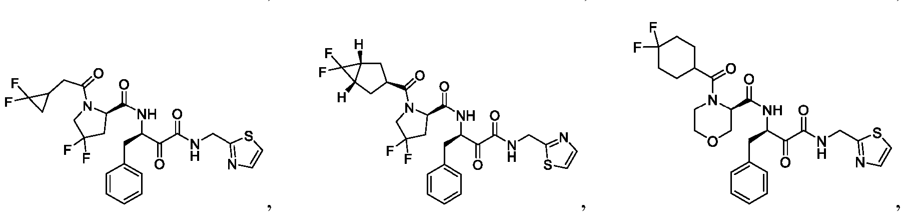

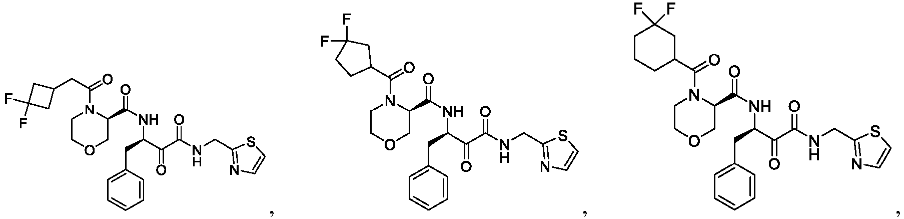

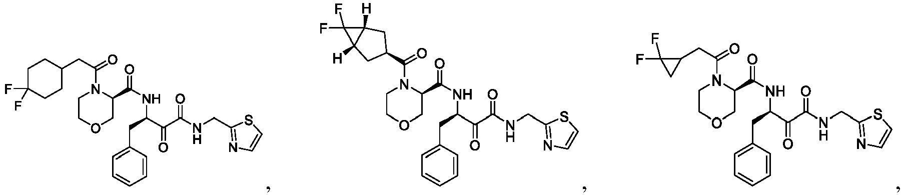

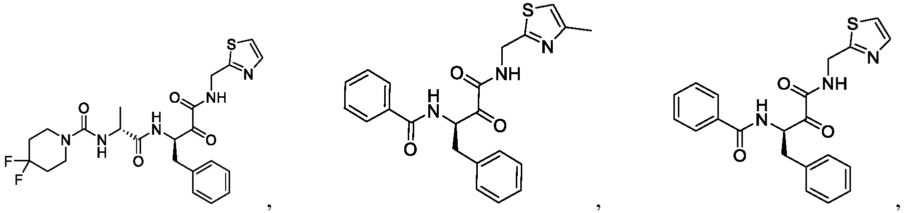

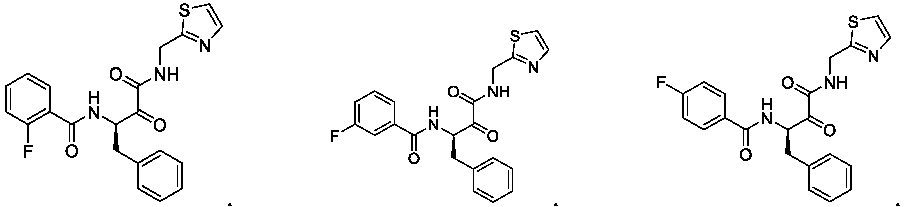

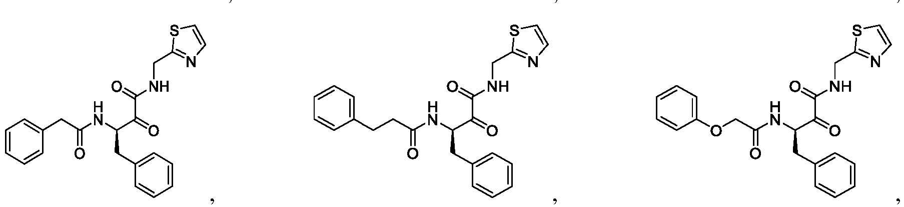

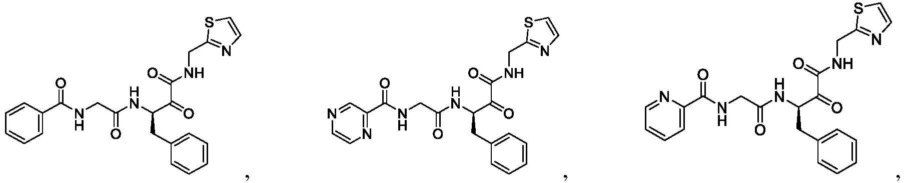

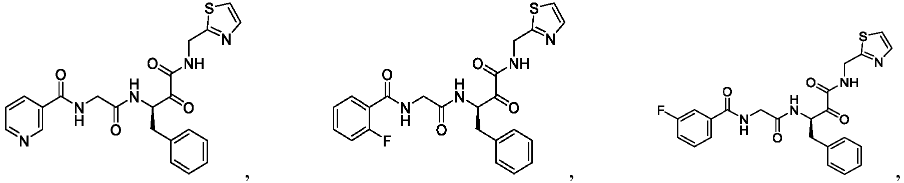

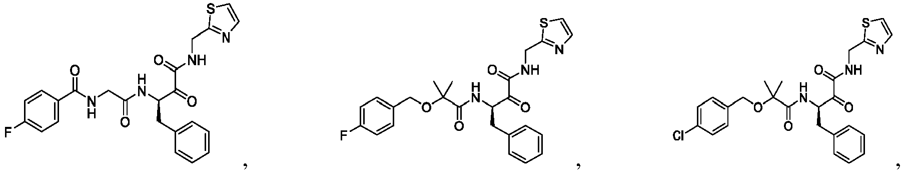

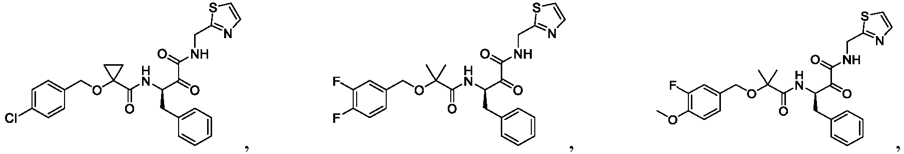

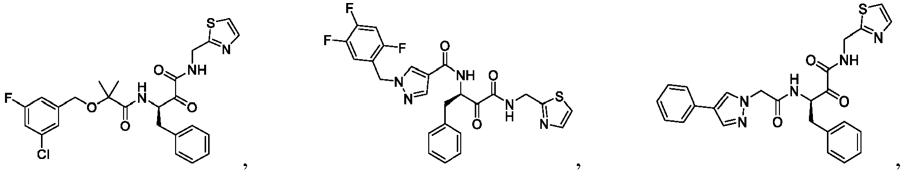

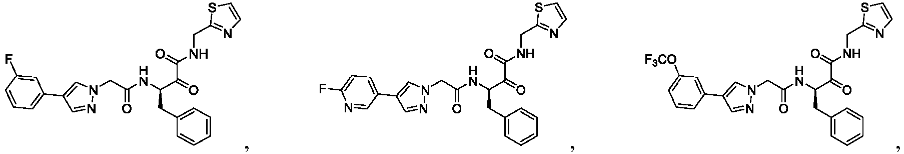

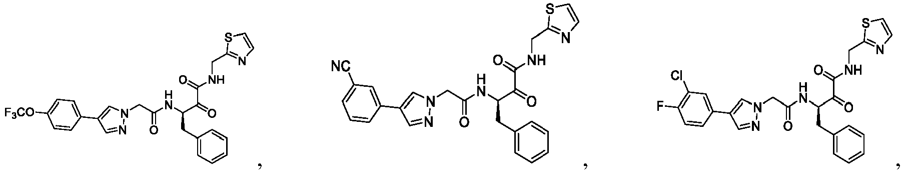

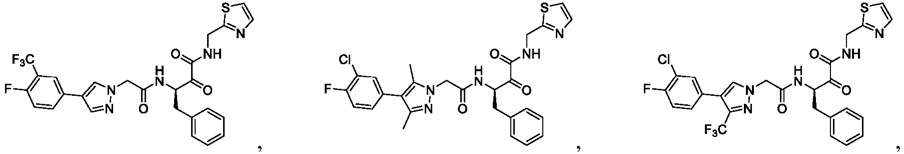

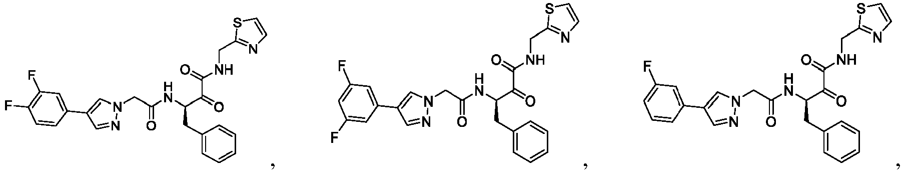

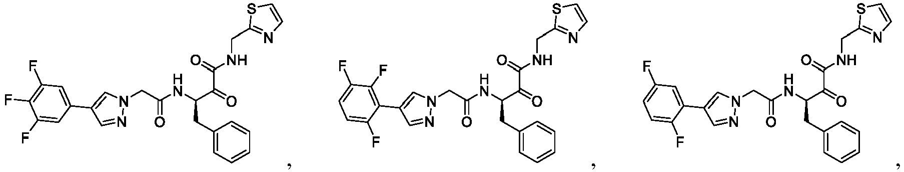

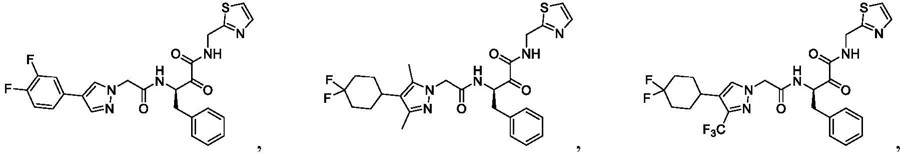

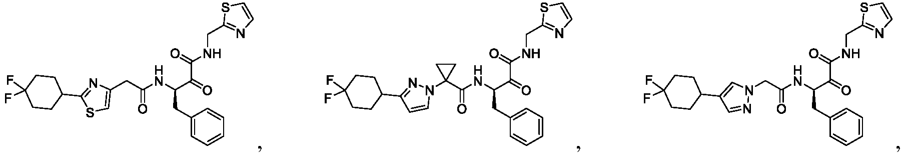

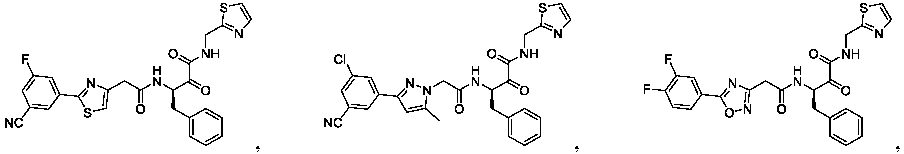

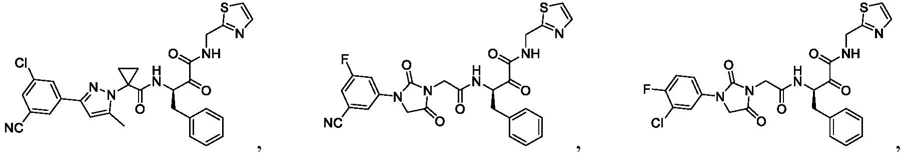

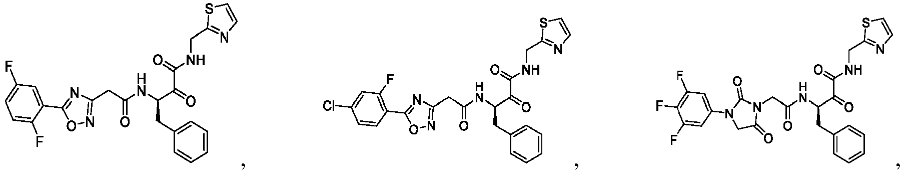

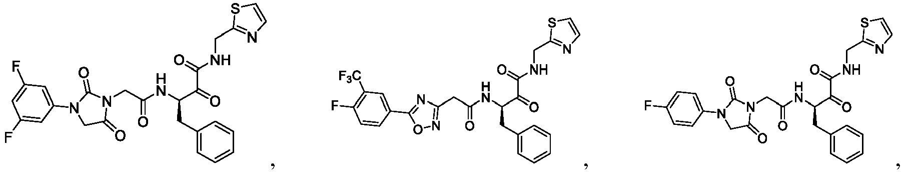

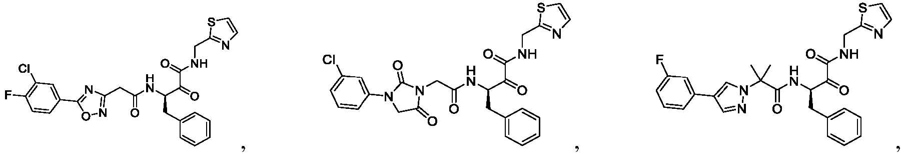

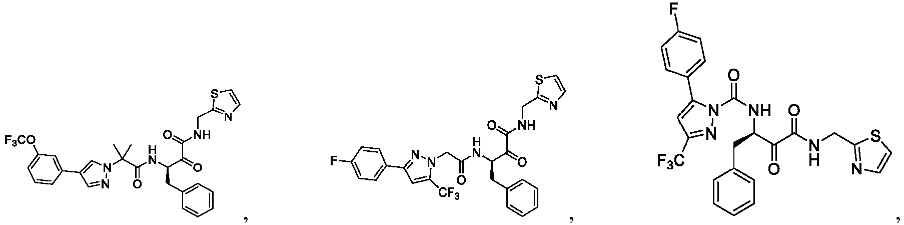

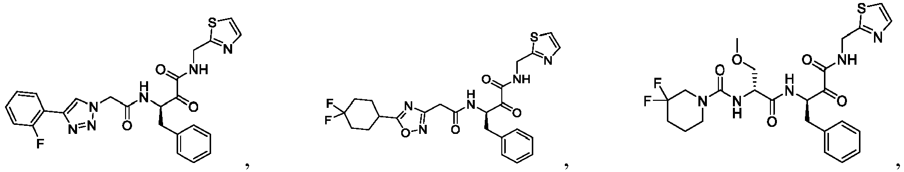

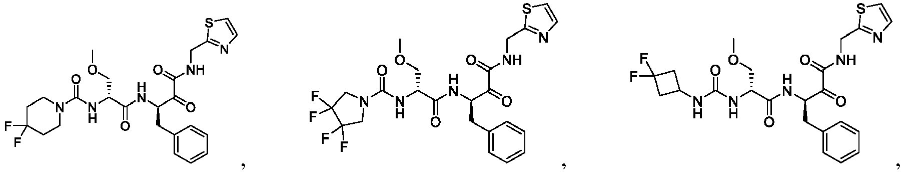

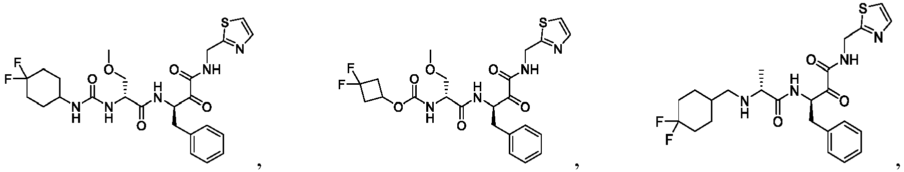

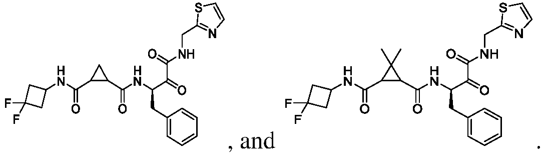

49. The compound according to claim 39, or a pharmaceutically acceptable salt thereof, or a stereoisomer thereof, or an optical isomer thereof, or a deuterated compound thereof, **characterized in that** the structure of the compound is as represented by formula II-A-I' -2:

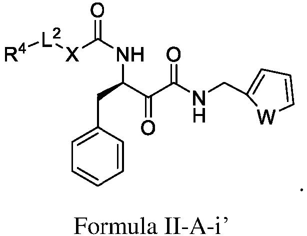

50. The compound according to claim 49, or a pharmaceutically acceptable salt thereof, or a stereoisomer thereof, or an optical isomer thereof, or a deuterated compound thereof, **characterized in that** the compound has any one of the following structures:

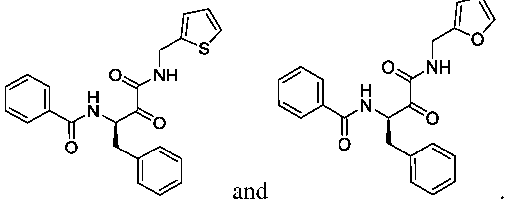

51. The compound according to claim 27, or a pharmaceutically acceptable salt thereof, or a stereoisomer thereof, or an optical isomer thereof, or a deuterated compound thereof, **characterized in that** the structure of the compound is as represented by formula II-A-j: 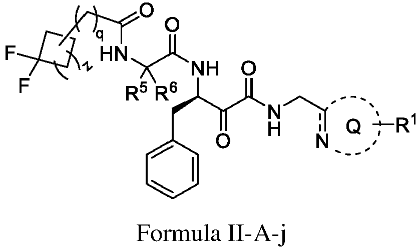
wherein, z is any integer from 1 to 3; q is any integer from 0 to 4; at least one of R⁵ and R⁶ is not H;
preferably, z is 1, 2, or 3; q is either 0 or 1.

52. The compound according to claim 51, or a pharmaceutically acceptable salt thereof, or a stereoisomer thereof, or an optical isomer thereof, or a deuterated compound thereof, **characterized in that** the structure of the compound is as represented by formula II-A-j-1:

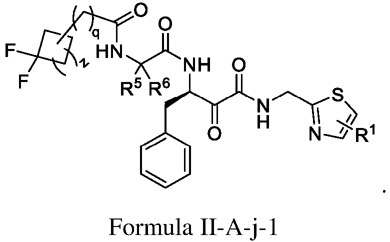

53. The compound according to claim 52, or a pharmaceutically acceptable salt thereof, or a stereoisomer thereof, or an optical isomer thereof, or a deuterated compound thereof, **characterized in that** the structure of the compound is as represented by any one of the following structures:

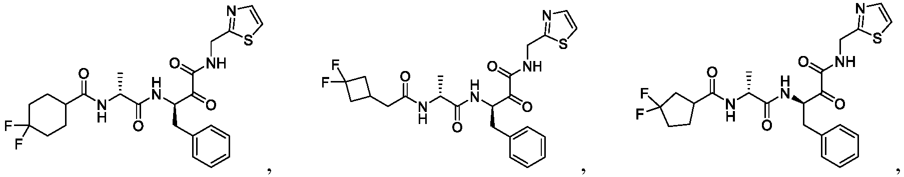

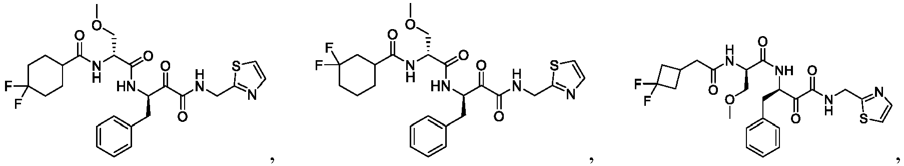

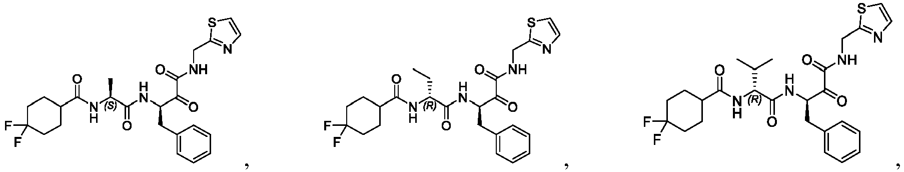

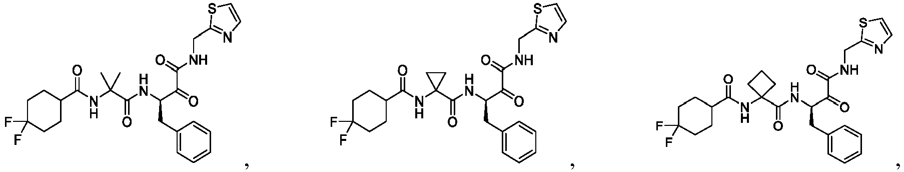

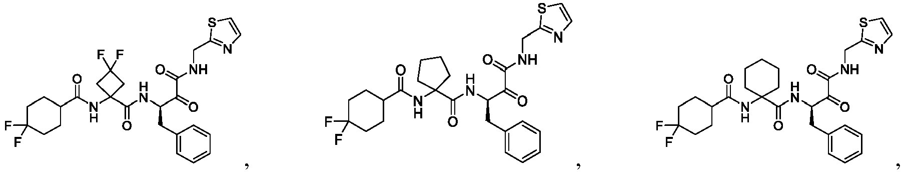

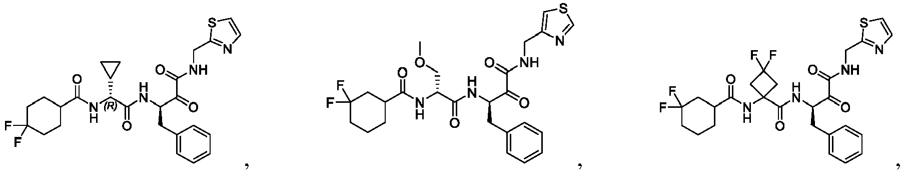

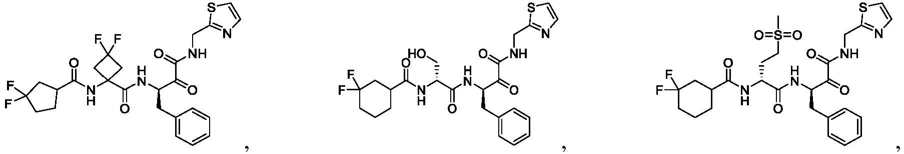

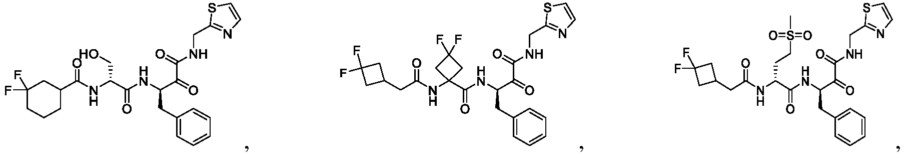

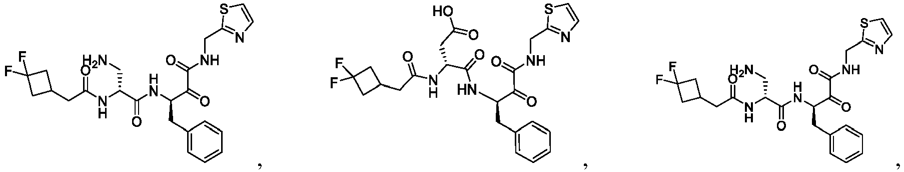

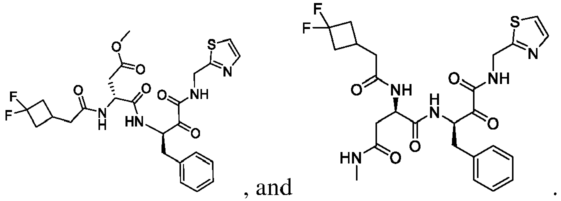

54. The compound according to claim 26, or a pharmaceutically acceptable salt thereof, or a stereoisomer thereof, or an optical isomer thereof, or a deuterated compound thereof, **characterized in that** the structure of the compound is as represented by formula II-B, II-C or II-D:

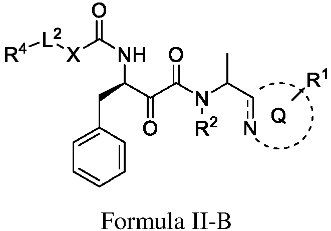

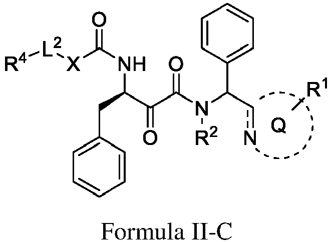

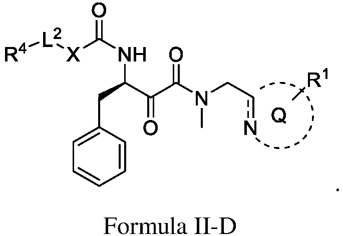

55. The compound according to claim 54, or a pharmaceutically acceptable salt thereof, or a stereoisomer thereof, or an optical isomer thereof, or a deuterated compound thereof, **characterized in that** the structure of the compound is as represented by formula II-B-a:

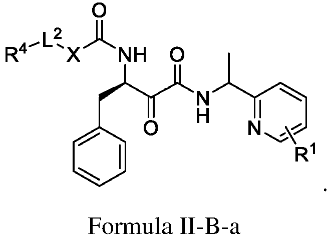

56. The compound according to claim 54, or a pharmaceutically acceptable salt thereof, or a stereoisomer thereof, or an optical isomer thereof, or a deuterated compound thereof, **characterized in that** the structure of the compound is as represented by formula II-B-a-1:

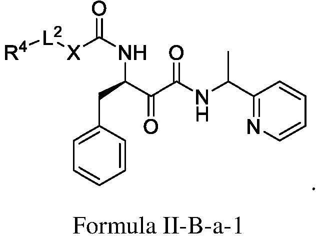

57. The compound according to claim 56, or a pharmaceutically acceptable salt thereof, or a stereoisomer thereof, or an optical isomer thereof, or a deuterated compound thereof, **characterized in that** the compound has the following structure:

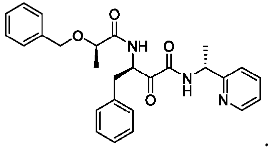

58. The compound according to claim 54, or a pharmaceutically acceptable salt thereof, or a stereoisomer thereof, or an optical isomer thereof, or a deuterated compound thereof, **characterized in that** the structure of the compound is as represented by formula II-C-a:

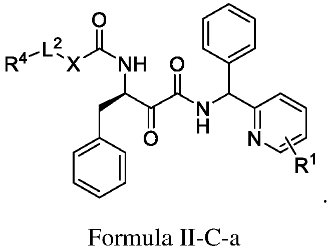

59. The compound according to claim 58, or a pharmaceutically acceptable salt thereof, or a stereoisomer thereof, or an optical isomer thereof, or a deuterated compound thereof, **characterized in that** the structure of the compound is as represented by formula II-C-a-1:

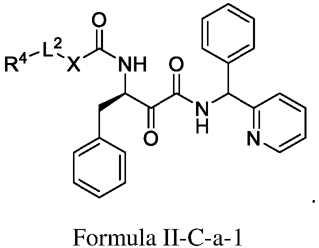

60. The compound according to claim 59, or a pharmaceutically acceptable salt thereof, or a stereoisomer thereof, or an optical isomer thereof, or a deuterated compound thereof, **characterized in that** the compound has the following structure:

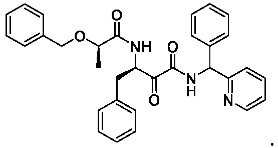

61. The compound according to claim 54, or a pharmaceutically acceptable salt thereof, or a stereoisomer thereof, or an optical isomer thereof, or a deuterated compound thereof, **characterized in that** the structure of the compound is as represented by formula II-D-a:

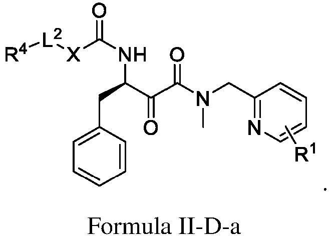

62. The compound according to claim 61, or a pharmaceutically acceptable salt thereof, or a stereoisomer thereof, or an optical isomer thereof, or a deuterated compound thereof, **characterized in that** the structure of the compound is as represented by formula II-D-a-1:

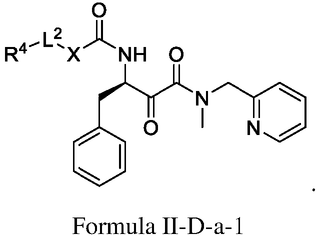

63. The compound according to claim 62, or a pharmaceutically acceptable salt thereof, or a stereoisomer thereof, or an optical isomer thereof, or a deuterated compound thereof, **characterized in that** the compound has the following structures:

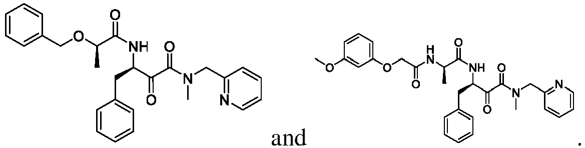

64. The compound according to claim 26, or a pharmaceutically acceptable salt thereof, or a stereoisomer thereof, or an optical isomer thereof, or a deuterated compound thereof, **characterized in that** the structure of the compound is as represented by formula III-A, formula III-B, formula III-C, formula III-D, formula III-E or formula III-F: 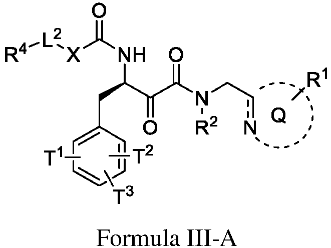 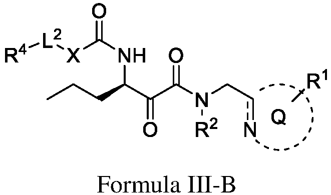 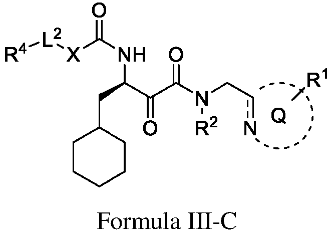 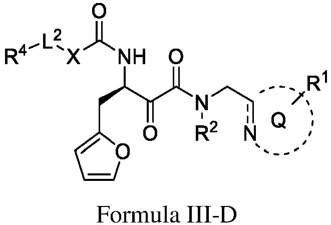 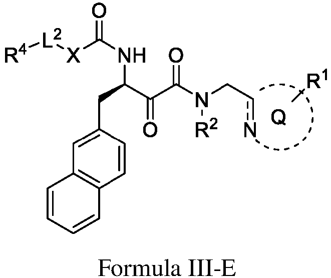 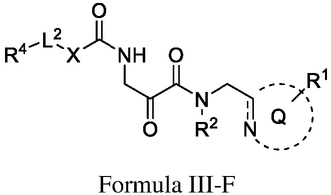 wherein, T¹, T², and T³ are each independently selected from H or halogen, and at least one of them is halogen.

65. The compound according to claim 64, or a pharmaceutically acceptable salt thereof, or a stereoisomer thereof, or an optical isomer thereof, or a deuterated compound thereof, **characterized in that** T is F.

66. The compound according to claim 64, or a pharmaceutically acceptable salt thereof, or a stereoisomer thereof, or an optical isomer thereof, or a deuterated compound thereof, **characterized in that** R¹ is Cl or H.

67. The compound according to claim 64, or a pharmaceutically acceptable salt thereof, or a stereoisomer thereof, or an optical isomer thereof, or a deuterated compound thereof, **characterized in that** the structure of the compound is as represented by formula III-A-a, formula III-A-b, formula III-B-a, formula III-C-a, formula III-C-b, formula III-D-a, formula III-D-b, formula III-E-a or formula III-F-a:

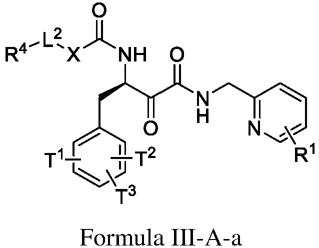

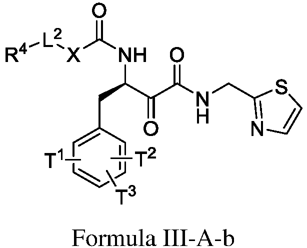

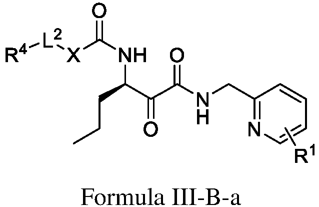

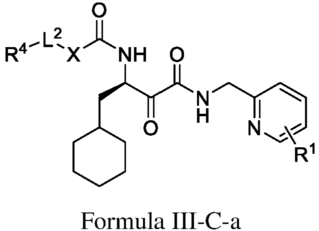

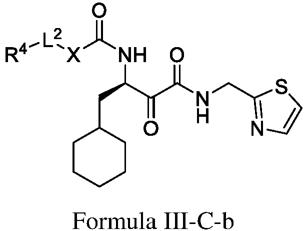

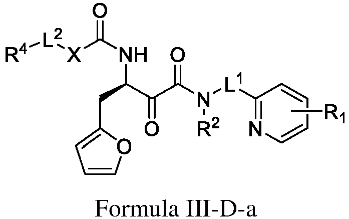

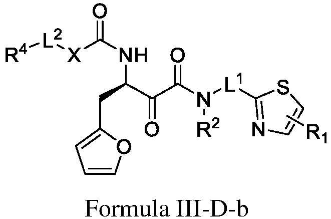

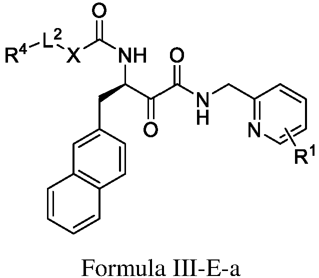

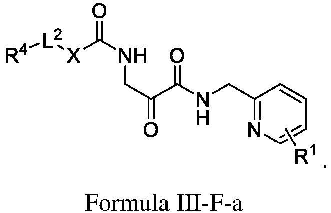

68. The compound according to claim 67, or a pharmaceutically acceptable salt thereof, or a stereoisomer thereof, or an optical isomer thereof, or a deuterated compound thereof, **characterized in that** the structure of the compound is as represented by any one of the following structures:

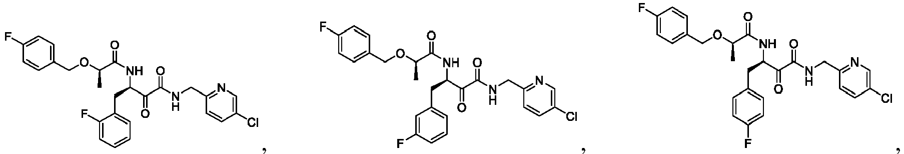

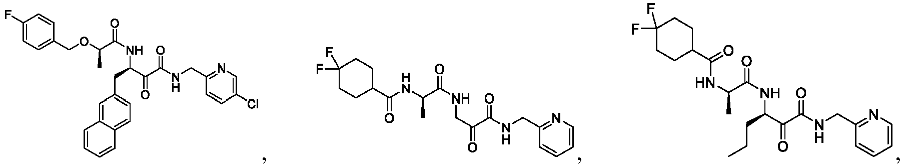

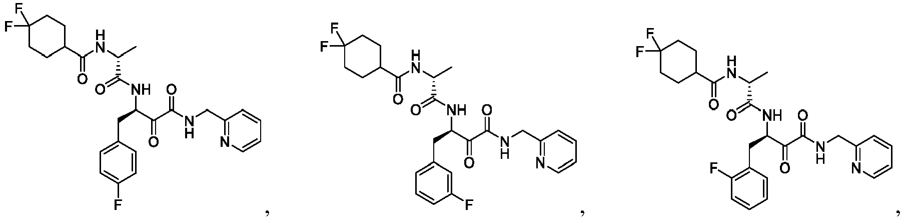

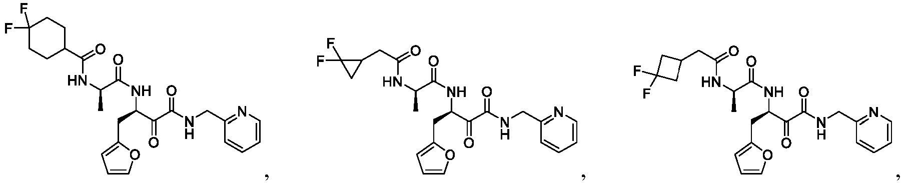

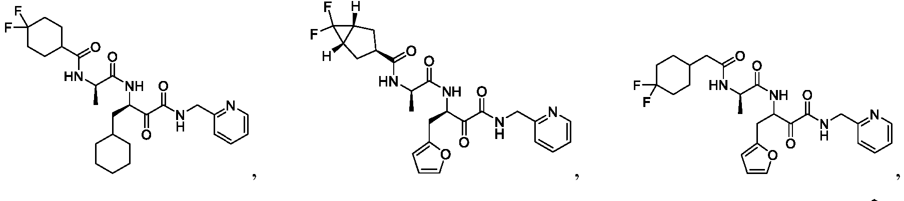

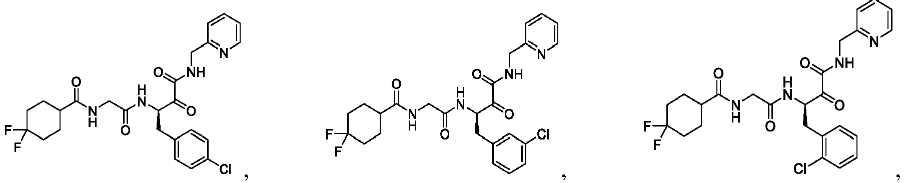

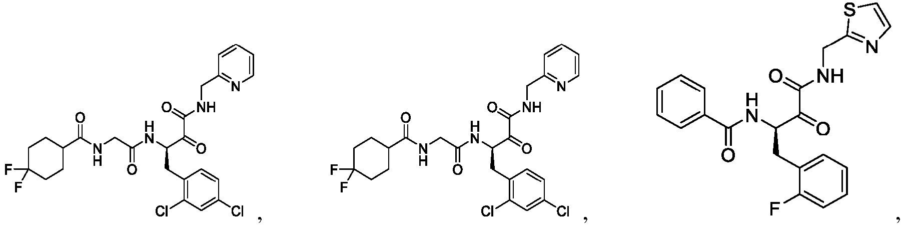

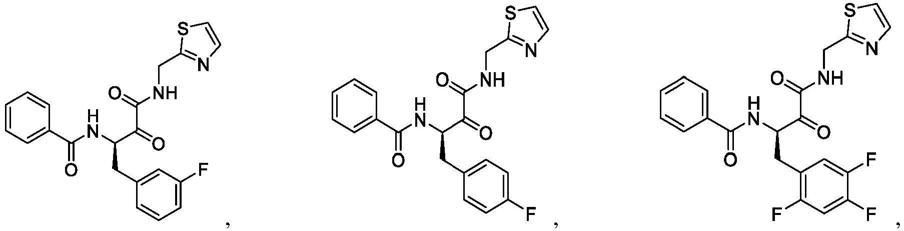

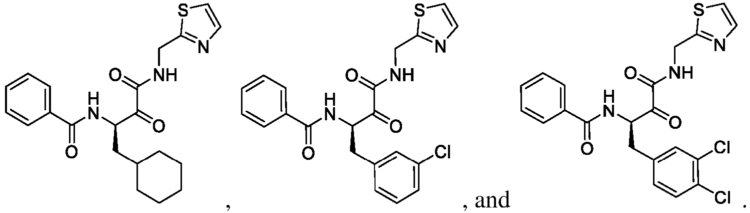

69. A pharmaceutical composition, **characterized in that** it is a preparation formed by the compound according to any one of claims 1 to 68, or a pharmaceutically acceptable salt thereof, or a stereoisomer thereof, or an optical isomer thereof, or a deuterated compound thereof, as the active ingredient, in association with pharmaceutically acceptable excipients.

70. The compound according to any one of claims 1 to 68, or a pharmaceutically acceptable salt thereof, or a stereoisomer thereof, or an optical isomer thereof, or a deuterated compound thereof for use in the manufacturer of medicaments for preventing and/or treating coronavirus-related diseases.

71. The use according to claim 70, **characterized in that** the medicaments for preventing and/or treating coronavirus-related diseases are anti-coronavirus medicaments.

72. The use according to claim 71, **characterized in that** the anti-coronavirus medicaments are those inhibiting coronavirus infection in cells.

73. The use according to claim 71, **characterized in that** the anti-coronavirus medicaments are the inhibitors of coronavirus proteolytic enzymes, and preferably are the inhibitors of coronavirus main proteases.

74. The use according to any one of claims 70 to 73, **characterized in that** the coronavirus is SARS-CoV-2, SARS-CoV, MERS-CoV, HcoV-229E, HcoV-NL63, HcoV-HKU1 or HcoV-OC43, and preferably is SARS-CoV-2

75. The use according to claim 74, **characterized in that** the medicaments for preventing and/or treating coronavirus-related diseases are those for preventing and/or treating Corona Virus Disease 2019 (COVID-19).

76. The use according to claim 74, **characterized in that** the inhibitors of coronavirus main proteases are SARS-CoV-2M^{pro} inhibitors.
